# EUROPÄISCHE PATENTSCHRIFT

(11) **EP 1 769 074 B1**
(45) Veröffentlichungstag und Bekanntmachung des Hinweises auf die Patenterteilung: **14.10.2009**
(21) Anmeldenummer: 05781182.0
(22) Anmeldetag: 15.07.2005
(51) Int. Cl.: C12N 15/82, C12N 15/55, C12N 9/20, C12N 15/53, C12N 9/04, C12N 15/60, C12N 9/88, A01H 5/00

(54) **VERFAHREN ZUR ERHÖHUNG DES GEHALTS AN MEHRFACH UNGESÄTTIGTEN LANGKETTIGEN FETTSÄUREN IN TRANSGENEN ORGANISMEN**
METHOD FOR INCREASING THE CONTENT OF POLYUNSATURATED LONG-CHAINED FATTY ACIDS IN TRANSGENIC ORGANISMS
PROCÉDÉ PERMETTANT D'AUGMENTER LA TENEUR D'ORGANISMES TRANSGÉNIQUES EN ACIDES GRAS À CHAINE LONGUE POLYINSATURES

(30) Priorität: 16.07.2004 DE 102004034442; 15.12.2004 DE 102004060340
(43) Veröffentlichungstag der Anmeldung: 04.04.2007
(73) Patentinhaber: BASF Plant Science GmbH, 67056 Ludwigshafen (DE)
(72) Erfinder: CIRPUS, Petra, 68163 Mannheim (DE); BAUER, Jörg, 67117 Limburgerhof (DE); QIU, Xiao, S7N 3S5 (CA); VRINTEN, Patricia, Saskatoon, Sk. S7J 4H5 (CA)
(74) Vertreter: Neuefeind, Regina
(86) Internationale Anmeldenummer: PCT/EP2005/007754
(87) Internationale Veröffentlichungsnummer: WO 2006/008099

(56) Entgegenhaltungen:
- WO-A-01/83788
- WO-A-97/37006
- WO-A-03/093482
- WO-A-20/04057001
- MAY C ET AL: "A PHOSPHOLIPASE A2 IS TRANSIENTLY SYNTHESIZED DURING SEED GERMINATION AND LOCALIZED TO LIPID BODIES" BIOCHIMICA ET BIOPHYSICA ACTA, AMSTERDAM, NL, Bd. 1393, 28. August 1998 (1998-08-28), Seiten 267-276, XP000978452 ISSN: 0006-3002
- SIX D A ET AL: "The expanding superfamily of phospholipase A2 enzymes: classification and characterization" BIOCHIMICA AND BIOPHYSICA ACTA. MOLECULAR AND CELL BIOLOGY OF LIPIDS, ELSEVIER, AMSTERDAM, NL, Bd. 1488, Nr. 1-2, 31. Oktober 2000 (2000-10-31), Seiten 1-19, XP004277419 ISSN: 1388-1981
- DREXLER H ET AL: "Metabolic engineering of fatty acids for breeding of new oilseed crops: strategies, problems and first results" JOURNAL OF PLANT PHYSIOLOGY, FISCHER, STUTTGART, DE, Bd. 160, Nr. 7, 2003, Seiten 779-802, XP004955255 ISSN: 0176-1617
- XU XIAOJIE ET AL: "Sequence analysis of the cloned glossy8 gene of maize suggests that it may code for a beta-ketoacyl reductase required for the biosynthesis of cuticular waxes" PLANT PHYSIOLOGY (ROCKVILLE), Bd. 115, Nr. 2, 1997, Seiten 501-510, XP002378066 ISSN: 0032-0889
- BEISSON FREDERIC ET AL: "Arabidopsis genes involved in acyl lipid metabolism. A 2003 census of the candidates, a study of the distribution of expressed sequence tags in organs, and a web-based database." PLANT PHYSIOLOGY (ROCKVILLE), Bd. 132, Nr. 2, Juni 2003 (2003-06), Seiten 681-697, XP002378071 ISSN: 0032-0889
- DERELLE EVELYNE ET AL: "DNA libraries for sequencing the genome of Ostreococcus tauri (Chlorophyta, Prasinophyceae): The smallest free-living eukaryotic cell." JOURNAL OF PHYCOLOGY, Bd. 38, Nr. 6, Dezember 2002 (2002-12), Seiten 1150-1156, XP002378067 ISSN: 0022-3646

## Beschreibung

Die vorliegende Erfindung betrifft ein Verfahren zur Erhöhung des Gehalts von mehrfach ungesättigten langkettigen Fettsäuren in Pfanzen oder Pflanzenzellen, indem Nukleinsäuren in die Pflanze oder Pflanzenzelle eingebracht werden, die für Polypeptide bzw. Proteine mit einer Phospholipase-Aktivität kodieren. Vorteilhaft stammen diese Enzyme aus Ostreococcus oder Thraustochytrium.

Weiterhin beschrieben sind diese Nukleinsäuresequenzen, die Nukleinsäurekonstrukte enthaltend diese Nukleinsäuresequenzen, die Vektoren enthaltend die Nukleinsäuresequenzen und/oder die Nukleinsäurekonstrukte, sowie die transgenen Pfanzen oder Pflanzenzellen enthaltend die vorgenannten Nukleinsäuresequenzen, Nukleinsäurekonstrukte und/oder Vektoren.

Vorteilhaft können diese vorgenannten Nukleinsäuresequenzen, Nukleinsäurekonstrukte und/oder Vektoren gegebenenfalls zusammen mit weiteren Nukleinsäuresequenzen, die für Polypeptide bzw. Proteine der Biosynthese des Fettsäure- oder Lipidstoffwechels kodieren, in dem Organismus exprimiert werden. Besonders vorteilhafte Nukleinsäuresequenzen des Fettsäure- oder Lipidstoffwechels sind dabei solche, die für eine Δ-9-Elongase-, Δ-8-Desaturase-, Δ-6-Desaturase-, eine Δ-5-Desaturase-, Δ-4-Desaturase-, Δ-12-Desaturase-, Δ-5-Elongase- und/oder Δ-6-Elongase-Aktivität kodieren. Vorteilhaft stammen diese Desaturasen und Elongasen aus Organismen wie Thalassiosira, Euglena, Isochrysis, Physcomitrella, Thraustochytrium, Borago, Phytophthora, Crypthecodinium, Oncorhynchus, Primula, Xenopus, Ciona, Arabidopsis, Mortierella, Caenorhabditis, Phaeodactylum, Ceratodon oder Ostreococcus.

Weiterhin beschrieben sind Öle, Lipide und/oder Fettsäuren, hergestellt nach dem erfmdungsgemäßen Verfahren, und deren Verwendung. Außerdem offenbart sind ungesättigte Fettsäuren sowie Triglyceride mit einem erhöhten Gehalt an ungesättigten Fettsäuren und deren Verwendung.

Fettsäuren und Triacylglyceride haben eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfach ungesättigte Fettsäuren wie Linol- und Linolensäure sind für Säugetiere essentiell, da sie nicht von diesen selbst hergestellt werden können. Deshalb stellen mehrfach ungesättigte ω-3-Fettsäuren und ω-6-Fettsäuren einen wichtigen Bestandteil der tierischen und menschlichen Nahrung dar.

Mehrfach ungesättigte langkettige ω-3-Fettsäuren wie Eicosapentaensäure (= EPA, C20:5^{Δ5,8,11,14,17}) oder Docosahexaensäure (= DHA, C22:6^{Δ4,7,10,13,16,19}) sind wichtige Komponenten der menschlichen Ernährung aufgrund ihrer verschiedenen Rollen in der Gesundheit, die Aspekte wie die Entwicklung des kindlichen Gehirns, die Funktionalität des Auges, die Synthese von Hormonen und anderen Signalstoffen, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, C22:6^{Δ4,7,10,13,16,19}) oder Eisosapentaensäure (= EPA, C20:5^{Δ5,8,11,14,17}) Babynahrung zur Erhöhung des Nährwertes zugesetzt. Der ungesättigten Fettsäure DHA wird dabei ein positiver Effekt auf die Entwicklung und Aufrechterhaltung von Gehirnfunktionen zugeschrieben.

Im folgenden werden mehrfach ungesättigte Fettsäuren als PUFA, PUFAs, LCPUFA oder LCPUFAs bezeichnet (**p**oly **u**nsaturated **f**atty **a**cids, **PUFA,** mehrfach ungesättigte Fettsäuren; **l**ong **c**hain **p**oly **u**nsaturated **f**atty **a**cids, **LCPUFA,** langkettige mehrfach ungesättigte Fettsäuren).

Hauptsächlich werden die verschiedenen Fettsäuren und Triglyceride aus Mikroorganismen wie Mortierella oder Schizochytrium oder aus Öl-produzierenden Pflanzen wie Soja, Raps oder Algen wie Crypthecodinium oder Phaeodactylum und anderen gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie DHA, EPA, Arachidonsäure (= ARA, C20:4^{Δ5,8,11,14}), Dihomo-γ-linolensäure (C20:3^{Δ8,11,14}) oder Docosapentaensäure (DPA, C22:5^{Δ7,10,13,16,19}) werden in Ölfruchtpflanzen wie Raps, Soja, Sonnenblume oder Färbersaflor nicht synthetisiert. Übliche natürliche Quellen für diese Fettsäuren sind Algen oder Fische wie Hering, Lachs, Sardine, Goldbarsch, Aal, Karpfen, Forelle, Heilbutt, Makrele, Zander oder Thunfisch.

Je nach Anwendungszweck werden Öle mit gesättigten oder ungesättigten Fettsäuren bevorzugt. So werden z.B. in der humanen Ernährung Lipide mit ungesättigten Fettsäuren, speziell mehrfach ungesättigten Fettsäuren, bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche, speziell chronisch entzündliche Prozesse im Rahmen immunologischer Erkrankungen wie rheumatroider Arthritis lassen sich durch ω-3-Fettsäuren positiv beeinflussen. Sie werden deshalb Lebensmitteln, speziell diätischen Lebensmitteln, zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.

ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, den Thromoxanen und Leukotrienen, die sich von der Arachidonsäure und der Eicosapentaensäure ableiten. Eicosanoide (sog. PG₂-Serie), die aus ω-6-Fettsäuren gebildet werden, fördern in der Regel Entzündungsreaktionen, während Eicosanoide (sog. PG₃-Serie) aus ω-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben, und auch die Anwendung zur Produktion in transgenen Organismen wird z.B. in WO98/46763 WO98/46764, WO9846765 beschrieben. Dabei wird auch die Expression verschiedener Desaturasen wie in WO99/64616 oder WO98/46776 und Bildung polyungesättigter Fettsäuren beschrieben und beansprucht. Bzgl. der Effektivität der Expression von Desaturasen und ihrem Einfluss auf die Bildung polyungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus ω-3- und ω-6-Fettsäuren erhalten.

Besonders geeignete Mikroorganismen zur Herstellung von PUFAs sind Mikroorganismen wie Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moose wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wann immer möglich, wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen, wobei diese in der Regel je nach verwendetem Mikroorganismus als Fettsäuregemische aus beispielsweise EPA, DPA und ARA anfallen.

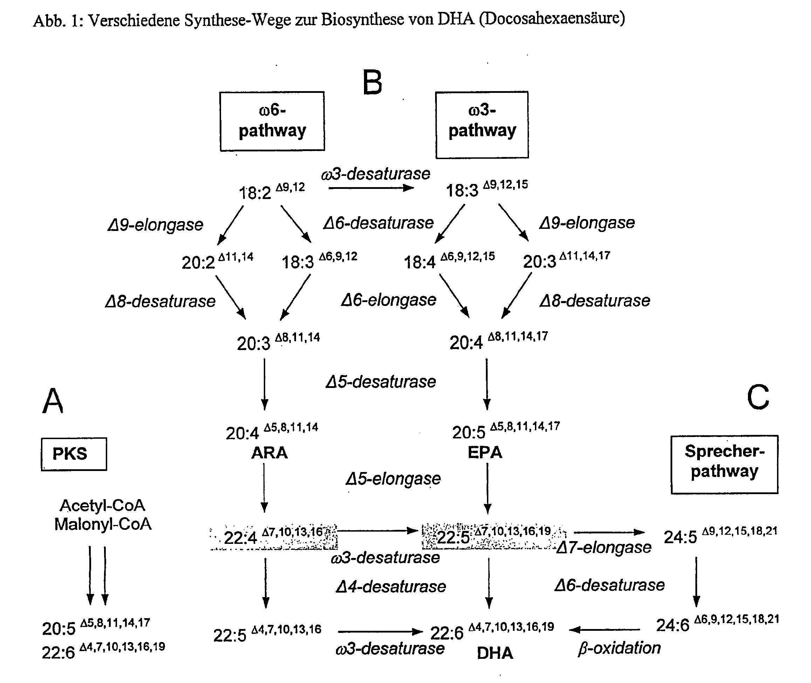

Für die Synthese von Arachidonsäure, Eicosapentaensäure (EPA) und Docosahexaensäure (DHA) werden verschiedene Synthesewege diskutiert (Figur 1). So erfolgt die Produktion von EPA bzw. DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731, 1997).

Ein alternative Strategie verläuft über die wechselnde Aktivität von Desaturasen und Elongasen (Zank, T.K. et al. Plant Journal 31:255-268, 2002; Sakuradani, E. et al. Gene 238:445-453, 1999). Eine Modifikation des beschriebenen Weges über Δ6-Desaturase, Δ6-Elongase, Δ5-Desaturase, Δ5-Elongase, Δ4-Desaturase ist der Sprecher-Syntheseweg (Sprecher 2000, Biochim. Biophys. Acta 1486:219-231) in Säugetieren. Anstelle der Δ4-Desaturierung erfolgt hier ein weiterer Elongationsschritt auf C₂₄, eine weitere Δ6-Desaturierung und abschliessend eine β-Oxidation auf die C₂₂-Kettenlänge. Für die Herstellung in Pflanzen und Mikroorganismen ist der sogenannte Sprecher-Syntheseweg (siehe Figur 1) allerdings nicht geeignet, da die Regulationsmechanismen nicht bekannt sind.

Die polyungesättigten Fettsäuren können entsprechend ihrem Desaturierungsmuster in zwei große Klassen, in ω-6- oder ω-3-Fettsäuren, eingeteilt werden, die metabolisch und funktionell unterschiedliche Aktivitäten haben (Fig. 1).

Als Ausgangsprodukt für den ω-6-Stoffwechselweg fungiert die Fettsäure Linolsäure (18:2^{Δ9,12}), während der ω-3-Weg über Linolensäure (18:3^{Δ9,12,15}) abläuft.

Linolensäure wird dabei durch die Aktivität einer ω-3-Desaturase gebildet (Tocher et al. 1998, Prog. Lipid Res. 37, 73-117; Domergue et al. 2002, Eur. J. Biochem. 269, 4105-4113).

Säugetiere und damit auch der Mensch verfügen über keine entsprechende Desaturase-Aktivität (Δ-12- und ω-3-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen polyungesättigten Fettsäuren Arachidonsäure (= ARA, 20:4^{Δ5,8,11,14}), eine ω-6-Fettsäure, und die beiden ω-3-Fettsäuren Eicosapentaen- (= EPA, 20:5^{Δ5,8,11,14,17}) und Docosahexaensäure (DHA, 22:6^{Δ4,7,10,13,17,19}) synthetisiert. Die Applikation von ω-3-Fettsäuren zeigt dabei die oben beschriebene therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthritis (Cleland und James 2000, J. Rheumatol. 27, 2305-2307).

Die Verlängerung von Fettsäuren durch Elongasen um 2 bzw. 4 C-Atome ist für die Produktion von C₂₀- bzw. C₂₂-PUFAs von entscheidender Bedeutung. Dieser Prozess verläuft über 4 Stufen. Der erste Schritt stellt die Kondensation von Malonyl-CoA an das Fettsäure-Acyl-CoA durch die Ketoacyl-CoA-Synthase (KCS, im weiteren Text als Elongase bezeichnet). Es folgt dann ein Reduktionschritt (Ketoacyl-CoA-Reduktase, KCR), ein Dehydratationsschritt (Dehydratase) und ein abschliessender Reduktionsschritt (Enoyl-CoA-Reduktase). Es wurde postuliert, dass die Aktivität der Elongase die Spezifität und Geschwindigkeit des gesamten Prozesses beeinflusst (Millar and Kunst, 1997 Plant Journal 12:121-131).

In der Vergangenheit wurden zahlreiche Versuche unternommen, Elongasegene zu erhalten. Millar und Kunst (1997, Plant Journal 12:121-131) und Millar et al. (1999, Plant Cell 11:825-838) beschreiben die Charakterisierung von pflanzlichen Elongasen zur Synthese von einfach ungesättigten langkettigen Fettsäuren (C22:1) bzw. zur Synthese von sehr langkettigen Fettsäuren für die Wachsbildung in Pflanzen (C₂₈-C₃₂). Beschreibungen zur Synthese von Arachidonsäure und EPA finden sich beispielsweise in WO0159128, WO0012720, WO02077213 und W00208401. Die Synthese von mehrfach ungesättigten C24-Fettsäuren ist beispielsweise in Tvrdik et al. (2000, JCB 149:707-717) oder WO0244320 beschrieben.

Höhere Pflanzen enthalten mehrfach ungesättigte Fettsäuren wie Linolsäure (C18:2) und Linolensäure (C18:3). ARA, EPA und DHA kommen im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden könnten. Hierzu müssen Gene, kodierend für Enzyme der Biosynthese von LCPUFAs, vorteilhaft über gentechnische Methoden in Ölsaaten eingeführt und exprimiert werden. Dies sind Gene, die beispielsweise für Δ-6-Desaturasen, Δ-6-Elongasen, Δ-5-Desaturasen oder Δ-4-Desaturasen kodieren. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos *Physcomitrella patens* und Δ-6-Elongase-Gene aus *P. patens* und dem Nematoden *C. elegans* isoliert werden.

Erste transgene Pflanzen, die Gene kodierend für Enzyme der LCPUFA-Biosynthese enthalten und exprimieren und LCPUFAs produzieren, wurden beispielsweise in DE 102 19 203 (Verfahren zur Herstellung mehrfach ungesättigter Fettsäuren in Pflanzen) erstmals beschrieben. Diese Pflanzen produzieren allerdings LCPUFAs in Mengen, die für eine Aufarbeitung der in den Pflanzen enthaltenen Öle noch weiter optimiert werden müssen.

Um eine Anreicherung der Nahrung und des Futters mit diesen mehrfach ungesättigten Fettsäuren zu ermöglichen, besteht daher ein großer Bedarf an einem einfachen, kostengünstigen Verfahren zur Herstellung dieser mehrfach ungesättigten Fettsäuren speziell in eukaryontischen Systemen.

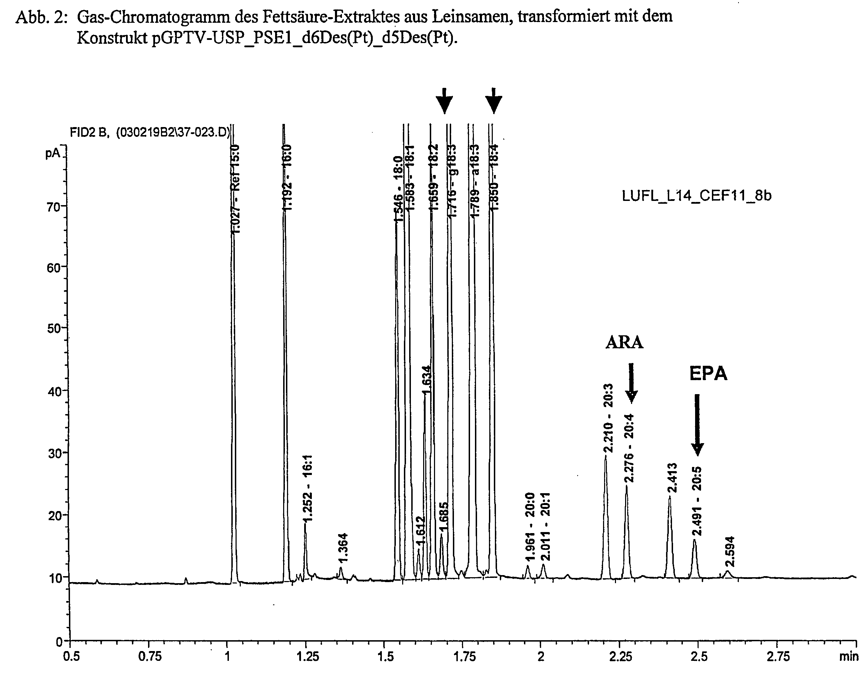

Nach wie vor bestehen eine Reihe von limitierenden Schritten in der Fettsäurebiosynthese, die einer Erhöhung des Gehalts an mehrfach ungesättigten langkettigen Fettsäuren im Wege stehen. So konnte beispielsweise in transgenen Pflanzen, wie sie beispielsweise in DE10219203 beschrieben wurden, gezeigt werden, dass die Elongation, d.h. die Kettenverlängerung, von C18- auf C20-Fettsäuren ein solcher limitierender Schritt ist (Figur 2). Figur 2 gibt das GasChromatogramm des Fettsäure-Extraktes aus Leinsamen, transformiert mit dem Konstrukt pGPTV-USP_PSE1_d6Des(Pt)_d5Des(Pt), gemäss den Beschreibungen aus DE10219203 wieder. Die neu entstandenen Produkte der Aktivitäten der Gene sind mit Pfeilen markiert. Die Synthese der Endprodukte Arachidonsäure (ARA) und Eicosapentaensäure (EPA) verläuft über γ-Linolensäure (g18:3) bzw. Stearidonsäure (18:4) (erster Schritt, siehe auch Figur 1). Im zweiten Schritt erfolgt dann die Elongation zu den Zwischenprodukten 20:3n-6 und 20:4n-3 (Elongationsschritt). Im letzten Schritt werden dann die Zwischenprodukte zu ARA und EPA umgesetzt. Eine starke Abnahme der Produktmengen vom ersten zum zweiten Schritt ist erkennbar. Figur 2 zeigt, dass nach dem ersten Schritt in der aeroben LCPUFA-Synthese, die Desaturierung von Linol- bzw. Linolensäure (siehe Figur 1), die Produktmenge drastisch reduziert ist Dies bedeutet möglicherweise, dass der Umsatz der Elongation von Δ6-desaturierten Fettsäuren in einem zu geringem Maße erfolgt. Die Konversionsrate ist dabei deutlich geringer, als dies in Hefeexperimenten, in denen die Δ6-desaturierten Fettsäuren gefüttert wurden, gezeigt werden konnte (Zank et al. 2002, Plant Journal 31:255-268).

Es bestand daher die Aufgabe, weitere Gene bzw. Enzyme, die für die Synthese von LCPUFAs geeignet sind, speziell Gene, die eine Phospholipase A2-Aktivität aufweisen, für die Herstellung von mehrfach ungesättigten Fettsäuren zur Verfügung zu stellen und mit diesen die Fettsäurebiosynthese in Ölen und/oder Lipiden weiter zu optimieren.

Weiterhin bestand die Aufgabe, ein Verfahren zur Herstellung von Ölen oder Lipiden mit einem hohen Anteil an ungesättigten Fettsäuren, vorteilhaft an mehrfach ungesättigten Fettsäuren, in einer Pflanze oder Pflanzenzelle, zu entwickeln. Diese Aufgabe wurde durch das erfindungsgemäße Verfahren zur Herstellung von Ölen oder Lipiden mit einem hohen Anteil an ungesättigten Fettsäuren in transgenen Pflanzen oder Pflanzenzellen gelöst. Dieses Verfahren ist **dadurch gekennzeichnet, dass** es folgende Verfahrensschritte umfasst:
a) Einbringen mindestens einer Nukleinsäuresequenz in einem Expressionskonstrukt in die Pflanze oder Pflanzenzelle, welche für eine Phospholipase A2-Aktivität kodiert,
b) Kultivierung und Ernte der transgenen Pflanze oder Pflanzenzelle.

Vorteilhaft werden die im Verfahren hergestellten Öle oder Lipide aus der transgenen Pflanze oder Pflanzenzelle isoliert, und gegegenenfalls werden die in den Ölen oder Lipiden enthaltenen Fettsäuren, vorteilhaft die ungesättigten Fettsäuren, aus diesen befreit.

Vorteilhaft enthalten die im erfindungsgemäßen Verfahren hergestellten mehrfach ungesättigten Fettsäuren mindestens zwei, vorteilhaft drei, vier, fünf oder sechs Doppelbindungen. Besonders vorteilhaft enthalten die Fettsäuren vier, fünf oder sechs Doppelbindungen. Im Verfahren hergestellte Fettsäuren haben vorteilhaft 18, 20 oder 22 C-Atome in der Fettsäurekette, bevorzugt enthalten die Fettsäuren 20 oder 22 Kohlenstoffatome in der Fettsäurekette. Diese hergestellten Fettsäuren können als einziges Produkt im Verfahren hergestellt werden oder in einem Fettsäuregemisch vorliegen.

Bei den im erfindungsgemäßen Verfahren verwendeten Nukleinsäuresequenzen handelt es sich um isolierte Nukleinsäuresequenzen, die für Polypeptide bzw. Proteine mit Phospholipase A2-Aktivität kodieren und vorteilhaft aus Organismen der Gattungen Ostreococcus oder Thraustochytrium stammen.

Bevorzugte im erfindungsgemäßen Verfahren verwendete Nukleinsäuresequenzen, die für Polypeptide bzw. Proteine mit Phospholipase A2- kodieren, sind ausgewählt aus der Gruppe bestehend aus:
a) einer Nukleinsäuresequenz mit der in SEQ ID NO: 1 dargestellten Sequenz, oder
b) Nukleinsäuresequenzen, die sich als Ergebnis des degenerierten genetischen Codes von der in SEQ ID NO: 2 dargestellten Aminosäuresequenz ableiten lassen, oder
c) Derivate der in SEQ ID NO: 1 dargestellten Nukleinsäuresequenz, die für Polypeptide bzw. Proteine mit mindestens 40 % Identität auf Aminosäureebene mit SEQ ID NO: 2 kodieren und eine Phospholipase A2- aufweisen.

Diese im erfindungsgemäßen Verfahren verwendeten Nukleinsäuresequenzen, die für Polypeptide bzw. Proteine mit Phospholipase A2- kodieren, können vorteilhaft in Kombination mit Nukleinsäuresequenzen, die für Polypeptide bzw. Proteine mit Δ-9-Elongase-, Δ-6-Desaturase-, Δ-8-Desaturase-, Δ-12-Desaturase-, Δ-6-Elongase-, Δ-5-Desaturase-, Δ-5-Elongase-, ω-3-Desaturase- und/oder Δ-4-Desaturase-Aktivität kodieren, im erfindungsgemäßen Verfahren verwendet werden. Diese im erfindungsgemäßen Verfahren verwendeten Nukleinsäuresequenzen und die durch sie kodierten Proteine führen zu einer Erhöhung des Gehalts an ungesättigten Fettsäuren, bevorzugt zu einer Erhöhung des Gehalts an LCPUFAs, in den transgenen Organismen. Unter dem Begriff "mit einem hohen Anteil" an ungesättigten Fettsäure bzw. unter dem Begriff "Erhöhung" ist eine Zunahme der ungestättigten Fettsäuren in den Ölen oder Lipiden oder in Form der freien Fettsäuren in den Organismen von mindestens 5, 6, 7, 8, 9 oder 10 %, vorteilhaft von mindestens 15, 20, 25, 30, 35, 40, 45 oder 50 %, bevorzugt von mindestens 55, 60, 65, 70, 75, 80, 85, 90, 95 oder 100 %, besonders bevorzugt von mindestens 105, 110, 115 oder 120 %, ganz besonders bevorzugt von mindestens 130, 135, 140, 145 oder 150 % gegenüber der Menge an ungestättigten Fettsäuren in den Ölen oder Lipiden oder in Form der freien Fettsäuren in den Pflanzen, die die im erfindungsgemäßen Verfahren mit den verwendeten Nukleinsäuresequenzen und die durch diese kodierten Proteine gegenüber der nicht transgenen Ausgangspflanze, beispielsweise einer Pflanze wie Arabidopsis oder Lein, beim Vergleich in der GC-Analyse (siehe Beispiele) erreicht werden. Die vorgenannten Prozentangaben beziehen sich auf die Steigerung der ungestättigten Fettsäuren in den Ölen oder Lipiden oder in Form der freien Fettsäuren in den Organismen bezogen auf den Gesamtlipidgehalt in Gewichtsprozent. Im erfindungsgemäßen Verfahren werden so die hergestellten LCPUFAs mit einem Gehalt von mindestens 3 Gew.-%, vorteilhaft von mindestens 5 Gew.-%, bevorzugt von mindestens 8 Gew.-%, besonders bevorzugt von mindestens 10 Gew.-% und ganz besonders bevorzugt von mindestens 15 Gew.-% bezogen auf die gesamten Fettsäuren in der transgenen Pflanze hergestellt.

Die im erfindungsgemäßen Verfahren verwendete Aktivität der Phospholipase A2 [= PLA2(Ot)] ist beschrieben als Hydrolase-Reaktion der Esterbindung der sn-2-Position von Triacylglyceriden (E.C. Nummer 3.1.1.4). Eine Erhöhung des LCPUFA-Gehalts kann aufgrund der Aktivität auf folgenden Reaktionsmechanismus zurückgeführt werden:

Der Reaktionsmechanismus von LCPUFA setzt sich aus den Schritten Δ6-Desaturierung, Δ6-Elongation und Δ5-Desaturierung zusammen (Figur 1). Diese Schritte erfolgen in unterschiedlichen Kompartimenten (Domergue et al. 2003, JBC, 278:35115-35126). Dabei erfolgt der erste Desaturierungsschritt an der sn-2-Position von Phospholipiden, hauptsächlich Phosphatidylcholin (Domergue et al. 2002, Eur. J. Biochem. 269:4105-4113). Für den folgenden Elongationsschritt muss die Fettsäure aus dem Phosphatidylcholin herausgelöst werden und dem Elongationskomplex in Form eines Acyl-CoA-Esters zugänglich gemacht werden. Organismen verfügen dabei über ein Set von Acyltransferasen, um diese Reaktion durchführen zu können.

In transgenen Pflanzen scheint dieser Schritt limitierend zu sein, d.h. das endogen vorhandene Set an Enzymen kann die Reaktion nicht effizient katalysieren.

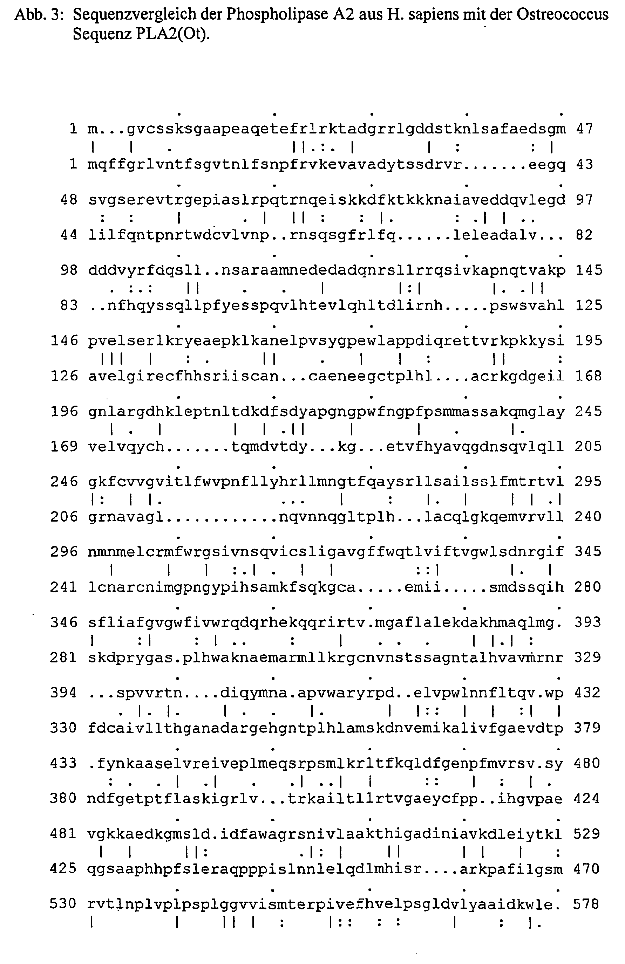

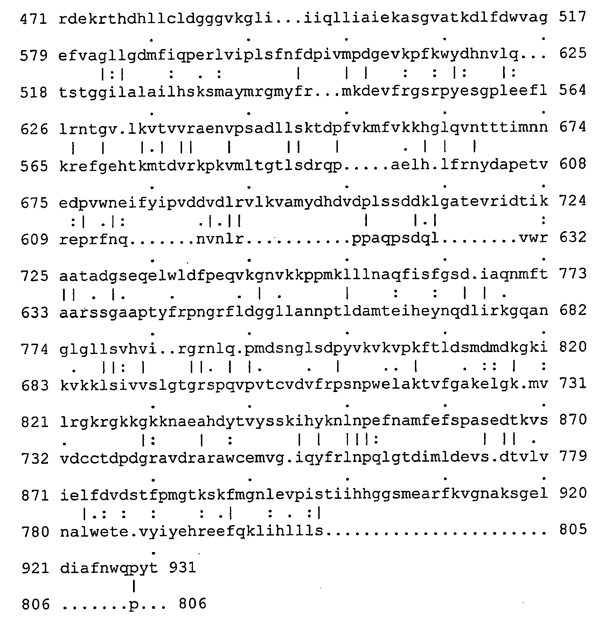

Aufgrund der Aktivität der PLA2(Ot) werden mehr Fettsäuren für die Elongation bereitgestellt, was zur Erhöhung des Gehalts an LCPUFA führt. Die PLA2(Ot) zeigt Homologien zu einer Phospholipase A2 aus Homo sapiens (siehe Figur 3).

Weiterhin beschrieben werden Enzyme des Elongationskomplexes. Neben der oben erwähnten Bereitstellung von Fettsäuren für die Elongation stellt die Aktivität des Elongationskomplexes ein wichtiges Potential zur Erhöhung des Gehalts an verlängerten Fettsäuren dar.

Aus der Alge Ostreococcus tauri sowie dem Pilz Thraustochytrium ssp. konnten Gene identifiziert werden, die für Proteine des Elongasekomplexes kodieren, deren Kombination in Organsimen zur Erhöhung des Gehalts an LCPUFA führt.

Der Prozess der Verlängerung von Fettsäuren verläuft über 4 Stufen (Biochemistry and Molecular Biology of Plants, 2000, ed. Buchanan, Gruissem, Jones, ASPP). Der erste Schritt stellt die Kondensation von Malonyl-CoA an das Fettsäure-Acyl-CoA durch die Ketoacyl-CoA-Synthase (KCS, im weiteren Text als Elongase bezeichnet) dar. Es folgt dann ein Reduktionschritt (Ketoacyl-CoA-Reduktase, KCR), ein Dehydratationsschritt (Dehydratase) und ein abschliessender Reduktionsschritt (Enoyl-CoA-Reduktase). Es wurde postuliert, dass die Aktivität der Elongase die Spezifität und Geschwindigkeit des gesamten Prozesses beeinflussen (Millar and Kunst, 1997 Plant Journal 12:121-131). Es konnte gezeigt werden, dass die verstärkte Bereitstellung einer der Komponenten des Elongasekomplexes zu einer Erhöhung der Menge an Elongationsprodukt führt (Beaudoin et al. 2001, JBC, 277:11481-11488).

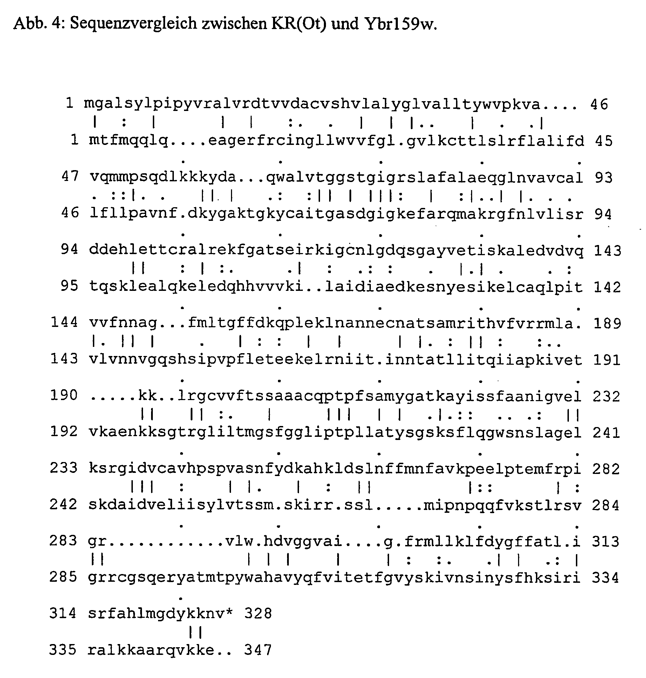

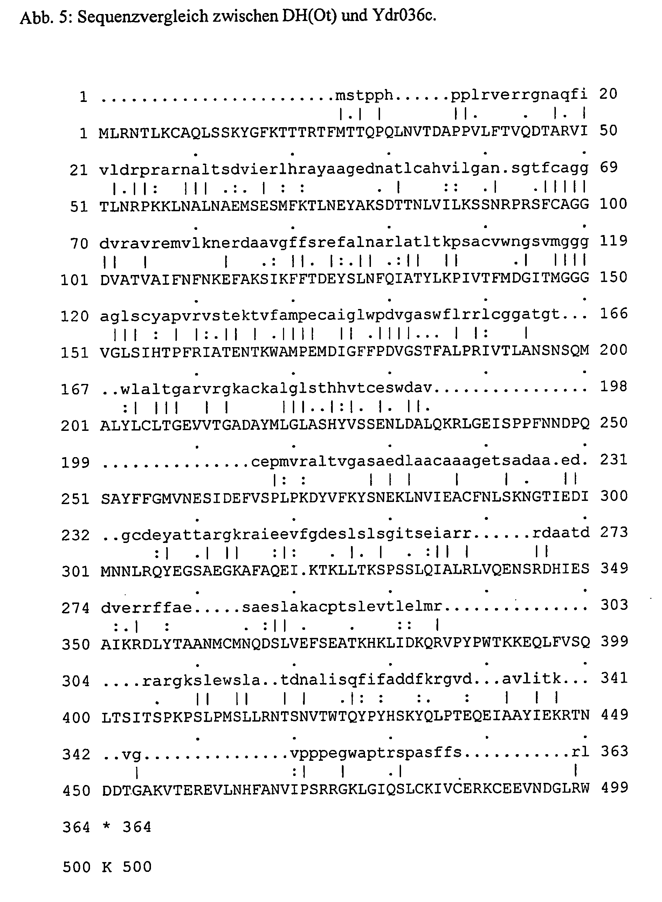

Überraschenderweise führt die kombinierte Expression der Gene für die Ketoacyl-CoA-Reduktase [KR(Ot)] und für die Dehydratase [DH (Ot)] aus der Alge Ostreococcus in Pflanzen zu einer Erhöhung bzw. weiteren Steigerung der Menge an LCPUFAs. Anhand von Sequenzvergleichen konnte gezeigt werden, dass die beiden identifizierten Gene Homologien zu Enzymen mit Ketoacyl-CoA-Reduktase (Ketoacyl-CoA-Reduktase von Saccharomyces cerevisiae GenBank Acc. NP009717; Ybr159w) bzw. Dehydratase-Aktivität (Dehydratase/ enoyl-Reduktase Aktivität von Saccharomyces cerevisiae GenBank Acc. S61591; Ydr036c) besitzen (siehe Figuren 4, 5 und 6).

Vorteilhaft werden, wie oben beschrieben, im erfindungsgemäßen Verfahren Nukleinsäuresequenzen, die für Polypeptide bzw. Proteine mit Phospholipase A2- kodieren, in Kombination mit Nukleinsäuresequenzen, die für Polypeptide bzw. Proteine mit Δ-9-Elongase-, Δ-6-Desaturase-, Δ-8-Desaturase-, Δ-12-Desaturase-, Δ-6-Elongase-, Δ-5-Desaturase-, Δ-5-Elongase- oder Δ-4-Desaturase-Aktivität kodieren, verwendet. Die Nukleinsäuresequenzen, die für Polypeptide bzw. Proteine mit Δ-9-Elongase-, Δ-6-Desaturase-, Δ-8-Desaturase-, Δ-12-Desaturase-, Δ-6-Elongase-, Δ-5-Desaturase-, Δ-5-Elongase-, ω-3-Desaturase- oder Δ-4-Desaturase-Aktivität kodieren, sind dabei vorteilhaft ausgewählt aus der Gruppe bestehend aus:
a) einer Nukleinsäuresequenz mit der in SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, SEQ ID NO: 136, SEQ ID NO: 138, SEQ ID NO: 140, SEQ ID NO: 142 oder SEQ ID NO: 144 dargestellten Sequenz, oder
b) Nukleinsäuresequenzen, die sich als Ergebnis des degenerierten genetischen Codes von den in SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO: 129, SEQ ID NO: 131, SEQ ID NO: 133, SEQ ID NO: 135, SEQ ID NO: 137, SEQ ID NO: 139, SEQ ID NO: 141, SEQ ID NO: 143 oder SEQ ID NO: 145 dargestellten Aminosäuresequenzen ableiten lassen, oder
c) Derivate der in SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, SEQ ID NO: 136, SEQ ID NO: 138, SEQ ID NO: 140, SEQ ID NO: 142 oder SEQ ID NO: 144 dargestellten Nukleinsäuresequenz, die für Polypeptide bzw. Proteine kodieren, die mindestens 40 % Identität auf Aminosäureebene mit SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO: 129, SEQ ID NO: 131, SEQ ID NO: 133, SEQ ID NO: 135, SEQ ID NO: 137, SEQ ID NO: 139, SEQ ID NO: 141, SEQ ID NO: 143 oder SEQ ID NO: 145 haben und eine Δ-9-Elongase-, Δ-6-Desaturase-, Δ-8-Desaturase-, Δ-6-Elongase-, Δ-5-Desaturase-, Δ-12-Desaturase-, ω-3-Desaturase-, Δ-5-Elongase- oder Δ-4-Desaturase-Aktivität aufweisen.

Die im Verfahren hergestellten Öle oder Lipide enthalten vorteilhaft einen hohen Anteil an mehrfach ungesättigten Fettsäuren, die vorteilhaft in Membranlipiden und/oder Triacylglyceriden gebunden sind. Die mehrfach ungesättigten Fettsäuren können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Glyceride vorliegen. Die in den Triacylglyceriden gebundenen verschieden Fettsäuren lassen sich dabei von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten. Bevorzugt sind die langkettigen Fettsäuren, besonders bevorzugt sind die langkettigen Fettsäuren LCPUFAs von C₁₈-, C₂₀- und/oder C₂₂-Fettsäuren.

Im erfindungsgemäßen Verfahren werden vorteilhaft Öle oder Lipide in Form ihrer Fettsäureester mit mehrfach ungesättigten C₁₈-, C₂₀- und/oder C₂₂-Fettsäuremolekülen mit mindestens zwei Doppelbindungen im Fettsäureester, vorteilhaft mit mindestens drei, vier, fünf oder sechs Doppelbindungen im Fettsäureester, besonders vorteilhaft mit mindestens fünf oder sechs Doppelbindungen im Fettsäureester hergestellt. Dies führt im Verfahren vorteilhaft zur Synthese von Linolsäure (=LA, C18:2^{Δ9,12}), γ-Linolensäure (= GLA, C18:3^{Δ6,9,12}), Stearidonsäure (= SDA, C18:4 ^{Δ6,9,12,15}), Dihomo-γ-Linolensäure (= DGLA, 20:3 ^{Δ8,11,14}), ω-3-Eicosatetraensäure (= ETA, C20:4 ^{Δ5,8,11,14}), Arachidonsäure (ARA, C20:4^{Δ5,8,11,14}), Eicosapentaensäure (EPA, C20:5^{Δ5,8,11,14,17}), ω-6-Docosapentaensäure (C22:5^{Δ4,7,10,13,16}), ω-6-Docosatetraensäure (C22:4^{Δ,7,10,13,16}), ω-3-Docosapentaensäure (= DPA, C22:5^{Δ7,10,13,16,19}), Docosahexaensäure (= DHA, C22:6^{Δ4,7,10,13,16,19}) oder deren Mischungen, bevorzugt zu ARA, EPA und/oder DHA. Ganz besonders bevorzugt werden ω-3-Fettsäuren wie EPA und/oder DHA hergestellt.

Die Fettsäureester mit mehrfach ungesättigten C₁₈-, C₂₀- und/oder C₂₂-Fettsäuremolekülen können aus den Organismen, die für die Herstellung der Fettsäureester verwendet wurden, in Form eines Öls oder Lipids, beispielsweise in Form von Verbindungen wie Sphingolipiden, Phosphoglyceriden, Lipiden, Glycolipiden wie Glycosphingolipiden, Phospholipiden wie Phosphatidylethanolamin, Phosphatidylcholin, Phosphatidylserin, Phosphatidylglycerol, Phosphatidylinositol oder Diphosphatidylglycerol, Monoacylglyceriden, Diacylglyceriden, Triacylglyceriden oder sonstigen Fettsäureestern wie die AcetylCoenzymA-Ester, die die mehrfach ungesättigten Fettsäuren mit mindestens zwei, drei, vier, fünf oder sechs, bevorzugt fünf oder sechs Doppelbindungen enthalten, isoliert werden. Vorteilhaft werden sie in der Form ihrer Diacylglyceride, Triacylglyceride und/oder in Form des Phosphatidylcholin isoliert, besonders bevorzugt in der Form der Triacylglyceride. Neben diesen Estern sind die mehrfach ungesättigten Fettsäuren auch als freie Fettsäuren oder gebunden in anderen Verbindungen in den Organismen, vorteilhaft den Pflanzen, enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 3 Gew.-%, vorteilhaft von mindestens 5 Gew.-%, bevorzugt von mindestens 8 Gew.-%, besonders bevorzugt von mindestens 10 Gew.-% und ganz besonders bevorzugt von mindestens 15 Gew.-% bezogen auf die gesamten Fettsäuren in der transgenen Pflanzehergestellt. Dabei werden vorteilhaft C₁₈-, C₂₀- und/oder C₂₂-Fettsäuren, die in der Wirtspflanze vorhanden sind, zu mindestens 10 %, vorteilhaft zu mindestens 20 %, besonders vorteilhaft zu mindestens 30 % und ganz besonders vorteilhaft zu mindestens 40 % in die entsprechenden Produkte wie DPA oder DHA, um nur zwei beispielhaft zu nennen, umgesetzt. Vorteilhaft werden die Fettsäuren in gebundener Form hergestellt. Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Weiterhin werden vorteilhaft Vorprodukte dieser Fettsäuren im erfindungsgemäßen Verfahren zur Verfügung gestellt. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA), Eicosapentaensäure (EPA), ω-6-Docosapentaensäure oder DHA nicht als absolute Reinprodukte an, es sind immer auch geringe Spuren der Vorstufen im Endprodukt enthalten. Sind in der Ausgangspflanze beispielsweise sowohl Linolsäure als auch Linolensäure vorhanden, so liegen die Endprodukte wie ARA, EPA oder DHA als Mischungen vor. Die Vorstufen sollten vorteilhaft nicht mehr als 20 Gew.-%, bevorzugt nicht mehr als 15 Gew.-%, besonders bevorzugt nicht als 10 Gew.-% und ganz besonders bevorzugt nicht mehr als 5 Gew.-% bezogen auf die Menge des jeweilige Endprodukts betragen. Vorteilhaft werden in einer transgenen Pflanze als Endprodukte nur ARA, EPA oder nur DHA im erfindungsgemäßen Verfahren gebunden oder als freie Säuren hergestellt. Werden die Verbindungen ARA, EPA und DHA gleichzeitig hergestellt, werden sie vorteilhaft in einem Verhältnis von mindesten 1:1:2 (EPA:ARA:DHA), vorteilhaft von mindestens 1:1:3, bevorzugt von 1:1:4 und besonders bevorzugt von 1:1:5 hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 bis 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureestern bzw. Fettsäuregemischen bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure, EPA und/oder DHA enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureestern bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7 %, 6 % oder 5 % und ganz besonders bevorzugt zu weniger als 4 %, 3 %, 2 % oder 1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren oder keine Buttersäure, kein Cholesterin, keine Clupanodonsäure (= Docosapentaensäure, C22:5Δ^{4,8,12,15,21}) sowie keine Nisinsäure (Tetracosahexaensäure, C23:6^{Δ3,8,12,15,18,21}).

Auch chemisch reine, mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise, beispielsweise über Extraktion, Destillation, Kristallisation, Chromatographie oder Kombinationen dieser Methoden, isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Als Pflanzen kommen prinzipiell alle Pflanzen in Frage, die in der Lage sind, Fettsäuren zu synthetisieren, wie alle dikotylen oder monokotylen Pflanzen, Algen oder Moose. Vorteilhafte Pflanzen sind ausgewählt aus der Gruppe der Pflanzenklassen bzw. -familien Adelotheciaceae, Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Crypthecodiniaceae, Cucurbitaceae, Ditrichaceae, Elaeagnaceae, Ericaceae, Euphorbiaceae, Fabaceae, Geraniaceae, Gramineae, Juglandaceae, Lauraceae, Leguminosae, Linaceae, Euglenacease oder Prasinophyceae. Auch Gemüsepflanzen oder Zierpflanzen wie Tagetes kommen in Betracht.

Beispielhaft seien die folgenden Pflanzen genannt, ausgewählt aus der Gruppe: Adelotheciaceae wie die Gattungen Physcomitrella z.B. die Gattung und Arten *Physcomitrella patens*, Anacardiaceae wie die Gattungen Pistacia, Mangifera, Anacardium z.B. die Gattung und Arten *Pistacia vera* [Pistazie], *Mangifer indica* [Mango] oder *Anacardium occidentale* [Cashew], Asteraceae wie die Gattungen Calendula, Carthamus, Centaurea, Cichorium, Cynara, Helianthus, Lactuca, Locusta, Tagetes, Valeriana z.B. die Gattung und Arten Calendula officinalis [Garten-Ringelblune], Carthamus tinctorius [Färberdistel, safflower], *Centaurea cyanus* [Kornblume], *Cichorium intybus* [Wegwarte], Cynara scolymus [Artichoke], *Helianthus annus* [Sonnenblume], *Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [Salat], *Tagetes lucida, Tagetes erecta* oder *Tagetes tenuifolia* [Studentenblume], Apiaceae wie die Gattung Daucus z.B. die Gattung und Art *Daucus carota* [Karotte], Betulaceae wie die Gattung Corylus z.B. die Gattungen und Arten *Corylus avellana* oder *Corylus colurna* [Haselnuss], Boraginaceae wie die Gattung Borago z.B. die Gattung und Art *Borago officinalis* [Borretsch], Brassicaceae wie die Gattungen Brassica, Camelina, Melanosinapis, Sinapis, Arabadopsis z.B. die Gattungen und Arten *Brassica napus, Brassica rapa* ssp. [Raps], *Sinapis arvensis Brassica juncea, Brassica juncea var. juncea, Brassica juncea var. crispifolia, Brassica juncea var. foliosa, Brassica nigra, Brassica sinapioides*, Camelina sativa, *Melanosinapis communis* [Senf], *Brassica oleracea* [Futterrübe] oder Arabidopsis thaliana, Bromeliaceae wie die Gattungen Anana, Bromelia (Ananas) z.B. die Gattungen und Arten *Anana comosus, Ananas ananas* oder *Bromelia comosa* [Ananas], Caricaceae wie die Gattung Carica wie die Gattung und Art *Carica papaya* [Papaya], Cannabaceae wie die Gattung Cannabis wie die Gattung und Art *Cannabis sative* [Hanf], Convolvulaceae wie die Gattungen Ipomea, Convolvulus z.B. die Gattungen und Arten *Ipomoea batatus, Ipomoea pandurata, Convolvulus batatas, Convolvulus tiliaceus, Ipomoea fastigiata, Ipomoea tiliacea, Ipomoea triloba* oder *Convolvulus panduratus* [Süßkartoffel, Batate], Chenopodiaceae wie die Gattung Beta wie die Gattungen und Arten *Beta vulgaris, Beta vulgaris var. altissima, Beta vulgaris var. Vulgaris, Beta maritima, Beta vulgaris var. perennis, Beta vulgaris var. conditiva* oder *Beta vulgaris var. esculenta* [Zuckerrübe], Crypthecodiniaceae wie die Gattung Crypthecodinium z.B. die Gattung und Art *Cryptecodinium cohnii*, Cucurbitaceae wie die Gattung Cucubita z.B. die Gattungen und Arten *Cucurbita maxima, Cucurbita mixta, Cucurbita pepo* oder *Cucurbita moschata* [Kürbis], Cymbellaceae wie die Gattungen Amphora, Cymbella, Okedenia, Phaeodactylum, Reimeria z.B. die Gattung und Art *Phaeodactylum tricornutum*, Ditrichaceae wie die Gattungen Ditrichaceae, Astomiopsis, Ceratodon, Chrysoblastella, Ditrichum, Distichium, Eccremidium, Lophidion, Philibertiella, Pleuridium, Saelania, Trichodon, Skottsbergia z.B. die Gattungen und Arten *Ceratodon antarcticus, Ceratodon columbiae, Ceratodon heteropfiyllus, Ceratodon purpurascens, Ceratodon purpureus, Ceratodon purpureus ssp. convolutus, Ceratodon purpureus ssp. stenocarpus, Ceratodon purpureus var. rotundifolius, Ceratodon ratodon, Ceratodon stenocarpus, Chrysoblastella chilensis, Ditrichum ambiguum, Ditrichum brevisetum, Ditrichum crispatissimum, Ditrichum difficile, Ditrichum fa*/*cifo*/*ium, Ditrichum flexicaule, Ditrichum giganteum, Ditrichum heteromallum, Ditrichum lineare, Ditrichum lineare, Ditrichum montanum, Ditrichum montanum, Ditrichum pallidum, Ditrichum punctulatum, Ditrichum pusi*/*lum, Ditrichum pusillum var. tortile, Ditrichum rhynchostegium, Ditrichum schimperi*, *Ditrichum tortile, Distichium capillaceum, Distichium hagenii, Distichium inclinatum, Distichium macounii, Eccremidium floridanum, Eccremidium whiteleggei, Lophidion strictus, Pleuridium acuminatum, Pleuridium alternifolium, Pleuridium holdridgei, Pleuridium mexicanum, Pleuridium ravenelii, Pleuridium subulatum, Saelania glaucescens, Trichodon borealis, Trichodon cylindricus oder Trichodon cylindricus var. oblongus*, Elaeagnaceae wie die Gattung Elaeagnus z.B. die Gattung und Art *Olea europaea* [Olive], Ericaceae wie die Gattung Kalmia z.B. die Gattungen und Arten *Kalmia latifolia, Kalmia angustifolia, Kalmia microphylla, Kalmia polifolia, Kalmia occidentalis, Cistus chamaerhodendros* oder *Kalmia lucida* [Berglorbeer], Euglenaceae wie die Gattungen Ascoglena, Astasia, Colacium, Cyclidiopsis, Euglena, Euglenopsis, Hyalaphacus, Khawkinea, Lepocinclis, Phacus, Strombomonas, Trachelomonas z.B. die Gattung und Art Euglena gracilis; Euphorbiaceae wie die Gattungen Manihot, Janipha, Jatropha, Ricinus z.B. die Gattungen und Arten *Manihot utilissima, Janipha manihot" Jatropha manihot*., *Manihot aipil, Manihot dulcis, Manihot manihot, Manihot melanobasis, Manihot esculenta* [Manihot] oder *Ricinus communis* [Rizinus], Fabaceae wie die Gattungen Pisum, Albizia, Cathormion, Feuillea, Inga, Pithecolobium, Acacia, Mimosa, Medicajo, Glycine, Dolichos, Phaseolus, Soja z.B. die Gattungen und Arten *Pisum sativum, Pisum arvense, Pisum humile* [Erbse], *Albizia berteriana, Albizia julibrissin, Albizia lebbeck Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [Seidenbaum], *Medicago sativa, Medicago falcata, Medicago varia* [Alfalfa] *Glycine max Dolichos soja, Glycine gracilis, Glycine hispida, Phaseolus max, Soja hispida* oder *Soja max* [Sojabohne], Funariaceae wie die Gattungen Aphanorrhegma, Entosthodon, Funaria, Physcomitrella, Physcomitrium z.B. die Gattungen und Arten *Aphanorrhegma serratum, Entosthodon attenuatus, Entosthodon bolanderi, Entosthodon bonplandii, Entosthodon californicus, Entosthodon drummondii, Entosthodon jamesonii, Entosthodon leibergii, Entosthodon neoscoticus, Entosthodon rubrisetus, Entosthodon spathulifolius, Entosthodon tucsoni, Funaria americana, Funaria bolanderi, Funaria calcarea, Funaria californica, Funaria calvescens, Funaria convoluta, Funariaflavicans, Funaria groutiana, Funaria hygrometrica, Funaria hygrometrica var. arctica, Funaria hygrometrica var. calvescens, Funaria hygrometrica var. convoluta, Funaria hygrometrica va. muralis, Funaria hygrometrica va. utahensis, Funaria microstoma, Funaria microstoma var. obtusifolia, Funaria muhlenbergii, Funaria orcuttii, Funaria plano-convexa, Funaria polaris, Funarici ravenelii, Funaria rubriseta, Funaria serrata, Funaria sorrorae, Funaria sublimbatus, Funaria tucsoni, Physcomitrella californica, Physcoinitra patens, Physcomitrella readeri, Physcomitrium australe, Physcomitrium californicum, Physcomitrium collenchymatum, Physeomitrium coloradense, Physcomitrium cupuliferum, Physcontitrium drummondii, Physcomitrium eurystomum, Physcomitrium flexifolium, Physcomitrium hookeri, Physeomitrium hookeri var. serratum, Physcomitrium immersum, Physcomitrium kellermanii, Physcomitrium megalocarpum, Physcomitrium pyriforme, Physcomitrium pyriforme var. serratum, Physcomitrium rufipes, Physcomitrium sandbergii, Physcomitrium subsphaericum, Physcomitrium washingtoniense*, Geraniaceae wie die Gattungen Pelargonium, Cocos, Oleum z.B. die Gattungen und Arten *Cocos nucifera, Pelargonium grossularioides* oder *Oleum cocois* [Kokusnuss], Gramineae wie die Gattung Saccharum z.B. die Gattung und Art Saccharum officinarum, Juglandaceae wie die Gattungen Juglans, Wallia z.B. die Gattungen und Arten *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* oder *Wallia nigra* [Walnuss], Lauraceae Wie die Gattungen Persea, Laurus z.B. die Gattungen und Arten *Laurus nobilis* [Lorbeer], *Persea americana, Persea gratissima* oder *Persea per sea* [Avocado], Leguminosae wie die Gattung Arachis z.B. die Gattung und Art *Arachis hypogaea* [Erdnuss], Linaceae wie die Gattungen Linum, Adenolinum z.B, die Gattungen und Arten *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* oder *Linum trigynum* [Lein], Lythrarieae wie die Gattung Punica z.B. die Gattung und Art *Punica granatum* [Granatapfel], Malvaceae wie die Gattung Gossypium z.B. die Gattungen und Arten *Gossypium hir sutum, Gossypium arboreum, Gossypium barbadense, Gossypium herbaceum* oder *Gossypium thurberi* [Baumwolle], Marchantiaceae wie die Gattung Marchantia z.B. die Gattungen und Arten *Marchantia berteroanci, Marchantia foliacea, Marchantia macropora*. Musaceae wie die Gattung Musa z.B. die Gattungen und Arten *Musa nana, Musa acuminata, Musa paradisiaca, Musa* spp. [Banane], Onagraceae wie die Gattungen Camissonia, Oenothera z.B. die Gattungen und Arten *Oenothera biennis* oder *Camissonia brevipes* [Nachtkerze], Palmae wie die Gattung Elacis z.B. die Gattung und Art *Elaeis guineensis* [Ölpalme], Papaveraceae wie die Gattung Papaver z.B. die Gattungen und Arten *Papaver orientale, Papaver rhoeas, Papaver dubium* [Mohn], Pedaliaceae wie die Gattung Sesamum z.B. die Gattung und Art *Sesamum indicum* [Sesam], Piperaceae wie die Gattungen Piper, Artanthe, Peperomia, Steffensia z.B. die Gattungen und Arten *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata*. [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten *Hordeum vulgare, Hordeum jubatum, Hordeum niurinum, Hordeum secalinum, Hordeum distichon Hordeum aegiceras, Hordeum hexastichon., Hordeum hexastichum, Hordeum irregulare, Hordeum sativum, Hordeum secalinum* [Gerste], *Secale cereale* [Roggen], Avena sativa, *Avena fatua, Avena byzantina, Avena fatua var. sativa, Avena hybrida* [Hafer], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia* [Reis], *Zea mays* [Mais] *Triticum aestivum, Triticum durum, Triticum turgidum, Triticum hybernum, Triticum macha, Triticum sativum* oder *Triticum vulgare* [Weizen], Porphyridiaceae wie die Gattungen Chroothece, Flintiella, Petrovanella, Porphyridium, Rhodella, Rhodosorus, Vanhoeffenia z.B. die Gattung und Art *Porphyridium cruentum*, Proteaceae wie die Gattung Macadamia z.B. die Gattung und Art *Macadamia intergrifolia* [Macadamia], Prasinophyceae wie die Gattungen Nephroselmis, Prasinococcus, Scherffelia, Tetraselmis, Mantoniella, Ostreococcus z.B. die Gattungen und Arten *Nephroselmis olivacea, Prasinococcus capsulatus, Scherffelia dubia, Tetraselmis chui, Tetraselmis suecica*, Mantoniella squamata, Ostreococcus tauri, Rubiaceae wie die Gattung Coffea z.B. die Gattungen und Arten Cofea spp., *Coffea arabica, Coffea canephora* oder *Coffea liberica* [Kaffee], Scrophulariaceae wie die Gattung Verbascum z.B. die Gattungen und Arten *Verbascum blattaria, Verbascum chaixii, Verbascum densiflorum, Verbascum lagurus, Verbascum longifolium, Verbascum lychnitis, Verbascum nigrum, Verbascum olynipicum, Verbascum phlomoides, Verbascunt phoenicum, Verbascum pulverulentum* oder *Verbascum thapsus* [*Königskerze*], Solanaceae wie die Gattungen Capsicum, Nicotiana, Solanum, Lycopersicon z.B. die Gattungen und Arten *Capsicum annuum, Capsicum annuum var. glabriusculum, Capsicum frutescens* [Pfeffer], *Capsicum annuum* [Paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [Tabak], *Solanum tuberosum* [Kartoffel], *Solanum melongena* [Aubergine] *Lycopersicon esculentum, Lycopersicon lycopersicum., Lycopersicon pyriforme, Solanum integrifolium* oder *Solantum lycopersicum* [Tomate], Sterculiaceae wie die Gattung Theobroma z.B. die Gattung und Art *Theobroma cacao* [Kakao] oder Theaceae wie die Gattung Camellia z.B. die Gattung und Art *Camellia sinensis* [Tee].

Vorteilhaft werden im erfindungsgemäßen Verfahren transgene Pflanzen wie zweikeimblättrige oder einkeimblättrige Pflanzen verwendet. Besonders vorteilhaft werden Pflanzen im erfindungsgemäßen Verfahren verwendet, die zu den Öl-produzierenden Pflanzen gehören, das heißt die für die Herstellung von Ölen verwendet werden, wie Crypthecodinium, Phaeodactylum oder Pflanzen, insbesondere Pflanzen bevorzugt Ölfruchtpflanzen, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Bonetsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Bevorzugte erfindungsgemäße Pflanzen sind Ölfruchtpflanzen, wie Erdnuss, Raps, Canola, Sonnenblume, Saflor, Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2-und/oder C18:3-Fettsäure-reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf.

Für das erfindungsgemäße beschriebene Verfahren ist es vorteilhaft, in die Pflanze zusätzlich zu den unter Verfahrensschritt (a) bis (c) eingebrachten Nukleinsäuren weitere Nukleinsäuren einzubringen, die für Enzyme des Fettsäure- oder Lipidstoffwechsels kodieren.

Im Prinzip können alle Gene des Fettsäure- oder Lipidstoffwechsels vorteilhaft in Kombination mit der erfinderischen Phospholipase A2 [im Sinne dieser Anmeldung soll der Plural den Singular und umgekehrt beinhalten] im Verfahren zur Herstellung mehrfach ungesättigter Fettsäuren verwendet werden. Vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) in Kombination mit der Phospholipase A2, Ketoacyl-CoA-Reduktase und/oder Dehydratase verwendet. Besonders bevorzugt werden Gene ausgewählt aus der Gruppe der ω-3-Desaturasen, Δ-4-Desaturasen, Δ-5-Desaturasen, Δ-6-Desaturasen, Δ-8-Desatuasen, Δ-9-Desaturasen, Δ-12-Desaturasen, Δ-6-Elongasen, Δ-5-Elongasen oder Δ-9-Elongasen in Kombination mit den vorgenannten Genen für die Phospholipase A2 verwendet, wobei einzelne Gene oder mehrere Gene in Kombination verwendet werden können.

Durch die enzymatische Aktivität der im erfindungsgemäßen Verfahren verwendeten Nukleinsäuren, die für Polypeptide bzw. Proteine mit Phospholipase A2-Aktivität kodieren, vorteilhaft in Kombination mit Nukleinsäuresequenzen, die für Polypeptide bzw. Proteine des Fettsäure- oder Lipidstoffwechsels wie weiteren Polypeptiden bzw. Proteinen mit ω-3-, Δ-4-, Δ-5-, Δ-6-, Δ-8-, Δ-12-Desaturase- oder Δ-5-, Δ-6-oder Δ-9-Elongase-Aktivität kodieren, können unterschiedlichste mehrfach ungesättigte Fettsäuren im erfindungsgemäßen Verfahren hergestellt werden. Je nach Auswahl der für das erfindungsgemäße Verfahren verwendeten Pflanze lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA, oder die sich von C18:3-Fettsäuren ableiten, wie SDA, ETA oder EPA. Liegt in der für das Verfahren verwendeten Pflanze als ungesättigte Fettsäure nur Linolsäure (= LA, C18:2^{Δ9,12}) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:3^{Δ9,12,15}) beispielsweise wie in Lein, so können als Produkte des Verfahrens nur SDA, ETA, EPA und/oder DHA entstehen, die wie oben beschrieben als freie Fettsäuren oder gebunden vorliegen können. Durch Modifikation der Aktivität der an der Synthese beteiligten Enzyme wie der Phospholipase A2- vorteilhaft in Kombination mit der ω-3-, Δ-4-, Δ-5-, Δ-6-, Δ-12-Desaturase und/oder Δ-6-, Δ-5-Elongase, oder der Δ-4-, Δ-5-, Δ-8-, Δ-12-Desaturase, und/oder Δ-9-, Δ-5-Elongase lassen sich gezielt in den vorgenannten Organismen, vorteilhaft in den vorgenannten Pflanzen, nur einzelne Produkte herstellten. Durch die Aktivität der Δ-6-Desaturase undä-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Werden die Δ-5-Desaturasen, die Δ-5-Elongase und die Δ-4-Desaturase zusätzlich in die Organismen, vorteilhaft in die Pflanze, eingebracht, so entstehen zusätzlich ARA, EPA und/oder DHA. Dies gilt auch für Organismen, in die vorher die Δ-8-Desaturase und Δ-9-Elongase eingebracht wurde. Vorteilhaft werden nur ARA, EPA oder DHA oder deren Mischungen synthetisiert, abhängig von der in im Organismus bzw. in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen, bzw. ARA, EPA, DHA oder deren Mischungen, vorteilhaft EPA oder DHA oder deren Mischungen.

Neben der Produktion der Ausgangsfettsäuren für die verwendete Phospholipase A2 direkt in der Pflanze können die Fettsäuren auch von außen gefüttert werden. Aus Kostengründen ist die Produktion in der Pflanze oder Pflanzenzelle bevorzugt. Bevorzugte Substrate der Phospholipase A2 sind Phospholipide, spezifischer Phosphatidylcholine und Phosphatidylethanolamine, am besten Phosphatidylcholine mit den Fettsäuren γ-Linolensäure (C18:3^{Δ6,9,12}), Stearidonsäure (C18:4^{Δ6,9,12,15}) und Eicosapentaensäure (C20:5^{Δ5,8,11,14,17}) an der sn-2-Position. Bevorzugte Substrate der Ketoacyl-CoA-Reduktase oder Dehydratase sind die CoA-Ester der γ-Linolensäure (C18:3^{Δ6,9,12}), Stearidonsäure (C18:4^{Δ6,9,12,15}), Arachidonsäure (C20:4^{Δ5,8,11,14}) und Eicosapentaensäure (C20:5^{Δ5,8,11,14,17}).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft, die Menge an Ausgangsprodukt für die Fettsäuresynthese zu steigern. Dies kann beispielsweise durch das Einbringen einer Nukleinsäure in die Pflanze, die für ein Polypeptid bzw. Protein mit Δ-12-Desaturase kodiert, erreicht werden. Dies ist besonders vorteilhaft in Öl-produzierenden Pflanzen wie der Familie der Brassicaceae wie der Gattung Brassica z.B. Raps; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max*, die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681), ist die Verwendung der genannten Δ-12-Desaturasen zur Herstellung des Ausgangsprodukts Linolsäure vorteilhaft.

Vorteilhaft werden im erfindungsgemäßen Verfahren die vorgenannten Nukleinsäuresequenzen oder deren Derivate oder Homologe verwendet, die für Polypeptide bzw. Proteine mit Phospholipase A2- Aktivität kodieren, die noch die enzymatische Aktivität der durch die Nukleinsäuresequenzen kodierten Proteine besitzen. Diese Sequenzen werden einzeln oder in Kombination mit den für die Δ-12-Desaturase, Δ-4-Desaturase, Δ-5-Desaturase, Δ-8-Desaturase, Δ-6-Desaurase, Δ-5-Elongase, Δ-6-Elongase, Δ-9-Elongase und/oder ω-3-Desaturase kodierenden Nukleinsäuresquenzen in Expressionskonstrukte cloniert und zum Einbringen und zur Expression in Pflanzen oder Pflanzenzellen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Pflanzenzelle oder einer ganzen Pflanze die die im Verfahren verwendeten Nukleinsäuresequenzen enthält, wobei die Zelle und/oder der Pflanze mit einer erfindungsgemäßen Nukleinsäuresequenz, die für die Phospholipase A2 kodiert, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels kodieren, transformiert wird. Bei einer weiteren bevorzugten Ausführungsform umfasst dieses Verfahren ferner den Schritt des Gewinnens der Öle, Lipide oder freien Fettsäuren aus der Pflanze oder Pflanzenzelle oder aus der Kultur. Bei der Kultur kann es sich beispielsweise um eine Fermentationskultur, oder um eine Treibhaus- oder Feldkultur einer Pflanze handeln. Die so hergestellte Zelle oder die so hergestellte Pflanze ist vorteilhaft eine Zelle einer Öl-produzierenden Pflanze wie einer Ölfruchtpflanze wie beispielsweise Erdnuss, Raps, Canola, Lein, Hanf, Erdnuss, Soja, Safflower, Hanf, Sonnenblumen oder Borretsch.

Unter Anzucht ist beispielsweise die Kultivierung im Falle von Pflanzenzellen, -geweben oder -organen auf oder in einem Nährmedium oder die Kultivierung der ganzen Pflanze auf bzw. in einem Substrat, beispielsweise in Hydrokultur, Blumentopferde oder auf einem Ackerboden zu verstehen.

"Transgen" bzw. "Rekombinant" im Sinne der Erfindung bedeutet bezüglich beispielsweise einer Nukleinsäuresequenz, einer Expressionskassette (= Genkonstrukt) oder einem Vektor enthaltend die beschriebene Nukleinsäuresequenz oder einem Organismus transformiert mit der Nukleinsäuresequenz, der Expressionskassette oder dem Vektor alle solche durch gentechnische Methoden zustandegekommenen Konstruktionen, in denen entweder
a) die beschriebene Nukleinsäuresequenz, oder
b) eine mit der beschriebenen Nukleinsäuresequenz funktionell verknüpfte genetische Kontrollsequenz, zum Beispiel ein Promotor, oder
c) (a) und (b)
sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. "Natürliche genetische Umgebung" meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp und ganz besonders bevorzugt mindestens 5000 bp. Eine natürlich vorkommende Expressionskassette - beispielsweise die natürlich vorkommende Kombination des natürlichen Promotors der beschriebenen Nukleinsäuresequenzen mit der entsprechenden Phospholipase A2- wird zu einer transgenen Expressionskassette, wenn diese durch nicht-natürliche, synthetische ("künstliche") Verfahren wie beispielsweise eine Mutagenisierung geändert wird. Entsprechende Verfahren sind beispielsweise beschrieben in US 5,565,350 oder WO 00/15815.

Unter "transgener Pflanze" im Sinne der Erfindung ist wie vorgenannt zu verstehen, dass die im Verfahren verwendeten Nukleinsäuren nicht an ihrer natürlichen Stelle im Genom der Pflanze sind. Dabei können die Nukleinsäuren homolog oder heterolog exprimiert werden. Transgen bedeutet aber auch wie genannt, dass die beschriebenen Nukleinsäuren an ihrem natürlichen Platz im Genom der Pflanze sind, dass jedoch die Sequenz gegenüber der natürlichen Sequenz verändert wurde und/oder dass die Regulationssequenzen gegenüber den natürlichen Sequenzen verändert wurden. Bevorzugt ist unter transgen die Expression der Nukleinsäuren an nicht natürlicher Stelle im Genom zu verstehen, das heißt eine homologe oder bevorzugt heterologe Expression der Nukleinsäuren liegt vor. Bevorzugte transgene Pflanzen sind die Ölfruchtpflanzen.

Als Organismen bzw. Wirtsorganismen für die im erfindungsgemäßen Verfahren verwendeten Nukleinsäuren, Expressionskassetten oder Vektoren eignen sich prinzipiell vorteilhaft alle Organismen, die in der Lage sind, Fettsäuren, speziell ungesättigte Fettsäuren, zu synthetisieren, bzw. die für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne, Mikroorganismen wie Pilze beispielsweise die Gattung Mortierella, Thraustochytrium, Saprolegnia, Phytophtora oder Pythium, Bakterien wie die Gattung Escherichia oder Shewanella, Hefen wie die Gattung Saccharomyces, Cyanobakterien, Ciliaten, Algen wie Mantoniella, Euglena oder Ostreococcus oder Protozoen wie Dinoflagellaten wie Thalassiosira oder Crypthecodinium genannt. Bevorzugt werden Organismen, die natürlicherweise Öle in größeren Mengen synthetisieren können wie Pilze wie Mortierella alpina, Pythium insidiosum, Phytophtora infestans oder Pflanzen wie Soja, Raps, Kokosnuss, Ölpalme, FärberSaflor, Flachs, Hanf, Rizinus, Calendula, Erdnuss, Kakaobohne oder Sonnenblume, oder Hefen wie Saccharomyces cerevisiae. Besonders bevorzugt werden Soja, Flachs, Raps, FärberSaflor, Sonnenblume, Calendula, Mortierella oder Saccharomyces cerevisiae. Prinzipiell sind als Wirtsorganismen neben den vorgenannten transgenen Organismen auch transgene Tiere, vorteilhaft nicht-humane Tiere geeignet, beispielsweise C. elegans, Ciöna intestinalis oder Xenopus laevis.

Nutzbare Wirtszellen sind weiterhin genannt in: Goeddel, Gene Expression Technology: Methods in Enzymology 185, Academic Press, San Diego, CA (1990).

Verwendbare Expressionsstämme wie z.B. solche, die eine geringere Proteaseaktivität aufweisen, sind beschrieben in: Gottesman, S., Gene Expression Technology: Methods in Enzymology 185, Academic Press, San Diego, California (1990) 119-128.

Zu den Pflanzenwirten gehören vorteilhaft auch Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, dies von der eigentlichen transgenen Pflanze abgeleitet sind und/oder dazu verwendet werden können, die transgene Pflanze hervorzubringen:

Transgene Pflanzen, die die im erfindungsgemäßen Verfahren synthetisierten mehrfach ungesättigten Fettsäuren enthalten, können vorteilhaft direkt vermarktet werden, ohne dass die synthetisierten Öle, Lipide oder Fettsäuren isoliert werden müssen. Unter Pflanzen im erfindungsgemäßen Verfahren sind ganze Pflanzen sowie alle Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe oder Zellkulturen gemeint, die sich von der transgenen Pflanze ableiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen, vorteilhaft Pflanzen, in Form ihrer Öle, Fette, Lipide und/oder freien Fettsäuren isoliert werden. Durch dieses Verfahren hergestellte mehrfach ungesättigten Fettsäuren lassen sich durch Ernten der Organismen entweder aus der Kultur, in der sie wachsen, oder vom Feld gewinnen. Dies kann über Pressen oder Extraktion der Pflanzenteile, bevorzugt der Pflanzensamen, erfolgen. Dabei können die Öle, Fette, Lipide und/oder freien Fettsäuren durch sogenanntes Kaltschlagen oder Kaltpressen ohne Zuführung von Wärme durch Pressen gewonnen werden. Damit sich die Pflanzenteile, speziell die Samen, leichter aufschließen lassen, werden sie vorher zerkleinert, gedämpft oder geröstet. Die so vorbehandelten Samen können anschließend gepresst werden oder mit Lösungsmittel wie warmem Hexan extrahiert werden. Anschließend wird das Lösungsmittel wieder entfernt. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base, beispielsweise Natronlauge, entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen, werden die Produkte einer Bleichung mit beispielsweise Bleicherde oder Aktivkohle unterzogen. Zum Schluss wird das Produkt beispielsweise mit Wasserdampf desodoriert.

Vorzugsweise sind die durch dieses Verfahren produzierten PUFAs bzw. LCPUFAs C₁₈-, C₂₀- oder C₂₂-Fettsäuremoleküle, vorteilhaft C₂₀- oder C₂₂-Fettsäuremoleküle mit mindestens zwei Doppelbindungen im Fettsäuremolekül, vorzugsweise drei, vier, fünf oder sechs Doppelbindungen. Diese C₁₈-, C₂₀- oder C₂₂-Fettsäuremoleküle lassen sich aus der Pflanze in Form eines Öls, Lipids oder einer freien Fettsäure isolieren. Geeignete Pflanzen sind beispielsweise die vorstehend erwähnten.

Weiterhin beschrieben werden Öle, Lipide, Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.

Diese Öle, Lipide oder Fettsäuren enthalten wie oben beschrieben vorteilhaft 6 bis 15 % Palmitinsäure, 1 bis 6 % Stearinsäure; 7 bis 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureestern bzw. Fettsäuregemischen bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure, EPA und/oder DHA enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vemonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure),Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureestern bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7 %, 6 % oder 5 %, ganz besonders bevorzugt zu weniger als 4 %, 3 %, 2 % oder 1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren oder keine Butterbuttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,12,15,18,21}).

Vorteilhaft enthalten die im erfindungsgemäßen Verfahren hergestellten Öle, Lipide oder Fettsäuren mindestens 0,5 %, 1 %, 2 %, 3 %, 4 % oder 5 %, vorteilhaft mindestens 6 %, 7 %, 8 %, 9 % oder 10 %, besonders vorteilhaft mindestens 11 %, 12 %, 13 %, 14 % oder 15 % ARA oder mindestens 0,5 %, 1 %, 2 %, 3 %, 4 % oder 5 %, vorteilhaft mindestens 6 %, oder 7 %, besonders vorteilhaft mindestens 8 %, 9 % oder 10 % EPA und/oder DHA bezogen auf den Gesamtfettsäuregehalt des Produktionsorganismus, vorteilhaft einer Pflanze, besonders vorteilhaft einer Ölfruchtpflanze wie Soja, Raps, Kokosnuss, Ölpalme, Färbersafflor, Flachs, Hanf, Rizinus, Calendula, Erdnuss, Kakaobohne, Sonnenblume oder den oben genannten weiteren ein- oder zweikeimblättrigen Ölfruchtpflanzen.

Außerdem beschrieben ist die Verwendung dieser Öle, Lipide, Fettsäuren und/oder Fettsäurezusammensetzungen in Futtermitteln,

Nahrungsmitteln, Kosmetika oder Pharmazeutika. Die Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-γ-linolensäure, Arachidonsäure, α-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Antéil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylester durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc. enthalten. Insbesondere kann je nach Ausgangsorganismus der Anteil der verschiedenen Fettsäuren in dem Öl oder Fett schwanken.

Bei den im Verfahren hergestellten mehrfach ungesättigte Fettsäuren mit vorteilhaft mindestens zwei Doppelbindungen handelt es sich wie oben beschrieben beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigten Fettsäuren mit vorteilhaft mindestens fünf oder sechs Doppelbindungen lassen sich die enthaltenden mehrfach ungesättigten Fettsäuren beispielsweise über eine Alkalibehandlung, beispielsweise wäßrige KOH oder NaOH oder saure Hydrolyse, vorteilhaft in Gegenwart eines Alkohols wie Methanol oder Ethanol oder über eine enzymatische Abspaltung freisetzen und beispielsweise über Phasentrennung und anschließender Ansäuerung über z.B. H₂SO₄ isolieren. Die Freisetzung der Fettsäuren kann auch direkt ohne die vorhergehend beschriebene Aufarbeitung erfolgen.

Die im Verfahren verwendeten Nukleinsäuren können nach Einbringung in einer Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen, vorteilhaft zur multiparallelen samenspezifischen Expression von Genen in die Pflanzen, gebracht.

Als Substrate der im erfindungsgemäßen Verfahren verwendeten Nukleinsäuren, die für Polypeptide bzw. Proteine mit Phospholipase A2- kodieren, und/oder den weiteren verwendeten Nukleinsäuren wie den Nukleinsäuren, die für Polypeptide bzw. Proteine des Fettsäure- oder Lipidstoffwechsels ausgewählt aus der Gruppe Δ-12-Desaturase(n), Δ-9-Elongase(n), Δ-8-Desaturase(n), Δ-6-Desaturase(n), Δ-6-Elongase(a), Δ-5-Desaturase(n), Δ-5-Elongase(n), ω-3-Desaturase(n), Δ-4-Desaturas(n), Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) kodieren, eignen sich vorteilhaft C₁₆-, C₁₈-, C₂₀- oder C₂₂-Fettsäuren. Bevorzugt werden die im Verfahren als Substrate umgesetzten Fettsäuren in Form ihrer Acyl-CoA-Ester und/oder ihrer Phospholipid-Ester umgesetzt.

Zur Herstellung der langkettigen PUFAs müssen die mehrfach ungesättigten C₁₈-Fettsäuren zunächst durch die enzymatische Aktivität einer Desaturase desaturiert und anschließend über eine Elongase um mindestens zwei Kohlenstoffatome verlängert werden. Nach einer Elongationsrunde führt diese Enzymaktivität zu C₂₀-Fettsäuren, und nach zwei Elongationsrunden zu C₂₂-Fettsäuren. Die Aktivität der im erfindungsgemäßen Verfahren verwendeten Desaturasen und Elongasen führt vorzugsweise zu C₁₈-, C₂₀- und/oder C₂₂-Fettsäuren, vorteilhaft mit mindestens zwei Doppelbindungen im Fettsäuremolekül, vorzugsweise mit drei, vier, fünf oder sechs Doppelbindungen, besonders bevorzugt zu C₂₀-und/oder C₂₂-Fettsäuren mit mindestens zwei Doppelbindungen im Fettsäuremolekül, vorzugsweise mit drei, vier, fünf oder sechs Doppelbindungen, ganz besonders bevorzugt mit fünf oder sechs Doppelbindungen im Molekül. Nachdem eine erste Desaturierung und die Verlängerung stattgefunden hat, können weitere Desaturierungs- und Elongierungsschritte wie z.B. eine solche Desaturierung in Δ-5- und Δ-4-Postition erfolgen. Besonders bevorzugt als Produkte des erfindungsgemäßen Verfahrens sind Dihomo-γ-linolensäure, Arachidonsäure, Eicosapentaensäure, Docosapetaensäure und/oder Docosahesaensäure. Die C₂₀-Fettsäuren mit mindestens zwei Doppelbindungen in der Fettsäure können durch enzymatische Aktivitäten in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fetten in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Durch das erfindungsgemäße Verfahren können die hergestellten mehrfach ungesättigten Fettsäuren in der im Verfahren verwendeten Pflanze prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in der transgenen Pflanze erhöht.

Die hergestellten mehrfach ungesättigten Fettsäuren können nach dem Fachmann bekannten Verfahren wie oben beschrieben aus den Pflanzen isoliert werden, beispielsweise über Extraktion, Destillation, Kristallisation, ggf. Salzfällung und/oder Chromatographie. Die Pflanzen können dazu vorher noch vorteilhaft aufgeschlossen werden.

Der pH-Wert wird dabei vorteilhaft zwischen pH 4 und 12, bevorzugt zwischen pH 6 und 9, besonders bevorzugt zwischen pH 7 und 8 gehalten.

Diese erfindungsgemäß einsetzbaren Medien umfassen wie oben beschrieben gewöhnlich eine oder mehrere Kohlenstoffquellen, Stickstoffquellen, anorganische Salze, Vitamine und/oder Spurenelemente.

Bevorzugte Kohlenstoffquellen sind Zucker wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen wie Melassen oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z.B. Sojaöl, Sonnenblumenöl, Erdnussöl und/oder Kokosfett, Fettsäuren wie z.B. Palmitinsäure, Stearinsäure und/oder Linolsäure, Alkohole und/oder Polyalkohole wie z. B. Glycerin, Methanol und/oder Ethanol und/oder organische Säuren wie z.B. Essigsäure und/oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger Form oder Gasform oder Ammoniumsalze wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide, aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden natriumhaltigen Salze verwendet werden.

Chelatbildner können zum Medium gegeben werden, um die Metallionen in Lösung zu halten. Besonders geeignete Chelatbildner umfassen Dihydroxyphenole wie Catechol oder Protocatechuat oder organische Säuren wie Citronensäure.

Sämtliche Medienkomponenten werden entweder durch Hitze (20 min bei 1,5 bar und 121°C) oder durch Sterilfiltration sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C, und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwicklung können Antischaummittel wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder sauerstoffhaltige Gasmischungen, wie z.B. Umgebungsluft, in die Kultur eingetragen. Die Temperatur der Kultur liegt normalerweise bei 20°C bis 45°C und vorzugsweise bei 25°C bis 40°C. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere mehrfach ungesättigte Fettsäuren enthaltenden Fermentationsbrühen haben üblicherweise eine Trockenmasse von 7,5 bis 25 Gew.-%.

Die Fermentationsbrühe kann anschließend weiter verarbeitet werden. Je nach Anforderung kann die Biomasse ganz oder teilweise durch Separationsmethoden wie z. B. Zentrifugation, Filtration, Dekantieren oder einer Kombination dieser Methoden aus der Fermentationsbrühe entfernt oder vollständig in ihr belassen werden. Vorteilhaft wird die Biomasse nach Abtrennung aufgearbeitet.

Die Fermentationsbrühe kann aber auch ohne Zellabtrennung mit bekannten Methoden wie z. B. mit Hilfe eines Rotationsverdampfers, Dünnschichtverdampfers, Fallfilmverdampfers, durch Umkehrosmose oder durch Nanofiltration eingedickt beziehungsweise aufkonzentriert werden. Diese aufkonzentrierte Fermentationsbrühe kann schließlich zur Gewinnung der darin enthaltenen Fettsäuren aufgearbeitet werden.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmaka, Nahrungsmitteln, Tierfutter oder Kosmetika verwendet werden.

Weiterhin beschrieben sind isolierte Nukleinsäuresequenzen, die für Polypeptide bzw. Proteine mit Phospholipase A2-aktivität kodieren.

Weiterhin offenbart sind isolierte Nukleinsäuresequenzen, die für Polypeptide bzw. Proteine mit Phospholipase A2-Aktivität kodieren, ausgewählt aus der Gruppe:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 2 dargestellten Aminosäuresequenz ableiten lassen, oder
c) Derivate der in SEQ ID NO: 1 dargestellten Nukleinsäuresequenz, die für Polypeptide bzw. Proteine kodieren, die mindestens 40 % Identität auf Aminosäureebene mit SEQ ID NO: 2 haben und eine Phospholipase A2-Aktivität aufweisen.

Weiterhin beschrieben sind Genkonstrukt, die die Nukleinsäuresequenz SEQ ID NO: 1, enthalten, wobei die Nukleinsäure funktionsfähig mit einem oder mehreren Regulationssignalen verbunden ist. Zusätzlich können weitere Biosynthesegene des Fettsäure- oder Lipidstoffwechsels ausgewählt aus der Gruppe Δ-4-Desaturase(n), Δ-5-Desaturase(n), Δ-6-Desaturase(n), Δ-8-Desatuase(n), Δ-12-Desaturase(n), Δ-6-Elongase(n), Δ-5-Elongase(n), Δ-9-Elongase(n), ω-3-Desaturase(n), Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) im Genkonstrukt enthalten sein. Vorteilhaft sind zusätzlich Biosynthesegene des Fettsäure- oder Lipidstoffwechsels ausgewählt aus der Gruppe der Δ-4-Desaturase, Δ-5-Desaturase, Δ-6-Desaturase, Δ-8-Desatuase, Δ-9-Desaturase, Δ-12-Desaturase, Δ-6-Elongase, Δ-5-Elongase, Δ-9-Elongase oder ω-3-Desaturase enthalten.

Moose und Algen sind die einzigen bekannten Pflanzensysteme, die erhebliche Mengen an mehrfach ungesättigten Fettsäuren wie Arachidonsäure (ARA) und/oder Eicosapentaensäure (EPA) und/oder Docosahexaensäure (DHA) herstellen. Moose enthalten PUFAs in Membranlipiden, während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt.

Die im erfindungsgemäßen Verfahren verwendete Nukleinsäuren stammen daher vorteilhaft aus Pflanzen wie Algen, beispielsweise Algen der Klasse der Prasinophyceae, wie aus den Gattungen Heteromastix, Mammella, Mantoniella, Micromonas, Nephroselmis, Ostreococcus, Prasinocladus, Prasinococcus, Pseudoscourfielda, Pycnococcus, Pyramimonas, Scherffelia oder Tetraselmis wie den Gattungen und Arten Heteromastix longifillis, Mamiella gilva, Mantoniella squamata, Micromonas pusilla, Nephroselmis olivacea, Nephroselmis pyriformis, Nephroselmis rotunda, Ostreococcus tauri, Ostreococcus sp. Prasinocladus ascus, Prasinocladus lubricus, Pycnococcus provasolii, Pyramimonas amylifera, Pyramimonas disomata, Pyramimonas obovata, Pyramimonas orientalis, Pyramimonas parkeae, Pyramimonas spinifera, Pyramimonas sp., Tetraselmis apiculata, Tetraselmis carteriaformis, Tetraselmis chui, Tetraselmis convolutae, Tetraselmis desikacharyi, Tetraselmis gracilis, Tetraselmis hazeni, Tetraselmis impellucida, Tetraselmis inconspicua, Tetraselmis levis, Tetraselmis maculata, Tetraselmis marina, Tetraselmis striata, Tetraselmis subcordiformis, Tetraselmis suecica, Tetraselmis tetrabrachia, Tetraselmis tetrathele, Tetraselmis verrucosa, Tetraselmis verrucosa fo. rubens oder Tetraselmis sp. oder aus Algen der Familie Pythiaceae oder der Familie Euglenaceae wie aus den Gattungen Ascoglena, Astasia, Colacium, Cyclidiopsis, Euglena, Euglenopsis, Hyalophacus, Khawkinea, Lepocinclis, Phacus, Strombomonas oder Trachelomonas wie die Gattungen und Art Euglena acus, Euglena geniculata, Euglena gracilis, Euglena mixocylindracea, Euglena rostrifera, Euglena viridis, Colacium stentorium, Trachelomonas cylindrica oder Trachelomonas volvocina. Vorteilhaft stammen die verwendeten Nukleinsäuren aus Algen der Gattungen Euglena, Mantonielle oder Ostreococcus.

Weitere vorteilhafte Pflanzen sind Algen wie Isochrysis oder Crypthecodinium, Diatomeen wie Thalassiosira, Crypthecodinium oder Phaeodactylum, Moose wie Physcomitrella oder Ceratodon sowie höhere Pflanzen wie Muscarioides, Borago, Primulaceae wie Aleuritia, Calendula stellata, Osteospermum spinescens oder Osteospermum hyoseroides. Außerdem vorteilhaft sind Mikroorganismen wie Pilze wie Phycomycota wie Thraustochytrium, Aspergillus, Phytophtora, Entomophthora, Mucor, Fusarium, Phytophthora oder Mortierella, Hefen wie Saccharomyces sowie Bakterien wie Shewanella.

Auch vorteilhaft sind Einzeller, Ciliaten, Dinoflagellaten sowie nicht-humane Tiere wie Nematoden wie Caenorhabditis, Ciona, Xenopus, Insekten, Seegurken oder Fische, bevorzugt aus der Ordnung der Salmoniformes wie der Familie der Salmonidae wie der Gattung Salmo, beispielsweise aus den Gattungen und Arten Oncorhynchus mykiss, Trutta trutta oder Salmo trutta fario.. Vorteilhaft stammen die erfindungsgemäßen isolierten Nukleinsäuresequenzen aus einem Tier aus der Ordnung der Vertebraten. Bevorzugt stammen die Nukleinsäuresequenzen aus der Klasse der Vertebrata; Euteleostomi, Actinopterygii; Neopterygü; Teleostei; Euteleostei, Protacanthopterygii, Salmoniformes; Salmonidae bzw. Oncorhynchus oder Vertebrata, Amphibia, Anura, Pipidae, Xenopus oder Evertebrata wie Protochordata, Tunicata, Holothuroidea, Cionidae wie Amaroucium constellatum, Botryllus schlosseri, Ciona intestinalis, Molgula citrina, Molgula manhattensis, Perophora viridis oder Styela partita.

Die im Verfahren verwendeten Nukleinsäuresequenzen, die für Proteine mit Phospholipase A2- kodieren, werden vorteilhaft allein oder bevorzugt in Kombination miteinander oder mit anderen Nukleinsäuresequenzen, die für Proteine mit ω-3-Desaturase-, Δ-4-Desaturase-, Δ-5-Desaturase-, Δ-6-Desaturase-, Δ-8-Desatuase-, Δ-12-Desaturase-, Δ-5-Elongase-, Δ-6-Elongase- oder Δ-9-Elongase-Aktivität kodieren, in einer Expressionskassette (= Nukleinsäurekonstrukt), die die Expression der Nukleinsäuren in einer Pflanze möglicht, eingebracht. Es kann im Nukleinsäurekonstrukt mehr als eine Nukleinsäuresequenz einer enzymatischen Aktivität wie z.B. einer Phospholipase A2, Ketoacyl-CoA-Reduktase, Dehydratase, Δ-12-Desaturase, Δ-4-Desaturase, Δ-5-Desaturase, Δ-6-Desaturase, Δ-5-Elongase, Δ-6-Elongase und/oder ω-3-Desaturase enthalten sein.

Zum Einbringen werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung. Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilzen gewährleisten, und die die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel **dadurch gekennzeichnet, dass** sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E.coli als auch in Agrobacterium zu replizieren. Zu diesen binären Vektoren gehören Vektoren der Serien pBIB-HYG, pPZP, pBecks und pGreen. Erfindungsgemäß bevorzugt verwendet werden Bin19, pBI101, pBinAR, pGPTV und pCAMBIA. Eine Übersicht über binäre Vektoren und ihre Verwendung gibt Hellens et al., Trends in Plant Science (2000) 5, 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittene und erforderlichenfalls gereinigte Amplifikat mit ähnlich präparierten Vektorfragmenten unter Einsatz einer Ligase kloniert. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere Escherichia coli und Agrobacterium tumefaciens, unter selektiven Bedingungen stabil propagieren und ermöglichen einen Transfer von heterologer DNA in Pflanzen oder Mikroorganismen.

Unter der vorteilhaften Verwendung von Klonierungsvektoren können die im Verfahren verwendeten Nukleinsäuren und Nukleinsäurekonstrukte in Pflanzen eingebracht werden und damit bei der Pflanzentransformation verwendet werden, wie diejenigen, die veröffentlicht sind und zitiert sind in: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)). Die im Verfahren verwendeten Nukleinsäuren und Nukleinsäurekonstrukte und/oder Vektoren lassen sich damit zur gentechnologischen Veränderung eines breiten Spektrums an Organismen, vorteilhaft an Pflanzen, verwenden, so dass diese bessere und/oder effizientere Produzenten von PUFAs werden.

Es gibt eine Reihe von Mechanismen, durch die eine Veränderung des Phospholipase A2- Proteins sowie der weiteren im Verfahren verwendeten Proteine wie die Δ-12-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Proteine möglich ist, so dass die Ausbeute, Produktion und/oder Effizienz der Produktion der vorteilhaft mehrfach ungesättigten Fettsäuren in einer Pflanze, bevorzugt in einer Ölfruchtpflanze oder einem Mikroorganismus, aufgrund dieses veränderten Proteins direkt beeinflusst werden kann. Die Anzahl oder Aktivität der Phospholipase A2-, Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongäse- und/oder Δ-4-Desaturase-Proteine und/oder -Gene kann erhöht werden, so dass größere Mengen der Genprodukte und damit letztlich größere Mengen der Verbindungen der allgemeinen Formel I hergestellt werden. Auch eine de novo Synthese in einem Organismus, dem die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines Phospholipase A2-, Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Gens in einen Organismus allein oder in Kombination mit anderen Genen in eine Zelle kann nicht nur den Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Öle, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PLTFAs, wie im folgenden beschrieben, weiter gesteigert wird. Durch Optimierung der Aktivität oder Erhöhung der Anzahl einer oder mehrerer Phospholipase A2-, Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Gene, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die im erfindungsgemäßen Verfahren verwendeten isolierten Nukleinsäuremoleküle kodieren für Proteine oder Teile von diesen, wobei die Proteine oder das einzelne Protein oder Teile davon eine Aminosäuresequenz enthalten, die ausreichend homolog zu einer Aminosäuresequenz ist, die in der Sequenz SEQ ID NO: 2 dargestellt ist, so dass die Proteine oder Teile davon noch eine Phospholipase A2- Aktivität aufweisen. Vorzugsweise haben die Proteine oder Teile davon, die von dem Nukleinsäuremolekül / den Nukleinsäuremolekülen kodiert werden, noch ihre wesentliche enzymatische Aktivität und die Fähigkeit, am Stoffwechsel von zum Aufbau von Zellmembranen oder Lipidkörperchen in Pflanzen, notwendigen Verbindungen oder am Transport von Molekülen über diese Membranen teilzunehmen. Vorteilhaft sind die von den Nukleinsäuremolekülen kodierten Proteine zu mindestens etwa 30 %, 35 %, 40 %, 45 % oder 50 %, vorzugsweise mindestens etwa 55 % oder 60 % und stärker bevorzugt mindestens etwa 70 %, 80 % oder 90 % und am stärksten bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr identisch zu der in SEQ ID NO: 2 dargestellten Aminosäuresequenz. Im Sinne der Erfindung ist unter "Homologie" oder "homolog" Identität oder identisch zu verstehen.

Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen, zur Verfügung. Dabei liefern die Algorithmen von Needleman und Wunsch oder Smith und Waterman besonders zuverlässige Ergebnisse. Für die Sequenzvergleiche wurde das Programm PileUp (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)] verwendet, die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 8, Length Weight: 2, Average Match: 2.778 und Average Mismatch: -2.248. Diese Einstellungen wurden, falls nicht anders angegeben, immer als Standardeinstellungen für Sequenzvergleiche verwendet.

Unter "wesentlicher enzymatischer Aktivität" der im erfindungsgemäßen Verfahren verwendeten Phospholipase A2 ist zu verstehen, dass sie gegenüber den durch die Sequenz mit SEQ ID NO: 1 und deren Derivate kodierten Proteinen/Enzymen im Vergleich noch eine enzymatische Aktivität von mindestens 10 %, bevorzugt 20 %, besonders bevorzugt 30 % und ganz besonders bevorzugt 40 % aufweisen und damit am Stoffwechsel von zum Aufbau von Fettsäuren, Fettsäureestern wie Diacylglyceriden und/oder Triacylglyceriden in einem Organismus, vorteilhaft einer Pflanze oder Pflanzenzelle, notwendigen Verbindungen oder am Transport von Molekülen über Membranen teilnehmen können, wobei C₁₈-, C₂₀- oder C₂₂-Kohlenstoffketten im Fettsäuremolekül mit Doppelbindungen an mindestens zwei, vorteilhaft drei, vier, fünf oder sechs Stellen gemeint sind.

Alternativ können im erfindungsgemäßen Verfahren Nukleotidsequenzen verwendet werden, die für eine Phospholipase A2 kodieren und die an eine Nukleotidsequenz, wie in SEQ ID NO: 1 dargestellt, vorteilhaft unter stringenten Bedingungen hybridisieren.

Die im Verfahren verwendeten Nukleinsäuresequenzen werden vorteilhaft in einer Expressionskassette, die die Expression der Nukleinsäuren in Pflanzen ermöglicht, eingebracht.

Dabei werden die Nukleinsäuresequenzen, die für die Phospholipase A2 kodieren, mit einem oder mehreren Regulationssignalen vorteilhafterweise zur Erhöhung der Genexpression funktionell verknüpft. Diese regulatorischen Sequenzen sollen die gezielte Expression der Gene und der Proteine ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen, an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette (= Expressionskonstrukt = Genkonstrukt) kann aber auch einfacher aufgebaut sein, das heißt es wurden keine zusätzlichen Regulationssignale vor die Nukleinsäuresequenz oder dessen Derivate inseriert, und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen wie weitere regulatorische Elemente oder Terminatoren inseriert werden. Die Phospholipase A2- Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen im Wirtsorganismus exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Weiterhin beschrieben sind ein oder mehrere Genkonstrukte, die eine oder mehrere Sequenzen enthalten, die durch SEQ ID NO: 1 oder deren Derivate definiert sind und für Polypeptide bzw. Proteine gemäß SEQ ID NO: 2 kodieren. Die genannten Phospholipase A2- Proteine führen dabei vorteilhaft zu einer Spaltung der Esterbindung von Fettsäuren an der sn-2 Position von Phospholipiden bzw. zur Reduktion und Dehydrierung von Fettsäuren, wobei das Substrat vorteilhaft ein, zwei, drei, vier, fünf oder sechs Doppelbindungen aufweist und vorteilhaft 18, 20 oder 22 Kohlenstoffatome im Fettsäuremolekül aufweist. Gleiches gilt für ihre Homologe, Derivate oder Analoga, die funktionsfähig mit einem oder mehreren Regulationssignalen, vorteilhafterweise zur Steigerung der Genexpression, verbunden sind.

Vorteilhafte Regulationssequenzen für das neue Verfahren liegen beispielsweise in Promotoren vor, wie dem cos-, tan-, trp-, tet-, trp-tet-, lpp-, lac-, lpp-lao-, lacIq-, T7-, T5-, T3-, gal-, trc-, ara-, SP6-, λ-PR- oder λ-PL-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclin-induzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der ausführungsgemäße USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen: lpt-2- oder lpt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren die im Embryo und/oder im Endosperm aktiv sind. Samen-spezifische Promotoren können prinzipiell sowohl aus dikotolydonen als auch aus monokotolydonen Pflanzen isoliert werden. Im folgenden sind vorteilhafte bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und lpt1(Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der Biosynthesegene in die transgene Pflanze über mehrere Generation sicherzustellen, sollte jede der im Verfahren verwendeten Nukleinsäuren, die für die Phospholipase A2 kodieren, vorteilhaft in Kombination mit den Nukleinsäuren, die für die Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase und/oder Δ-4-Desaturase kodieren, unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu exprimierenden Nukleinsäure folgt, vorteilhaft in einem Polylinker, und anschließend gegebenenfalls ein Terminator hinter dem Polylinker liegt. Diese Abfolge wiederholt sich mehrfach, bevorzugt drei-, vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu dreimal. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator. Derartige vorteilhafte Konstrukte werden beispielsweise in DE 10102337 oder DE 10102338 offenbart. Es ist aber auch möglich, mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4 oder DC3-Promotor und unterschiedliche Terminatoren verwendet werden. Es ist aber auch möglich, nur einen Promotortyp in der Kassette zu verwenden. Dies kann jedoch zu unerwünschten Rekombinationsereignissen führen.

Wie oben beschrieben, sollte die Transkription der eingebrachte Gene vorteilhaft durch geeignete Terminatoren am 3'-Ende der eingebrachten Biosynthesegene (hinter dem Stopcodon) abgebrochen werden. Verwendet werden kann hier z.B. der OCS 1 Terminator. Wie auch für die Promotoren, so sollten hier für jedes Gen unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure- oder Lipidstoffwechsels enthalten sein oder aber diese Gene können auf einem weiteren oder mehreren weiteren Nukleinsäurekonstrukten liegen. Vorteilhaft werden als weitere Biosynthesegene des Fettsäure- oder Lipidstoffwechsels im Genkonstrukt ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) oder deren Kombinationen verwendet. Besonders vorteilhafte Nukleinsäuresequenzen sind Biosynthesegene des Fettsäure- oder Lipidstoffwechsels ausgewählt aus der Gruppe der Acyl-CoA:Lysophospholipid-Acyltransferase, ω-3-Desaturase, Δ-4-Desaturase, Δ-5-Desaturase, Δ-6-Desaturase, Δ-8-Desatuase, Δ-9-Desaturase, Δ-12-Desaturase, Δ-5-Elongase, Δ-6-Elongase und/oder Δ-9-Elongase.

Dabei können die vorgenannten Nukleinsäuren bzw. Gene in Kombination mit anderen Elongasen und Desaturasen in Expressionskassetten wie den vorgenannten kloniert werden und zur Transformation von Pflanzen mit Hilfe von Agrobakterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Die Expressionskassetten können prinzipiell direkt zum Einbringen in die Pflanze verwendet werden oder aber in einen Vektoren eingebracht werden.

Diese vorteilhaften Vektoren, vorzugsweise Expressionsvektoren, enthalten die im Verfahren verwendeten Nukleinsäuren, die für die Phospholipasen A2 kodieren und die vorteilhaft mit Nukleinsäuren kombiniert werden können, die für die Δ-12-Desaturasen, ω-3-Desaturasen, Δ-9-Elongasen, Δ-6-Desaturasen, Δ-8-Desaturasen, Δ-9-Desaturasen, Δ-6-Elongasen, Δ-5-Desaturasen, Δ-5-Elongasen oder Δ-4-Desaturasen kodieren, oder ein Nukleinsäurekonstrukt, das die verwendete Nukleinsäure allein oder in Kombination mit weiteren Biosynthesegenen des Fettsäure- oder Lipidstoffwechsels wie den Acyl-CoA:Lysophospholipid-Acyltransferasen, ω-3-Desaturasen, Δ-4-Desaturasen, Δ-5-Desaturasen, Δ-6-Desaturasen, Δ-8-Desatuasen, Δ-9-Desaturasen, Δ-12-Desaturasen, ω3-Desaturasen, Δ-5-Elongasen, Δ-6-Elongasen und/oder Δ-9-Elongasen enthält. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", das für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden beim Einbringen in die Wirtszelle vorteilhaft in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Der Begriff soll jedoch die anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren wie SV40, CMV oder TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die oben beschriebenen Nukleinsäuren oder das oben beschriebene Genkonstrukt in einer Form, die sich zur Expression der verwendeten Nukleinsäuren in einer Wirtszelle eignen, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfassen. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder in: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren wie der Auswahl der zu transformierenden Wirtszelle, dem Ausmaß der Expression des gewünschten Proteins usw. abhängen kann.

Die verwendeten rekombinanten Expressionsvektoren können zur Expression von Phospholipasen A2, Ketoacyl-CoA-Reduktasen, Dehydratasen, Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongase und/oder Δ-4-Desaturasen in prokaryotischen oder eukaryotischen Zellen gestaltet sein. Dies ist vorteilhaft, da häufig Zwischenschritte der Vektorkonstruktion der Einfachheit halber in Mikroorganismen durchgeführt werden. Beispielsweise können die Phospholipase A2-, Ketoacyl-CoA-Reduktase-, Dehydratase-, Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen sind in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989 beschrieben.

Bei einer weiteren Ausführungsform des Verfahrens können die Phospholipasen A2 vorteilhaft in Kombination mit den Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilizatiön, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion wie Termination der Transkription erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon. Auch alle anderen in Pflanzen funktionell aktiven Terminatoren sind geeignet.

Da die Pflanzengenexpression sehr oft nicht auf die Transkriptionsebene beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren wie der in US 4,962,028 beschriebene Promotor der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9), sowie Promotoren, welche die samenspezifische Expression in Monokotyledonen-Pflanzen wie Mais, Gerste, Weizen, Roggen, Reis usw. herbeiführen. Geeignete beachtenswerte Promotoren sind der lpt2- oder lptl-Gen-Promotor aus Gerste (WO 95/15389 und WO 95/23230) oder die in WO 99/16890 beschriebenen Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen.

Insbesondere kann die multiparallele Expression der im Verfahren verwendeten Phospholipasen A2 vorteilhaft in Kombination mit den Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Wirtszellen, die im Prinzip zum Aufnehmen der beschriebenen Nukleinsäure, des Genproduktes oder des Vektors geeignet sind, sind alle prokaryotischen oder eukaryotischen Organismen. Vorteilhaft verwendete Wirtsorganismen sind Mikroorganismen wie Pilze oder Hefen oder Pflanzenzellen, vorzugsweise Pflanzen oder Teile davon. Pilze, Hefen oder Pflanzen werden vorzugsweise verwendet, besonders bevorzugt Pflanzen, ganz besonders bevorzugt Pflanzen wie Ölfruchtpflanzen, die große Mengen an Lipidverbindungen enthalten, wie Raps, Nachtkerze, Hanf, Diestel, Erdnuss, Canola, Lein, Soja, Saflor, Sonnenblume, Borretsch, oder Pflanzen wie Mais, Weizen, Roggen, Hafer, Triticale, Reis, Gerste, Baumwolle, Maniok, Pfeffer, Tagetes, Solanaceen-Pflanzen wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) sowie ausdauernde Gräser und Futterfeldfrüchte. Besonders bevorzugte erfindungsgemäße Pflanzen sind Ölfruchtpflanzen wie Soja, Erdnuss, Raps, Canola, Lein, Hanf, Nachtkerze, Sonnenblume, Saflor, Bäume (Ölpalme, Kokosnuss).

Weiterhin offenbart sind wie oben beschrieben isolierte Nukleinsäuresequenzen, die für Polypeptide bzw. Proteine mit Phospholipase A2-Aktivität kodieren, wobei die durch die Nukleinsäuresequenzen kodierten Phospholipasen A2 vorteilhaft an der sn2-Position der Phospholipide gebundene Fettsäuren abhydrolisieren.

Bevorzugte Nukleinsäuresequenzen, die für Polypeptide bzw. Proteine mit Phospholipase A2-Aktivität kodieren, sind Sequenzen ausgewählt aus der Gruppe:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 2 dargestellten Aminosäuresequenz ableiten lassen, oder
c) Derivate der in SEQ ID NO: 1 dargestellten Nukleinsäuresequenz, die für Polypeptide bzw. Proteine kodieren, die mindestens 40 % Homologie auf Aminosäureebene mit SEQ ID NO: 2 haben und eine Phospholipase A2-Aktivität aufweisen.

Weitere nicht zur Erfindung gehörende Gegenstände sind die im folgenden aufgezählten Nukleinsäuresequenzen, die für Ketoacyl-CoA-Reduktasen oder Dehydratasen kodieren.

Weitere nicht zur Erfindung gehörende isolierte Nukleinsäuresequenzen sind Sequenzen, die für Polypeptide bzw. Proteine mit Ketoacyl-CoA-Reduktase-Aktivität kodieren, ausgewählt aus der Gruppe:
a) einer Nukleinsäuresequenz mit der in SEQ ID NO: 3 dargestellten Sequenz,
b) Nukleinsäuresequenzen, die sich als Ergebnis des degenerierten genetischen Codes von der in SEQ ID NO: 4 dargestellten Aminosäuresequenz ableiten lassen, oder
c) Derivate der in SEQ ID NO: 3 dargestellten Nukleinsäuresequenz, die für Polypeptide bzw. Proteine kodieren, die mindestens 40 % Homologe auf Aminosäureebene mit SEQ ID NO: 4 haben und eine Ketoacyl-CoA-Reduktase-Aktivität aufweisen.

Weitere nicht zur Erfindung gehörende isolierte Nukleinsäuresequenzen sind Sequenzen, die für Polypeptide bzw. Proteine mit Dehydratase-Aktivität kodieren, ausgewählt aus der Gruppe:
a) einer Nukleinsäuresequenz mit der in SEQ ID NO: 5 oder SEQ ID NO: 7 dargestellten Sequenz,
b) Nukleinsäuresequenzen, die sich als Ergebnis des degenerierten genetischen Codes von der in SEQ ID NO: 6 oder SEQ ID NO: 8 dargestellten Aminosäuresequenzen ableiten lassen, oder
c) Derivate der in SEQ ID NO: 5 oder SEQ ID NO: 7 dargestellten Nukleinsäuresequenzen, die für Polypeptide bzw. Proteine kodieren, die mindestens 40 % Identität auf Aminosäureebene mit SEQ ID NO: 6 oder SEQ ID NO: 8 haben und eine Dehydratase-Aktivität aufweisen.

Die oben genannten Nukleinsäuren stammen vorteilhaft von vorgenannten Organismen.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Phospholipase A2- molekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt, flankieren.

Die im Verfahren verwendeten Nukleinsäuremoleküle, z.B. ein Nukleinsäuremolekül mit einer Nukleotidsequenz der SEQ ID NO: 1 oder eines Teils davon, kann unter Verwendung molekularbiologischer Standardtechniken und der hier bereitgestellten Sequenzinformation isoliert werden. Auch kann mit Hilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lässt sich ein Nukleinsäuremolekül, umfassend eine vollständige Sequenz der SEQ ID NO: 1, SEQ ID NO: 3, SEQ ID NO: 5 oder SEQ ID NO: 7 oder einen Teil davon, durch Polymerasekettenreaktion isolieren, wobei Oligonukleotidprimer, die auf der Basis dieser Sequenz oder von Teilen davon verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, SEQ ID NO: 3, SEQ ID NO: 5 oder SEQ ID NO: 7 gezeigten Sequenzen oder mit Hilfe der in SEQ ID NO: 2, SEQ ID NO: 4, SEQ ID NO: 6 oder SEQ ID NO: 8 dargestellten Aminosäuresequenzen erstellen. Eine Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Homologe der verwendeten Phospholipase A2- Nukleinsäuresequenzen mit der Sequenz SEQ ID NO: 1 bedeuten beispielsweise allelische Varianten mit mindestens etwa 30, 35, 40, 45, 50, 55 oder 60 %, vorzugsweise mindestens etwa 60, 65 oder 70 %, stärker bevorzugt mindestens etwa 70 oder 80 %, 90 % oder 95 % und noch stärker bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr Identität bzw. Homologie zu der in SEQ ID NO: 1 gezeigten Nukleotidsequenz oder ihren Homologen, Derivaten oder Analoga oder Teilen davon. Weiterhin sind isolierte Nukleinsäuremoleküle einer Nukleotidsequenz, die an der in SEQ ID NO: 1 gezeigten Nukleotidsequenz oder einen Teil davon hybridisieren, z.B. unter stringenten Bedingungen hybridisiert. Unter einem Teil gemäß der Erfindung ist dabei zu verstehen, dass mindestens 25 Basenpaare (= bp), 50 bp, 75 bp, 100 bp, 125 bp oder 150 bp, bevorzugt mindestens 175 bp, 200 bp, 225 bp, 250 bp, 275 bp oder 300 bp, besonders bevorzugt 350 bp, 400 bp, 450 bp, 500 bp oder mehr Basenpaare für die Hybridisierung verwendet werden. Es kann auch vorteilhaft die Gesamtsequenz verwendet werden. Allelische Varianten umfassen insbesondere funktionelle Varianten, die sich durch Deletion, Insertion oder Substitution von Nukleotiden aus/in der in SEQ ID NO: 1 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der Phospholipase A2 besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 1 kodierten Protein. Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)] verwendet, die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 8, Length Weight: 2, Average Match: 2.778 und Average Mismatch: -2.248. Diese wurden, falls nicht anders angegeben, als Standardeinstellungen immer für Sequenzvergleiche verwendet.

Beschrieben sind zudem Nukleinsäuremoleküle, die sich von der in SEQ ID NO: 1 gezeigten Nukleotidsequenz (und Teilen davon) aufgrund des degenerierten genetischen Codes unterscheidet und somit die gleiche Phospholipase A2 kodiert wie diejenige, die von den in SEQ ID NO: 1 gezeigten Nukleotidsequenz kodiert wird.

Zusätzlich zu der in SEQ ID NO: 1 gezeigten Phospholipasen A2 erkennt der Fachmann, dass DNA-Sequenzpolymorphismen, die zu Änderungen in den Aminosäuresequenzen der Phospholipase A2 führen, innerhalb einer Population existieren können. Diese genetischen Polymorphismen im Phospholipase A2- Gen können zwischen Individuen innerhalb einer Population aufgrund von natürlicher Variation existieren. Diese natürlichen Varianten bewirken üblicherweise eine Varianz von 1 bis 5 % in der Nukleotidsequenz des Phospholipase A2- Gens. Alle diese Nukleotidvariationen und die daraus resultierenden Aminosäurepolymorphismen in der Phospholipase A2 die das Ergebnis natürlicher Variation sind und die funktionelle Aktivität der Enzyme nicht verändern, sind im erfindungsgemäßen Verfahren verwendbar.

Für das erfindungsgemäße Verfahren vorteilhafte Nukleinsäuremoleküle können auf der Grundlage ihrer Homologie zu den hier offenbarten Phospholipase A2- Nukleinsäuren unter Verwendung der Sequenzen oder eines Teils davon als Hybridisienmgssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und unter stringenten Bedingungen mit den Nukleinsäuremolekülen, die die Nukleotidsequenz der SEQ ID NO: 1 umfassen, hybridisieren. Es können auch Nukleinsäuren mit mindestens 25, 50,100, 250 oder mehr Nukleotiden verwendet werden. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6. finden. Ein bevorzugtes, nicht einschränkendes Beispiel für stringente Hybridisierungsbedingungen sind Hybridisierungen in 6 x Natriumohlorid/Natriumoitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im oben genannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp Länge und einem G+C-Gehalt von 50 % in Abwesenheit von Formamid bestimmt. Der Fachmann weiß, wie die erforderlichen Hybridisierungsbedingungen anhand von Lehrbüchern wie dem vorstehend erwähnten oder aus den folgenden Lehrbüchern: Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford bestimmt werden können.

Zur Bestimmung der prozentualen Homologie von zwei Aminosäuresequenzen (z.B. einer der Sequenzen der SEQ ID NO: 2, SEQ ID NO: 4, SEQ ID NO: 6 oder SEQ ID NO: 8) oder von zwei Nukleinsäuresequenzen (z.B. SEQ ID NO: 1, SEQ ID NO: 3, SEQ ID NO: 5 oder SEQ ID NO: 7) werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die Begriffe Homologie und Identität sind damit als Synonym anzusehen. Die verwendeten Programme bzw. Algorithmen sind oben beschrieben.

Ein isoliertes Nukleinsäuremolekül, das für eine Phospholipase A2, Ketoacyl-CoA-Reduktase oder Dehydratase kodiert, ausgewählt aus der Gruppe SEQ ID NO: 1, SEQ ID NO: 3, SEQ ID NO: 5 oder SEQ ID NO: 7, die zu einer Proteinsequenz der SEQ ID NO: 2, SEQ ID NO: 4, SEQ ID NO: 6 oder SEQ ID NO: 8 homolog ist, kann durch Einbringen einer oder mehrerer Nukleotidsubstitutionen, -additionen oder -deletionen in eine Nukleotidsequenz der SEQ ID NO: 1, SEQ ID NO: 3, SEQ ID NO: 5 oder SEQ ID NO: 7 erzeugt werden, so dass eine oder mehrere Aminosäuresubstitutionen, -additionen oder -deletionen in das kodierte Protein eingebracht werden. Mutationen können in eine der Sequenzen der SEQ ID NO: 1, SEQ ID NO: 3, SEQ ID NO: 5 oder SEQ ID NO: 7 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nicht-essentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer Phospholipase A2, Ketoacyl-CoA-Reduktase oder Dehydratase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der Phospholipase A2, Ketoacyl-CoA-Reduktase oder Dehydratase kodierenden Sequenz eingebracht werden, z.B. durch Sättigungsmutagenese, und die resultierenden Mutanten können nach der hier beschriebenen Phospholipase A2-, Ketoacyl-CoA-Reduktase- oder Dehydratase-Aktivität durchmustert werden, um Mutanten zu identifizieren, die die Phospholipase A2-, Ketoacyl-CoA-Reduktase- oder Dehydratase-Aktivität beibehalten haben. Nach der Mutagenese einer der Sequenzen SEQ ID NO: 1, SEQ ID NO: 3, SEQ ID NO: 5 oder SEQ ID NO: 7 kann das kodierte Protein rekombinant exprimiert werden, und die Aktivität des Proteins kann z.B. unter Verwendung der hier beschriebenen Tests bestimmt werden.

Homologe der SEQ ID NO: 1, SEQ ID NO: 3, SEQ ID NO: 5 oder SEQ ID NO: 7 bedeuten beispielsweise auch bakterielle, Pilz- und Pflanzenhomologe, verkürzte Sequenzen, einzelsträngige DNA oder RNA der kodierenden und nicht-kodierenden DNA-Sequenz.

Homologe der SEQ ID NO: 1, SEQ ID NO: 3, SEQ ID NO: 5 oder SEQ ID NO: 7 bedeutet auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die vorgenannten Nukleinsäuren und Proteinmoleküle mit Phospholipase A2- vorteilhaft in Kombination mit den Nukleinsäuren und Proteinmolekülen mit Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Aktivität, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Organismen, vorteilhaft in Pflanzen wie Mais, Weizen, Roggen, Hafer, Triticale, Reis, Gerste, Sojabohne, Erdnuss, Baumwolle, Linum-Arten wie Öl- oder Faserlein, Brassica-Arten wie Raps, Canola und Rübsen, Pfeffer, Sonnenblume, Borretsch, Nachtkerze und Tagetes, Solanacaen-Pflanzen wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekte Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat, da mehrfach ungesättigte Fettsäuren (= PUFAs) nicht nur einfach in Triacylglycerin, sondern auch in

Membranlipide eingebaut werden.

Besonders zur Herstellung von PUFAs, beispielsweise Stearidonsäure , Eicosapentaensäure und Docosahexaensäure, eignen sich Brasicaceae, Boraginaceen, Primulaceen oder Linaceen. Besonders vorteilhaft eignet sich Lein (Linum usitatissimum) zur Herstellung von PUFAS mit den offenbarten Nukleinsäuresequenzen, wie beschrieben, in Kombination mit weiteren Desaturasen und Elongasen.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E.coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten, an Phospholipide gebundenen Fettsäuren müssen anschließend wieder für die weitere Elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estem auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese C18-Kohlenstoff-Fettsäuren müssen auf C₂₀ und C₂₂ verlängert werden, damit Fettsäuren vom Eicosa- und Docosa-Kettentyp erhalten werden. Mit Hilfe der im Verfahren verwendeten Phospholipasen A2-, Ketoacyl-CoA-Reduktasen oder Dehydratasen in Kombination mit weiteren Enzymen wie Desaturasen wie der Δ-12-, ω3-, Δ-4-, Δ-5-, Δ-6- und Δ-8-Desaturasen und/oder Elongasen wie der Δ-5-, Δ-6-, Δ-9-Elongasen können Arachidonsäure, Eicosapentaensäure, Docosapentaensäure oder Docosahexaensäure, vorteilhaft Eicosapentaensäure und/oder Docosahexaensäure, hergestellt werden und anschließend für verschiedene Zwecke bei Nahrungsmittel-, Futter-, Kosmetik- oder pharmazeutischen Anwendungen verwendet werden. Mit den genannten Enzymen können Öle oder Lipide mit einem hohen Anteil an C₁₈-, C₂₀- und/oder C₂₂-Fettsäuren mit mindestens zwei, vorteilhaft mindestens drei, vier, fünf oder sechs Doppelbindungen im Fettsäuremolekül, vorzugsweise C₂₀- oder C₂₂-Fettsäuren mit vorteilhaft vier, fünf oder sechs Doppelbindungen im Fettsäuremolekül hergestellt werden. Vorteilhaft lassen sich im Verfahren Fettsäuren wie Linolsäure, γ-Linolensäure, Dihomo-γlinolensäure, Arachidonsäure, Stearidonsäure, Eicosatetraensäure oder Eicosapentaensäure, Docosapentaensäure, Docosatetraensäure, Docosapentaensäure, Docosahexaensäure oder deren Mischungen herstellen. Substrate der verwendeten Enzyme im erfindungsgemäßen Verfahren sind C₁₆-, C₁₈- oder C₂₀-Fettsäuren wie zum Beispiel Linolsäure, γ-Linolensäure, α-Linolensäure, Dihomo-γ-linolensäure, Eicosatetraensäure oder Stearidonsäure. Bevorzugte Substrate sind Linolsäure, γ-Linolensäure und/oder α-Linolensäure, Dihomo-γ-linolensäure bzw. Arachidonsäure, Eicosatetraensäure oder Eicosapentaensäure. Die synthetisierten vorteilhaften C₂₀- oder C₂₂-Fettsäuren mit mindestens zwei, drei, vier, fünf oder sechs Doppelbindungen in der Fettsäure fallen im erfindungsgemäßen Verfahren in Form der freien Fettsäure oder in Form ihrer Ester, beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt. Diese Glyceride kommen letztlich in den Ölen oder Lipiden als eine Substanzgruppe vor.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, die Desaturierung, den Lipidstoffwechsel und den Membrantransport von fetthaltigen Verbindungen, die Betaoxidation, Fettsäuremodifikation und Cofaktoren, Triacylglycerin-Speicherung und -Assemblierung siehe in den folgenden Artikeln einschließlich der Literaturstellen darin: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs umfassen eine Gruppe von Molekülen, die höhere Tiere nicht mehr synthetisieren können und somit aufnehmen müssen, oder die höhere Tiere nicht mehr ausreichend selbst herstellen können und somit zusätzlich aufnehmen müssen, obwohl sie leicht von anderen Organismen, wie Bakterien, synthetisiert werden. Beispielsweise können Katzen Arachidonsäure nicht mehr synthetisieren.

Unter Phospholipiden im Sinne der Erfindung sind zu verstehen: Phosphatidylcholin, Phosphatidylethanolamin, Phosphatidylserin, Phosphatidylglycerin und/oder Phosphatidylinositol, vorteilhafterweise Phosphatidylcholin. Die Begriffe "Produktion" oder "Produktivität" sind im Fachgebiet bekannt und beinhalten die Konzentration des Fermentationsproduktes, das in einer bestimmten Zeitspanne und einem bestimmten Fermentationsvolumen gebildet wird (z.B. kg Produkt pro Stunde pro Liter). Es umfasst auch die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Der Begriff "Effizienz der Produktion" umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff "Ausbeute" oder "Produkt/Kohlenstoff-Ausbeute" ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe "Biosynthese" oder "Biosyntheseweg" sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe "Abbau" oder "Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff "Stoffwechsel" ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Weiterhin beschrieben sind transgene Pflanzen oder Pflanzenzellen, die die Nukleinsäure SEQ ID NO: 1 enthalten oder ein Genkonstrukt oder einen Vektor enthalten, die diese erfindungsgemäß Nukleinsäuresequenz enthalten.

Diese Erfindung wird durch die nachstehenden Beispiele weiter veranschaulicht, die nicht als beschränkend aufgefasst werden sollten.

### Beispiele

### Beispiel 1: Allgemeine Klonierungsverfahren

Die Klonierungsverfahren wie z.B. Restriktionsspaltungen, Agarose-Gelelektrophorese, Reinigung von DNA-Fragmenten, Transfer von Nukleinsäuren auf Nitrozellulose- und Nylon-Membranen, Verknüpfen von DNA-Fragmenten, Transformation von Escherichia coli-Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase-Kettenreaktion wurden zur Vermeidung von Polymerasefehlern in zu exprimierenden Konstrukten sequenziert und überprüft.

### Beispiel 3: Klonierung von Genen aus Ostreococcus tauri

Durch Suche von homologen Bereichen in Proteinsequenzen konnten Sequenzen mit entsprechenden Motiven in einer Ostreococcus tauri Sequenzdatenbank (genomische Sequenzen) identifiziert werden. Die Alignments zum Auffinden von Homologien der einzelnen Gene wurden mit dem tBLASTn-Algorithmus (Altschul et al., J. Mol. Biol. 1990, 215: 403 - 410) durchgeführt. Es handelt sich dabei um die folgenden Sequenzen:

| Gen-Name | SEQ ID | Aminosäuren |
|---|---|---|
| PLA2(Ot) | SEQ ID NO: 1 | 930 |
| KR(Ot) | SEQ ID NO: 3 | 327 |
| DH(Ot) | SEQ ID NO: 5 | 362 |

Die Klonierung wird wie folgt durchgeführt:
40 ml einer *Ostreococcus tauri* Kultur in der stationären Phase werden abzentrifugiert, in 100 µl Aqua bidest resuspendiert und bei -20°C gelagert. Auf der Basis des PCR-Verfahrens werden die zugehörigen genomischen DNAs amplifiziert. Die entsprechenden Primerpaare werden so ausgewählt, dass sie die Hefe-Konsensus-Sequenz für hocheffiziente Translation (Kozak, Cell 1986, 44:283-292) neben dem Startcodon tragen. Die Amplifizierung der Ot-DNAs wird jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR sind wie folgt: erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

### Beispiel 4: Klonierung eines Dehydratase-Genes aus Thraustochytrium ssp.

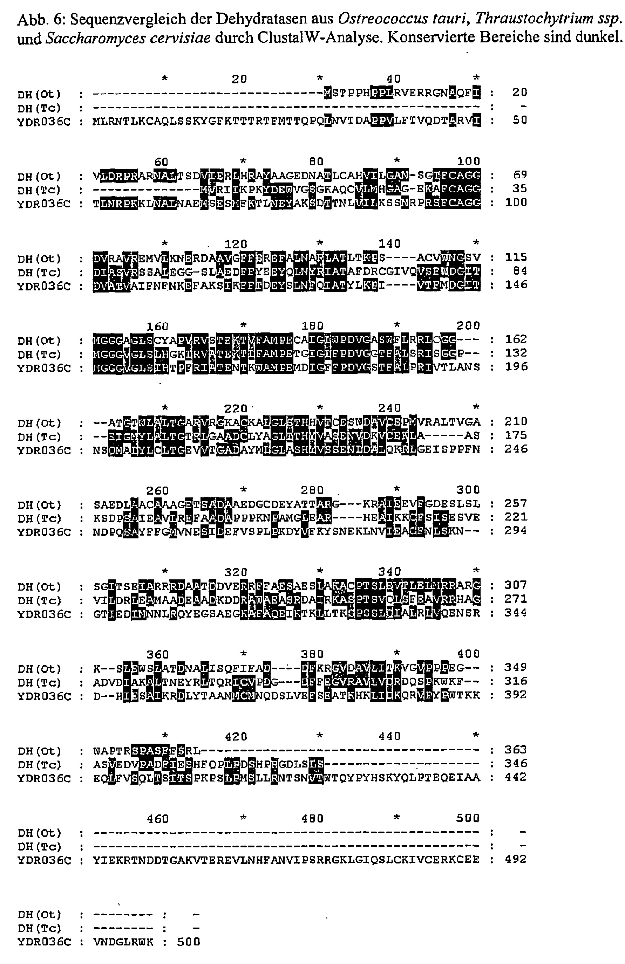

Durch Vergleiche der verschiedenen in dieser Anmeldung gefundenen Dehydratase-Proteinsequenzen konnten konservierte Nukleinsäurebereiche definiert werden (Figur 6: Phe-Cys-Ala-Gly-Gly-Asp, Phe-Phe-X-X-Glu-Phe-X-Leu-Asn, Thr-X-Phe-Ala-Met-Pro-Glu, Pro-Asp-Valin-Gly-X-Thr/Ser-Phe/Trp). Mit Hilfe dieser Sequenzen wurde eine EST-Datenbank von Thraustochytrium ssp. nach Dehydratasen durchsucht.

| Gen-Name | cDNA | Codierende Sequenz | Aminosäuren | SEQ ID |
|---|---|---|---|---|
| DH(Tc) | 1171 bp | 1041 bp | 346 | SEQ ID NO: 7 |

Gesamt-RNA von Thraustochytrium ssp. wurde mit Hilfe des RNAeasy Kits der Firma Qiagen (Valencia, CA, US) isoliert. Aus der Gesamt-RNA wurde mit Hilfe des PolyATract Isolierungssystems (Promega) mRNA isoliert. Die mRNA wurde mit dem Marathon cDNA Amplification-Kit (BD Biosciences) revers transkribiert und entsprechend der Herstellerangaben Adaptoren ligiert. Die cDNA-Bank wurde dann für die PCR zur Klonierung von Expressionsplasmiden mittels 5'- und 3'-RACE (rapid amplification of cDNA ends) verwendet.

### Beispiel 5: Klonierung von Expressionsplasmiden zur heterologen Expression in Hefen:

Zur Charakterisierung der Funktion der identifizierten Gene aus *Ostreococcus tauri* und Thraustochytrium werden die offenen Leserahmen der jeweiligen DNAs stromabwärts des Galactose-induzierbaren GAL1-Promotors von pYES2.1/V5-His-TOPO (Invitrogen) kloniert, wobei pYES2-PLA2(Ot), pYES2-KR(Ot),pYES2-DH(Ot) und pYES2(DH(Tc) erhalten werden. Folgende Primersequenzen werden eingesetzt:

| Gen | Primersequenz | SEQ ID NO: |
|---|---|---|
| PLA2(Ot) | Forward: caccatgggcgtgtgttcctc | SEQ ID NO: 9 |
| | Reverse: tcacgtgtatggttgccagttg | SEQ ID NO: 10 |
| KR(Ot) | Forward: caccatgggcgccctgagctatc | SEQ ID NO: 11 |
| | Reverse: ttacacgttcttcttgtaat | SEQ ID NO: 12 |
| DH(Ot) | Forward: caccatgtccaccccaccccatccac | SEQ ID NO: 13 |
| | Reverse: ttacaagcgagagaagaagg | SEQ ID NO: 14 |
| DH(Tc) | Forward: caccatggtgcgcatcatcaagcc | SEQ ID NO: 15 |
| | Reverse: ctaggagaggctgagatcg | SEQ ID NO: 16 |

Der *Saccharomyces cerevisiae-Stamm* 334 wird durch Elektroporation (1500 V) mit den Vektoren pYES2-PLA2(Ot), pYES2-KR(Ot), pYES2-DH(Ot) und pYES2-DH(Tc) transformiert. Als Kontrolle wird eine Hefe verwendet, die mit dem leeren Vektor pYES2 transformiert wird. Die Selektion der transformierten Hefen erfolgt aufKomplett-Mininalmedium (CMdum)-Agarplatten mit 2% Glucose, aber ohne Uracil. Nach der Selektion werden je drei Transformanten zur weiteren funktionellen Expression ausgewählt.

Für die Expresssion der Ot-Gene sowie des DH(Tc) Gens werden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.
5 ml CMdum-Flüssigmedium (ohne Uracil) mit 2% Raffinose und 300 µM verschiedener Fettsäuren werden dann mit den Vorkulturen auf eine OD₆₀₀ von 0,05 angeimpft. Die Expression wird durch die Zugabe von 2% (w/v) Galactose induziert. Die Kulturen werden für weitere 96 h bei 20°C inkubiert. Zur Charakterisierung der Gene kann nach folgenden beschriebenen Verfahren vorgegangen werden:
PLA2(Ot): Lee et al., 2003, Mol. Cells, 16:361-367
KR(Ot): Beaudoin et al. 2001, JBC, 277:11481-11488
DH(Ot) und DH(Tc): Garcia et al. 2004, The Acyl-CoA elongase in Arabidopsis thaliana : characterization of a candidate gene presumably encoding the 3-hydroxyacyl-CoA dehydratase. Posterpräsentation 16^{th} Plant Lipid Symposium, Budapest.

### Beispiel 6: Klonierung von Expressionsplasmiden zur Samen-spezifischen Expression in Pflanzen

Für die Transformation von Pflanzen wird ein weiterer Transformationsvektor auf Basis des binäre Plasmids pSUN-USP erzeugt. Dazu werden mittels PCR NotI-Schnittstellen am 5' und 3'-Ende der kodierenden Sequenzen eingefügt. Die entsprechenden Primersequenzen werden von den 5'- und 3'-Bereichen von PLA2(Ot), KR(Ot), DH(Ot) und DH(Tc) abgeleitet.

Zusammensetzung des PCR-Ansatzes (50 µL):
5,00 µL Template cDNA
5,00 µL 10x Puffer (Advantage-Polymerase)+ 25mM MgCl₂
5,00 µL 2mM dNTP
1,25 µL je Primer (10 pmol/µL)
0,50 µL Advantage-Polymerase (Clontech)

Reaktionsbedingungen der PCR:
Anlagerung: 1 min 55°C
Denaturierung: 1 min 94°C
Elongation: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicher Weise inkubiert. Anschließend werden die PCR Produkte sowie der Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschliessend werden Vektor und PCR-Produkte ligiert. Dazu wird das Rapid Ligation Kit von Roche verwendet. Die entstandenen Plasmide pSUN-PLA2(Ot), pSUN-KR(Ot), pSUN-DH(Ot) und pSUN-DH(Tc) werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopin-Synthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 17). Das PCR-Fragment wurde mit EcoRI/SalI nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.

### Beispiel 7: Expression von PLA2(Ot), KR(Ot),DH(Ot) und DH(Tc) in Hefen

Hefen, die wie unter Beispiel 5 mit den Plasmiden pYES2, pYES2-PLA2(Ot), pYES2-KR(Ot), pYES2-DH(Ot) und pYES2-DH(Tc) transformiert werden, werden folgendermaßen analysiert:

Die Hefezellen aus den Hauptkulturen werden durch Zentrifugation (100 x g, 5 min, 20°C) geerntet und mit 100 mM NaHCO₃, pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgt durch zweimalige Extraktion mit Petrolether (PE). Zur Entfernung nicht derivatisierter Fettsäuren werden die organischen Phasen je einmal mit 2 ml 100 mM NaHCO₃, pH 8,0 und 2 ml Aqua dest. gewaschen. Anschließend werden die PE-Phasen mit Na₂SO₄ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse sind wie folgt: Die Ofentemperatur wird von 50°C bis 250°C mit einer Rate von 5°C/min und schließlich 10 min bei 250°C(halten) programmiert.

Die Identifikation der Signale erfolgt durch Vergleiche der Retentionszeiten mit entsprechenden Fettsäurestandards (Sigma). Die Methodik ist beschrieben zum

Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Beispiel 8: Erzeugung von transgenen Pflanzen

### a) Erzeugung transgener Rapspflanzen (verändert nach Moloney et al., 1992, Plant Cell Reports, 8:238-242)

Zur Erzeugung transgener Rapspflanzen werden die binären Vektoren in Agrobacterium tumefaciens C58C1:pGV2260 oder Escherichia coli genutzt (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland) wird eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 cm²) werden dazu in einer Petrischale mit einer 1:50 Agrobakterienverdünnung für 5-10 Minuten inkubiert. Es folgt eine 3-tägige Koinkubation in Dunkelheit bei 25°C auf 3MS-Medium mit 0,8 % Bacto-Agar. Die Kultivierung erfolgt dann 3 Tage mit 16 Stunden Licht / 8 Stunden Dunkelheit. In wöchentlichem Rhythmus auf MS-Medium mit 500 mg/l Claforan (Cefotaxime-Natrium), 50 mg/l Kanamycin, 20 mikroM Benzylaminopurin (BAP) wird dann mit 1,6 g/l Glukose weiterinkubiert. Wachsende Sprosse werden auf MS-Medium mit 2 % Saccharose, 250 mg/l Claforan und 0,8 % Bacto-Agar überführt, Bildeten sich nach drei Wochen keine Wurzeln, so wird als Wachstumshormon 2-Indolbuttersäure zum Bewurzeln zum Medium gegeben.

Regenerierte Sprossen werden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Elongase-Expression wie Δ-5-Elongase- oder Δ-6-Elongase-Aktivität mittels Lipidanalysen untersucht. Linien mit erhöhten Gehalten an C20- und C22 mehrfach ungesättigten Fettsäuren können so identifiziert werden.

### b) Herstellung von transgenen Leinpflanzen

Die Herstellung von transgenen Leinpflanzen kann zum Beispiel nach der Methode von Bell et al., 1999, In Vitro Cell. Dev. Biol.-Plant. 35(6):456-465 mittels particle bombartment erfolgen. Agrobakterien-vermittelte Transformationen können zum Beispiel nach Mlynarova et al. (1994), Plant Cell Report 13: 282-285 hergestellt werden.

### Beispiel 9: Lipidextraktion aus Hefen und Samen

Die Auswirkung der genetischen Modifikation in Pflanzen, Pilzen, Algen oder Ciliaten auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145 beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan 1 h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

## Patentansprüche

1. Verfahren zur Herstellung von Ölen oder Lipiden mit einem hohen Anteil an mehrfach ungesättigten Fettsäuren enthaltend mindestens zwei Doppelbindungen in transgenen Pflanzen oder transgenen Pflanzenzellen,
**dadurch gekennzeichnet, dass** es folgende Verfahrensschritte umfasst:
a) Einbringen mindestens einer für eine Phospholipase A2-Aktivität kodierenden rekombinanten Nukleinsäuresequenz in einem Expressionskonstrukt in die Pflanzen oder Pflanzenzellen, und
b) Kultivieren und Ernten der transgenen Pflanze oder Pflanzenzellen.

2. Verfahren nach Anspruch 1,
**dadurch gekennzeichnet, dass** das Öl oder Lipid aus der transgenen Pflanze oder den transgenen Pflanzenzellen isoliert wird.

3. Verfahren nach einem der Ansprüche 1 oder 2,
**dadurch gekennzeichnet, dass** die Nukleinsäuresequenzen, die für Polypeptide bzw. Proteine mit Phospholipase A2-Aktivität kodieren, ausgewählt sind aus der Gruppe bestehend aus:
a) einer Nukleinsäuresequenz mit der in SEQ ID NO: 1 dargestellten Sequenz, oder
b) Nukleinsäuresequenzen, die sich als Ergebnis des degenerierten genetischen Codes von der in SEQ ID NO: 2 dargestellten Aminosäuresequenzen ableiten lassen, oder
c) Derivate der in SEQ ID NO: 1 dargestellten Nukleinsäuresequenz, die für Polypeptide bzw. Proteine mit mindestens 40 % Identität auf Aminosäureebene mit SEQ ID NO: 2 kodieren und eine Phospholipase A2-Aktivität aufweisen.

4. Verfahren nach einem der Ansprüche 1 bis 3,
**dadurch gekennzeichnet, dass** zusätzlich in die Pflanze oder die Pflanzenzellen eine Nukleinsäuresequenz eingebracht wird, die für Polypeptide bzw. Proteine mit Δ-12-Desaturase-, A-9-Elongase-, Δ-8-Desaturase-, Δ-6-Desaturase-, Δ-6-Elongase-, Δ-5-Desaturase-, Δ-5-Elongase-, ω-3-Desaturase-, Δ-4-Desaturase-Aktivität kodiert.

5. Verfahren nach einem der Ansprüche 1 bis 4,
**dadurch gekennzeichnet, dass** die Öle oder Lipide einen hohen Anteil an einer ungesättigten Fettsäure ausgewählt aus der Gruppe der Linolsäure, γ-Linolensäure, Stearidonsäure, Dihomo-γ-Linolensäure, ω-3-Eicosatetiaensäure, Arachidonsäure, Eicosapentaensäure, ω-6-Docosapentaensäure, ω-6-Docosatetraensäure, ω-3-Docosapentaensäure und Docosahexaensäure enthalten.

6. Verfahren nach einem der Ansprüche 1 bis 5,
**dadurch gekennzeichnet, dass** die ungesättigte Fettsäure aus den Ölen oder Lipiden in Form ihrer freien Fettsäure isoliert wird.

7. Verfahren nach einem der Ansprüche 1 bis 6,
**dadurch gekennzeichnet, dass** die transgene Pflanze eine Öl-produzierende Pflanze, eine Gemüsepflanze oder Zierpflanze ist.

8. Verfahren nach einem der Ansprüche 1 bis 7,
**dadurch gekennzeichnet, dass** die transgene Pflanze ausgewählt ist aus der Gruppe der Pflanzenklassen bzw. -familien: Adelotheciaceae, Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Crypthecodiniaceae, Cucurbitaceae, Ditrichaceae, Elaeagnaceae, Ericaceae, Euphorbiaceae, Fabaceae, Geraniaceae, Gramineae, Juglandaceae, Lauraceae, Leguminosae, Linaceae oder Prasinophyceae.

## Claims

1. Method for producing oils or lipids having a high content of unsaturated fatty acids containing at least two double bonds in transgenic plants or transgenic plant cells,
**characterized in that** it comprises the following steps:
a) introducing at least one recombinant nucleic acid sequence in an expression construct coding for a phospholipase A2 activity into the plants or plant cells, and
b) cultivating and harvesting the transgenic plant or plant cells.

2. Method according to claim 1,
**characterized in that** the oil or lipid is isolated from the transgenic plant or the transgenic plant cells.

3. Method according to claim 1 or 2,
**characterized in that** the nucleic acid sequences coding for polypeptides or proteins having phospholipase A1 activity are selected from the group consisting of:
a) a nucleic acid sequence having the sequence depicted in SEQ ID NO: 1, or
b) nucleic acid sequences that can be derived due to the degenerate genetic code from the amino acid sequences depicted in SEQ ID NO:2, or
c) derivatives of the nucleic acid sequence depicted in SEQ ID NO: 1 which code for polypeptides or proteins having at least 40% identity on the amino acid level to SEQ ID NO: 2 and exhibiting a phospholipase A1 activity.

4. Method according to any of claims 1 to 3,
**characterized in that** a nucleic acid sequence coding for polypeptides or proteins having Δ-12-desaturase, Δ-9-elongase, Δ-8-desaturase, Δ-6-desaturase, Δ-6-elongase, Δ-5-desaturase, Δ-5-elongase, ω-3-desaturase or Δ-4-desaturase activity is additionally introduced into the plant or the plant cells.

5. Method according to any of claims 1 to 4,
**characterized in that** the oils or lipids contain a high content of an unsaturated fatty acid selected from the group consisting of linoleic acid, γ-linoleic acid, stearidonic acid, dihomo-γ-linoleic acid, ω-3-eicosatetraenoic acid, arachidonic acid, eicosapentaenoic acid, ω-6-docosapentaenoic acid, ω-6-docosatetraenoic acid, ω-3-docosapentaenoic acids and docosahexaenoic acid.

6. Method according to any of claims 1 to 5,
**characterized in that** the unsaturated fatty acid is isolated from the oils or lipids in form of its free fatty acid.

7. Method according to any of claims 1 to 6,
**characterized in that** the transgenic plant is an oil-producing plant, a vegetable plant or an ornamental plant.

8. Method according to any of claims 1 to 7,
**characterized in that** the transgenic plant is selected from the group of plant classes or families: Adelotheciaceae, Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Crypthecodiniaceae, Cucurbitaceae, Ditrichaceae, Elaeagnaceae, Ericaceae, Euphorbiaceae, Fabaceae, Geraniaceae, Gramineae, Juglandaceae, Lauraceae, Leguminosae, Linaceae or Prasinophyceae.

## Revendications

1. Procédé pour la fabrication, dans des plantes transgéniques ou dans des cellules végétales transgéniques, des huiles ou des lipides ayant une proportion forte d'acides gras polyinsaturés contenant au moins deux liaisons doubles,
**caractérisé par le fait qu**'il comprend les étapes suivantes :
a) introduire dans la plante ou la cellule végétale au moins une séquence d'acide nucléique recombinante codant pour une activité de phospholipase A2 dans une construction d'expression, et
b) cultiver et récolter la plante transgénique ou la cellule végétale transgéniques.

2. Procédé selon la revendication 1,
**caractérisé par le fait que** l'huile ou le lipide est isolé à partir de la plante transgénique ou la cellule végétale.

3. Procédé selon l'une des revendications 1 ou 2,
**caractérisé par le fait que** les séquences d'acide nucléique codant pour des polypeptides et des protéines, respectivement, ayant de l'activité de phospholipase A2 sont sélectionnées parmi le groupe consistant en :
a) une séquence d'acide nucléique ayant la séquence représentée dans SEQ ID NO : 1 ou
b) des séquences d'acide nucléique qui se font dériver de la séquence d'acide aminé représentée dans SEQ ID NO : 2 par suite du code dégénéré, ou
c) des dérivés de la séquence d'acide nucléique représentée dans SEQ ID NO : 1 codant pour des polypeptides et des protéines, respectivement, ayant au moins 40% d'identité avec SEQ ID NO : 2 au niveau d'acide aminé et possédant une activité de phospholipase A2.

4. Procédé selon l'une des revendications 1 à 3,
**caractérisé par le fait qu**'une séquence d'acide nucléique codant pour des polypeptides et protéines, respectivement, ayant une activité de Δ-12-désaturase, Δ-9-élongase, Δ-8-désaturase, Δ**-**6-désaturase, Δ-6-élongase, Δ-5-désaturase, Δ-5-élongase, ω-3-désaturase, Δ-4-désaturase est introduite additionellement dans la plante ou la cellule végétale.

5. Procédé selon l'une des revendications 1 à 4,
**caractérisé par le fait que** les huiles ou les lipides contiennent une proportion forte d'un acide gras insaturé sélectionné parmi le groupe de l'acide linoléique, l'acide γ-linolénique, l'acide stéaridonique, l'acide dihomo-γ-linolénique, l'acide ω-3-eicosatetraénoïque, l'acide arachidonique, l'acide eicosapentaénoïque, l'acide ω-6-docosapentaénoïque, l'acide ω-6-docosatetraénoïque, ω-3-docosapentaénoïque et l'acide docosahexaénoïque.

6. Procédé selon l'une des revendications 1 à 5,
**caractérisé par le fait que** l'acide gras insaturé est isolé à partir des huiles ou lipides dans la forme de son acide gras libre.

7. Procédé selon l'une des revendications 1 à 6,
**caractérisé par le fait que** la plante transgène est une plante produisant de l'huile, une plante légumière ou une plante ornementale.

8. Procédé selon l'une des revendications 1 à 7,
**caractérisé par le fait que** la plante transgène est sélectionné parmi le groupe des classifications et des familles de plantes respectivement : adelotheciaceae, anacardiaceae, asteraceae, apiaceae, betulaceae, Boraginaceae, brassicaceae, bromeliaceae, caricaceae, cannabaceae, convolvulaceae, chenopodiaceae, crypthecodiniaceae, cucurbitaceae, ditrichaceae, elaeagnaceae, ericaceae, euphorbiaceae, fabaceae, geraniaceae, gramineae, juglandaceae, lauraceae, leguminosae, linaceae ou prasinophyceae.


### SEQUENCE LISTING

<110> BASF AG
<120> verfahren zur Erhöhung des Gehalts an mehrfach ungesättigten langkettigen Fettsäuren in transgenen Organismen
<130> B 7767
<150> DE 10 2004 034 442.6
   <151> 2004-07-16
<150> DE 10 2004 060 340.5
   <151> 2004-12-15
<160> 145
<170> PatentIn version 3.3
<210> 1
   <211> 2793
   <212> DNA
   <213> ostreococcus tauri
<220>
   <221> CDS
   <222> (1)..(2793)
   <223> Phospholipase 2
<400> 1
   atg ggc gtg tgt tcc tcc aag agc ggc gcc gcg ccc gag gcc cag gag      48
   Met Gly Val Cys Ser Ser Lys Ser Gly Ala Ala Pro Glu Ala Gln Glu
   1               5                   10                  15
   acg gag ttt cgc ttg cgc aag acg gcg gat gga aga aga ttg ggc gac      96
   Thr Glu Phe Arg Leu Arg Lys Thr Ala Asp Gly Arg Arg Leu Gly Asp
               20                  25                  30
   gat tcg acg aag aac ctc agc gcg ttc gct gag gac agc gga atg tcg     144
   Asp Ser Thr Lys Asn Leu Ser Ala Phe Ala Glu Asp Ser Gly Met Ser
           35                  40                  45
   gtc ggg agc gaa cga gag gtg acg agg ggg gaa ccg atc gca tcg ctt     192
   Val Gly Ser Glu Arg Glu Val Thr Arg Gly Glu Pro Ile Ala Ser Leu
       50                  55                  60
   cga cct caa acc agg aat cag gag att tcg aaa aaa gac ttc aag acg     240
   Arg Pro Gln Thr Arg Asn Gln Glu Ile Ser Lys Lys Asp Phe Lys Thr
   65                  70                  75                  80
   aag aag aag aac gcg atc gcg gtg gag gac gac cag gtg ttg gaa gga     288
   Lys Lys Lys Asn Ala Ile Ala Val Glu Asp Asp Gln Val Leu Glu Gly
                   85                  90                  95
   gac gat gac gac gtg tat cgc ttc gat cag tca ctg cta aac agt gct     336
   Asp Asp Asp Asp Val Tyr Arg Phe Asp Gln Ser Leu Leu Asn Ser Ala
               100                 105                 110
   cgg gcg gcg atg aac gag gac gag gac gcc gat caa aac cgt agc ctg     384
   Arg Ala Ala Met Asn Glu Asp Glu Asp Ala Asp Gln Asn Arg Ser Leu
           115                 120                 125
   ctt cga aga caa tcg atc gtg aag gct ccg aac cag acg gtc gcc aag     432
   Leu Arg Arg Gln Ser Ile Val Lys Ala Pro Asn Gln Thr Val Ala Lys
       130                 135                 140
   cca ccc gtc gag ctg agc gag cgt ctc aaa agg tac gag gcc gaa cca     480
   Pro Pro Val Glu Leu Ser Glu Arg Leu Lys Arg Tyr Glu Ala Glu Pro
   145                 150                 155                 160
   aaa tta aag gcg aat gag tta ccg gtt tca tat ggt cca gaa tgg ctg      528
   Lys Leu Lys Ala Asn Glu Leu Pro Val Ser Tyr Gly Pro Glu Trp Leu
                   165                 170                 175
   gca ccg ccg gat ata caa cga gag acg acg gtg aga aaa cca aaa aag      576
   Ala Pro Pro Asp Ile Gln Arg Glu Thr Thr Val Arg Lys Pro Lys Lys
               180                 185                 190
   tac tcc att ggc aac ctt gca aga ggt gat cat aag ttg gaa ccg acg      624
   Tyr Ser Ile Gly Asn Leu Ala Arg Gly Asp His Lys Leu Glu Pro Thr
           195                 200                 205
   aat ttg acg gac aaa gac ttc tct gat tat gct ccc ggg aac ggg ccg      672
   Asn Leu Thr Asp Lys Asp Phe Ser Asp Tyr Ala Pro Gly Asn Gly Pro
       210                 215                 220
   tgg ttt aac ggt ccg ttc ccg agc atg atg gca tcg tca gca aag caa      720
   Trp Phe Asn Gly Pro Phe Pro Ser Met Met Ala Ser Ser Ala Lys Gln
   225                 230                 235                 240
   atg ggt tta gcg tac ggt aaa ttc tgc gta gtc ggt gtc atc acg ttg      768
   Met Gly Leu Ala Tyr Gly Lys Phe Cys Val Val Gly Val Ile Thr Leu
                   245                 250                 255
   ttt tgg gtg cca aac ttt ctt ctt tac cat agg ctg cta atg aac ggc      816
   Phe Trp Val Pro Asn Phe Leu Leu Tyr His Arg Leu Leu Met Asn Gly
               260                 265                 270
   acg ttt cag gca tat tcc agg ctc ctt tct gca atc tta tcg agc ctt      864
   Thr Phe Gln Ala Tyr Ser Arg Leu Leu Ser Ala Ile Leu Ser Ser Leu
           275                 280                 285
   ttt atg acg cga act gtg ctc aac atg aac atg gag ttg tgt cgc atg      912
   Phe Met Thr Arg Thr Val Leu Asn Met Asn Met Glu Leu Cys Arg Met
       290                 295                 300
   ttc tgg cga ggt tca ata gtg aat tcg cag gtt atc tgc tcc ttg ata      960
   Phe Trp Arg Gly Ser Ile Val Asn Ser Gln Val Ile Cys Ser Leu Ile
   305                 310                 315                 320
   ggc gcg gtc ggc ttc ttc tgg caa act ctg gtc atc ttc acc gtt gga     1008
   Gly Ala Val Gly Phe Phe Trp Gln Thr Leu Val Ile Phe Thr Val Gly
                   325                 330                 335
   tgg ctg agc gac aat cgc ggt atc ttt tca ttc ctg atc gcc ttt ggt     1056
   Trp Leu Ser Asp Asn Arg Gly Ile Phe Ser Phe Leu Ile Ala Phe Gly
               340                 345                 350
   gtt ggt tgg ttt atc gtt tgg agg caa gat cag cgc cat gaa aag caa     1104
   Val Gly Trp Phe Ile Val Trp Arg Gln Asp Gln Arg His Glu Lys Gln
           355                 360                 365
   cag cgc atc agg aca gtg atg ggt gct ttc ctt gcg ttg gaa aaa gat     1152
   Gln Arg Ile Arg Thr Val Met Gly Ala Phe Leu Ala Leu Glu Lys Asp
       370                 375                 380
   gcg aag cac atg gcc caa ctc atg ggc tca ccg gtg gtt cgt aca aac     1200
   Ala Lys His Met Ala Gln Leu Met Gly Ser Pro Val Val Arg Thr Asn
   385                 390                 395                 400
   gac atc caa tac atg aat gcg gcg ccc gtt tgg gcg aga tac cgc cca     1248
   Asp Ile Gln Tyr Met Asn Ala Ala Pro Val Trp Ala Arg Tyr Arg Pro
                   405                 410                 415
   gat gag ctt gtt ccg tgg ctg aac aat ttc ttg aca cag gtc tgg ccc     1296
   Asp Glu Leu Val Pro Trp Leu Asn Asn Phe Leu Thr Gln Val Trp Pro
               420                 425                 430
   ttc tac aac aaa gcg gcg agc gag ctg gtt cgc gag atc gtt gag ccg    1344
   Phe Tyr Asn Lys Ala Ala Ser Glu Leu Val Arg Glu Ile Val Glu Pro
           435                 440                 445
   ttg atg gag caa tct cgc ccc tcc atg ttg aag agg ctg act ttc aag    1392
   Leu Met Glu Gln Ser Arg Pro Ser Met Leu Lys Arg Leu Thr Phe Lys
       450                 455                 460
   cag ctc gat ttc ggc gaa aac cct ttc atg gtg cga agc gtc tca tat    1440
   Gln Leu Asp Phe Gly Glu Asn Pro Phe Met Val Arg Ser Val Ser Tyr
   465                 470                 475                 480
   gtc ggg aaa aaa gcc gag gac aaa ggt atg agc ctt gat ata gat ttt    1488
   Val Gly Lys Lys Ala Glu Asp Lys Gly Met Ser Leu Asp Ile Asp Phe
                   485                 490                 495
   gcc tgg gct gga agg tca aac atc gtt ctt gcg gca aaa acc cac ata    1536
   Ala Trp Ala Gly Arg Ser Asn Ile Val Leu Ala Ala Lys Thr His Ile
               500                 505                 510
   gga gca gac att aac atc gca gtt aaa gat ctt gag att tac acc aag    1584
   Gly Ala Asp Ile Asn Ile Ala Val Lys Asp Leu Glu Ile Tyr Thr Lys
           515                 520                 525
   ctc cgg gtg aca ctg aat ccc ctt gtg ccg ctg cca agc ccg ctt ggt    1632
   Leu Arg Val Thr Leu Asn Pro Leu Val Pro Leu Pro Ser Pro Leu Gly
       530                 535                 540
   ggt gtc gta att tct atg aca gag aga cct atc gtt gag ttt cac gta    1680
   Gly Val Val Ile Ser Met Thr Glu Arg Pro Ile Val Glu Phe His Val
   545                 550                 555                 560
   gag tta ccg agt ggg ttg gat gtg ctc tac gcc gca att gac aag tgg    1728
   Glu Leu Pro Ser Gly Leu Asp Val Leu Tyr Ala Ala Ile Asp Lys Trp
                   565                 570                 575
   ctc gag gag ttt gtc gcc ggt ctc ctt ggc gac atg ttt att cag ccc    1776
   Leu Glu Glu Phe Val Ala Gly Leu Leu Gly Asp Met Phe Ile Gln Pro
               580                 585                 590
   gag cgt ctg gtc ata ccg ttg agc ttc aat ttt gat cca atc gtc atg    1824
   Glu Arg Leu Val Ile Pro Leu Ser Phe Asn Phe Asp Pro Ile Val Met
           595                 600                 605
   ccg gac ggc gag gtg aag cca ttc aag tgg tac gac cat aac gtt ttg    1872
   Pro Asp Gly Glu Val Lys Pro Phe Lys Trp Tyr Asp His Asn Val Leu
       610                 615                 620
   cag ctg cgc aac act ggg gta ctc aag gta act gtt gtg cga gct gag    1920
   Gln Leu Arg Asn Thr Gly Val Leu Lys Val Thr Val Val Arg Ala Glu
   625                 630                 635                 640
   aac gtt cca agc gcg gat ctc ttg tcc aag acg gat cca ttc gtg aag    1968
   Asn Val Pro Ser Ala Asp Leu Leu Ser Lys Thr Asp Pro Phe Val Lys
                   645                 650                 655
   atg ttt gtc aag aaa cat ggg ttg caa gta aac acg acg acg att atg    2016
   Met Phe Val Lys Lys His Gly Leu Gln Val Asn Thr Thr Thr Ile Met
               660                 665                 670
   aac aac gaa gac cca gtc tgg aat gaa atc ttt tac att cca gtc gat    2064
   Asn Asn Glu Asp Pro Val Trp Asn Glu Ile Phe Tyr Ile Pro Val Asp
           675                 680                 685
   gat gtc gac ttg aga gtg ctc aag gtc gcc atg tac gat cac gac gta    2112
   Asp Val Asp Leu Arg Val Leu Lys Val Ala Met Tyr Asp His Asp Val
       690                 695                 700
   gat cct ctt agt agc gat gac aag ttg ggt gcc acg gaa gta cgc ata      2160
   Asp Pro Leu Ser Ser Asp Asp Lys Leu Gly Ala Thr Glu Val Arg Ile
   705                 710                 715                 720
   gac acg atc aaa gcc gcg aca gct gat ggg agt gaa caa gaa ctt tgg      2208
   Asp Thr Ile Lys Ala Ala Thr Ala Asp Gly Ser Glu Gln Glu Leu Trp
                   725                 730                 735
   ctc gat ttc ccg gag caa gtc aag ggg aat gtg aaa aaa ccg ccc atg      2256
   Leu Asp Phe Pro Glu Gln Val Lys Gly Asn Val Lys Lys Pro Pro Met
               740                 745                 750
   aaa ctg ttg ctg aat gca cag ttc atc tcg ttc ggc agc gac atc gca      2304
   Lys Leu Leu Leu Asn Ala Gln Phe Ile Ser Phe Gly Ser Asp Ile Ala
           755                 760                 765
   caa aat atg ttc act ggt ctt ggg cta ctg tcg gtt cac gtg atc cgc      2352
   Gln Asn Met Phe Thr Gly Leu Gly Leu Leu Ser Val His Val Ile Arg
       770                 775                 780
   ggc cga aat ttg caa cca atg gat tcg aat gga ttg tca gat cct tac      2400
   Gly Arg Asn Leu Gln Pro Met Asp Ser Asn Gly Leu Ser Asp Pro Tyr
   785                 790                 795                 800
   gtt aag gtc aag gtg cca aag ttc acc ctt gat agt atg gac atg gac      2448
   Val Lys Val Lys Val Pro Lys Phe Thr Leu Asp Ser Met Asp Met Asp
                   805                 810                 815
   aag ggc aaa ata ctg agg ggc aag aga ggt aag aag ggc aag aag aat      2496
   Lys Gly Lys Ile Leu Arg Gly Lys Arg Gly Lys Lys Gly Lys Lys Asn
               820                 825                 830
   gcg gaa gct cat gat tac acg gtt tat agc tca aag att cat tac aag      2544
   Ala Glu Ala His Asp Tyr Thr Val Tyr Ser Ser Lys Ile His Tyr Lys
           835                 840                 845
   aac ctg aat cca gag ttt aac gcc atg ttt gag ttc tct cct gca agt      2592
   Asn Leu Asn Pro Glu Phe Asn Ala Met Phe Glu Phe Ser Pro Ala Ser
       850                 855                 860
   gaa gac acg aag gtg tcg ata gag ctg ttt gac gtt gat tcg acg ttt      2640
   Glu Asp Thr Lys Val Ser Ile Glu Leu Phe Asp Val Asp Ser Thr Phe
   865                 870                 875                 880
   ccg atg ggg act aag agt aaa ttc atg ggg aat ctt gaa gtg ccg att      2688
   Pro Met Gly Thr Lys Ser Lys Phe Met Gly Asn Leu Glu Val Pro Ile
                   885                 890                 895
   tcg act atc att cac cac ggt gga tcg atg gag gcg agg ttt aaa gtt      2736
   Ser Thr Ile Ile His His Gly Gly Ser Met Glu Ala Arg Phe Lys Val
               900                 905                 910
   ggc aac gct aag tcg ggt gag cta gac atc gcg ttc aac tgg caa cca      2784
   Gly Asn Ala Lys Ser Gly Glu Leu Asp Ile Ala Phe Asn Trp Gln Pro
           915                 920                 925
   tac acg tga                                                          2793
   Tyr Thr
       930
<210> 2
   <211> 930
   <212> PRT
   <213> ostreococcus tauri
<400> 2
   Met Gly Val Cys Ser Ser Lys Ser Gly Ala Ala Pro Glu Ala Gln Glu
   1               5                   10                  15
   Thr Glu Phe Arg Leu Arg Lys Thr Ala Asp Gly Arg Arg Leu Gly Asp
               20                  25                  30
   Asp Ser Thr Lys Asn Leu Ser Ala Phe Ala Glu Asp Ser Gly Met Ser
           35                  40                  45
   Val Gly Ser Glu Arg Glu Val Thr Arg Gly Glu Pro Ile Ala Ser Leu
       50                  55                  60
   Arg Pro Gln Thr Arg Asn Gln Glu Ile Ser Lys Lys Asp Phe Lys Thr
   65                  70                  75                  80
   Lys Lys Lys Asn Ala Ile Ala Val Glu Asp Asp Gln Val Leu Glu Gly
                   85                  90                  95
   Asp Asp Asp Asp Val Tyr Arg Phe Asp Gln Ser Leu Leu Asn Ser Ala
               100                 105                 110
   Arg Ala Ala Met Asn Glu Asp Glu Asp Ala Asp Gln Asn Arg Ser Leu
           115                 120                 125
   Leu Arg Arg Gln Ser Ile Val Lys Ala Pro Asn Gln Thr Val Ala Lys
       130                 135                 140
   Pro Pro Val Glu Leu Ser Glu Arg Leu Lys Arg Tyr Glu Ala Glu Pro
   145                 150                 155                 160
   Lys Leu Lys Ala Asn Glu Leu Pro Val Ser Tyr Gly Pro Glu Trp Leu
                   165                 170                 175
   Ala Pro Pro Asp Ile Gln Arg Glu Thr Thr Val Arg Lys Pro Lys Lys
               180                 185                 190
   Tyr Ser Ile Gly Asn Leu Ala Arg Gly Asp His Lys Leu Glu Pro Thr
           195                 200                 205
   Asn Leu Thr Asp Lys Asp Phe Ser Asp Tyr Ala Pro Gly Asn Gly Pro
       210                 215                 220
   Trp Phe Asn Gly Pro Phe Pro Ser Met Met Ala Ser Ser Ala Lys Gln
   225                 230                 235                 240
   Met Gly Leu Ala Tyr Gly Lys Phe Cys Val Val Gly Val Ile Thr Leu
                   245                 250                 255
   Phe Trp Val Pro Asn Phe Leu Leu Tyr His Arg Leu Leu Met Asn Gly
   260 265 270
   Thr Phe Gln Ala Tyr Ser Arg Leu Leu Ser Ala Ile Leu Ser Ser Leu
   275 280 285
   Phe Met Thr Arg Thr Val Leu Asn Met Asn Met Glu Leu Cys Arg Met
   290 295 300
   Phe Trp Arg Gly Ser Ile Val Asn Ser Gln Val Ile Cys Ser Leu Ile
   305 310 315 320
   Gly Ala Val Gly Phe Phe Trp Gln Thr Leu Val Ile Phe Thr Val Gly
   325 330 335
   Trp Leu Ser Asp Asn Arg Gly Ile Phe Ser Phe Leu Ile Ala Phe Gly
   340 345 350
   Val Gly Trp Phe Ile Val Trp Arg Gln Asp Gln Arg His Glu Lys Gln
   355 360 365
   Gln Arg Ile Arg Thr Val Met Gly Ala Phe Leu Ala Leu Glu Lys Asp
   370 375 380
   Ala Lys His Met Ala Gln Leu Met Gly Ser Pro Val Val Arg Thr Asn
   385 390 395 400
   Asp Ile Gln Tyr Met Asn Ala Ala Pro Val Trp Ala Arg Tyr Arg Pro
   405 410 415
   Asp Glu Leu Val Pro Trp Leu Asn Asn Phe Leu Thr Gln Val Trp Pro
   420 425 430
   Phe Tyr Asn Lys Ala Ala Ser Glu Leu Val Arg Glu Ile Val Glu Pro
   435 440 445
   Leu Met Glu Gln Ser Arg Pro Ser Met Leu Lys Arg Leu Thr Phe Lys
   450 455 460
   Gln Leu Asp Phe Gly Glu Asn Pro Phe Met Val Arg Ser Val Ser Tyr
   465 470 475 480
   Val Gly Lys Lys Ala Glu Asp Lys Gly Met Ser Leu Asp Ile Asp Phe
   485 490 495
   Ala Trp Ala Gly Arg Ser Asn Ile Val Leu Ala Ala Lys Thr His Ile
   500 505 510
   Gly Ala Asp Ile Asn Ile Ala Val Lys Asp Leu Glu Ile Tyr Thr Lys
   515 520 525
   Leu Arg Val Thr Leu Asn Pro Leu Val Pro Leu Pro Ser Pro Leu Gly
   530 535 540
   Gly Val Val Ile Ser Met Thr Glu Arg Pro Ile Val Glu Phe His Val
   545 550 555 560
   Glu Leu Pro Ser Gly Leu Asp Val Leu Tyr Ala Ala Ile Asp Lys Trp
   565 570 575
   Leu Glu Glu Phe Val Ala Gly Leu Leu Gly Asp Met Phe Ile Gln Pro
   580 585 590
   Glu Arg Leu Val Ile Pro Leu Ser Phe Asn Phe Asp Pro Ile Val Met
   595 600 605
   Pro Asp Gly Glu Val Lys Pro Phe Lys Trp Tyr Asp His Asn Val Leu
   610 615 620
   Gln Leu Arg Asn Thr Gly Val Leu Lys Val Thr Val Val Arg Ala Glu
   625 630 635 640
   Asn Val Pro Ser Ala Asp Leu Leu Ser Lys Thr Asp Pro Phe Val Lys
   645 650 655
   Met Phe Val Lys Lys His Gly Leu Gln Val Asn Thr Thr Thr Ile Met
   660 665 670
   Asn Asn Glu Asp Pro Val Trp Asn Glu Ile Phe Tyr Ile Pro Val Asp
   675 680 685
   Asp Val Asp Leu Arg Val Leu Lys Val Ala Met Tyr Asp His Asp Val
   690 695 700
   Asp Pro Leu Ser Ser Asp Asp Lys Leu Gly Ala Thr Glu Val Arg Ile
   705 710 715 720
   Asp Thr Ile Lys Ala Ala Thr Ala Asp Gly Ser Glu Gln Glu Leu Trp
   725 730 735
   Leu Asp Phe Pro Glu Gln Val Lys Gly Asn Val Lys Lys Pro Pro Met
   740 745 750
   Lys Leu Leu Leu Asn Ala Gln Phe Ile Ser Phe Gly Ser Asp Ile Ala
   755 760 765
   Gln Asn Met Phe Thr Gly Leu Gly Leu Leu Ser Val His Val Ile Arg
   770 775 780
   Gly Arg Asn Leu Gln Pro Met Asp Ser Asn Gly Leu Ser Asp Pro Tyr
   785 790 795 800
   Val Lys Val Lys Val Pro Lys Phe Thr Leu Asp Ser Met Asp Met Asp
   805 810 815
   Lys Gly Lys Ile Leu Arg Gly Lys Arg Gly Lys Lys Gly Lys Lys Asn
   820 825 830
   Ala Glu Ala His Asp Tyr Thr Val Tyr Ser Ser Lys Ile His Tyr Lys
   835 840 845
   Asn Leu Asn Pro Glu Phe Asn Ala Met Phe Glu Phe Ser Pro Ala Ser
   850 855 860
   Glu Asp Thr Lys Val Ser Ile Glu Leu Phe Asp Val Asp Ser Thr Phe
   865 870 875 880
   Pro Met Gly Thr Lys Ser Lys Phe Met Gly Asn Leu Glu Val Pro Ile
   885 890 895
   Ser Thr Ile Ile His His Gly Gly Ser Met Glu Ala Arg Phe Lys Val
   900 905 910
   Gly Asn Ala Lys Ser Gly Glu Leu Asp Ile Ala Phe Asn Trp Gln Pro
   915 920 925
   Tyr Thr
   930
<210> 3
   <211> 984
   <212> DNA
   <213> Ostreococcus tauri
<220>
   <221> CDS
   <222> (1)..(984)
   <223> Ketoacyl-CoA-Reduktase
<400> 3
   atg ggc gcc ctg agc tat ctc ccg att ccc tac gtc cgc gcg ctg gtg      48
   Met Gly Ala Leu Ser Tyr Leu Pro Ile Pro Tyr Val Arg Ala Leu Val
   1               5                   10                  15
   cgg gat acc gtc gtc gat gcg tgc gtc tcg cac gtc ttg gcg ctc tac      96
   Arg Asp Thr Val Val Asp Ala Cys Val Ser His Val Leu Ala Leu Tyr
               20                  25                  30
   ggc ctc gtc gcg ctg ttg acg tat tgg gtg cca aag gtg gcg gtg cag     144
   Gly Leu Val Ala Leu Leu Thr Tyr Trp Val Pro Lys Val Ala Val Gln
           35                  40                  45
   atg atg ccg agt cag gat tta aag aag aag tac gac gcg cag tgg gcg     192
   Met Met Pro Ser Gln Asp Leu Lys Lys Lys Tyr Asp Ala Gln Trp Ala
       50                  55                  60
   ctc gtc acc ggt ggg tcc acg gga atc gga cga tcg ttg gcg ttc gcg     240
   Leu Val Thr Gly Gly Ser Thr Gly Ile Gly Arg Ser Leu Ala Phe Ala
   65                  70                  75                  80
   ctc gcg gaa caa ggg ctg aac gtc gcg gtg tgc gcg ctg gat gac gaa     288
   Leu Ala Glu Gln Gly Leu Asn Val Ala Val Cys Ala Leu Asp Asp Glu
                   85                  90                  95
   cat ctg gag acg acg tgt cgg gcg ctg cgg gag aaa ttc ggc gcg acg      336
   His Leu Glu Thr Thr Cys Arg Ala Leu Arg Glu Lys Phe Gly Ala Thr
               100                 105                 110
   agc gag att cga aaa att gga tgt aat ctc ggc gat cag tcg ggg gcg      384
   Ser Glu Ile Arg Lys Ile Gly Cys Asn Leu Gly Asp Gln Ser Gly Ala
           115                 120                 125
   tac gtg gag acg atc tcg aag gcg ttg gag gac gtg gat gtg cag gtg      432
   Tyr Val Glu Thr Ile Ser Lys Ala Leu Glu Asp Val Asp Val Gln Val
       130                 135                 140
   gtg ttc aat aac gcc ggg ttc atg ctc acg ggc ttt ttc gat aag cag      480
   Val Phe Asn Asn Ala Gly Phe Met Leu Thr Gly Phe Phe Asp Lys Gln
   145                 150                 155                 160
   ccg ttg gag aag ttg aac gcg aac aac gag tgc aac gcg acg agc gcg      528
   Pro Leu Glu Lys Leu Asn Ala Asn Asn Glu Cys Asn Ala Thr Ser Ala
                   165                 170                 175
   atg cga atc acg cac gtc ttc gtg cga agg atg ttg gca aaa aaa ctg      576
   Met Arg Ile Thr His Val Phe Val Arg Arg Met Leu Ala Lys Lys Leu
               180                 185                 190
   aga ggg tgc gtt gtt ttc acc tcg agc gcg gcg gcg tgt caa ccc acg      624
   Arg Gly Cys Val Val Phe Thr Ser Ser Ala Ala Ala Cys Gln Pro Thr
           195                 200                 205
   ccg ttt tcg gcg atg tac ggg gcg acg aag gcg tac att tcg agt ttc      672
   Pro Phe Ser Ala Met Tyr Gly Ala Thr Lys Ala Tyr Ile Ser Ser Phe
       210                 215                 220
   gcc gcc aac atc ggc gtc gag tta aag tct cga gga atc gac gtg tgc      720
   Ala Ala Asn Ile Gly Val Glu Leu Lys Ser Arg Gly Ile Asp Val Cys
   225                 230                 235                 240
   gcc gtg cat ccg agc ccg gtg gcg agt aac ttt tac gac aaa gcg cat      768
   Ala Val His Pro Ser Pro Val Ala Ser Asn Phe Tyr Asp Lys Ala His
                   245                 250                 255
   aaa ttg gac tca ctc aac ttt ttc atg aac ttt gcg gtg aag ccg gag      816
   Lys Leu Asp Ser Leu Asn Phe Phe Met Asn Phe Ala Val Lys Pro Glu
               260                 265                 270
   gag tta ccg acg gag atg ttc cgc ccg atc ggt cgc gtc ttg tgg cac      864
   Glu Leu Pro Thr Glu Met Phe Arg Pro Ile Gly Arg Val Leu Trp His
           275                 280                 285
   gac gtc ggc ggc gtc gcc atc ggt ttc cgc atg ctc ctc aag ctc ttc      912
   Asp Val Gly Gly Val Ala Ile Gly Phe Arg Met Leu Leu Lys Leu Phe
       290                 295                 300
   gac tac ggt ttc ttc gcg acg ctc atc tcg aga ttc gcc cac ctc atg      960
   Asp Tyr Gly Phe Phe Ala Thr Leu Ile Ser Arg Phe Ala His Leu Met
   305                 310                 315                 320
   ggc gat tac aag aag aac gtg taa                                      984
   Gly Asp Tyr Lys Lys Asn Val
                   325
<210> 4
   <211> 327
   <212> PRT
   <213> Ostreococcus tauri
<400> 4
   Met Gly Ala Leu Ser Tyr Leu Pro Ile Pro Tyr Val Arg Ala Leu Val
   1               5                   10                  15
   Arg Asp Thr Val Val Asp Ala Cys Val Ser His Val Leu Ala Leu Tyr
               20                  25                  30
   Gly Leu Val Ala Leu Leu Thr Tyr Trp Val Pro Lys Val Ala Val Gln
           35                  40                  45
   Met Met Pro Ser Gln Asp Leu Lys Lys Lys Tyr Asp Ala Gln Trp Ala
       50                  55                  60
   Leu Val Thr Gly Gly Ser Thr Gly Ile Gly Arg Ser Leu Ala Phe Ala
   65                  70                  75                  80
   Leu Ala Glu Gln Gly Leu Asn Val Ala Val Cys Ala Leu Asp Asp Glu
                   85                  90                  95
   His Leu Glu Thr Thr Cys Arg Ala Leu Arg Glu Lys Phe Gly Ala Thr
               100                 105                 110
   Ser Glu Ile Arg Lys Ile Gly Cys Asn Leu Gly Asp Gln Ser Gly Ala
           115                 120                 125
   Tyr Val Glu Thr Ile Ser Lys Ala Leu Glu Asp Val Asp Val Gln Val
       130                 135                 140
   Val Phe Asn Asn Ala Gly Phe Met Leu Thr Gly Phe Phe Asp Lys Gln
   145                 150                 155                 160
   Pro Leu Glu Lys Leu Asn Ala Asn Asn Glu Cys Asn Ala Thr Ser Ala
                   165                 170                 175
   Met Arg Ile Thr His Val Phe Val Arg Arg Met Leu Ala Lys Lys Leu
               180                 185                 190
   Arg Gly Cys Val Val Phe Thr Ser Ser Ala Ala Ala Cys Gln Pro Thr
           195                 200                 205
   Pro Phe Ser Ala Met Tyr Gly Ala Thr Lys Ala Tyr Ile Ser Ser Phe
       210                 215                 220
   Ala Ala Asn Ile Gly Val Glu Leu Lys Ser Arg Gly Ile Asp Val Cys
   225                 230                 235                 240
   Ala Val His Pro Ser Pro Val Ala Ser Asn Phe Tyr Asp Lys Ala His
                   245                 250                 255
   Lys Leu Asp Ser Leu Asn Phe Phe Met Asn Phe Ala Val Lys Pro Glu
   260 265 270
   Glu Leu Pro Thr Glu Met Phe Arg Pro Ile Gly Arg Val Leu Trp His
   275 280 285
   Asp Val Gly Gly Val Ala Ile Gly Phe Arg Met Leu Leu Lys Leu Phe
   290 295 300
   Asp Tyr Gly Phe Phe Ala Thr Leu Ile Ser Arg Phe Ala His Leu Met
   305 310 315 320
   Gly Asp Tyr Lys Lys Asn Val
   325
<210> 5
   <211> 1092
   <212> DNA
   <213> Ostreococcus tauri
<220>
   <221> CDS
   <222> (1)..(1092)
   <223> Dehydratase
<400> 5
   atg tcc acc cca ccc cat cca ccg ctc cgc gtc gag cgg cgt gga aac      48
   Met Ser Thr Pro Pro His Pro Pro Leu Arg Val Glu Arg Arg Gly Asn
   1               5                   10                  15
   gcc cag ttc atc gtc ctc gac cgt ccg aga gcg cgt aac gcg tta acg      96
   Ala Gln Phe Ile Val Leu Asp Arg Pro Arg Ala Arg Asn Ala Leu Thr
               20                  25                  30
   tcg gac gtc atc gag cgt tta cac cgc gcg tac gcc gcg ggc gag gac     144
   Ser Asp Val Ile Glu Arg Leu His Arg Ala Tyr Ala Ala Gly Glu Asp
           35                  40                  45
   aat gcg acg ctc tgc gcg cac gtc ata ctc ggc gcg aac tct gga acg     192
   Asn Ala Thr Leu Cys Ala His Val Ile Leu Gly Ala Asn Ser Gly Thr
       50                  55                  60
   ttc tgc gcc gga ggg gac gtg cgc gcg gtt cgc gaa atg gtg ctg aag     240
   Phe Cys Ala Gly Gly Asp Val Arg Ala Val Arg Glu Met Val Leu Lys
   65                  70                  75                  80
   aac gag cgg gac gcg gcg gtg ggc ttt ttc tcg aga gag ttc gcg ctg     288
   Asn Glu Arg Asp Ala Ala Val Gly Phe Phe Ser Arg Glu Phe Ala Leu
                   85                  90                  95
   aac gcg cgc ttg gcg acg ctg aca aag ccg tcg gcg tgc gtg tgg aac     336
   Asn Ala Arg Leu Ala Thr Leu Thr Lys Pro Ser Ala Cys Val Trp Asn
               100                 105                 110
   gga agc gtc atg ggc ggt ggg gcg gga ttg agc tgt tac gcc ccg gtg     384
   Gly Ser Val Met Gly Gly Gly Ala Gly Leu Ser Cys Tyr Ala Pro Val
           115                 120                 125
   cgg gtg tcg acg gag aag acg gtt ttt gcg atg ccg gag tgc gcg atc     432
   Arg Val Ser Thr Glu Lys Thr Val Phe Ala Met Pro Glu Cys Ala Ile
       130                 135                 140
   ggg ctt tgg ccc gac gtg ggg gcg tcg tgg ttt ttg aga agg ttg tgc     480
   Gly Leu Trp Pro Asp Val Gly Ala Ser Trp Phe Leu Arg Arg Leu Cys
   145                 150                 155                 160
   ggc ggc gcg acc ggg acg tgg ttg gcg ctc acg ggc gcg cgt gtt cgt      528
   Gly Gly Ala Thr Gly Thr Trp Leu Ala Leu Thr Gly Ala Arg Val Arg
                   165                 170                 175
   gga aaa gcg tgc aag gct tta gga tta tcc acg cat cac gtc acg tgc      576
   Gly Lys Ala Cys Lys Ala Leu Gly Leu Ser Thr His His Val Thr Cys
               180                 185                 190
   gag tca tgg gat gcg gtg tgc gaa ccg atg gtt cga gcg ctc acg gtt      624
   Glu Ser Trp Asp Ala Val Cys Glu Pro Met Val Arg Ala Leu Thr Val
           195                 200                 205
   ggg gcg agc gcg gag gat tta gcg gcg tgc gcg gcg gcg ggc gag acc      672
   Gly Ala Ser Ala Glu Asp Leu Ala Ala Cys Ala Ala Ala Gly Glu Thr
       210                 215                 220
   tcg gcg gac gcg gcg gaa gac ggg tgc gac gag tac gcg acg acg gcg      720
   Ser Ala Asp Ala Ala Glu Asp Gly Cys Asp Glu Tyr Ala Thr Thr Ala
   225                 230                 235                 240
   cgt ggg aag cgc gcg atc gag gag gtt ttc gga gat gaa tct cta tcg      768
   Arg Gly Lys Arg Ala Ile Glu Glu Val Phe Gly Asp Glu Ser Leu Ser
                   245                 250                 255
   ttg agc ggg atc acg agc gaa atc gct cgc agg cgc gac gcg gca acg      816
   Leu Ser Gly Ile Thr Ser Glu Ile Ala Arg Arg Arg Asp Ala Ala Thr
               260                 265                 270
   gac gac gta gaa cga cga ttc ttc gcc gaa tcc gcc gag tcg ctc gca      864
   Asp Asp Val Glu Arg Arg Phe Phe Ala Glu Ser Ala Glu Ser Leu Ala
           275                 280                 285
   aag gcg tgc ccg acg agc ctc gag gtc acg ctc gag ctc atg cgt cgc      912
   Lys Ala Cys Pro Thr Ser Leu Glu Val Thr Leu Glu Leu Met Arg Arg
       290                 295                 300
   gcg cga ggg aaa agt ttg gag tgg agc ttg gcg acg gac aac gcg ttg      960
   Ala Arg Gly Lys Ser Leu Glu Trp Ser Leu Ala Thr Asp Asn Ala Leu
   305                 310                 315                 320
   atc tcg cag ttc atc ttc gcc gac gat ttc aag cgc ggc gtc gac gcg     1008
   Ile Ser Gln Phe Ile Phe Ala Asp Asp Phe Lys Arg Gly Val Asp Ala
                   325                 330                 335
   gtg ctc atc aca aaa gtc ggc gtc ccg ccg ccc gag gga tgg gcg ccg     1056
   Val Leu Ile Thr Lys Val Gly Val Pro Pro Pro Glu Gly Trp Ala Pro
               340                 345                 350
   acg cgt tcg ccc gct tcc ttc ttc tct cgc ttg taa                     1092
   Thr Arg Ser Pro Ala Ser Phe Phe Ser Arg Leu
           355                 360
<210> 6
   <211> 363
   <212> PRT
   <213> Ostreococcus tauri
<400> 6
   Met Ser Thr Pro Pro His Pro Pro Leu Arg Val Glu Arg Arg Gly Asn
   1               5                   10                  15
   Ala Gln Phe Ile Val Leu Asp Arg Pro Arg Ala Arg Asn Ala Leu Thr
                   20                  25                  30
   Ser Asp Val Ile Glu Arg Leu His Arg Ala Tyr Ala Ala Gly Glu Asp
           35                  40                  45
   Asn Ala Thr Leu Cys Ala His Val Ile Leu Gly Ala Asn Ser Gly Thr
       50                  55                  60
   Phe Cys Ala Gly Gly Asp Val Arg Ala Val Arg Glu Met Val Leu Lys
   65                  70                  75                  80
   Asn Glu Arg Asp Ala Ala Val Gly Phe Phe Ser Arg Glu Phe Ala Leu
                   85                  90                  95
   Asn Ala Arg Leu Ala Thr Leu Thr Lys Pro Ser Ala Cys Val Trp Asn
               100                 105                 110
   Gly Ser Val Met Gly Gly Gly Ala Gly Leu Ser Cys Tyr Ala Pro Val
           115                 120                 125
   Arg Val Ser Thr Glu Lys Thr Val Phe Ala Met Pro Glu Cys Ala Ile
       130                 135                 140
   Gly Leu Trp Pro Asp Val Gly Ala Ser Trp Phe Leu Arg Arg Leu Cys
   145                 150                 155                 160
   Gly Gly Ala Thr Gly Thr Trp Leu Ala Leu Thr Gly Ala Arg Val Arg
                   165                 170                 175
   Gly Lys Ala Cys Lys Ala Leu Gly Leu Ser Thr His His Val Thr Cys
               180                 185                 190
   Glu Ser Trp Asp Ala Val Cys Glu Pro Met Val Arg Ala Leu Thr Val
           195                 200                 205
   Gly Ala Ser Ala Glu Asp Leu Ala Ala Cys Ala Ala Ala Gly Glu Thr
       210                 215                 220
   Ser Ala Asp Ala Ala Glu Asp Gly Cys Asp Glu Tyr Ala Thr Thr Ala
   225                 230                 235                 240
   Arg Gly Lys Arg Ala Ile Glu Glu Val Phe Gly Asp Glu Ser Leu Ser
                   245                 250                 255
   Leu Ser Gly Ile Thr Ser Glu Ile Ala Arg Arg Arg Asp Ala Ala Thr
               260                 265                 270
   Asp Asp Val Glu Arg Arg Phe Phe Ala Glu Ser Ala Glu Ser Leu Ala
           275                 280                 285
   Lys Ala Cys Pro Thr Ser Leu Glu Val Thr Leu Glu Leu Met Arg Arg
   290 295 300
   Ala Arg Gly Lys Ser Leu Glu Trp Ser Leu Ala Thr Asp Asn Ala Leu
   305 310 315 320
   Ile Ser Gln Phe Ile Phe Ala Asp Asp Phe Lys Arg Gly Val Asp Ala
   325 330 335
   Val Leu Ile Thr Lys Val Gly Val Pro Pro Pro Glu Gly Trp Ala Pro
   340 345 350
   Thr Arg Ser Pro Ala Ser Phe Phe Ser Arg Leu
   355 360
<210> 7
   <211> 1171
   <212> DNA
   <213> Thraustochytrium
<220>
   <221> gene
   <222> (1)..(1171)
   <223> putative Dehydratase
<400> 7
   acgagcgagg tgctgctcaa caagccgaag aagctcaacg cgctggacct ggagatggtg 60
   cgcatcatca agcccaagta cgacgaatgg gtcggctccg gcaaggcgca gtgcgtgctc 120
   atgcacggcg ccggcgagaa ggccttttgc gccggcggcg acatcgcctc ggtgcgctcg 180
   agcgcgctcg agggcggctc gctcgcagag gacttcttct acgaagagta tcagctcaac 240
   taccgcattg ccacggcctt tgaccgctgc ggcatcgtcc aagttagctt ttgggatggc 300
   atcaccatgg gcggcggcgt cggcctcagc ctgcacggca agatccgcgt cgccacggag 360
   aagacgcttt tcgccatgcc ggaaaccggc atcggcctct tcccggatgt cggcggcacc 420
   tttgcgctct cccgcatttc gggcggacct gagatcggca tgtacctcgc gctcacgggg 480
   actcgcctcg gcgcggccga ctgcctctac gcgggcctca ctacgcacta cgtggccagc 540
   gagaacgtcg acaaggtctg cgaaaagctc gccgcgtcca agtcggaccc ctcagccatt 600
   gaggccgttc tgcgcgagtt cgcggcggac gcgccgccgc ccaagaaccc tgccatgggc 660
   ctcgaggcgc gccacgaggc catcaaaaag tgctttagca tcagcgagtc cgtcgaggtt 720
   atcctcgacc gtctcgaggc catggccgcc gacgaggccg ccgacaagga cgaccgcgcc 780
   tgggccgagg cctcgcgcga tgccattcgt aaggcctcgc ccacctctgt gtgcctctcg 840
   ttcgaagccg tgcgccgcca cgctggcgcc gacgtcgaca tcgccaaggc gctcaccaac 900
   gagtaccgcc tcacgcagcg catctgcgtg ccggatggcg atttcttcga aggcgtccgc 960
   gccgtcctcg tcgaccgaga ccagtctccc aagtggaaat ttgcttcggt cgaggacgtc 1020
   cccgccgact tcatcgagag ccactttcag ccattgcctg actcgcatcc gcgcggcgat 1080
   ctcagcctct cctaggctag gctagccgtt tgcggacttt agactctgct aatcacaagt 1140
   ttactatgca gcgaaaaaaa aaaaaaaaaa a 1171
<210> 8
   <211> 346
   <212> PRT
   <213> Thraustochytrium
<400> 8
   Met Val Arg Ile Ile Lys Pro Lys Tyr Asp Glu Trp Val Gly Ser Gly
   1 5 10 15
   Lys Ala Gln Cys Val Leu Met His Gly Ala Gly Glu Lys Ala Phe Cys
   20 25 30
   Ala Gly Gly Asp Ile Ala Ser Val Arg Ser Ser Ala Leu Glu Gly Gly
   35 40 45
   Ser Leu Ala Glu Asp Phe Phe Tyr Glu Glu Tyr Gln Leu Asn Tyr Arg
   50 55 60
   Ile Ala Thr Ala Phe Asp Arg Cys Gly Ile Val Gln Val Ser Phe Trp
   65 70 75 80
   Asp Gly Ile Thr Met Gly Gly Gly Val Gly Leu Ser Leu His Gly Lys
   85 90 95
   Ile Arg Val Ala Thr Glu Lys Thr Leu Phe Ala Met Pro Glu Thr Gly
   100 105 110
   Ile Gly Leu Phe Pro Asp Val Gly Gly Thr Phe Ala Leu Ser Arg Ile
   115 120 125
   Ser Gly Gly Pro Glu Ile Gly Met Tyr Leu Ala Leu Thr Gly Thr Arg
   130 135 140
   Leu Gly Ala Ala Asp Cys Leu Tyr Ala Gly Leu Thr Thr His Tyr Val
   145 150 155 160
   Ala Ser Glu Asn Val Asp Lys Val Cys Glu Lys Leu Ala Ala Ser Lys
   165 170 175
   Ser Asp Pro Ser Ala Ile Glu Ala Val Leu Arg Glu Phe Ala Ala Asp
   180 185 190
   Ala Pro Pro Pro Lys Asn Pro Ala Met Gly Leu Glu Ala Arg His Glu
   195 200 205
   Ala Ile Lys Lys Cys Phe Ser Ile Ser Glu Ser Val Glu Val Ile Leu
   210 215 220
   Asp Arg Leu Glu Ala Met Ala Ala Asp Glu Ala Ala Asp Lys Asp Asp
   225                 230                 235                 240
   Arg Ala Trp Ala Glu Ala Ser Arg Asp Ala Ile Arg Lys Ala Ser Pro
                   245                 250                 255
   Thr Ser Val Cys Leu Ser Phe Glu Ala Val Arg Arg His Ala Gly Ala
               260                 265                 270
   Asp Val Asp Ile Ala Lys Ala Leu Thr Asn Glu Tyr Arg Leu Thr Gln
           275                 280                 285
   Arg Ile Cys Val Pro Asp Gly Asp Phe Phe Glu Gly Val Arg Ala Val
       290                 295                 300
   Leu Val Asp Arg Asp Gln Ser Pro Lys Trp Lys Phe Ala Ser Val Glu
   305                 310                 315                 320
   Asp Val Pro Ala Asp Phe Ile Glu Ser His Phe Gln Pro Leu Pro Asp
                   325                 330                 335
   Ser His Pro Arg Gly Asp Leu Ser Leu Ser
               340                 345
<210> 9
   <211> 21
   <212> DNA
   <213> Artificial
<220>
   <223> Primer
<400> 9
   caccatgggc gtgtgttcct c 21
<210> 10
   <211> 22
   <212> DNA
   <213> Artificial
<220>
   <223> Primer
<400> 10
   tcacgtgtat ggttgccagt tg 22
<210> 11
   <211> 23
   <212> DNA
   <213> Artificial
<220>
   <223> Primer
<400> 11
   caccatgggc gccctgagct atc 23
<210> 12
   <211> 20
   <212> DNA
   <213> Artificial
<220>
   <223> Primer
<400> 12
   ttacacgttc ttcttgtaat 20
<210> 13
   <211> 26
   <212> DNA
   <213> Artificial
<220>
   <223> Primer
<400> 13
   caccatgtcc accccacccc atccac 26
<210> 14
   <211> 20
   <212> DNA
   <213> Artificial
<220>
   <223> Primer
<400> 14
   ttacaagcga gagaagaagg 20
<210> 15
   <211> 24
   <212> DNA
   <213> Artificial
<220>
   <223> Primer
<400> 15
   caccatggtg cgcatcatca agcc 24
<210> 16
   <211> 19
   <212> DNA
   <213> Artificial
<220>
   <223> Primer
<400> 16
   ctaggagagg ctgagatcg 19
<210> 17
   <211> 60
   <212> DNA
   <213> Artificial
<220>
   <223> Primer
<400> 17
   gtcgacccgc ggactagtgg gccctctaga cccgggggat ccggatctgc tggctatgaa 60
<210> 18
   <211> 1266
   <212> DNA
   <213> Euglena gracilis
<220>
   <221> CDS
   <222> (1)..(1266)
   <223> delta-8-Desaturase
<400> 18
   atg aag tca aag cgc caa gcg ctt ccc ctt aca att gat gga aca aca      48
   Met Lys Ser Lys Arg Gln Ala Leu Pro Leu Thr Ile Asp Gly Thr Thr
   1               5                   10                  15
   tat gat gtg tct gcc tgg gtc aat ttc cac cct ggt ggt gcg gaa att      96
   Tyr Asp Val Ser Ala Trp Val Asn Phe His Pro Gly Gly Ala Glu Ile
               20                  25                  30
   ata gag aat tac caa gga agg gat gcc act gat gcc ttc atg gtt atg     144
   Ile Glu Asn Tyr Gln Gly Arg Asp Ala Thr Asp Ala Phe Met Val Met
           35                  40                  45
   cac tct caa gaa gcc ttc gac aag ctc aag cgc atg ccc aaa atc aat     192
   His Ser Gln Glu Ala Phe Asp Lys Leu Lys Arg Met Pro Lys Ile Asn
       50                  55                  60
   ccc agt tct gag ttg cca ccc cag gct gca gtg aat gaa gct caa gag     240
   Pro Ser Ser Glu Leu Pro Pro Gln Ala Ala Val Asn Glu Ala Gln Glu
   65                  70                  75                  80
   gat ttc cgg aag ctc cga gaa gag ttg atc gca act ggc atg ttt gat     288
   Asp Phe Arg Lys Leu Arg Glu Glu Leu Ile Ala Thr Gly Met Phe Asp
                   85                  90                  95
   gcc tcc ccc ctc tgg tac tca tac aaa atc agc acc aca ctg ggc ctt     336
   Ala Ser Pro Leu Trp Tyr Ser Tyr Lys Ile Ser Thr Thr Leu Gly Leu
               100                 105                 110
   gga gtg ctg ggt tat ttc ctg atg gtt cag tat cag atg tat ttc att     384
   Gly Val Leu Gly Tyr Phe Leu Met Val Gln Tyr Gln Met Tyr Phe Ile
           115                 120                 125
   ggg gca gtg ttg ctt ggg atg cac tat caa cag atg ggc tgg ctt tct     432
   Gly Ala Val Leu Leu Gly Met His Tyr Gln Gln Met Gly Trp Leu Ser
       130                 135                 140
   cat gac att tgc cac cac cag act ttc aag aac cgg aac tgg aac aac     480
   His Asp Ile Cys His His Gln Thr Phe Lys Asn Arg Asn Trp Asn Asn
   145                 150                 155                 160
   ctc gtg gga ctg gta ttt ggc aat ggt ctg caa ggt ttt tcc gtg aca     528
   Leu Val Gly Leu Val Phe Gly Asn Gly Leu Gln Gly Phe Ser Val Thr
                   165                 170                 175
   tgc tgg aag gac aga cac aat gca cat cat tcg gca acc aat gtt caa     576
   Cys Trp Lys Asp Arg His Asn Ala His His Ser Ala Thr Asn Val Gln
               180                 185                 190
   ggg cac gac cct gat att gac aac ctc ccc ctc tta gcc tgg tct gag     624
   Gly His Asp Pro Asp Ile Asp Asn Leu Pro Leu Leu Ala Trp Ser Glu
           195                 200                 205
   gat gac gtc aca cgg gcg tca ccg att tcc cgc aag ctc att cag ttc     672
   Asp Asp Val Thr Arg Ala Ser Pro Ile Ser Arg Lys Leu Ile Gln Phe
       210                 215                 220
   cag cag tat tat ttc ttg gtc atc tgt atc ttg ttg cgg ttc att tgg      720
   Gln Gln Tyr Tyr Phe Leu Val Ile Cys Ile Leu Leu Arg Phe Ile Trp
   225                 230                 235                 240
   tgt ttc cag agc gtg ttg acc gtg cgc agt ctg aag gac aga gat aac      768
   Cys Phe Gln Ser Val Leu Thr Val Arg Ser Leu Lys Asp Arg Asp Asn
                   245                 250                 255
   caa ttc tat cgc tct cag tat aag aag gag gcc att ggc ctc gcc ctg      816
   Gln Phe Tyr Arg Ser Gln Tyr Lys Lys Glu Ala Ile Gly Leu Ala Leu
               260                 265                 270
   cat tgg aca ttg aag gcc ctg ttc cac tta ttc ttt atg ccc agc atc      864
   His Trp Thr Leu Lys Ala Leu Phe His Leu Phe Phe Met Pro Ser Ile
           275                 280                 285
   ctc aca tcg ctg ttg gta ttt ttc gtt tcg gag ctg gtt ggc ggc ttc      912
   Leu Thr Ser Leu Leu Val Phe Phe Val Ser Glu Leu Val Gly Gly Phe
       290                 295                 300
   ggc att gcg atc gtg gtg ttc atg aac cac tac cca ctg gag aag atc      960
   Gly Ile Ala Ile Val Val Phe Met Asn His Tyr Pro Leu Glu Lys Ile
   305                 310                 315                 320
   ggg gac tcg gtc tgg gat ggc cat gga ttc tcg gtt ggc cag atc cat     1008
   Gly Asp Ser Val Trp Asp Gly His Gly Phe Ser Val Gly Gln Ile His
                   325                 330                 335
   gag acc atg aac att cgg cga ggg att atc aca gat tgg ttt ttc gga     1056
   Glu Thr Met Asn Ile Arg Arg Gly Ile Ile Thr Asp Trp Phe Phe Gly
               340                 345                 350
   ggc ttg aac tac cag atc gag cac cat ttg tgg ccg acc ctc cct cgc     1104
   Gly Leu Asn Tyr Gln Ile Glu His His Leu Trp Pro Thr Leu Pro Arg
           355                 360                 365
   cac aac ctg aca gcg gtt agc tac cag gtg gaa cag ctg tgc cag aag     1152
   His Asn Leu Thr Ala Val Ser Tyr Gln Val Glu Gln Leu Cys Gln Lys
       370                 375                 380
   cac aac ctg ccg tat cgg aac ccg ctg ccc cat gaa ggg ttg gtc atc     1200
   His Asn Leu Pro Tyr Arg Asn Pro Leu Pro His Glu Gly Leu Val Ile
   385                 390                 395                 400
   ctg ctg cgc tat ctg gcg gtg ttc gcc cgg atg gcg gag aag caa ccc     1248
   Leu Leu Arg Tyr Leu Ala Val Phe Ala Arg Met Ala Glu Lys Gln Pro
                   405                 410                 415
   gcg ggg aag gct cta taa                                             1266
   Ala Gly Lys Ala Leu
               420
<210> 19
   <211> 421
   <212> PRT
   <213> Euglena gracilis
<400> 19
   Met Lys Ser Lys Arg Gln Ala Leu Pro Leu Thr Ile Asp Gly Thr Thr
   1 5 10 15
   Tyr Asp Val Ser Ala Trp Val Asn Phe His Pro Gly Gly Ala Glu Ile
   20 25 30
   Ile Glu Asn Tyr Gln Gly Arg Asp Ala Thr Asp Ala Phe Met Val Met
   35 40 45
   His Ser Gln Glu Ala Phe Asp Lys Leu Lys Arg Met Pro Lys Ile Asn
   50 55 60
   Pro Ser Ser Glu Leu Pro Pro Gln Ala Ala Val Asn Glu Ala Gln Glu
   65 70 75 80
   Asp Phe Arg Lys Leu Arg Glu Glu Leu Ile Ala Thr Gly Met Phe Asp
   85 90 95
   Ala Ser Pro Leu Trp Tyr Ser Tyr Lys Ile Ser Thr Thr Leu Gly Leu
   100 105 110
   Gly Val Leu Gly Tyr Phe Leu Met Val Gln Tyr Gln Met Tyr Phe Ile
   115 120 125
   Gly Ala Val Leu Leu Gly Met His Tyr Gln Gln Met Gly Trp Leu Ser
   130 135 140
   His Asp Ile Cys His His Gln Thr Phe Lys Asn Arg Asn Trp Asn Asn
   145 150 155 160
   Leu Val Gly Leu Val Phe Gly Asn Gly Leu Gln Gly Phe Ser Val Thr
   165 170 175
   Cys Trp Lys Asp Arg His Asn Ala His His Ser Ala Thr Asn Val Gln
   180 185 190
   Gly His Asp Pro Asp Ile Asp Asn Leu Pro Leu Leu Ala Trp Ser Glu
   195 200 205
   Asp Asp Val Thr Arg Ala Ser Pro Ile Ser Arg Lys Leu Ile Gln Phe
   210 215 220
   Gln Gln Tyr Tyr Phe Leu Val Ile Cys Ile Leu Leu Arg Phe Ile Trp
   225 230 235 240
   Cys Phe Gln Ser Val Leu Thr Val Arg Ser Leu Lys Asp Arg Asp Asn
   245 250 255
   Gln Phe Tyr Arg Ser Gln Tyr Lys Lys Glu Ala Ile Gly Leu Ala Leu
   260 265 270
   His Trp Thr Leu Lys Ala Leu Phe His Leu Phe Phe Met Pro Ser Ile
   275 280 285
   Leu Thr Ser Leu Leu Val Phe Phe Val Ser Glu Leu Val Gly Gly Phe
   290 295 300
   Gly Ile Ala Ile Val Val Phe Met Asn His Tyr Pro Leu Glu Lys Ile
   305 310 315 320
   Gly Asp Ser Val Trp Asp Gly His Gly Phe Ser Val Gly Gln Ile His
   325 330 335
   Glu Thr Met Asn Ile Arg Arg Gly Ile Ile Thr Asp Trp Phe Phe Gly
   340 345 350
   Gly Leu Asn Tyr Gln Ile Glu His His Leu Trp Pro Thr Leu Pro Arg
   355 360 365
   His Asn Leu Thr Ala Val Ser Tyr Gln Val Glu Gln Leu Cys Gln Lys
   370 375 380
   His Asn Leu Pro Tyr Arg Asn Pro Leu Pro His Glu Gly Leu Val Ile
   385 390 395 400
   Leu Leu Arg Tyr Leu Ala Val Phe Ala Arg Met Ala Glu Lys Gln Pro
   405 410 415
   Ala Gly Lys Ala Leu
   420
<210> 20
   <211> 777
   <212> DNA
   <213> Isochrysis galbana
<220>
   <221> CDS
   <222> (1)..(777)
   <223> delta-9-Elongase
<400> 20
   atg gcc ctc gca aac gac gcg gga gag cgc atc tgg gcg gct gtg acc      48
   Met Ala Leu Ala Asn Asp Ala Gly Glu Arg Ile Trp Ala Ala Val Thr
   1               5                   10                  15
   gac ccg gaa atc ctc att ggc acc ttc tcg tac ttg cta ctc aaa ccg      96
   Asp Pro Glu Ile Leu Ile Gly Thr Phe Ser Tyr Leu Leu Leu Lys Pro
               20                  25                  30
   ctg ctc cgc aat tcc ggg ctg gtg gat gag aag aag ggc gca tac agg      144
   Leu Leu Arg Asn Ser Gly Leu Val Asp Glu Lys Lys Gly Ala Tyr Arg
           35                  40                  45
   acg tcc atg atc tgg tac aac gtt ctg ctg gcg ctc ttc tct gcg ctg      192
   Thr Ser Met Ile Trp Tyr Asn Val Leu Leu Ala Leu Phe Ser Ala Leu
       50                  55                  60
   agc ttc tac gtg acg gcg acc gcc ctc ggc tgg gac tat ggt acg ggc      240
   Ser Phe Tyr Val Thr Ala Thr Ala Leu Gly Trp Asp Tyr Gly Thr Gly
   65                  70                  75                  80
   gcg tgg ctg cgc agg caa acc ggc gac aca ccg cag ccg ctc ttc cag      288
   Ala Trp Leu Arg Arg Gln Thr Gly Asp Thr Pro Gln Pro Leu Phe Gln
                   85                  90                  95
   tgc ccg tcc ccg gtt tgg gac tcg aag ctc ttc aca tgg acc gcc aag      336
   Cys Pro Ser Pro Val Trp Asp Ser Lys Leu Phe Thr Trp Thr Ala Lys
               100                 105                 110
   gca ttc tat tac tcc aag tac gtg gag tac ctc gac acg gcc tgg ctg      384
   Ala Phe Tyr Tyr Ser Lys Tyr Val Glu Tyr Leu Asp Thr Ala Trp Leu
           115                 120                 125
   agg gtc tcc ttt ctc cag gcc ttc cac cac ttt ggc gcg ccg tgg gat      432
   Arg Val Ser Phe Leu Gln Ala Phe His His Phe Gly Ala Pro Trp Asp
       130                 135                 140
   gtg tac ctc ggc att cgg ctg cac aac gag ggc gta tgg atc ttc atg      480
   Val Tyr Leu Gly Ile Arg Leu His Asn Glu Gly Val Trp Ile Phe Met
   145                 150                 155                 160
   ttt ttc aac tcg ttc att cac acc atc atg tac acc tac tac ggc ctc      528
   Phe Phe Asn Ser Phe Ile His Thr Ile Met Tyr Thr Tyr Tyr Gly Leu
                   165                 170                 175
   acc gcc gcc ggg tat aag ttc aag gcc aag ccg ctc atc acc gcg atg      576
   Thr Ala Ala Gly Tyr Lys Phe Lys Ala Lys Pro Leu Ile Thr Ala Met
               180                 185                 190
   cag atc tgc cag ttc gtg ggc ggc ttc ctg ttg gtc tgg gac tac atc      624
   Gln Ile Cys Gln Phe Val Gly Gly Phe Leu Leu Val Trp Asp Tyr Ile
           195                 200                 205
   aac gtc ccc tgc ttc aac tcg gac aaa ggg aag ttg ttc agc tgg gct      672
   Asn Val Pro Cys Phe Asn Ser Asp Lys Gly Lys Leu Phe Ser Trp Ala
       210                 215                 220
   ttc aac tat gca tac gtc ggc tcg gtc ttc ttg ctc ttc tgc cac ttt      720
   Phe Asn Tyr Ala Tyr Val Gly Ser Val Phe Leu Leu Phe Cys His Phe
   225                 230                 235                 240
   ttc tac cag gac aac ttg gca acg aag aaa tcg gcc aag gcg ggc aag      768
   Phe Tyr Gln Asp Asn Leu Ala Thr Lys Lys Ser Ala Lys Ala Gly Lys
                   245                 250                 255
   cag ctc tag                                                          777
   Gln Leu
<210> 21
   <211> 258
   <212> PRT
   <213> Isochrysis galbana
<400> 21
   Met Ala Leu Ala Asn Asp Ala Gly Glu Arg Ile Trp Ala Ala Val Thr
   1 5 10 15
   Asp Pro Glu Ile Leu Ile Gly Thr Phe Ser Tyr Leu Leu Leu Lys Pro
   20 25 30
   Leu Leu Arg Asn Ser Gly Leu Val Asp Glu Lys Lys Gly Ala Tyr Arg
   35 40 45
   Thr Ser Met Ile Trp Tyr Asn Val Leu Leu Ala Leu Phe Ser Ala Leu
   50 55 60
   Ser Phe Tyr Val Thr Ala Thr Ala Leu Gly Trp Asp Tyr Gly Thr Gly
   65 70 75 80
   Ala Trp Leu Arg Arg Gln Thr Gly Asp Thr Pro Gln Pro Leu Phe Gln
   85 90 95
   Cys Pro Ser Pro Val Trp Asp Ser Lys Leu Phe Thr Trp Thr Ala Lys
   100 105 110
   Ala Phe Tyr Tyr Ser Lys Tyr Val Glu Tyr Leu Asp Thr Ala Trp Leu
   115 120 125
   Arg Val Ser Phe Leu Gln Ala Phe His His Phe Gly Ala Pro Trp Asp
   130 135 140
   Val Tyr Leu Gly Ile Arg Leu His Asn Glu Gly Val Trp Ile Phe Met
   145 150 155 160
   Phe Phe Asn Ser Phe Ile His Thr Ile Met Tyr Thr Tyr Tyr Gly Leu
   165 170 175
   Thr Ala Ala Gly Tyr Lys Phe Lys Ala Lys Pro Leu Ile Thr Ala Met
   180 185 190
   Gln Ile Cys Gln Phe Val Gly Gly Phe Leu Leu Val Trp Asp Tyr Ile
   195 200 205
   Asn Val Pro Cys Phe Asn Ser Asp Lys Gly Lys Leu Phe Ser Trp Ala
   210 215 220
   Phe Asn Tyr Ala Tyr Val Gly Ser Val Phe Leu Leu Phe Cys His Phe
   225 230 235 240
   Phe Tyr Gln Asp Asn Leu Ala Thr Lys Lys Ser Ala Lys Ala Gly Lys
   245 250 255
   Gln Leu
<210> 22
   <211> 1410
   <212> DNA
   <213> Phaeodactylum tricornutum
<220>
   <221> CDS
   <222> (1)..(1410)
   <223> delta-5-Desaturase
<400> 22
   atg gct ccg gat gcg gat aag ctt cga caa cgc cag acg act gcg gta 48
   Met Ala Pro Asp Ala Asp Lys Leu Arg Gln Arg Gln Thr Thr Ala Val
   1               5                   10                  15
   gcg aag cac aat gct gct acc ata tcg acg cag gaa cgc ctt tgc agt      96
   Ala Lys His Asn Ala Ala Thr Ile Ser Thr Gln Glu Arg Leu Cys Ser
               20                  25                  30
   ctg tct tcg ctc aaa ggc gaa gaa gtc tgc atc gac gga atc atc tat     144
   Leu Ser Ser Leu Lys Gly Glu Glu Val Cys Ile Asp Gly Ile Ile Tyr
           35                  40                  45
   gac ctc caa tca ttc gat cat ccc ggg ggt gaa acg atc aaa atg ttt     192
   Asp Leu Gln Ser Phe Asp His Pro Gly Gly Glu Thr Ile Lys Met Phe
       50                  55                  60
   ggt ggc aac gat gtc act gta cag tac aag atg att cac ccg tac cat     240
   Gly Gly Asn Asp Val Thr Val Gln Tyr Lys Met Ile His Pro Tyr His
   65                  70                  75                  80
   acc gag aag cat ttg gaa aag atg aag cgt gtc ggc aag gtg acg gat     288
   Thr Glu Lys His Leu Glu Lys Met Lys Arg Val Gly Lys Val Thr Asp
                   85                  90                  95
   ttc gtc tgc gag tac aag ttc gat acc gaa ttt gaa cgc gaa atc aaa     336
   Phe Val Cys Glu Tyr Lys Phe Asp Thr Glu Phe Glu Arg Glu Ile Lys
               100                 105                 110
   cga gaa gtc ttc aag att gtg cga cga ggc aag gat ttc ggt act ttg     384
   Arg Glu Val Phe Lys Ile Val Arg Arg Gly Lys Asp Phe Gly Thr Leu
           115                 120                 125
   gga tgg ttc ttc cgt gcg ttt tgc tac att gcc att ttc ttc tac ctg     432
   Gly Trp Phe Phe Arg Ala Phe Cys Tyr Ile Ala Ile Phe Phe Tyr Leu
       130                 135                 140
   cag tac cat tgg gtc acc acg gga acc tct tgg ctg ctg gcc gtg gcc     480
   Gln Tyr His Trp Val Thr Thr Gly Thr Ser Trp Leu Leu Ala Val Ala
   145                 150                 155                 160
   tac gga atc tcc caa gcg atg att ggc atg aat gtc cag cac gat gcc     528
   Tyr Gly Ile Ser Gln Ala Met Ile Gly Met Asn Val Gln His Asp Ala
                   165                 170                 175
   aac cac ggg gcc acc tcc aag cgt ccc tgg gtc aac gac atg cta ggc     576
   Asn His Gly Ala Thr Ser Lys Arg Pro Trp Val Asn Asp Met Leu Gly
               180                 185                 190
   ctc ggt gcg gat ttt att ggt ggt tcc aag tgg ctc tgg cag gaa caa     624
   Leu Gly Ala Asp Phe Ile Gly Gly Ser Lys Trp Leu Trp Gln Glu Gln
           195                 200                 205
   cac tgg acc cac cac gct tac acc aat cac gcc gag atg gat ccc gat     672
   His Trp Thr His His Ala Tyr Thr Asn His Ala Glu Met Asp Pro Asp
       210                 215                 220
   agc ttt ggt gcc gaa cca atg ctc cta ttc aac gac tat ccc ttg gat     720
   Ser Phe Gly Ala Glu Pro Met Leu Leu Phe Asn Asp Tyr Pro Leu Asp
   225                 230                 235                 240
   cat ccc gct cgt acc tgg cta cat cgc ttt caa gca ttc ttt tac atg     768
   His Pro Ala Arg Thr Trp Leu His Arg Phe Gln Ala Phe Phe Tyr Met
                   245                 250                 255
   ccc gtc ttg gct gga tac tgg ttg tcc gct gtc ttc aat cca caa att     816
   Pro Val Leu Ala Gly Tyr Trp Leu Ser Ala Val Phe Asn Pro Gln Ile
               260                 265                 270
   ctt gac ctc cag caa cgc ggc gca ctt tcc gtc ggt atc cgt ctc gac     864
   Leu Asp Leu Gln Gln Arg Gly Ala Leu Ser Val Gly Ile Arg Leu Asp
           275                 280                 285
   aac gct ttc att cac tcg cga cgc aag tat gcg gtt ttc tgg cgg gct      912
   Asn Ala Phe Ile His Ser Arg Arg Lys Tyr Ala Val Phe Trp Arg Ala
       290                 295                 300
   gtg tac att gcg gtg aac gtg att gct ccg ttt tac aca aac tcc ggc      960
   Val Tyr Ile Ala Val Asn Val Ile Ala Pro Phe Tyr Thr Asn Ser Gly
   305                 310                 315                 320
   ctc gaa tgg tcc tgg cgt gtc ttt gga aac atc atg ctc atg ggt gtg     1008
   Leu Glu Trp Ser Trp Arg Val Phe Gly Asn Ile Met Leu Met Gly Val
                   325                 330                 335
   gcg gaa tcg ctc gcg ctg gcg gtc ctg ttt tcg ttg tcg cac aat ttc     1056
   Ala Glu Ser Leu Ala Leu Ala Val Leu Phe Ser Leu Ser His Asn Phe
               340                 345                 350
   gaa tcc gcg gat cgc gat ccg acc gcc cca ctg aaa aag acg gga gaa     1104
   Glu Ser Ala Asp Arg Asp Pro Thr Ala Pro Leu Lys Lys Thr Gly Glu
           355                 360                 365
   cca gtc gac tgg ttc aag aca cag gtc gaa act tcc tgc act tac ggt     1152
   Pro Val Asp Trp Phe Lys Thr Gln Val Glu Thr Ser Cys Thr Tyr Gly
       370                 375                 380
   gga ttc ctt tcc ggt tgc ttc acg gga ggt ctc aac ttt cag gtt gaa     1200
   Gly Phe Leu Ser Gly Cys Phe Thr Gly Gly Leu Asn Phe Gln Val Glu
   385                 390                 395                 400
   cac cac ttg ttc cca cgc atg agc agc gct tgg tat ccc tac att gcc     1248
   His His Leu Phe Pro Arg Met Ser Ser Ala Trp Tyr Pro Tyr Ile Ala
                   405                 410                 415
   ccc aag gtc cgc gaa att tgc gcc aaa cac ggc gtc cac tac gcc tac     1296
   Pro Lys Val Arg Glu Ile Cys Ala Lys His Gly Val His Tyr Ala Tyr
               420                 425                 430
   tac ccg tgg atc cac caa aac ttt ctc tcc acc gtc cgc tac atg cac     1344
   Tyr Pro Trp Ile His Gln Asn Phe Leu Ser Thr Val Arg Tyr Met His
           435                 440                 445
   gcg gcc ggg acc ggt gcc aac tgg cgc cag atg gcc aga gaa aat ccc     1392
   Ala Ala Gly Thr Gly Ala Asn Trp Arg Gln Met Ala Arg Glu Asn Pro
       450                 455                 460
   ttg acc gga cgg gcg taa                                             1410
   Leu Thr Gly Arg Ala
   465
<210> 23
   <211> 469
   <212> PRT
   <213> Phaeodactylum tricornutum
<400> 23
   Met Ala Pro Asp Ala Asp Lys Leu Arg Gln Arg Gln Thr Thr Ala Val
   1               5                   10                  15
   Ala Lys His Asn Ala Ala Thr Ile Ser Thr Gln Glu Arg Leu Cys Ser
               20                  25                  30
   Leu Ser Ser Leu Lys Gly Glu Glu Val Cys Ile Asp Gly Ile Ile Tyr
           35                  40                  45
   Asp Leu Gln Ser Phe Asp His Pro Gly Gly Glu Thr Ile Lys Met Phe
       50                  55                  60
   Gly Gly Asn Asp Val Thr Val Gln Tyr Lys Met Ile His Pro Tyr His
   65                  70                  75                  80
   Thr Glu Lys His Leu Glu Lys Met Lys Arg Val Gly Lys Val Thr Asp
                   85                  90                  95
   Phe Val Cys Glu Tyr Lys Phe Asp Thr Glu Phe Glu Arg Glu Ile Lys
               100                 105                 110
   Arg Glu Val Phe Lys Ile Val Arg Arg Gly Lys Asp Phe Gly Thr Leu
           115                 120                 125
   Gly Trp Phe Phe Arg Ala Phe Cys Tyr Ile Ala Ile Phe Phe Tyr Leu
       130                 135                 140
   Gln Tyr His Trp Val Thr Thr Gly Thr Ser Trp Leu Leu Ala Val Ala
   145                 150                 155                 160
   Tyr Gly Ile Ser Gln Ala Met Ile Gly Met Asn Val Gln His Asp Ala
                   165                 170                 175
   Asn His Gly Ala Thr Ser Lys Arg Pro Trp Val Asn Asp Met Leu Gly
               180                 185                 190
   Leu Gly Ala Asp Phe Ile Gly Gly Ser Lys Trp Leu Trp Gln Glu Gln
           195                 200                 205
   His Trp Thr His His Ala Tyr Thr Asn His Ala Glu Met Asp Pro Asp
       210                 215                 220
   Ser Phe Gly Ala Glu Pro Met Leu Leu Phe Asn Asp Tyr Pro Leu Asp
   225                 230                 235                 240
   His Pro Ala Arg Thr Trp Leu His Arg Phe Gln Ala Phe Phe Tyr Met
                   245                 250                 255
   Pro Val Leu Ala Gly Tyr Trp Leu Ser Ala Val Phe Asn Pro Gln Ile
               260                 265                 270
   Leu Asp Leu Gln Gln Arg Gly Ala Leu Ser Val Gly Ile Arg Leu Asp
           275                 280                 285
   Asn Ala Phe Ile His Ser Arg Arg Lys Tyr Ala Val Phe Trp Arg Ala
       290                 295                 300
   Val Tyr Ile Ala Val Asn Val Ile Ala Pro Phe Tyr Thr Asn Ser Gly
   305 310 315 320
   Leu Glu Trp Ser Trp Arg Val Phe Gly Asn Ile Met Leu Met Gly Val
   325 330 335
   Ala Glu Ser Leu Ala Leu Ala Val Leu Phe Ser Leu Ser His Asn Phe
   340 345 350
   Glu Ser Ala Asp Arg Asp Pro Thr Ala Pro Leu Lys Lys Thr Gly Glu
   355 360 365
   Pro Val Asp Trp Phe Lys Thr Gln Val Glu Thr Ser Cys Thr Tyr Gly
   370 375 380
   Gly Phe Leu Ser Gly Cys Phe Thr Gly Gly Leu Asn Phe Gln Val Glu
   385 390 395 400
   His His Leu Phe Pro Arg Met Ser Ser Ala Trp Tyr Pro Tyr Ile Ala
   405 410 415
   Pro Lys Val Arg Glu Ile Cys Ala Lys His Gly Val His Tyr Ala Tyr
   420 425 430
   Tyr Pro Trp Ile His Gln Asn Phe Leu Ser Thr Val Arg Tyr Met His
   435 440 445
   Ala Ala Gly Thr Gly Ala Asn Trp Arg Gln Met Ala Arg Glu Asn Pro
   450 455 460
   Leu Thr Gly Arg Ala
   465
<210> 24
   <211> 1344
   <212> DNA
   <213> Ceratodon purpureus
<220>
   <221> CDS
   <222> (1)..(1344)
   <223> delta-5-Desaturase
<400> 24
   atg gta tta cga gag caa gag cat gag cca ttc ttc att aaa att gat        48
   Met Val Leu Arg Glu Gln Glu His Glu Pro Phe Phe Ile Lys Ile Asp
   1               5                   10                  15
   gga aaa tgg tgt caa att gac gat gct gtc ctg aga tca cat cca ggt        96
   Gly Lys Trp Cys Gln Ile Asp Asp Ala Val Leu Arg Ser His Pro Gly
               20                  25                  30
   ggt agt gca att act acc tat aaa aat atg gat gcc act acc gta ttc       144
   Gly Ser Ala Ile Thr Thr Tyr Lys Asn Met Asp Ala Thr Thr Val Phe
           35                  40                  45
   cac aca ttc cat act ggt tct aaa gaa gcg tat caa tgg ctg aca gaa       192
   His Thr Phe His Thr Gly Ser Lys Glu Ala Tyr Gln Trp Leu Thr Glu
       50                  55                  60
   ttg aaa aaa gag tgc cct aca caa gaa cca gag atc cca gat att aag      240
   Leu Lys Lys Glu Cys Pro Thr Gln Glu Pro Glu Ile Pro Asp Ile Lys
   65                  70                  75                  80
   gat gac cca atc aaa gga att gat gat gtg aac atg gga act ttc aat      288
   Asp Asp Pro Ile Lys Gly Ile Asp Asp Val Asn Met Gly Thr Phe Asn
                   85                  90                  95
   att tct gag aaa cga tct gcc caa ata aat aaa agt ttc act gat cta      336
   Ile Ser Glu Lys Arg Ser Ala Gln Ile Asn Lys Ser Phe Thr Asp Leu
               100                 105                 110
   cgt atg cga gtt cgt gca gaa gga ctt atg gat gga tct cct ttg ttc      384
   Arg Met Arg Val Arg Ala Glu Gly Leu Met Asp Gly Ser Pro Leu Phe
           115                 120                 125
   tac att aga aaa att ctt gaa aca atc ttc aca att ctt ttt gca ttc      432
   Tyr Ile Arg Lys Ile Leu Glu Thr Ile Phe Thr Ile Leu Phe Ala Phe
       130                 135                 140
   tac ctt caa tac cac aca tat tat ctt cca tca gct att cta atg gga      480
   Tyr Leu Gln Tyr His Thr Tyr Tyr Leu Pro Ser Ala Ile Leu Met Gly
   145                 150                 155                 160
   gtt gcg tgg caa caa ttg gga tgg tta atc cat gaa ttc gca cat cat      528
   Val Ala Trp Gln Gln Leu Gly Trp Leu Ile His Glu Phe Ala His His
                   165                 170                 175
   cag ttg ttc aaa aac aga tac tac aat gat ttg gcc agc tat ttc gtt      576
   Gln Leu Phe Lys Asn Arg Tyr Tyr Asn Asp Leu Ala Ser Tyr Phe Val
               180                 185                 190
   gga aac ttt tta caa gga ttc tca tct ggt ggt tgg aaa gag cag cac      624
   Gly Asn Phe Leu Gln Gly Phe Ser Ser Gly Gly Trp Lys Glu Gln His
           195                 200                 205
   aat gtg cat cac gca gcc aca aat gtt gtt gga cga gac gga gat ctt      672
   Asn Val His His Ala Ala Thr Asn Val Val Gly Arg Asp Gly Asp Leu
       210                 215                 220
   gat tta gtc cca ttc tat gct aca gtg gca gaa cat ctc aac aat tat      720
   Asp Leu Val Pro Phe Tyr Ala Thr Val Ala Glu His Leu Asn Asn Tyr
   225                 230                 235                 240
   tct cag gat tca tgg gtt atg act cta ttc aga tgg caa cat gtt cat      768
   Ser Gln Asp Ser Trp Val Met Thr Leu Phe Arg Trp Gln His Val His
                   245                 250                 255
   tgg aca ttc atg tta cca ttc ctc cgt ctc tcg tgg ctt ctt cag tca      816
   Trp Thr Phe Met Leu Pro Phe Leu Arg Leu Ser Trp Leu Leu Gln Ser
               260                 265                 270
   atc att ttt gtt agt cag atg cca act cat tat tat gac tat tac aga      864
   Ile Ile Phe Val Ser Gln Met Pro Thr His Tyr Tyr Asp Tyr Tyr Arg
           275                 280                 285
   aat act gcg att tat gaa cag gtt ggt ctc tct ttg cac tgg gct tgg      912
   Asn Thr Ala Ile Tyr Glu Gln Val Gly Leu Ser Leu His Trp Ala Trp
       290                 295                 300
   tca ttg ggt caa ttg tat ttc cta ccc gat tgg tca act aga ata atg      960
   Ser Leu Gly Gln Leu Tyr Phe Leu Pro Asp Trp Ser Thr Arg Ile Met
   305                 310                 315                 320
   ttc ttc ctt gtt tct cat ctt gtt gga ggt ttc ctg ctc tct cat gta     1008
   Phe Phe Leu Val Ser His Leu Val Gly Gly Phe Leu Leu Ser His Val
                   325                 330                 335
   gtt act ttc aat cat tat tca gtg gag aag ttt gca ttg agc tcg aac      1056
   Val Thr Phe Asn His Tyr Ser Val Glu Lys Phe Ala Leu Ser Ser Asn
               340                 345                 350
   atc atg tca aat tac gct tgt ctt caa atc atg acc aca aga aat atg      1104
   Ile Met Ser Asn Tyr Ala Cys Leu Gln Ile Met Thr Thr Arg Asn Met
           355                 360                 365
   aga cct gga aga ttc att gac tgg ctt tgg gga ggt ctt aac tat cag      1152
   Arg Pro Gly Arg Phe Ile Asp Trp Leu Trp Gly Gly Leu Asn Tyr Gln
       370                 375                 380
   att gag cac cat ctt ttc cca acg atg cca cga cac aac ttg aac act      1200
   Ile Glu His His Leu Phe Pro Thr Met Pro Arg His Asn Leu Asn Thr
   385                 390                 395                 400
   gtt atg cca ctt gtt aag gag ttt gca gca gca aat ggt tta cca tac      1248
   Val Met Pro Leu Val Lys Glu Phe Ala Ala Ala Asn Gly Leu Pro Tyr
                   405                 410                 415
   atg gtc gac gat tat ttc aca gga ttc tgg ctt gaa att gag caa ttc      1296
   Met Val Asp Asp Tyr Phe Thr Gly Phe Trp Leu Glu Ile Glu Gln Phe
               420                 425                 430
   cga aat att gca aat gtt gct gct aaa ttg act aaa aag att gcc tag      1344
   Arg Asn Ile Ala Asn Val Ala Ala Lys Leu Thr Lys Lys Ile Ala
           435                 440                 445
<210> 25
   <211> 447
   <212> PRT
   <213> Ceratodon purpureus
<400> 25
   Met Val Leu Arg Glu Gln Glu His Glu Pro Phe Phe Ile Lys Ile Asp
   1               5                   10                  15
   Gly Lys Trp Cys Gln Ile Asp Asp Ala Val Leu Arg Ser His Pro Gly
               20                  25                  30
   Gly Ser Ala Ile Thr Thr Tyr Lys Asn Met Asp Ala Thr Thr Val Phe
           35                  40                  45
   His Thr Phe His Thr Gly Ser Lys Glu Ala Tyr Gln Trp Leu Thr Glu
       50                  55                  60
   Leu Lys Lys Glu Cys Pro Thr Gln Glu Pro Glu Ile Pro Asp Ile Lys
   65                  70                  75                  80
   Asp Asp Pro Ile Lys Gly Ile Asp Asp Val Asn Met Gly Thr Phe Asn
                   85                  90                  95
   Ile Ser Glu Lys Arg Ser Ala Gln Ile Asn Lys Ser Phe Thr Asp Leu
               100                 105                 110
   Arg Met Arg Val Arg Ala Glu Gly Leu Met Asp Gly Ser Pro Leu Phe
   115 120 125
   Tyr Ile Arg Lys Ile Leu Glu Thr Ile Phe Thr Ile Leu Phe Ala Phe
   130 135 140
   Tyr Leu Gln Tyr His Thr Tyr Tyr Leu Pro Ser Ala Ile Leu Met Gly
   145 150 155 160
   Val Ala Trp Gln Gln Leu Gly Trp Leu Ile His Glu Phe Ala His His
   165 170 175
   Gln Leu Phe Lys Asn Arg Tyr Tyr Asn Asp Leu Ala Ser Tyr Phe Val
   180 185 190
   Gly Asn Phe Leu Gln Gly Phe Ser Ser Gly Gly Trp Lys Glu Gln His
   195 200 205
   Asn Val His His Ala Ala Thr Asn Val Val Gly Arg Asp Gly Asp Leu
   210 215 220
   Asp Leu Val Pro Phe Tyr Ala Thr Val Ala Glu His Leu Asn Asn Tyr
   225 230 235 240
   Ser Gln Asp Ser Trp Val Met Thr Leu Phe Arg Trp Gln His Val His
   245 250 255
   Trp Thr Phe Met Leu Pro Phe Leu Arg Leu Ser Trp Leu Leu Gln Ser
   260 265 270
   Ile Ile Phe Val Ser Gln Met Pro Thr His Tyr Tyr Asp Tyr Tyr Arg
   275 280 285
   Asn Thr Ala Ile Tyr Glu Gln Val Gly Leu Ser Leu His Trp Ala Trp
   290 295 300
   Ser Leu Gly Gln Leu Tyr Phe Leu Pro Asp Trp Ser Thr Arg Ile Met
   305 310 315 320
   Phe Phe Leu Val Ser His Leu Val Gly Gly Phe Leu Leu Ser His Val
   325 330 335
   Val Thr Phe Asn His Tyr Ser Val Glu Lys Phe Ala Leu Ser Ser Asn
   340 345 350
   Ile Met Ser Asn Tyr Ala Cys Leu Gln Ile Met Thr Thr Arg Asn Met
   355 360 365
   Arg Pro Gly Arg Phe Ile Asp Trp Leu Trp Gly Gly Leu Asn Tyr Gln
   370 375 380
   Ile Glu His His Leu Phe Pro Thr Met Pro Arg His Asn Leu Asn Thr
   385 390 395 400
   Val Met Pro Leu Val Lys Glu Phe Ala Ala Ala Asn Gly Leu Pro Tyr
   405 410 415
   Met Val Asp Asp Tyr Phe Thr Gly Phe Trp Leu Glu Ile Glu Gln Phe
   420 425 430
   Arg Asn Ile Ala Asn Val Ala Ala Lys Leu Thr Lys Lys Ile Ala
   435 440 445
<210> 26
   <211> 1443
   <212> DNA
   <213> Physcomitrella patens
<220>
   <221> CDS
   <222> (1)..(1443)
   <223> delta-5-Desaturase
<400> 26
   atg gcg ccc cac tct gcg gat act gct ggg ctc gtg cct tct gac gaa      48
   Met Ala Pro His Ser Ala Asp Thr Ala Gly Leu Val Pro Ser Asp Glu
   1               5                   10                  15
   ttg agg cta cga acg tcg aat tca aag ggt ccc gaa caa gag caa act      96
   Leu Arg Leu Arg Thr Ser Asn Ser Lys Gly Pro Glu Gln Glu Gln Thr
               20                  25                  30
   ttg aag aag tac acc ctt gaa gat gtc agc cgc cac aac acc cca gca     144
   Leu Lys Lys Tyr Thr Leu Glu Asp Val Ser Arg His Asn Thr Pro Ala
           35                  40                  45
   gat tgt tgg ttg gtg ata tgg ggc aaa gtc tac gat gtc aca agc tgg     192
   Asp Cys Trp Leu Val Ile Trp Gly Lys Val Tyr Asp Val Thr Ser Trp
       50                  55                  60
   att ccc aat cat ccg ggg ggc agt ctc atc cac gta aaa gca ggg cag     240
   Ile Pro Asn His Pro Gly Gly Ser Leu Ile His Val Lys Ala Gly Gln
   65                  70                  75                  80
   gat tcc act cag ctt ttc gat tcc tat cac ccc ctt tat gtc agg aaa     288
   Asp Ser Thr Gln Leu Phe Asp Ser Tyr His Pro Leu Tyr Val Arg Lys
                   85                  90                  95
   atg ctc gcg aag tac tgt att ggg gaa tta gta ccg tct gct ggt gat     336
   Met Leu Ala Lys Tyr Cys Ile Gly Glu Leu Val Pro Ser Ala Gly Asp
               100                 105                 110
   gac aag ttt aag aaa gca act ctg gag tat gca gat gcc gaa aat gaa     384
   Asp Lys Phe Lys Lys Ala Thr Leu Glu Tyr Ala Asp Ala Glu Asn Glu
           115                 120                 125
   gat ttc tat ttg gtt gtg aag caa cga gtt gaa tct tat ttc aag agt     432
   Asp Phe Tyr Leu Val Val Lys Gln Arg Val Glu Ser Tyr Phe Lys Ser
       130                 135                 140
   aac aag ata aac ccc caa att cat cca cat atg atc ctg aag tca ttg     480
   Asn Lys Ile Asn Pro Gln Ile His Pro His Met Ile Leu Lys Ser Leu
   145                 150                 155                 160
   ttc att ctt ggg gga tat ttc gcc agt tac tat tta gcg ttc ttc tgg     528
   Phe Ile Leu Gly Gly Tyr Phe Ala Ser Tyr Tyr Leu Ala Phe Phe Trp
                   165                 170                 175
   tct tca agt gtc ctt gtt tct ttg ttt ttc gca ttg tgg atg ggg ttc      576
   Ser Ser Ser Val Leu Val Ser Leu Phe Phe Ala Leu Trp Met Gly Phe
               180                 185                 190
   ttc gca gcg gaa gtc ggc gtg tcg att caa cat gat gga aat cat ggt      624
   Phe Ala Ala Glu Val Gly Val Ser Ile Gln His Asp Gly Asn His Gly
           195                 200                 205
   tca tac act aaa tgg cgt ggc ttt gga tat atc atg gga gcc tcc cta      672
   Ser Tyr Thr Lys Trp Arg Gly Phe Gly Tyr Ile Met Gly Ala Ser Leu
       210                 215                 220
   gat cta gtc gga gcc agt agc ttc atg tgg aga cag caa cac gtt gtg      720
   Asp Leu Val Gly Ala Ser Ser Phe Met Trp Arg Gln Gln His Val Val
   225                 230                 235                 240
   gga cat cac tcg ttt aca aat gtg gac aac tac gat cct gat att cgt      768
   Gly His His Ser Phe Thr Asn Val Asp Asn Tyr Asp Pro Asp Ile Arg
                   245                 250                 255
   gtg aaa gat cca gat gtc agg agg gtt gcg acc aca caa cca aga caa      816
   Val Lys Asp Pro Asp Val Arg Arg Val Ala Thr Thr Gln Pro Arg Gln
               260                 265                 270
   tgg tat cat gcg tat cag cat atc tac ctg gca gta tta tat gga act      864
   Trp Tyr His Ala Tyr Gln His Ile Tyr Leu Ala Val Leu Tyr Gly Thr
           275                 280                 285
   cta gct ctt aag agt att ttt cta gat gat ttc ctt gcg tac ttc aca      912
   Leu Ala Leu Lys Ser Ile Phe Leu Asp Asp Phe Leu Ala Tyr Phe Thr
       290                 295                 300
   gga tca att ggc cct gtc aag gtg gcg aaa atg acc ccc ctg gag ttc      960
   Gly Ser Ile Gly Pro Val Lys Val Ala Lys Met Thr Pro Leu Glu Phe
   305                 310                 315                 320
   aac atc ttc ttt cag gga aag ctg cta tat gcg ttc tac atg ttc gtg     1008
   Asn Ile Phe Phe Gln Gly Lys Leu Leu Tyr Ala Phe Tyr Met Phe Val
                   325                 330                 335
   ttg cca tct gtg tac ggt gtt cac tcc gga gga act ttc ttg gca cta     1056
   Leu Pro Ser Val Tyr Gly Val His Ser Gly Gly Thr Phe Leu Ala Leu
               340                 345                 350
   tat gtg gct tct cag ctc att aca ggt tgg atg tta gct ttt ctt ttt     1104
   Tyr Val Ala Ser Gln Leu Ile Thr Gly Trp Met Leu Ala Phe Leu Phe
           355                 360                 365
   caa gta gca cat gtc gtg gat gat gtt gca ttt cct aca cca gaa ggt     1152
   Gln Val Ala His Val Val Asp Asp Val Ala Phe Pro Thr Pro Glu Gly
       370                 375                 380
   ggg aag gtg aag gga gga tgg gct gca atg cag gtt gca aca act acg     1200
   Gly Lys Val Lys Gly Gly Trp Ala Ala Met Gln Val Ala Thr Thr Thr
   385                 390                 395                 400
   gat ttc agt cca cgc tca tgg ttc tgg ggt cat gtc tct gga gga tta     1248
   Asp Phe Ser Pro Arg Ser Trp Phe Trp Gly His Val Ser Gly Gly Leu
                   405                 410                 415
   aac aac caa att gag cat cat ctg ttt cca gga gtg tgc cat gtt cat     1296
   Asn Asn Gln Ile Glu His His Leu Phe Pro Gly Val Cys His Val His
               420                 425                 430
   tat cca gcc att cag cct att gtc gag aag acg tgc aag gaa ttc gat     1344
   Tyr Pro Ala Ile Gln Pro Ile Val Glu Lys Thr Cys Lys Glu Phe Asp
           435                 440                     445
   gtg cct tat gta gcc tac cca act ttt tgg act gcg ttg aga gcc cac      1392
   Val Pro Tyr Val Ala Tyr Pro Thr Phe Trp Thr Ala Leu Arg Ala His
       450                 455                     460
   ttt gcg cat ttg aaa aag gtt gga ttg aca gag ttt cgg ctc gat ggc      1440
   Phe Ala His Leu Lys Lys Val Gly Leu Thr Glu Phe Arg Leu Asp Gly
   465                 470                     475                 480
   tga                                                                  1443
<210> 27
   <211> 480
   <212> PRT
   <213> Physcomitrella patens
<400> 27
   Met Ala Pro His Ser Ala Asp Thr Ala Gly Leu Val Pro Ser Asp Glu
   1               5                   10                  15
   Leu Arg Leu Arg Thr Ser Asn Ser Lys Gly Pro Glu Gln Glu Gln Thr
               20                  25                  30
   Leu Lys Lys Tyr Thr Leu Glu Asp Val Ser Arg His Asn Thr Pro Ala
           35                  40                  45
   Asp Cys Trp Leu Val Ile Trp Gly Lys Val Tyr Asp Val Thr Ser Trp
       50                  55                  60
   Ile Pro Asn His Pro Gly Gly Ser Leu Ile His Val Lys Ala Gly Gln
   65                  70                  75                  80
   Asp Ser Thr Gln Leu Phe Asp Ser Tyr His Pro Leu Tyr Val Arg Lys
                   85                  90                  95
   Met Leu Ala Lys Tyr Cys Ile Gly Glu Leu Val Pro Ser Ala Gly Asp
               100                 105                 110
   Asp Lys Phe Lys Lys Ala Thr Leu Glu Tyr Ala Asp Ala Glu Asn Glu
           115                 120                 125
   Asp Phe Tyr Leu Val Val Lys Gln Arg Val Glu Ser Tyr Phe Lys Ser
       130                 135                 140
   Asn Lys Ile Asn Pro Gln Ile His Pro His Met Ile Leu Lys Ser Leu
   145                 150                 155                 160
   Phe Ile Leu Gly Gly Tyr Phe Ala Ser Tyr Tyr Leu Ala Phe Phe Trp
                   165                 170                 175
   Ser Ser Ser Val Leu Val Ser Leu Phe Phe Ala Leu Trp Met Gly Phe
               180                 185                 190
   Phe Ala Ala Glu Val Gly Val Ser Ile Gln His Asp Gly Asn His Gly
   195 200 205
   Ser Tyr Thr Lys Trp Arg Gly Phe Gly Tyr Ile Met Gly Ala Ser Leu
   210 215 220
   Asp Leu Val Gly Ala Ser Ser Phe Met Trp Arg Gln Gln His Val Val
   225 230 235 240
   Gly His His Ser Phe Thr Asn Val Asp Asn Tyr Asp Pro Asp Ile Arg
   245 250 255
   Val Lys Asp Pro Asp Val Arg Arg Val Ala Thr Thr Gln Pro Arg Gln
   260 265 270
   Trp Tyr His Ala Tyr Gln His Ile Tyr Leu Ala Val Leu Tyr Gly Thr
   275 280 285
   Leu Ala Leu Lys Ser Ile Phe Leu Asp Asp Phe Leu Ala Tyr Phe Thr
   290 295 300
   Gly Ser Ile Gly Pro Val Lys Val Ala Lys Met Thr Pro Leu Glu Phe
   305 310 315 320
   Asn Ile Phe Phe Gln Gly Lys Leu Leu Tyr Ala Phe Tyr Met Phe Val
   325 330 335
   Leu Pro Ser Val Tyr Gly Val His Ser Gly Gly Thr Phe Leu Ala Leu
   340 345 350
   Tyr Val Ala Ser Gln Leu Ile Thr Gly Trp Met Leu Ala Phe Leu Phe
   355 360 365
   Gln Val Ala His Val Val Asp Asp Val Ala Phe Pro Thr Pro Glu Gly
   370 375 380
   Gly Lys Val Lys Gly Gly Trp Ala Ala Met Gln Val Ala Thr Thr Thr
   385 390 395 400
   Asp Phe Ser Pro Arg Ser Trp Phe Trp Gly His Val Ser Gly Gly Leu
   405 410 415
   Asn Asn Gln Ile Glu His His Leu Phe Pro Gly Val Cys His Val His
   420 425 430
   Tyr Pro Ala Ile Gln Pro Ile Val Glu Lys Thr Cys Lys Glu Phe Asp
   435 440 445
   Val Pro Tyr Val Ala Tyr Pro Thr Phe Trp Thr Ala Leu Arg Ala His
   450 455 460
   Phe Ala His Leu Lys Lys Val Gly Leu Thr Glu Phe Arg Leu Asp Gly
   465 470 475 480
<210> 28
   <211> 1320
   <212> DNA
   <213> Thraustochytrium
<220>
   <221> CDS
   <222> (1)..(1320)
<400> 28
   atg ggc aag ggc agc gag ggc cgc agc gcg gcg cgc gag atg acg gcc      48
   Met Gly Lys Gly Ser Glu Gly Arg Ser Ala Ala Arg Glu Met Thr Ala
   1               5                   10                  15
   gag gcg aac ggc gac aag cgg aaa acg att ctg atc gag ggc gtc ctg      96
   Glu Ala Asn Gly Asp Lys Arg Lys Thr Ile Leu Ile Glu Gly Val Leu
               20                  25                  30
   tac gac gcg acg aac ttt aag cac ccg ggc ggt tcg atc atc aac ttc     144
   Tyr Asp Ala Thr Asn Phe Lys His Pro Gly Gly Ser Ile Ile Asn Phe
           35                  40                  45
   ttg acc gag ggc gag gcc ggc gtg gac gcg acg cag gcg tac cgc gag     192
   Leu Thr Glu Gly Glu Ala Gly Val Asp Ala Thr Gln Ala Tyr Arg Glu
       50                  55                  60
   ttt cat cag cgg tcc ggc aag gcc gac aag tac ctc aag tcg ctg ccg     240
   Phe His Gln Arg Ser Gly Lys Ala Asp Lys Tyr Leu Lys Ser Leu Pro
   65                  70                  75                  80
   aag ctg gat gcg tcc aag gtg gag tcg cgg ttc tcg gcc aaa gag cag     288
   Lys Leu Asp Ala Ser Lys Val Glu Ser Arg Phe Ser Ala Lys Glu Gln
                   85                  90                  95
   gcg cgg cgc gac gcc atg acg cgc gac tac gcg gcc ttt cgc gag gag     336
   Ala Arg Arg Asp Ala Met Thr Arg Asp Tyr Ala Ala Phe Arg Glu Glu
               100                 105                 110
   ctc gtc gcc gag ggg tac ttt gac ccg tcg atc ccg cac atg att tac     384
   Leu Val Ala Glu Gly Tyr Phe Asp Pro Ser Ile Pro His Met Ile Tyr
           115                 120                 125
   cgc gtc gtg gag atc gtg gcg ctc ttc gcg ctc tcg ttc tgg ctc atg     432
   Arg Val Val Glu Ile Val Ala Leu Phe Ala Leu Ser Phe Trp Leu Met
       130                 135                 140
   tcc aag gcc tcg ccc acc tcg ctc gtg ctg ggc gtg gtg atg aac ggc     480
   Ser Lys Ala Ser Pro Thr Ser Leu Val Leu Gly Val Val Met Asn Gly
   145                 150                 155                 160
   att gcg cag ggc cgc tgc ggc tgg gtc atg cac gag atg ggc cac ggg     528
   Ile Ala Gln Gly Arg Cys Gly Trp Val Met His Glu Met Gly His Gly
                   165                 170                 175
   tcg ttc acg ggc gtc atc tgg ctc gac gac cgg atg tgc gag ttc ttc     576
   Ser Phe Thr Gly Val Ile Trp Leu Asp Asp Arg Met Cys Glu Phe Phe
               180                 185                 190
   tac ggc gtc ggc tgc ggc atg agc ggg cac tac tgg aag aac cag cac     624
   Tyr Gly Val Gly Cys Gly Met Ser Gly His Tyr Trp Lys Asn Gln His
           195                 200                 205
   agc aag cac cac gcc gcg ccc aac cgc ctc gag cac gat gtc gat ctc      672
   Ser Lys His His Ala Ala Pro Asn Arg Leu Glu His Asp Val Asp Leu
       210                 215                 220
   aac acg ctg ccc ctg gtc gcc ttt aac gag cgc gtc gtg cgc aag gtc      720
   Asn Thr Leu Pro Leu Val Ala Phe Asn Glu Arg Val Val Arg Lys Val
   225                 230                 235                 240
   aag ccg gga tcg ctg ctg gcg ctc tgg ctg cgc gtg cag gcg tac ctc      768
   Lys Pro Gly Ser Leu Leu Ala Leu Trp Leu Arg Val Gln Ala Tyr Leu
                   245                 250                 255
   ttt gcg ccc gtc tcg tgc ctg ctc atc ggc ctt ggc tgg acg ctc tac      816
   Phe Ala Pro Val Ser Cys Leu Leu Ile Gly Leu Gly Trp Thr Leu Tyr
               260                 265                 270
   ctg cac ccg cgc tac atg ctg cgc acc aag cgg cac atg gag ttc gtc      864
   Leu His Pro Arg Tyr Met Leu Arg Thr Lys Arg His Met Glu Phe Val
           275                 280                 285
   tgg atc ttc gcg cgc tac att ggc tgg ttc tcg ctc atg ggc gct ctc      912
   Trp Ile Phe Ala Arg Tyr Ile Gly Trp Phe Ser Leu Met Gly Ala Leu
       290                 295                 300
   ggc tac tcg ccg ggc acc tcg gtc ggg atg tac ctg tgc tcg ttc ggc      960
   Gly Tyr Ser Pro Gly Thr Ser Val Gly Met Tyr Leu Cys Ser Phe Gly
   305                 310                 315                 320
   ctc ggc tgc att tac att ttc ctg cag ttc gcc gtc agc cac acg cac     1008
   Leu Gly Cys Ile Tyr Ile Phe Leu Gln Phe Ala Val Ser His Thr His
                   325                 330                 335
   ctg ccg gtg acc aac ccg gag gac cag ctg cac tgg ctc gag tac gcg     1056
   Leu Pro Val Thr Asn Pro Glu Asp Gln Leu His Trp Leu Glu Tyr Ala
               340                 345                 350
   gcc gac cac acg gtg aac att agc acc aag tcc tgg ctc gtc acg tgg     1104
   Ala Asp His Thr Val Asn Ile Ser Thr Lys Ser Trp Leu Val Thr Trp
           355                 360                 365
   tgg atg tcg aac ctg aac ttt cag atc gag cac cac ctc ttc ccc acg     1152
   Trp Met Ser Asn Leu Asn Phe Gln Ile Glu His His Leu Phe Pro Thr
       370                 375                 380
   gcg ccg cag ttc cgc ttc aag gaa atc agt cct cgc gtc gag gcc ctc     1200
   Ala Pro Gln Phe Arg Phe Lys Glu Ile Ser Pro Arg Val Glu Ala Leu
   385                 390                 395                 400
   ttc aag cgc cac aac ctc ccg tac tac gac ctg ccc tac acg agc gcg     1248
   Phe Lys Arg His Asn Leu Pro Tyr Tyr Asp Leu Pro Tyr Thr Ser Ala
                   405                 410                 415
   gtc tcg acc acc ttt gcc aat ctt tat tcc gtc ggc cac tcg gtc ggc     1296
   Val Ser Thr Thr Phe Ala Asn Leu Tyr Ser Val Gly His Ser Val Gly
               420                 425                 430
   gcc gac acc aag aag cag gac tga                                     1320
   Ala Asp Thr Lys Lys Gln Asp
           435
<210> 29
   <211> 439
   <212> PRT
   <213> Thraustochytrium
<400> 29
   Met Gly Lys Gly Ser Glu Gly Arg Ser Ala Ala Arg Glu Met Thr Ala
   1               5                   10                  15
   Glu Ala Asn Gly Asp Lys Arg Lys Thr Ile Leu Ile Glu Gly Val Leu
               20                  25                  30
   Tyr Asp Ala Thr Asn Phe Lys His Pro Gly Gly Ser Ile Ile Asn Phe
           35                  40                  45
   Leu Thr Glu Gly Glu Ala Gly Val Asp Ala Thr Gln Ala Tyr Arg Glu
       50                  55                  60
   Phe His Gln Arg Ser Gly Lys Ala Asp Lys Tyr Leu Lys Ser Leu Pro
   65                  70                  75                  80
   Lys Leu Asp Ala Ser Lys Val Glu Ser Arg Phe Ser Ala Lys Glu Gln
                   85                  90                  95
   Ala Arg Arg Asp Ala Met Thr Arg Asp Tyr Ala Ala Phe Arg Glu Glu
               100                 105                 110
   Leu Val Ala Glu Gly Tyr Phe Asp Pro Ser Ile Pro His Met Ile Tyr
           115                 120                 125
   Arg Val Val Glu Ile Val Ala Leu Phe Ala Leu Ser Phe Trp Leu Met
       130                 135                 140
   Ser Lys Ala Ser Pro Thr Ser Leu Val Leu Gly Val Val Met Asn Gly
   145                 150                 155                 160
   Ile Ala Gln Gly Arg Cys Gly Trp Val Met His Glu Met Gly His Gly
                   165                 170                 175
   Ser Phe Thr Gly Val Ile Trp Leu Asp Asp Arg Met Cys Glu Phe Phe
               180                 185                 190
   Tyr Gly Val Gly Cys Gly Met Ser Gly His Tyr Trp Lys Asn Gln His
           195                 200                 205
   Ser Lys His His Ala Ala Pro Asn Arg Leu Glu His Asp Val Asp Leu
       210                 215                 220
   Asn Thr Leu Pro Leu Val Ala Phe Asn Glu Arg Val Val Arg Lys Val
   225                 230                 235                 240
   Lys Pro Gly Ser Leu Leu Ala Leu Trp Leu Arg Val Gln Ala Tyr Leu
                   245                 250                 255
   Phe Ala Pro Val Ser Cys Leu Leu Ile Gly Leu Gly Trp Thr Leu Tyr
               260                 265                 270
   Leu His Pro Arg Tyr Met Leu Arg Thr Lys Arg His Met Glu Phe Val
   275 280 285
   Trp Ile Phe Ala Arg Tyr Ile Gly Trp Phe Ser Leu Met Gly Ala Leu
   290 295 300
   Gly Tyr Ser Pro Gly Thr Ser Val Gly Met Tyr Leu Cys Ser Phe Gly
   305 310 315 320
   Leu Gly Cys Ile Tyr Ile Phe Leu Gln Phe Ala Val Ser His Thr His
   325 330 335
   Leu Pro Val Thr Asn Pro Glu Asp Gln Leu His Trp Leu Glu Tyr Ala
   340 345 350
   Ala Asp His Thr Val Asn Ile Ser Thr Lys Ser Trp Leu Val Thr Trp
   355 360 365
   Trp Met Ser Asn Leu Asn Phe Gln Ile Glu His His Leu Phe Pro Thr
   370 375 380
   Ala Pro Gln Phe Arg Phe Lys Glu Ile Ser Pro Arg Val Glu Ala Leu
   385 390 395 400
   Phe Lys Arg His Asn Leu Pro Tyr Tyr Asp Leu Pro Tyr Thr Ser Ala
   405 410 415
   Val Ser Thr Thr Phe Ala Asn Leu Tyr Ser Val Gly His Ser Val Gly
   420 425 430
   Ala Asp Thr Lys Lys Gln Asp
   435
<210> 30
   <211> 1341
   <212> DNA
   <213> Mortierella alpina
<220>
   <221> CDS
   <222> (1)..(1341)
   <223> delta-5-Desaturase
<400> 30
   atg gga acg gac caa gga aaa acc ttc acc tgg gaa gag ctg gcg gcc      48
   Met Gly Thr Asp Gln Gly Lys Thr Phe Thr Trp Glu Glu Leu Ala Ala
   1               5                   10                  15
   cat aac acc aag gac gac cta ctc ttg gcc atc cgc ggc agg gtg tac      96
   His Asn Thr Lys Asp Asp Leu Leu Leu Ala Ile Arg Gly Arg Val Tyr
               20                  25                  30
   gat gtc aca aag ttc ttg agc cgc cat cct ggt gga gtg gac act ctc     144
   Asp Val Thr Lys Phe Leu Ser Arg His Pro Gly Gly Val Asp Thr Leu
           35                  40                  45
   ctg ctc gga gct ggc cga gat gtt act ccg gtc ttt gag atg tat cac      192
   Leu Leu Gly Ala Gly Arg Asp Val Thr Pro Val Phe Glu Met Tyr His
       50                  55                  60
   gcg ttt ggg gct gca gat gcc att atg aag aag tac tat gtc ggt aca      240
   Ala Phe Gly Ala Ala Asp Ala Ile Met Lys Lys Tyr Tyr Val Gly Thr
   65                  70                  75                  80
   ctg gtc tcg aat gag ctg ccc atc ttc ccg gag cca acg gtg ttc cac      288
   Leu Val Ser Asn Glu Leu Pro Ile Phe Pro Glu Pro Thr Val Phe His
                   85                  90                  95
   aaa acc atc aag acg aga gtc gag ggc tac ttt acg gat cgg aac att      336
   Lys Thr Ile Lys Thr Arg Val Glu Gly Tyr Phe Thr Asp Arg Asn Ile
               100                 105                 110
   gat ccc aag aat aga cca gag atc tgg gga cga tac gct ctt atc ttt      384
   Asp Pro Lys Asn Arg Pro Glu Ile Trp Gly Arg Tyr Ala Leu Ile Phe
           115                 120                 125
   gga tcc ttg atc gct tcc tac tac gcg cag ctc ttt gtg cct ttc gtt      432
   Gly Ser Leu Ile Ala Ser Tyr Tyr Ala Gln Leu Phe Val Pro Phe Val
       130                 135                 140
   gtc gaa cgc aca tgg ctt cag gtg gtg ttt gca atc atc atg gga ttt      480
   Val Glu Arg Thr Trp Leu Gln Val Val Phe Ala Ile Ile Met Gly Phe
   145                 150                 155                 160
   gcg tgc gca caa gtc gga ctc aac cct ctt cat gat gcg tct cac ttt      528
   Ala Cys Ala Gln Val Gly Leu Asn Pro Leu His Asp Ala Ser His Phe
                   165                 170                 175
   tca gtg acc cac aac ccc act gtc tgg aag att ctg gga gcc acg cac      576
   Ser Val Thr His Asn Pro Thr Val Trp Lys Ile Leu Gly Ala Thr His
               180                 185                 190
   gac ttt ttc aac gga gca tcg tac ctg gtg tgg atg tac caa cat atg      624
   Asp Phe Phe Asn Gly Ala Ser Tyr Leu Val Trp Met Tyr Gln His Met
           195                 200                 205
   ctc ggc cat cac ccc tac acc aac att gct gga gca gat ccc gac gtg      672
   Leu Gly His His Pro Tyr Thr Asn Ile Ala Gly Ala Asp Pro Asp Val
       210                 215                 220
   tcg acg tct gag ccc gat gtt cgt cgt atc aag ccc aac caa aag tgg      720
   Ser Thr Ser Glu Pro Asp Val Arg Arg Ile Lys Pro Asn Gln Lys Trp
   225                 230                 235                 240
   ttt gtc aac cac atc aac cag cac atg ttt gtt cct ttc ctg tac gga      768
   Phe Val Asn His Ile Asn Gln His Met Phe Val Pro Phe Leu Tyr Gly
                   245                 250                 255
   ctg ctg gcg ttc aag gtg cgc att cag gac atc aac att ttg tac ttt      816
   Leu Leu Ala Phe Lys Val Arg Ile Gln Asp Ile Asn Ile Leu Tyr Phe
               260                 265                 270
   gtc aag acc aat gac gct att cgt gtc aat ccc atc tcg aca tgg cac      864
   Val Lys Thr Asn Asp Ala Ile Arg Val Asn Pro Ile Ser Thr Trp His
           275                 280                 285
   act gtg atg ttc tgg ggc ggc aag gct ttc ttt gtc tgg tat cgc ctg      912
   Thr Val Met Phe Trp Gly Gly Lys Ala Phe Phe Val Trp Tyr Arg Leu
       290                 295                 300
   att gtt ccc ctg cag tat ctg ccc ctg ggc aag gtg ctg ctc ttg ttc      960
   Ile Val Pro Leu Gln Tyr Leu Pro Leu Gly Lys Val Leu Leu Leu Phe
   305                 310                 315                 320
   acg gtc gcg gac atg gtg tcg tct tac tgg ctg gcg ctg acc ttc cag      1008
   Thr Val Ala Asp Met Val Ser Ser Tyr Trp Leu Ala Leu Thr Phe Gln
                   325                 330                 335
   gcg aac cac gtt gtt gag gaa gtt cag tgg ccg ttg cct gac gag aac      1056
   Ala Asn His Val Val Glu Glu Val Gln Trp Pro Leu Pro Asp Glu Asn
               340                 345                 350
   ggg atc atc caa aag gac tgg gca gct atg cag gtc gag act acg cag      1104
   Gly Ile Ile Gln Lys Asp Trp Ala Ala Met Gln Val Glu Thr Thr Gln
           355                 360                 365
   gat tac gca cac gat tcg cac ctc tgg acc agc atc act ggc agc ttg      1152
   Asp Tyr Ala His Asp Ser His Leu Trp Thr Ser Ile Thr Gly Ser Leu
       370                 375                 380
   aac tac cag gct gtg cac cat ctg ttc ccc aac gtg tcg cag cac cat      1200
   Asn Tyr Gln Ala Val His His Leu Phe Pro Asn Val Ser Gln His His
   385                 390                 395                 400
   tat ccc gat att ctg gcc atc atc aag aac acc tgc agc gag tac aag      1248
   Tyr Pro Asp Ile Leu Ala Ile Ile Lys Asn Thr Cys Ser Glu Tyr Lys
                   405                 410                 415
   gtt cca tac ctt gtc aag gat acg ttt tgg caa gca ttt gct tca cat      1296
   Val Pro Tyr Leu Val Lys Asp Thr Phe Trp Gln Ala Phe Ala Ser His
               420                 425                 430
   ttg gag cac ttg cgt gtt ctt gga ctc cgt ccc aag gaa gag tag          1341
   Leu Glu His Leu Arg Val Leu Gly Leu Arg Pro Lys Glu Glu
           435                 440                 445
<210> 31
   <211> 446
   <212> PRT
   <213> Mortierella alpina
<400> 31
   Met Gly Thr Asp Gln Gly Lys Thr Phe Thr Trp Glu Glu Leu Ala Ala
   1               5                   10                  15
   His Asn Thr Lys Asp Asp Leu Leu Leu Ala Ile Arg Gly Arg Val Tyr
               20                  25                  30
   Asp Val Thr Lys Phe Leu Ser Arg His Pro Gly Gly Val Asp Thr Leu
           35                  40                  45
   Leu Leu Gly Ala Gly Arg Asp Val Thr Pro Val Phe Glu Met Tyr His
       50                  55                  60
   Ala Phe Gly Ala Ala Asp Ala Ile Met Lys Lys Tyr Tyr Val Gly Thr
   65                  70                  75                  80
   Leu Val Ser Asn Glu Leu Pro Ile Phe Pro Glu Pro Thr Val Phe His
                   85                  90                  95
   Lys Thr Ile Lys Thr Arg Val Glu Gly Tyr Phe Thr Asp Arg Asn Ile
               100                 105                 110
   Asp Pro Lys Asn Arg Pro Glu Ile Trp Gly Arg Tyr Ala Leu Ile Phe
           115                 120                 125
   Gly Ser Leu Ile Ala Ser Tyr Tyr Ala Gln Leu Phe Val Pro Phe Val
       130                 135                 140
   Val Glu Arg Thr Trp Leu Gln Val Val Phe Ala Ile Ile Met Gly Phe
   145                 150                 155                 160
   Ala Cys Ala Gln Val Gly Leu Asn Pro Leu His Asp Ala Ser His Phe
                   165                 170                 175
   Ser Val Thr His Asn Pro Thr Val Trp Lys Ile Leu Gly Ala Thr His
               180                 185                 190
   Asp Phe Phe Asn Gly Ala Ser Tyr Leu Val Trp Met Tyr Gln His Met
           195                 200                 205
   Leu Gly His His Pro Tyr Thr Asn Ile Ala Gly Ala Asp Pro Asp Val
       210                 215                 220
   Ser Thr Ser Glu Pro Asp Val Arg Arg Ile Lys Pro Asn Gln Lys Trp
   225                 230                 235                 240
   Phe Val Asn His Ile Asn Gln His Met Phe Val Pro Phe Leu Tyr Gly
                   245                 250                 255
   Leu Leu Ala Phe Lys Val Arg Ile Gln Asp Ile Asn Ile Leu Tyr Phe
               260                 265                 270
   Val Lys Thr Asn Asp Ala Ile Arg Val Asn Pro Ile Ser Thr Trp His
           275                 280                 285
   Thr Val Met Phe Trp Gly Gly Lys Ala Phe Phe Val Trp Tyr Arg Leu
       290                 295                 300
   Ile Val Pro Leu Gln Tyr Leu Pro Leu Gly Lys Val Leu Leu Leu Phe
   305                 310                 315                 320
   Thr Val Ala Asp Met Val Ser Ser Tyr Trp Leu Ala Leu Thr Phe Gln
                   325                 330                 335
   Ala Asn His Val Val Glu Glu Val Gln Trp Pro Leu Pro Asp Glu Asn
               340                 345                 350
   Gly Ile Ile Gln Lys Asp Trp Ala Ala Met Gln Val Glu Thr Thr Gln
           355                 360                 365
   Asp Tyr Ala His Asp Ser His Leu Trp Thr Ser Ile Thr Gly Ser Leu
       370                 375                 380
   Asn Tyr Gln Ala Val His His Leu Phe Pro Asn Val Ser Gln His His
   385                 390                 395                 400
   Tyr Pro Asp Ile Leu Ala Ile Ile Lys Asn Thr Cys Ser Glu Tyr Lys
                   405                 410                 415
   Val Pro Tyr Leu Val Lys Asp Thr Phe Trp Gln Ala Phe Ala Ser His
               420                 425                 430
   Leu Glu His Leu Arg Val Leu Gly Leu Arg Pro Lys Glu Glu
           435                 440                 445
<210> 32
   <211> 1344
   <212> DNA
   <213> Caenorhabditis elegans
<220>
   <221> CDS
   <222> (1)..(1344)
   <223> delta-5-Desaturase
<400> 32
   atg gta tta cga gag caa gag cat gag cca ttc ttc att aaa att gat      48
   Met Val Leu Arg Glu Gln Glu His Glu Pro Phe Phe Ile Lys Ile Asp
   1               5                   10                  15
   gga aaa tgg tgt caa att gac gat gct gtc ctg aga tca cat cca ggt      96
   Gly Lys Trp Cys Gln Ile Asp Asp Ala Val Leu Arg Ser His Pro Gly
               20                  25                  30
   ggt agt gca att act acc tat aaa aat atg gat gcc act acc gta ttc      144
   Gly Ser Ala Ile Thr Thr Tyr Lys Asn Met Asp Ala Thr Thr Val Phe
           35                  40                  45
   cac aca ttc cat act ggt tct aaa gaa gcg tat caa tgg ctg aca gaa      192
   His Thr Phe His Thr Gly Ser Lys Glu Ala Tyr Gln Trp Leu Thr Glu
       50                  55                  60
   ttg aaa aaa gag tgc cct aca caa gaa cca gag atc cca gat att aag      240
   Leu Lys Lys Glu Cys Pro Thr Gln Glu Pro Glu Ile Pro Asp Ile Lys
   65                  70                  75                  80
   gat gac cca atc aaa gga att gat gat gtg aac atg gga act ttc aat      288
   Asp Asp Pro Ile Lys Gly Ile Asp Asp Val Asn Met Gly Thr Phe Asn
                   85                  90                  95
   att tct gag aaa cga tct gcc caa ata aat aaa agt ttc act gat cta      336
   Ile Ser Glu Lys Arg Ser Ala Gln Ile Asn Lys Ser Phe Thr Asp Leu
               100                 105                 110
   cgt atg cga gtt cgt gca gaa gga ctt atg gat gga tct cct ttg ttc      384
   Arg Met Arg Val Arg Ala Glu Gly Leu Met Asp Gly Ser Pro Leu Phe
           115                 120                 125
   tac att aga aaa att ctt gaa aca atc ttc aca att ctt ttt gca ttc      432
   Tyr Ile Arg Lys Ile Leu Glu Thr Ile Phe Thr Ile Leu Phe Ala Phe
       130                 135                 140
   tac ctt caa tac cac aca tat tat ctt cca tca gct att cta atg gga      480
   Tyr Leu Gln Tyr His Thr Tyr Tyr Leu Pro Ser Ala Ile Leu Met Gly
   145                 150                 155                 160
   gtt gcg tgg caa caa ttg gga tgg tta atc cat gaa ttc gca cat cat      528
   Val Ala Trp Gln Gln Leu Gly Trp Leu Ile His Glu Phe Ala His His
                   165                 170                 175
   cag ttg ttc aaa aac aga tac tac aat gat ttg gcc agc tat ttc gtt      576
   Gln Leu Phe Lys Asn Arg Tyr Tyr Asn Asp Leu Ala Ser Tyr Phe Val
               180                 185                 190
   gga aac ttt tta caa gga ttc tca tct ggt ggt tgg aaa gag cag cac      624
   Gly Asn Phe Leu Gln Gly Phe Ser Ser Gly Gly Trp Lys Glu Gln His
           195                 200                 205
   aat gtg cat cac gca gcc aca aat gtt gtt gga cga gac gga gat ctt      672
   Asn Val His His Ala Ala Thr Asn Val Val Gly Arg Asp Gly Asp Leu
       210                 215                 220
   gat tta gtc cca ttc tat gct aca gtg gca gaa cat ctc aac aat tat      720
   Asp Leu Val Pro Phe Tyr Ala Thr Val Ala Glu His Leu Asn Asn Tyr
   225                 230                 235                 240
   tct cag gat tca tgg gtt atg act cta ttc aga tgg caa cat gtt cat      768
   Ser Gln Asp Ser Trp Val Met Thr Leu Phe Arg Trp Gln His Val His
                   245                 250                 255
   tgg aca ttc atg tta cca ttc ctc cgt ctc tcg tgg ctt ctt cag tca      816
   Trp Thr Phe Met Leu Pro Phe Leu Arg Leu Ser Trp Leu Leu Gln Ser
               260                 265                 270
   atc att ttt gtt agt cag atg cca act cat tat tat gac tat tac aga      864
   Ile Ile Phe Val Ser Gln Met Pro Thr His Tyr Tyr Asp Tyr Tyr Arg
           275                 280                 285
   aat act gcg att tat gaa cag gtt ggt ctc tct ttg cac tgg gct tgg      912
   Asn Thr Ala Ile Tyr Glu Gln Val Gly Leu Ser Leu His Trp Ala Trp
       290                 295                 300
   tca ttg ggt caa ttg tat ttc cta ccc gat tgg tca act aga ata atg      960
   Ser Leu Gly Gln Leu Tyr Phe Leu Pro Asp Trp Ser Thr Arg Ile Met
   305                 310                 315                 320
   ttc ttc ctt gtt tct cat ctt gtt gga ggt ttc ctg ctc tct cat gta     1008
   Phe Phe Leu Val Ser His Leu Val Gly Gly Phe Leu Leu Ser His Val
                   325                 330                 335
   gtt act ttc aat cat tat tca gtg gag aag ttt gca ttg agc tcg aac     1056
   Val Thr Phe Asn His Tyr Ser Val Glu Lys Phe Ala Leu Ser Ser Asn
               340                 345                 350
   atc atg tca aat tac gct tgt ctt caa atc atg acc aca aga aat atg     1104
   Ile Met Ser Asn Tyr Ala Cys Leu Gln Ile Met Thr Thr Arg Asn Met
           355                 360                 365
   aga cct gga aga ttc att gac tgg ctt tgg gga ggt ctt aac tat cag     1152
   Arg Pro Gly Arg Phe Ile Asp Trp Leu Trp Gly Gly Leu Asn Tyr Gln
       370                 375                 380
   att gag cac cat ctt ttc cca acg atg cca cga cac aac ttg aac act     1200
   Ile Glu His His Leu Phe Pro Thr Met Pro Arg His Asn Leu Asn Thr
   385                 390                 395                 400
   gtt atg cca ctt gtt aag gag ttt gca gca gca aat ggt tta cca tac     1248
   Val Met Pro Leu Val Lys Glu Phe Ala Ala Ala Asn Gly Leu Pro Tyr
                   405                 410                 415
   atg gtc gac gat tat ttc aca gga ttc tgg ctt gaa att gag caa ttc     1296
   Met Val Asp Asp Tyr Phe Thr Gly Phe Trp Leu Glu Ile Glu Gln Phe
               420                 425                 430
   cga aat att gca aat gtt gct gct aaa ttg act aaa aag att gcc tag      1344
   Arg Asn Ile Ala Asn Val Ala Ala Lys Leu Thr Lys Lys Ile Ala
           435                 440                     445
<210> 33
   <211> 447
   <212> PRT
   <213> Caenorhabditis elegans
<400> 33
   Met Val Leu Arg Glu Gln Glu His Glu Pro Phe Phe Ile Lys Ile Asp
   1               5                   10                  15
   Gly Lys Trp Cys Gln Ile Asp Asp Ala Val Leu Arg Ser His Pro Gly
               20                  25                  30
   Gly Ser Ala Ile Thr Thr Tyr Lys Asn Met Asp Ala Thr Thr Val Phe
           35                  40                  45
   His Thr Phe His Thr Gly Ser Lys Glu Ala Tyr Gln Trp Leu Thr Glu
       50                  55                  60
   Leu Lys Lys Glu Cys Pro Thr Gln Glu Pro Glu Ile Pro Asp Ile Lys
   65                  70                  75                  80
   Asp Asp Pro Ile Lys Gly Ile Asp Asp Val Asn Met Gly Thr Phe Asn
                   85                  90                  95
   Ile Ser Glu Lys Arg Ser Ala Gln Ile Asn Lys Ser Phe Thr Asp Leu
               100                 105                 110
   Arg Met Arg Val Arg Ala Glu Gly Leu Met Asp Gly Ser Pro Leu Phe
           115                 120                 125
   Tyr Ile Arg Lys Ile Leu Glu Thr Ile Phe Thr Ile Leu Phe Ala Phe
       130                 135                 140
   Tyr Leu Gln Tyr His Thr Tyr Tyr Leu Pro Ser Ala Ile Leu Met Gly
   145                 150                 155                 160
   Val Ala Trp Gln Gln Leu Gly Trp Leu Ile His Glu Phe Ala His His
                   165                 170                 175
   Gln Leu Phe Lys Asn Arg Tyr Tyr Asn Asp Leu Ala Ser Tyr Phe Val
               180                 185                 190
   Gly Asn Phe Leu Gln Gly Phe Ser Ser Gly Gly Trp Lys Glu Gln His
           195                 200                 205
   Asn Val His His Ala Ala Thr Asn Val Val Gly Arg Asp Gly Asp Leu
       210                 215                 220
   Asp Leu Val Pro Phe Tyr Ala Thr Val Ala Glu His Leu Asn Asn Tyr
   225 230 235 240
   Ser Gln Asp Ser Trp Val Met Thr Leu Phe Arg Trp Gln His Val His
   245 250 255
   Trp Thr Phe Met Leu Pro Phe Leu Arg Leu Ser Trp Leu Leu Gln Ser
   260 265 270
   Ile Ile Phe Val Ser Gln Met Pro Thr His Tyr Tyr Asp Tyr Tyr Arg
   275 280 285
   Asn Thr Ala Ile Tyr Glu Gln Val Gly Leu Ser Leu His Trp Ala Trp
   290 295 300
   Ser Leu Gly Gln Leu Tyr Phe Leu Pro Asp Trp Ser Thr Arg Ile Met
   305 310 315 320
   Phe Phe Leu Val Ser His Leu Val Gly Gly Phe Leu Leu Ser His Val
   325 330 335
   Val Thr Phe Asn His Tyr Ser Val Glu Lys Phe Ala Leu Ser Ser Asn
   340 345 350
   Ile Met Ser Asn Tyr Ala Cys Leu Gln Ile Met Thr Thr Arg Asn Met
   355 360 365
   Arg Pro Gly Arg Phe Ile Asp Trp Leu Trp Gly Gly Leu Asn Tyr Gln
   370 375 380
   Ile Glu His His Leu Phe Pro Thr Met Pro Arg His Asn Leu Asn Thr
   385 390 395 400
   Val Met Pro Leu Val Lys Glu Phe Ala Ala Ala Asn Gly Leu Pro Tyr
   405 410 415
   Met Val Asp Asp Tyr Phe Thr Gly Phe Trp Leu Glu Ile Glu Gln Phe
   420 425 430
   Arg Asn Ile Ala Asn Val Ala Ala Lys Leu Thr Lys Lys Ile Ala
   435 440 445
<210> 34
   <211> 606
   <212> DNA
   <213> Ostreococcus tauri
<220>
   <221> CDS
   <222> (1)..(606)
   <223> delta-5-Desaturase
<400> 34
   atg tac ggt ttg cta tcg ctc aag tcg tgc ttc gtc gac gat ttc aac      48
   Met Tyr Gly Leu Leu Ser Leu Lys Ser Cys Phe Val Asp Asp Phe Asn
   1               5                   10                  15
   gcc tac ttc tcc gga cgc atc ggc tgg gtc aag gtg atg aag ttc acc      96
   Ala Tyr Phe Ser Gly Arg Ile Gly Trp Val Lys Val Met Lys Phe Thr
               20                  25                  30
   cgc ggc gag gcg atc gca ttt tgg ggc acc aag ctc ttg tgg gcc gcg      144
   Arg Gly Glu Ala Ile Ala Phe Trp Gly Thr Lys Leu Leu Trp Ala Ala
           35                  40                  45
   tat tac ctc gcg ttg ccg cta aag atg tcg cat cgg ccg ctc gga gaa      192
   Tyr Tyr Leu Ala Leu Pro Leu Lys Met Ser His Arg Pro Leu Gly Glu
       50                  55                  60
   ctc ctc gca ctc tgg gcc gtc acc gag ttc gtc acc gga tgg ctg ttg      240
   Leu Leu Ala Leu Trp Ala Val Thr Glu Phe Val Thr Gly Trp Leu Leu
   65                  70                  75                  80
   gcg ttc atg ttc caa gtc gcc cac gtc gtc ggc gag gtt cac ttc ttc      288
   Ala Phe Met Phe Gln Val Ala His Val Val Gly Glu Val His Phe Phe
                   85                  90                  95
   acc ctc gac gcg aag aac cgc gtg aac ttg gga tgg gga gag gca cag      336
   Thr Leu Asp Ala Lys Asn Arg Val Asn Leu Gly Trp Gly Glu Ala Gln
               100                 105                 110
   ctc atg tcg agc gcg gat ttc gcc cac gga tcc aag ttt tgg acg cac      384
   Leu Met Ser Ser Ala Asp Phe Ala His Gly Ser Lys Phe Trp Thr His
           115                 120                 125
   ttc tcc gga ggc tta aac tac caa gtc gtc cac cat ctc ttc ccg ggc      432
   Phe Ser Gly Gly Leu Asn Tyr Gln Val Val His His Leu Phe Pro Gly
       130                 135                 140
   gtc tgc cac gtg cac tat ccc gcg ctc gcg cca att att aag gcg gca      480
   Val Cys His Val His Tyr Pro Ala Leu Ala Pro Ile Ile Lys Ala Ala
   145                 150                 155                 160
   gct gag aag cac ggc ctc cac tac cag att tac ccc acg ttt tgg tcc      528
   Ala Glu Lys His Gly Leu His Tyr Gln Ile Tyr Pro Thr Phe Trp Ser
                   165                 170                 175
   gcc ctg cgc gcg cac ttc cgg cac ctc gcc aac gtc ggc cgc gcc gcg      576
   Ala Leu Arg Ala His Phe Arg His Leu Ala Asn Val Gly Arg Ala Ala
               180                 185                 190
   tac gta ccg tcc ctc caa acc gtc gga tga                              606
   Tyr Val Pro Ser Leu Gln Thr Val Gly
           195                 200
<210> 35
   <211> 201
   <212> PRT
   <213> Ostreococcus tauri
<400> 35
   Met Tyr Gly Leu Leu Ser Leu Lys Ser Cys Phe Val Asp Asp Phe Asn
   1 5 10 15
   Ala Tyr Phe Ser Gly Arg Ile Gly Trp Val Lys Val Met Lys Phe Thr
   20 25 30
   Arg Gly Glu Ala Ile Ala Phe Trp Gly Thr Lys Leu Leu Trp Ala Ala
   35 40 45
   Tyr Tyr Leu Ala Leu Pro Leu Lys Met Ser His Arg Pro Leu Gly Glu
   50 55 60
   Leu Leu Ala Leu Trp Ala Val Thr Glu Phe Val Thr Gly Trp Leu Leu
   65 70 75 80
   Ala Phe Met Phe Gln Val Ala His Val Val Gly Glu Val His Phe Phe
   85 90 95
   Thr Leu Asp Ala Lys Asn Arg Val Asn Leu Gly Trp Gly Glu Ala Gln
   100 105 110
   Leu Met Ser Ser Ala Asp Phe Ala His Gly Ser Lys Phe Trp Thr His
   115 120 125
   Phe Ser Gly Gly Leu Asn Tyr Gln Val Val His His Leu Phe Pro Gly
   130 135 140
   Val Cys His Val His Tyr Pro Ala Leu Ala Pro Ile Ile Lys Ala Ala
   145 150 155 160
   Ala Glu Lys His Gly Leu His Tyr Gln Ile Tyr Pro Thr Phe Trp Ser
   165 170 175
   Ala Leu Arg Ala His Phe Arg His Leu Ala Asn Val Gly Arg Ala Ala
   180 185 190
   Tyr Val Pro Ser Leu Gln Thr Val Gly
   195 200
<210> 36
   <211> 714
   <212> DNA
   <213> Ostreococcus tauri
<220>
   <221> CDS
   <222> (1)..(714)
   <223> delta-5-Desaturase
<400> 36
   atg gtg agc cat cac tcg tac tgt aac gac gcg gat ttg gat cag gat      48
   Met Val Ser His His Ser Tyr Cys Asn Asp Ala Asp Leu Asp Gln Asp
   1               5                   10                  15
   gtg tac acc gca ctg ccg ctc ctg cgc ctg gac ccg tct cag gag ttg      96
   Val Tyr Thr Ala Leu Pro Leu Leu Arg Leu Asp Pro Ser Gln Glu Leu
               20                  25                  30
   aag tgg ttt cat cga tac cag gcg ttt tac gcc ccg ctc atg tgg ccg     144
   Lys Trp Phe His Arg Tyr Gln Ala Phe Tyr Ala Pro Leu Met Trp Pro
           35                  40                  45
   ttt ttg tgg ctc gcg gcg cag ttt ggc gac gcg cag aac atc ctg atc     192
   Phe Leu Trp Leu Ala Ala Gln Phe Gly Asp Ala Gln Asn Ile Leu Ile
       50                  55                  60
   gac cga gcg tcg ccg ggc gtc gcg tac aag gga ttg atg gcg aac gag     240
   Asp Arg Ala Ser Pro Gly Val Ala Tyr Lys Gly Leu Met Ala Asn Glu
   65                  70                  75                  80
   gtc gcg ctg tac gtt ctc ggt aag gtt tta cac ttt ggt ctt ctc ctc     288
   Val Ala Leu Tyr Val Leu Gly Lys Val Leu His Phe Gly Leu Leu Leu
                   85                  90                  95
   ggc gtt cct gcg tac ttg cac gga ttg tcc aac gcg atc gtt cca ttc     336
   Gly Val Pro Ala Tyr Leu His Gly Leu Ser Asn Ala Ile Val Pro Phe
               100                 105                 110
   ttg gcg tac ggc gca ttc ggc tcc ttc gtc ctg tgc tgg ttc ttc atc     384
   Leu Ala Tyr Gly Ala Phe Gly Ser Phe Val Leu Cys Trp Phe Phe Ile
           115                 120                 125
   gtc agc cat aac ctc gaa gcg ctg aca ccc gtt aac ctt aac aag tcc     432
   Val Ser His Asn Leu Glu Ala Leu Thr Pro Val Asn Leu Asn Lys Ser
       130                 135                 140
   acg aag aac gac tgg ggg gcg tgg cag atc gag aca tcg gcg tct tgg     480
   Thr Lys Asn Asp Trp Gly Ala Trp Gln Ile Glu Thr Ser Ala Ser Trp
   145                 150                 155                 160
   ggc aac gcg ttc tgg agc ttc ttc tct gga ggt ctg aac ctg caa atc     528
   Gly Asn Ala Phe Trp Ser Phe Phe Ser Gly Gly Leu Asn Leu Gln Ile
                   165                 170                 175
   gag cac cac ctc ttc ccg ggc atg gcg cac aac ctg tac ccg aag atg     576
   Glu His His Leu Phe Pro Gly Met Ala His Asn Leu Tyr Pro Lys Met
               180                 185                 190
   gtg ccg atc atc aag gac gag tgt gcg aaa gcg ggc gtt cgc tac acc     624
   Val Pro Ile Ile Lys Asp Glu Cys Ala Lys Ala Gly Val Arg Tyr Thr
           195                 200                 205
   ggt tac ggt ggc tac acc ggc ctg ctc ccg atc acc cgc gac atg ttc     672
   Gly Tyr Gly Gly Tyr Thr Gly Leu Leu Pro Ile Thr Arg Asp Met Phe
       210                 215                 220
   tcc tac ctc cat aag tgt ggc cga acg gcg aaa cta gcc taa             714
   Ser Tyr Leu His Lys Cys Gly Arg Thr Ala Lys Leu Ala
   225                 230                 235
<210> 37
   <211> 237
   <212> PRT
   <213> Ostreococcus tauri
<400> 37
   Met Val Ser His His Ser Tyr Cys Asn Asp Ala Asp Leu Asp Gln Asp
   1               5                   10                  15
   Val Tyr Thr Ala Leu Pro Leu Leu Arg Leu Asp Pro Ser Gln Glu Leu
               20                  25                  30
   Lys Trp Phe His Arg Tyr Gln Ala Phe Tyr Ala Pro Leu Met Trp Pro
           35                  40                  45
   Phe Leu Trp Leu Ala Ala Gln Phe Gly Asp Ala Gln Asn Ile Leu Ile
   50 55 60
   Asp Arg Ala Ser Pro Gly Val Ala Tyr Lys Gly Leu Met Ala Asn Glu
   65 70 75 80
   Val Ala Leu Tyr Val Leu Gly Lys Val Leu His Phe Gly Leu Leu Leu
   85 90 95
   Gly Val Pro Ala Tyr Leu His Gly Leu Ser Asn Ala Ile Val Pro Phe
   100 105 110
   Leu Ala Tyr Gly Ala Phe Gly Ser Phe Val Leu Cys Trp Phe Phe Ile
   115 120 125
   Val Ser His Asn Leu Glu Ala Leu Thr Pro Val Asn Leu Asn Lys Ser
   130 135 140
   Thr Lys Asn Asp Trp Gly Ala Trp Gln Ile Glu Thr Ser Ala Ser Trp
   145 150 155 160
   Gly Asn Ala Phe Trp Ser Phe Phe Ser Gly Gly Leu Asn Leu Gln Ile
   165 170 175
   Glu His His Leu Phe Pro Gly Met Ala His Asn Leu Tyr Pro Lys Met
   180 185 190
   Val Pro Ile Ile Lys Asp Glu Cys Ala Lys Ala Gly Val Arg Tyr Thr
   195 200 205
   Gly Tyr Gly Gly Tyr Thr Gly Leu Leu Pro Ile Thr Arg Asp Met Phe
   210 215 220
   Ser Tyr Leu His Lys Cys Gly Arg Thr Ala Lys Leu Ala
   225 230 235
<210> 38
   <211> 1431
   <212> DNA
   <213> Thalassiosira pseudonana
<220>
   <221> CDS
   <222> (1)..(1431)
   <223> delta-5-Desaturase
<400> 38
   atg ccc ccc aac gcc gat atc tcc cgc atc cgc aac cgc atc ccc acc        48
   Met Pro Pro Asn Ala Asp Ile Ser Arg Ile Arg Asn Arg Ile Pro Thr
   1               5                   10                  15
   aaa aca ggt acc gtt gcc tct gcc gac aac aac gac ccc gcc acc caa        96
   Lys Thr Gly Thr Val Ala Ser Ala Asp Asn Asn Asp Pro Ala Thr Gln
               20                  25                  30
   tcc gtc cga acc ctc aaa tct ctc aag ggc aac gag gtc gtc atc aac 144
   Ser Val Arg Thr Leu Lys Ser Leu Lys Gly Asn Glu Val Val Ile Asn
   35 40 45
   ggc aca att tat gac att gct gac ttt gtc cat cct gga gga gag gtt 192
   Gly Thr Ile Tyr Asp Ile Ala Asp Phe Val His Pro Gly Gly Glu Val
   50 55 60
   gtc aag ttc ttt ggt ggg aat gat gtt act att cag tat aat atg att 240
   Val Lys Phe Phe Gly Gly Asn Asp Val Thr Ile Gln Tyr Asn Met Ile
   65 70 75 80
   cat ccg tat cat acg ggg aaa cat ctg gag aag atg aag gct gtt gga 288
   His Pro Tyr His Thr Gly Lys His Leu Glu Lys Met Lys Ala Val Gly
   85 90 95
   aag gtt gta gat tgg cag tcg gac tac aag ttc gac acc ccc ttt gaa 336
   Lys Val Val Asp Trp Gln Ser Asp Tyr Lys Phe Asp Thr Pro Phe Glu
   100 105 110
   cga gag atc aaa tca gaa gtg ttc aag atc gta cgt cgc ggg cgt gag 384
   Arg Glu Ile Lys Ser Glu Val Phe Lys Ile Val Arg Arg Gly Arg Glu
   115 120 125
   ttc ggc aca aca ggc tac ttc ctc cgt gcc ttt ttc tac atc gct ctc 432
   Phe Gly Thr Thr Gly Tyr Phe Leu Arg Ala Phe Phe Tyr Ile Ala Leu
   130 135 140
   ttc ttc acc atg caa tac act ttc gcc aca tgc acc acc ttc acc acc 480
   Phe Phe Thr Met Gln Tyr Thr Phe Ala Thr Cys Thr Thr Phe Thr Thr
   145 150 155 160
   tac gat cac tgg tat cag agt ggt gta ttc atc gca att gtg ttt ggt 528
   Tyr Asp His Trp Tyr Gln Ser Gly Val Phe Ile Ala Ile Val Phe Gly
   165 170 175
   att tca cag gca ttc att ggg ttg aat gtc cag cac gat gcc aat cac 576
   Ile Ser Gln Ala Phe Ile Gly Leu Asn Val Gln His Asp Ala Asn His
   180 185 190
   gga gct gcc agt aag cgt ccc tgg gtg aat gac ttg ttg gga ttt gga 624
   Gly Ala Ala Ser Lys Arg Pro Trp Val Asn Asp Leu Leu Gly Phe Gly
   195 200 205
   acg gat ttg att gga tct aac aaa tgg aat tgg atg gca cag cat tgg 672
   Thr Asp Leu Ile Gly Ser Asn Lys Trp Asn Trp Met Ala Gln His Trp
   210 215 220
   act cat cac gct tac act aac cat agt gag aag gat ccc gat agc ttc 720
   Thr His His Ala Tyr Thr Asn His Ser Glu Lys Asp Pro Asp Ser Phe
   225 230 235 240
   agc tcg gaa cct atg ttt gca ttc aat gac tat ccc att gga cac ccg 768
   Ser Ser Glu Pro Met Phe Ala Phe Asn Asp Tyr Pro Ile Gly His Pro
   245 250 255
   aag aga aag tgg tgg cat agg ttc cag gga ggg tac ttc ctc ttc atg 816
   Lys Arg Lys Trp Trp His Arg Phe Gln Gly Gly Tyr Phe Leu Phe Met
   260 265 270
   ctt gga ctt tac tgg ctc tcg act gta ttc aat ccg caa ttc att gat 864
   Leu Gly Leu Tyr Trp Leu Ser Thr Val Phe Asn Pro Gln Phe Ile Asp
   275 280 285
   ctt cgt caa cgt ggg gct cag tac gtc gga att caa atg gag aat gat 912
   Leu Arg Gln Arg Gly Ala Gln Tyr Val Gly Ile Gln Met Glu Asn Asp
   290 295 300
   ttc att gtc aag agg agg aag tac gcc gtt gca ttg agg atg atg tac      960
   Phe Ile Val Lys Arg Arg Lys Tyr Ala Val Ala Leu Arg Met Met Tyr
   305                 310                 315                 320
   att tac ttg aac att gtc agc ccc ttc atg aac aat ggt ttg agc tgg     1008
   Ile Tyr Leu Asn Ile Val Ser Pro Phe Met Asn Asn Gly Leu Ser Trp
                   325                 330                 335
   tct acc ttt gga atc atc atg ttg atg gga atc agc gag agt ctc act     1056
   Ser Thr Phe Gly Ile Ile Met Leu Met Gly Ile Ser Glu Ser Leu Thr
               340                 345                 350
   ctc agt gtg ctc ttc tcg ttg tct cac aac ttc atc aat tcg gat cgt     1104
   Leu Ser Val Leu Phe Ser Leu Ser His Asn Phe Ile Asn Ser Asp Arg
           355                 360                 365
   gat cct acg gct gac ttc aaa aag acc gga gaa caa gtg tgc tgg ttc     1152
   Asp Pro Thr Ala Asp Phe Lys Lys Thr Gly Glu Gln Val Cys Trp Phe
       370                 375                 380
   aag tcg cag gtg gag act tcg tct acc tat ggg ggt ttt att tcc gga     1200
   Lys Ser Gln Val Glu Thr Ser Ser Thr Tyr Gly Gly Phe Ile Ser Gly
   385                 390                 395                 400
   tgt ctt acg gga gga ctc aac ttt cag gtg gaa cat cat ctc ttt ccc     1248
   Cys Leu Thr Gly Gly Leu Asn Phe Gln Val Glu His His Leu Phe Pro
                   405                 410                 415
   cgt atg agc agt gct tgg tat cct tac att gca cct acg gtt cgt gag     1296
   Arg Met Ser Ser Ala Trp Tyr Pro Tyr Ile Ala Pro Thr Val Arg Glu
               420                 425                 430
   gtt tgc aag aag cac ggg gtg aac tac gct tat tat cct tgg att ggg     1344
   Val Cys Lys Lys His Gly Val Asn Tyr Ala Tyr Tyr Pro Trp Ile Gly
           435                 440                 445
   cag aat ttg gta tca aca ttc aaa tac atg cat cgc gct ggt agt gga     1392
   Gln Asn Leu Val Ser Thr Phe Lys Tyr Met His Arg Ala Gly Ser Gly
       450                 455                 460
   gcc aac tgg gag ctc aag ccg ttg tct gga agt gcc taa                 1431
   Ala Asn Trp Glu Leu Lys Pro Leu Ser Gly Ser Ala
   465                 470                 475
<210> 39
   <211> 476
   <212> PRT
   <213> Thalassiosira pseudonana
<400> 39
   Met Pro Pro Asn Ala Asp Ile Ser Arg Ile Arg Asn Arg Ile Pro Thr
   1               5                   10                  15
   Lys Thr Gly Thr Val Ala Ser Ala Asp Asn Asn Asp Pro Ala Thr Gln
               20                  25                  30
   Ser Val Arg Thr Leu Lys Ser Leu Lys Gly Asn Glu Val Val Ile Asn
           35                  40                  45
   Gly Thr Ile Tyr Asp Ile Ala Asp Phe Val His Pro Gly Gly Glu Val
       50                  55                  60
   Val Lys Phe Phe Gly Gly Asn Asp Val Thr Ile Gln Tyr Asn Met Ile
   65 70 75 80
   His Pro Tyr His Thr Gly Lys His Leu Glu Lys Met Lys Ala Val Gly
   85 90 95
   Lys Val Val Asp Trp Gln Ser Asp Tyr Lys Phe Asp Thr Pro Phe Glu
   100 105 110
   Arg Glu Ile Lys Ser Glu Val Phe Lys Ile Val Arg Arg Gly Arg Glu
   115 120 125
   Phe Gly Thr Thr Gly Tyr Phe Leu Arg Ala Phe Phe Tyr Ile Ala Leu
   130 135 140
   Phe Phe Thr Met Gln Tyr Thr Phe Ala Thr Cys Thr Thr Phe Thr Thr
   145 150 155 160
   Tyr Asp His Trp Tyr Gln Ser Gly Val Phe Ile Ala Ile Val Phe Gly
   165 170 175
   Ile Ser Gln Ala Phe Ile Gly Leu Asn Val Gln His Asp Ala Asn His
   180 185 190
   Gly Ala Ala Ser Lys Arg Pro Trp Val Asn Asp Leu Leu Gly Phe Gly
   195 200 205
   Thr Asp Leu Ile Gly Ser Asn Lys Trp Asn Trp Met Ala Gln His Trp
   210 215 220
   Thr His His Ala Tyr Thr Asn His Ser Glu Lys Asp Pro Asp Ser Phe
   225 230 235 240
   Ser Ser Glu Pro Met Phe Ala Phe Asn Asp Tyr Pro Ile Gly His Pro
   245 250 255
   Lys Arg Lys Trp Trp His Arg Phe Gln Gly Gly Tyr Phe Leu Phe Met
   260 265 270
   Leu Gly Leu Tyr Trp Leu Ser Thr Val Phe Asn Pro Gln Phe Ile Asp
   275 280 285
   Leu Arg Gln Arg Gly Ala Gln Tyr Val Gly Ile Gln Met Glu Asn Asp
   290 295 300
   Phe Ile Val Lys Arg Arg Lys Tyr Ala Val Ala Leu Arg Met Met Tyr
   305 310 315 320
   Ile Tyr Leu Asn Ile Val Ser Pro Phe Met Asn Asn Gly Leu Ser Trp
   325 330 335
   Ser Thr Phe Gly Ile Ile Met Leu Met Gly Ile Ser Glu Ser Leu Thr
   340 345 350
   Leu Ser Val Leu Phe Ser Leu Ser His Asn Phe Ile Asn Ser Asp Arg
   355 360 365
   Asp Pro Thr Ala Asp Phe Lys Lys Thr Gly Glu Gln Val Cys Trp Phe
   370 375 380
   Lys Ser Gln Val Glu Thr Ser Ser Thr Tyr Gly Gly Phe Ile Ser Gly
   385 390 395 400
   Cys Leu Thr Gly Gly Leu Asn Phe Gln Val Glu His His Leu Phe Pro
   405 410 415
   Arg Met Ser Ser Ala Trp Tyr Pro Tyr Ile Ala Pro Thr Val Arg Glu
   420 425 430
   Val Cys Lys Lys His Gly Val Asn Tyr Ala Tyr Tyr Pro Trp Ile Gly
   435 440 445
   Gln Asn Leu Val Ser Thr Phe Lys Tyr Met His Arg Ala Gly Ser Gly
   450 455 460
   Ala Asn Trp Glu Leu Lys Pro Leu Ser Gly Ser Ala
   465 470 475
<210> 40
   <211> 1449
   <212> DNA
   <213> Thalassiosira pseudonana
<220>
   <221> CDS
   <222> (1)..(1449)
   <223> delta-5-Desaturase
<400> 40
   atg cca ccc aac gcc gag gtc aaa aac ctc cgt tca cgt tcc atc cca      48
   Met Pro Pro Asn Ala Glu Val Lys Asn Leu Arg Ser Arg Ser Ile Pro
   1               5                   10                  15
   acg aag aag tcc agt tca tcg tca tcc acc gcg aac gac gat ccg gct      96
   Thr Lys Lys Ser Ser Ser Ser Ser Ser Thr Ala Asn Asp Asp Pro Ala
               20                  25                  30
   acc caa tcc acc tca cct gtg aac cga acc ctc aag tct ttg aat gga     144
   Thr Gln Ser Thr Ser Pro Val Asn Arg Thr Leu Lys Ser Leu Asn Gly
           35                  40                  45
   aac gaa ata gct att gac ggt gtc atc tat gat att gat ggc ttt gtc     192
   Asn Glu Ile Ala Ile Asp Gly Val Ile Tyr Asp Ile Asp Gly Phe Val
       50                  55                  60
   cat cct gga gga gag gtt att agc ttc ttt gga ggc aac gat gtg act     240
   His Pro Gly Gly Glu Val Ile Ser Phe Phe Gly Gly Asn Asp Val Thr
   65                  70                  75                  80
   gta cag tac aaa atg att cat ccg tat cat aat agt aag cat ctc gag     288
   Val Gln Tyr Lys Met Ile His Pro Tyr His Asn Ser Lys His Leu Glu
                   85                  90                  95
   aag atg aga gcc gtt gga aag att gca gac tac tcc aca gag tac aag     336
   Lys Met Arg Ala Val Gly Lys Ile Ala Asp Tyr Ser Thr Glu Tyr Lys
               100                 105                 110
   ttc gac aca ccc ttt gaa cga gag atc aaa tcc gaa gtg ttc aaa atc     384
   Phe Asp Thr Pro Phe Glu Arg Glu Ile Lys Ser Glu Val Phe Lys Ile
           115                 120                 125
   gtc cgt cga gga cgt gaa ttc ggt aca aca gga tat ttc ctc cgt gcc     432
   Val Arg Arg Gly Arg Glu Phe Gly Thr Thr Gly Tyr Phe Leu Arg Ala
       130                 135                 140
   ttc ttc tac att gct ctc ttc ttc acc atg caa tac acc ttc gcc aca     480
   Phe Phe Tyr Ile Ala Leu Phe Phe Thr Met Gln Tyr Thr Phe Ala Thr
   145                 150                 155                 160
   tgc act acc ttc acc acc tac gat cat tgg tat caa agt ggt gta ttc     528
   Cys Thr Thr Phe Thr Thr Tyr Asp His Trp Tyr Gln Ser Gly Val Phe
                   165                 170                 175
   atc gcc att gtg ttt ggt atc tca caa gct ttc att ggg ttg aat gta     576
   Ile Ala Ile Val Phe Gly Ile Ser Gln Ala Phe Ile Gly Leu Asn Val
               180                 185                 190
   caa cat gat gcc aat cac gga gct gct agc aaa cga cct tgg gtg aat     624
   Gln His Asp Ala Asn His Gly Ala Ala Ser Lys Arg Pro Trp Val Asn
           195                 200                 205
   gat ctc ctt gga tct gga gct gat ctc atc ggt gga tgc aaa tgg aac     672
   Asp Leu Leu Gly Ser Gly Ala Asp Leu Ile Gly Gly Cys Lys Trp Asn
       210                 215                 220
   tgg ttg gct cag cat tgg act cat cat gcg tat acc aat cac gct gat     720
   Trp Leu Ala Gln His Trp Thr His His Ala Tyr Thr Asn His Ala Asp
   225                 230                 235                 240
   aaa gat cct gat agc ttt agt tcc gag ccg gtc ttc aac ttt aac gat     768
   Lys Asp Pro Asp Ser Phe Ser Ser Glu Pro Val Phe Asn Phe Asn Asp
                   245                 250                 255
   tat ccc att ggt cac ccc aaa aga aag tgg tgg cat agg ttc caa ggg     816
   Tyr Pro Ile Gly His Pro Lys Arg Lys Trp Trp His Arg Phe Gln Gly
               260                 265                 270
   ctc tac ttc cta atc atg ctg agt ttc tat tgg gta tcg atg gta ttc     864
   Leu Tyr Phe Leu Ile Met Leu Ser Phe Tyr Trp Val Ser Met Val Phe
           275                 280                 285
   aac cca caa gtt atc gac ctc cgt cat gct gga gct gcc tac gtt gga     912
   Asn Pro Gln Val Ile Asp Leu Arg His Ala Gly Ala Ala Tyr Val Gly
       290                 295                 300
   ttt cag atg gag aac gac ttt atc gtc aaa cgg aga aag tat gca atg     960
   Phe Gln Met Glu Asn Asp Phe Ile Val Lys Arg Arg Lys Tyr Ala Met
   305                 310                 315                 320
   gca ctt cgt gca atg tac ttc tat ttc aac atc tat tgt ccg att gtc    1008
   Ala Leu Arg Ala Met Tyr Phe Tyr Phe Asn Ile Tyr Cys Pro Ile Val
                   325                 330                 335
   aac aat gga ttg act tgg tcg aca gtt gga atc atc ctc tta atg gga    1056
   Asn Asn Gly Leu Thr Trp Ser Thr Val Gly Ile Ile Leu Leu Met Gly
               340                 345                 350
   gtt agc gaa agc ttc atg ctc tcc ggt cta ttc gta ctc tca cac aac      1104
   Val Ser Glu Ser Phe Met Leu Ser Gly Leu Phe Val Leu Ser His Asn
           355                 360                 365
   ttt gaa aat tcc gaa cgt gat cct acc tct gag tat cgc aag act ggt      1152
   Phe Glu Asn Ser Glu Arg Asp Pro Thr Ser Glu Tyr Arg Lys Thr Gly
       370                 375                 380
   gag caa gta tgt tgg ttc aag tct caa gtg gag act tct tct acc tac      1200
   Glu Gln Val Cys Trp Phe Lys Ser Gln Val Glu Thr Ser Ser Thr Tyr
   385                 390                 395                 400
   gga ggt atc gtt gct ggg tgt ctc act ggt gga ctc aac ttt caa gtg      1248
   Gly Gly Ile Val Ala Gly Cys Leu Thr Gly Gly Leu Asn Phe Gln Val
                   405                 410                 415
   gag cat cat ttg ttc ccg agg atg agc agt gct tgg tat cct ttc atc      1296
   Glu His His Leu Phe Pro Arg Met Ser Ser Ala Trp Tyr Pro Phe Ile
               420                 425                 430
   gcg ccg aag gtt aga gag att tgt aag aag cat gga gtt aga tac gct      1344
   Ala Pro Lys Val Arg Glu Ile Cys Lys Lys His Gly Val Arg Tyr Ala
           435                 440                 445
   tac tat ccg tac atc tgg cag aac ttg cat tct acc gtg agt tac atg      1392
   Tyr Tyr Pro Tyr Ile Trp Gln Asn Leu His Ser Thr Val Ser Tyr Met
       450                 455                 460
   cat ggg acg gga acg gga gct aga tgg gag ctt cag ccg ttg tct gga      1440
   His Gly Thr Gly Thr Gly Ala Arg Trp Glu Leu Gln Pro Leu Ser Gly
   465                 470                 475                 480
   agg gcg tag                                                          1449
   Arg Ala
<210> 41
   <211> 482
   <212> PRT
   <213> Thalassiosira pseudonana
<400> 41
   Met Pro Pro Asn Ala Glu Val Lys Asn Leu Arg Ser Arg Ser Ile Pro
   1               5                   10                  15
   Thr Lys Lys Ser Ser Ser Ser Ser Ser Thr Ala Asn Asp Asp Pro Ala
               20                  25                  30
   Thr Gln Ser Thr Ser Pro Val Asn Arg Thr Leu Lys Ser Leu Asn Gly
           35                  40                  45
   Asn Glu Ile Ala Ile Asp Gly Val Ile Tyr Asp Ile Asp Gly Phe Val
       50                  55                  60
   His Pro Gly Gly Glu Val Ile Ser Phe Phe Gly Gly Asn Asp Val Thr
   65                  70                  75                  80
   Val Gln Tyr Lys Met Ile His Pro Tyr His Asn Ser Lys His Leu Glu
                   85                  90                  95
   Lys Met Arg Ala Val Gly Lys Ile Ala Asp Tyr Ser Thr Glu Tyr Lys
   100 105 110
   Phe Asp Thr Pro Phe Glu Arg Glu Ile Lys Ser Glu Val Phe Lys Ile
   115 120 125
   Val Arg Arg Gly Arg Glu Phe Gly Thr Thr Gly Tyr Phe Leu Arg Ala
   130 135 140
   Phe Phe Tyr Ile Ala Leu Phe Phe Thr Met Gln Tyr Thr Phe Ala Thr
   145 150 155 160
   Cys Thr Thr Phe Thr Thr Tyr Asp His Trp Tyr Gln Ser Gly Val Phe
   165 170 175
   Ile Ala Ile Val Phe Gly Ile Ser Gln Ala Phe Ile Gly Leu Asn Val
   180 185 190
   Gln His Asp Ala Asn His Gly Ala Ala Ser Lys Arg Pro Trp Val Asn
   195 200 205
   Asp Leu Leu Gly Ser Gly Ala Asp Leu Ile Gly Gly Cys Lys Trp Asn
   210 215 220
   Trp Leu Ala Gln His Trp Thr His His Ala Tyr Thr Asn His Ala Asp
   225 230 235 240
   Lys Asp Pro Asp Ser Phe Ser Ser Glu Pro Val Phe Asn Phe Asn Asp
   245 250 255
   Tyr Pro Ile Gly His Pro Lys Arg Lys Trp Trp His Arg Phe Gln Gly
   260 265 270
   Leu Tyr Phe Leu Ile Met Leu Ser Phe Tyr Trp Val Ser Met Val Phe
   275 280 285
   Asn Pro Gln Val Ile Asp Leu Arg His Ala Gly Ala Ala Tyr Val Gly
   290 295 300
   Phe Gln Met Glu Asn Asp Phe Ile Val Lys Arg Arg Lys Tyr Ala Met
   305 310 315 320
   Ala Leu Arg Ala Met Tyr Phe Tyr Phe Asn Ile Tyr Cys Pro Ile Val
   325 330 335
   Asn Asn Gly Leu Thr Trp Ser Thr Val Gly Ile Ile Leu Leu Met Gly
   340 345 350
   Val Ser Glu Ser Phe Met Leu Ser Gly Leu Phe Val Leu Ser His Asn
   355 360 365
   Phe Glu Asn Ser Glu Arg Asp Pro Thr Ser Glu Tyr Arg Lys Thr Gly
       370                 375                 380
   Glu Gln Val Cys Trp Phe Lys Ser Gln Val Glu Thr Ser Ser Thr Tyr
   385                 390                 395                 400
   Gly Gly Ile Val Ala Gly Cys Leu Thr Gly Gly Leu Asn Phe Gln Val
                   405                 410                 415
   Glu His His Leu Phe Pro Arg Met Ser Ser Ala Trp Tyr Pro Phe Ile
               420                 425                 430
   Ala Pro Lys Val Arg Glu Ile Cys Lys Lys His Gly Val Arg Tyr Ala
           435                 440                 445
   Tyr Tyr Pro Tyr Ile Trp Gln Asn Leu His Ser Thr Val Ser Tyr Met
       450                 455                 460
   His Gly Thr Gly Thr Gly Ala Arg Trp Glu Leu Gln Pro Leu Ser Gly
   465                 470                 475                 480
   Arg Ala
<210> 42
   <211> 1683
   <212> DNA
   <213> Borago officinalis
<220>
   <221> CDS
   <222> (42)..(1388)
   <223> delta-6-Desaturase
<400> 42
   tatctgccta ccctcccaaa gagagtagtc atttttcatc a atg gct gct caa atc     56
                                                 Met Ala Ala Gln Ile
                                                 1               5
   aag aaa tac att acc tca gat gaa ctc aag aac cac gat aaa ccc gga      104
   Lys Lys Tyr Ile Thr Ser Asp Glu Leu Lys Asn His Asp Lys Pro Gly
                   10                  15                  20
   gat cta tgg atc tcg att caa ggg aaa gcc tat gat gtt tcg gat tgg      152
   Asp Leu Trp Ile Ser Ile Gln Gly Lys Ala Tyr Asp Val Ser Asp Trp
               25                  30                  35
   gtg aaa gac cat cca ggt ggc agc ttt ccc ttg aag agt ctt gct ggt      200
   Val Lys Asp His Pro Gly Gly Ser Phe Pro Leu Lys Ser Leu Ala Gly
           40                  45                  50
   caa gag gta act gat gca ttt gtt gca ttc cat cct gcc tct aca tgg      248
   Gln Glu Val Thr Asp Ala Phe Val Ala Phe His Pro Ala Ser Thr Trp
       55                  60                  65
   aag aat ctt gat aag ttt ttc act ggg tat tat ctt aaa gat tac tct      296
   Lys Asn Leu Asp Lys Phe Phe Thr Gly Tyr Tyr Leu Lys Asp Tyr Ser
   70                  75                  80                  85
   gtt tct gag gtt tct aaa gat tat agg aag ctt gtg ttt gag ttt tct      344
   Val Ser Glu Val Ser Lys Asp Tyr Arg Lys Leu Val Phe Glu Phe Ser
                   90                  95                  100
   aaa atg ggt ttg tat gac aaa aaa ggt cat att atg ttt gca act ttg      392
   Lys Met Gly Leu Tyr Asp Lys Lys Gly His Ile Met Phe Ala Thr Leu
               105                 110                 115
   tgc ttt ata gca atg ctg ttt gct atg agt gtt tat ggg gtt ttg ttt      440
   Cys Phe Ile Ala Met Leu Phe Ala Met Ser Val Tyr Gly Val Leu Phe
           120                 125                 130
   tgt gag ggt gtt ttg gta cat ttg ttt tct ggg tgt ttg atg ggg ttt      488
   Cys Glu Gly Val Leu Val His Leu Phe Ser Gly Cys Leu Met Gly Phe
       135                 140                 145
   ctt tgg att cag agt ggt tgg att gga cat gat gct ggg cat tat atg      536
   Leu Trp Ile Gln Ser Gly Trp Ile Gly His Asp Ala Gly His Tyr Met
   150                 155                 160                 165
   gta gtg tct gat tca agg ctt aat aag ttt atg ggt att ttt gct gca      584
   Val Val Ser Asp Ser Arg Leu Asn Lys Phe Met Gly Ile Phe Ala Ala
                   170                 175                 180
   aat tgt ctt tca gga ata agt att ggt tgg tgg aaa tgg aac cat aat      632
   Asn Cys Leu Ser Gly Ile Ser Ile Gly Trp Trp Lys Trp Asn His Asn
               185                 190                 195
   gca cat cac att gcc tgt aat agc ctt gaa tat gac cct gat tta caa      680
   Ala His His Ile Ala Cys Asn Ser Leu Glu Tyr Asp Pro Asp Leu Gln
           200                 205                 210
   tat ata cca ttc ctt gtt gtg tct tcc aag ttt ttt ggt tca ctc acc      728
   Tyr Ile Pro Phe Leu Val Val Ser Ser Lys Phe Phe Gly Ser Leu Thr
       215                 220                 225
   tct cat ttc tat gag aaa agg ttg act ttt gac tct tta tca aga ttc      776
   Ser His Phe Tyr Glu Lys Arg Leu Thr Phe Asp Ser Leu Ser Arg Phe
   230                 235                 240                 245
   ttt gta agt tat caa cat tgg aca ttt tac cct att atg tgt gct gct      824
   Phe Val Ser Tyr Gln His Trp Thr Phe Tyr Pro Ile Met Cys Ala Ala
                   250                 255                 260
   agg ctc aat atg tat gta caa tct ctc ata atg ttg ttg acc aag aga      872
   Arg Leu Asn Met Tyr Val Gln Ser Leu Ile Met Leu Leu Thr Lys Arg
               265                 270                 275
   aat gtg tcc tat cga gct cag gaa ctc ttg gga tgc cta gtg ttc tcg      920
   Asn Val Ser Tyr Arg Ala Gln Glu Leu Leu Gly Cys Leu Val Phe Ser
           280                 285                 290
   att tgg tac ccg ttg ctt gtt tct tgt ttg cct aat tgg ggt gaa aga      968
   Ile Trp Tyr Pro Leu Leu Val Ser Cys Leu Pro Asn Trp Gly Glu Arg
       295                 300                 305
   att atg ttt gtt att gca agt tta tca gtg act gga atg caa caa gtt     1016
   Ile Met Phe Val Ile Ala Ser Leu Ser Val Thr Gly Met Gln Gln Val
   310                 315                 320                 325
   cag ttc tcc ttg aac cac ttc tct tca agt gtt tat gtt gga aag cct     1064
   Gln Phe Ser Leu Asn His Phe Ser Ser Ser Val Tyr Val Gly Lys Pro
                   330                 335                 340
   aaa ggg aat aat tgg ttt gag aaa caa acg gat ggg aca ctt gac att     1112
   Lys Gly Asn Asn Trp Phe Glu Lys Gln Thr Asp Gly Thr Leu Asp Ile
               345                 350                 355
   tct tgt cct cct tgg atg gat tgg ttt cat ggt gga ttg caa ttc caa      1160
   Ser Cys Pro Pro Trp Met Asp Trp Phe His Gly Gly Leu Gln Phe Gln
           360                 365                 370
   att gag cat cat ttg ttt ccc aag atg cct aga tgc aac ctt agg aaa      1208
   Ile Glu His His Leu Phe Pro Lys Met Pro Arg Cys Asn Leu Arg Lys
       375                 380                 385
   atc tcg ccc tac gtg atc gag tta tgc aag aaa cat aat ttg cct tac      1256
   Ile Ser Pro Tyr Val Ile Glu Leu Cys Lys Lys His Asn Leu Pro Tyr
   390                 395                 400                     405
   aat tat gca tct ttc tcc aag gcc aat gaa atg aca ctc aga aca ttg      1304
   Asn Tyr Ala Ser Phe Ser Lys Ala Asn Glu Met Thr Leu Arg Thr Leu
                   410                 415                 420
   agg aac aca gca ttg cag gct agg gat ata acc aag ccg ctc ccg aag      1352
   Arg Asn Thr Ala Leu Gln Ala Arg Asp Ile Thr Lys Pro Leu Pro Lys
               425                 430                 435
   aat ttg gta tgg gaa gct ctt cac act cat ggt taa aattaccctt           1398
   Asn Leu Val Trp Glu Ala Leu His Thr His Gly
           440                 445
   agttcatgta ataatttgag attatgtatc tcctatgttt gtgtcttgtc ttggttctac    1458
   ttgttggagt cattgcaact tgtcttttat ggtttattag atgtttttta atatatttta    1518
   gaggttttgc tttcatctcc attattgatg aataaggagt tgcatattgt caattgttgt    1578
   gctcaatatc tgatattttg gaatgtactt tgtaccactg tgttttcagt tgaagctcat    1638
   gtgtacttct atagactttg tttaaatggt tatgtcatgt tattt                    1683
<210> 43
   <211> 448
   <212> PRT
   <213> Borago officinalis
<400> 43
   Met Ala Ala Gln Ile Lys Lys Tyr Ile Thr Ser Asp Glu Leu Lys Asn
   1               5                   10                  15
   His Asp Lys Pro Gly Asp Leu Trp Ile Ser Ile Gln Gly Lys Ala Tyr
               20                  25                  30
   Asp Val Ser Asp Trp Val Lys Asp His Pro Gly Gly Ser Phe Pro Leu
           35                  40                  45
   Lys Ser Leu Ala Gly Gln Glu Val Thr Asp Ala Phe Val Ala Phe His
       50                  55                  60
   Pro Ala Ser Thr Trp Lys Asn Leu Asp Lys Phe Phe Thr Gly Tyr Tyr
   65                  70                  75                  80
   Leu Lys Asp Tyr Ser Val Ser Glu Val Ser Lys Asp Tyr Arg Lys Leu
                   85                  90                  95
   Val Phe Glu Phe Ser Lys Met Gly Leu Tyr Asp Lys Lys Gly His Ile
               100                 105                 110
   Met Phe Ala Thr Leu Cys Phe Ile Ala Met Leu Phe Ala Met Ser Val
           115                 120                 125
   Tyr Gly Val Leu Phe Cys Glu Gly Val Leu Val His Leu Phe Ser Gly
       130                 135                 140
   Cys Leu Met Gly Phe Leu Trp Ile Gln Ser Gly Trp Ile Gly His Asp
   145                 150                 155                 160
   Ala Gly His Tyr Met Val Val Ser Asp Ser Arg Leu Asn Lys Phe Met
                   165                 170                 175
   Gly Ile Phe Ala Ala Asn Cys Leu Ser Gly Ile Ser Ile Gly Trp Trp
               180                 185                 190
   Lys Trp Asn His Asn Ala His His Ile Ala Cys Asn Ser Leu Glu Tyr
           195                 200                 205
   Asp Pro Asp Leu Gln Tyr Ile Pro Phe Leu Val Val Ser Ser Lys Phe
       210                 215                 220
   Phe Gly Ser Leu Thr Ser His Phe Tyr Glu Lys Arg Leu Thr Phe Asp
   225                 230                 235                 240
   Ser Leu Ser Arg Phe Phe Val Ser Tyr Gln His Trp Thr Phe Tyr Pro
                   245                 250                 255
   Ile Met Cys Ala Ala Arg Leu Asn Met Tyr Val Gln Ser Leu Ile Met
               260                 265                 270
   Leu Leu Thr Lys Arg Asn Val Ser Tyr Arg Ala Gln Glu Leu Leu Gly
           275                 280                 285
   Cys Leu Val Phe Ser Ile Trp Tyr Pro Leu Leu Val Ser Cys Leu Pro
       290                 295                 300
   Asn Trp Gly Glu Arg Ile Met Phe Val Ile Ala Ser Leu Ser Val Thr
   305                 310                 315                 320
   Gly Met Gln Gln Val Gln Phe Ser Leu Asn His Phe Ser Ser Ser Val
                   325                 330                 335
   Tyr Val Gly Lys Pro Lys Gly Asn Asn Trp Phe Glu Lys Gln Thr Asp
               340                 345                 350
   Gly Thr Leu Asp Ile Ser Cys Pro Pro Trp Met Asp Trp Phe His Gly
           355                 360                 365
   Gly Leu Gln Phe Gln Ile Glu His His Leu Phe Pro Lys Met Pro Arg
       370                 375                 380
   Cys Asn Leu Arg Lys Ile Ser Pro Tyr Val Ile Glu Leu Cys Lys Lys
   385 390 395 400
   His Asn Leu Pro Tyr Asn Tyr Ala Ser Phe Ser Lys Ala Asn Glu Met
   405 410 415
   Thr Leu Arg Thr Leu Arg Asn Thr Ala Leu Gln Ala Arg Asp Ile Thr
   420 425 430
   Lys Pro Leu Pro Lys Asn Leu Val Trp Glu Ala Leu His Thr His Gly
   435 440 445
<210> 44
   <211> 1563
   <212> DNA
   <213> Ceratodon purpureus
<220>
   <221> CDS
   <222> (1)..(1563)
   <223> delta-6-Desaturase
<400> 44
   atg gtg tcc cag ggc ggc ggt ctc tcg cag ggt tcc att gaa gaa aac      48
   Met Val Ser Gln Gly Gly Gly Leu Ser Gln Gly Ser Ile Glu Glu Asn
   1               5                   10                  15
   att gac gtt gag cac ttg gca acg atg ccc ctc gtc agt gac ttc cta      96
   Ile Asp Val Glu His Leu Ala Thr Met Pro Leu Val Ser Asp Phe Leu
               20                  25                  30
   aat gtc ctg gga acg act ttg ggc cag tgg agt ctt tcc act aca ttc     144
   Asn Val Leu Gly Thr Thr Leu Gly Gln Trp Ser Leu Ser Thr Thr Phe
           35                  40                  45
   gct ttc aag agg ctc acg act aag aaa cac agt tcg gac atc tcg gtg     192
   Ala Phe Lys Arg Leu Thr Thr Lys Lys His Ser Ser Asp Ile Ser Val
       50                  55                  60
   gag gca caa aaa gaa tcg gtt gcg cgg ggg cca gtt gag aat att tct     240
   Glu Ala Gln Lys Glu Ser Val Ala Arg Gly Pro Val Glu Asn Ile Ser
   65                  70                  75                  80
   caa tcg gtt gcg cag ccc atc agg cgg agg tgg gtg cag gat aaa aag     288
   Gln Ser Val Ala Gln Pro Ile Arg Arg Arg Trp Val Gln Asp Lys Lys
                   85                  90                  95
   ccg gtt act tac agc ctg aag gat gta gct tcg cac gat atg ccc cag     336
   Pro Val Thr Tyr Ser Leu Lys Asp Val Ala Ser His Asp Met Pro Gln
               100                 105                 110
   gac tgc tgg att ata atc aaa gag aag gtg tat gat gtg agc acc ttc     384
   Asp Cys Trp Ile Ile Ile Lys Glu Lys Val Tyr Asp Val Ser Thr Phe
           115                 120                 125
   gct gag cag cac cct gga ggc acg gtt atc aac acc tac ttc gga cga     432
   Ala Glu Gln His Pro Gly Gly Thr Val Ile Asn Thr Tyr Phe Gly Arg
       130                 135                 140
   gac gcc aca gat gtt ttc tct act ttc cac gca tcc acc tca tgg aag     480
   Asp Ala Thr Asp Val Phe Ser Thr Phe His Ala Ser Thr Ser Trp Lys
   145                 150                 155                 160
   att ctt cag aat ttc tac atc ggg aac ctt gtt agg gag gag ccg act      528
   Ile Leu Gln Asn Phe Tyr Ile Gly Asn Leu Val Arg Glu Glu Pro Thr
                   165                 170                 175
   ttg gag ctg ctg aag gag tac aga gag ttg aga gcc ctt ttc ttg aga      576
   Leu Glu Leu Leu Lys Glu Tyr Arg Glu Leu Arg Ala Leu Phe Leu Arg
               180                 185                 190
   gaa cag ctt ttc aag agt tcc aaa tcc tac tac ctt ttc aag act ctc      624
   Glu Gln Leu Phe Lys Ser Ser Lys Ser Tyr Tyr Leu Phe Lys Thr Leu
           195                 200                 205
   ata aat gtt tcc att gtt gcc aca agc att gcg ata atc agt ctg tac      672
   Ile Asn Val Ser Ile Val Ala Thr Ser Ile Ala Ile Ile Ser Leu Tyr
       210                 215                 220
   aag tct tac cgg gcg gtt ctg tta tca gcc agt ttg atg ggc ttg ttt      720
   Lys Ser Tyr Arg Ala Val Leu Leu Ser Ala Ser Leu Met Gly Leu Phe
   225                 230                 235                 240
   att caa cag tgc gga tgg ttg tct cac gat ttt cta cac cat cag gta      768
   Ile Gln Gln Cys Gly Trp Leu Ser His Asp Phe Leu His His Gln Val
                   245                 250                 255
   ttt gag aca cgc tgg ctc aat gac gtt gtt ggc tat gtg gtc ggc aac      816
   Phe Glu Thr Arg Trp Leu Asn Asp Val Val Gly Tyr Val Val Gly Asn
               260                 265                 270
   gtt gtt ctg gga ttc agt gtc tcg tgg tgg aag acc aag cac aac ctg      864
   Val Val Leu Gly Phe Ser Val Ser Trp Trp Lys Thr Lys His Asn Leu
           275                 280                 285
   cat cat gct gct ccg aat gaa tgc gac caa aag tac aca ccg att gat      912
   His His Ala Ala Pro Asn Glu Cys Asp Gln Lys Tyr Thr Pro Ile Asp
       290                 295                 300
   gag gat att gat act ctc ccc atc att gct tgg agt aaa gat ctc ttg      960
   Glu Asp Ile Asp Thr Leu Pro Ile Ile Ala Trp Ser Lys Asp Leu Leu
   305                 310                 315                 320
   gcc act gtt gag agc aag acc atg ttg cga gtt ctt cag tac cag cac     1008
   Ala Thr Val Glu Ser Lys Thr Met Leu Arg Val Leu Gln Tyr Gln His
                   325                 330                 335
   cta ttc ttt ttg gtt ctt ttg acg ttt gcc cgg gcg agt tgg cta ttt     1056
   Leu Phe Phe Leu Val Leu Leu Thr Phe Ala Arg Ala Ser Trp Leu Phe
               340                 345                 350
   tgg agc gcg gcc ttc act ctc agg ccc gag ttg acc ctt ggc gag aag     1104
   Trp Ser Ala Ala Phe Thr Leu Arg Pro Glu Leu Thr Leu Gly Glu Lys
           355                 360                 365
   ctt ttg gag agg gga acg atg gct ttg cac tac att tgg ttt aat agt     1152
   Leu Leu Glu Arg Gly Thr Met Ala Leu His Tyr Ile Trp Phe Asn Ser
       370                 375                 380
   gtt gcg ttt tat ctg ctc ccc gga tgg aaa cca gtt gta tgg atg gtg     1200
   Val Ala Phe Tyr Leu Leu Pro Gly Trp Lys Pro Val Val Trp Met Val
   385                 390                 395                 400
   gtc agc gag ctc atg tct ggt ttc ctg ctg gga tac gta ttt gta ctc     1248
   Val Ser Glu Leu Met Ser Gly Phe Leu Leu Gly Tyr Val Phe Val Leu
                   405                 410                 415
   agt cac aat gga atg gag gtg tac aat acg tca aag gac ttc gtg aat     1296
   Ser His Asn Gly Met Glu Val Tyr Asn Thr Ser Lys Asp Phe Val Asn
                   420                     425                     430
   gcc cag att gca tcg act cgc gac atc aaa gca ggg gtg ttt aat gat      1344
   Ala Gln Ile Ala Ser Thr Arg Asp Ile Lys Ala Gly Val Phe Asn Asp
               435                     440                     445
   tgg ttc acc gga ggt ctc aac aga cag att gag cat cat cta ttt cca      1392
   Trp Phe Thr Gly Gly Leu Asn Arg Gln Ile Glu His His Leu Phe Pro
       450                     455                     460
   acg atg ccc agg cac aac ctt aat aaa att tct cct cac gtg gag act      1440
   Thr Met Pro Arg His Asn Leu Asn Lys Ile Ser Pro His Val Glu Thr
   465                     470                     475                 480
   ttg tgc aag aag cat gga ctg gtc tac gaa gac gtg agc atg gct tcg      1488
   Leu Cys Lys Lys His Gly Leu Val Tyr Glu Asp Val Ser Met Ala Ser
                   485                     490                     495
   ggc act tac cgg gtt ttg aaa aca ctt aag gac gtt gcc gat gct gct      1536
   Gly Thr Tyr Arg Val Leu Lys Thr Leu Lys Asp Val Ala Asp Ala Ala
               500                     505                     510
   tca cac cag cag ctt gct gcg agt tga                                  1563
   Ser His Gln Gln Leu Ala Ala Ser
           515                     520
<210> 45
   <211> 520
   <212> PRT
   <213> Ceratodon purpureus
<400> 45
   Met Val Ser Gln Gly Gly Gly Leu Ser Gln Gly Ser Ile Glu Glu Asn
   1               5                   10                  15
   Ile Asp Val Glu His Leu Ala Thr Met Pro Leu Val Ser Asp Phe Leu
               20                  25                  30
   Asn Val Leu Gly Thr Thr Leu Gly Gln Trp Ser Leu Ser Thr Thr Phe
           35                  40                  45
   Ala Phe Lys Arg Leu Thr Thr Lys Lys His Ser Ser Asp Ile Ser Val
       50                  55                  60
   Glu Ala Gln Lys Glu Ser Val Ala Arg Gly Pro Val Glu Asn Ile Ser
   65                  70                  75                  80
   Gln Ser Val Ala Gln Pro Ile Arg Arg Arg Trp Val Gln Asp Lys Lys
                   85                  90                  95
   Pro Val Thr Tyr Ser Leu Lys Asp Val Ala Ser His Asp Met Pro Gln
               100                 105                 110
   Asp Cys Trp Ile Ile Ile Lys Glu Lys Val Tyr Asp Val Ser Thr Phe
           115                 120                 125
   Ala Glu Gln His Pro Gly Gly Thr Val Ile Asn Thr Tyr Phe Gly Arg
       130                 135                 140
   Asp Ala Thr Asp Val Phe Ser Thr Phe His Ala Ser Thr Ser Trp Lys
   145                 150                 155                 160
   Ile Leu Gln Asn Phe Tyr Ile Gly Asn Leu Val Arg Glu Glu Pro Thr
                   165                 170                 175
   Leu Glu Leu Leu Lys Glu Tyr Arg Glu Leu Arg Ala Leu Phe Leu Arg
               180                 185                 190
   Glu Gln Leu Phe Lys Ser Ser Lys Ser Tyr Tyr Leu Phe Lys Thr Leu
           195                 200                 205
   Ile Asn Val Ser Ile Val Ala Thr Ser Ile Ala Ile Ile Ser Leu Tyr
       210                 215                 220
   Lys Ser Tyr Arg Ala Val Leu Leu Ser Ala Ser Leu Met Gly Leu Phe
   225                 230                 235                 240
   Ile Gln Gln Cys Gly Trp Leu Ser His Asp Phe Leu His His Gln Val
                   245                 250                 255
   Phe Glu Thr Arg Trp Leu Asn Asp Val Val Gly Tyr Val Val Gly Asn
               260                 265                 270
   Val Val Leu Gly Phe Ser Val Ser Trp Trp Lys Thr Lys His Asn Leu
           275                 280                 285
   His His Ala Ala Pro Asn Glu Cys Asp Gln Lys Tyr Thr Pro Ile Asp
       290                 295                 300
   Glu Asp Ile Asp Thr Leu Pro Ile Ile Ala Trp Ser Lys Asp Leu Leu
   305                 310                 315                 320
   Ala Thr Val Glu Ser Lys Thr Met Leu Arg Val Leu Gln Tyr Gln His
                   325                 330                 335
   Leu Phe Phe Leu Val Leu Leu Thr Phe Ala Arg Ala Ser Trp Leu Phe
               340                 345                 350
   Trp Ser Ala Ala Phe Thr Leu Arg Pro Glu Leu Thr Leu Gly Glu Lys
           355                 360                 365
   Leu Leu Glu Arg Gly Thr Met Ala Leu His Tyr Ile Trp Phe Asn Ser
       370                 375                 380
   Val Ala Phe Tyr Leu Leu Pro Gly Trp Lys Pro Val Val Trp Met Val
   385                 390                 395                 400
   Val Ser Glu Leu Met Ser Gly Phe Leu Leu Gly Tyr Val Phe Val Leu
                   405                 410                 415
   Ser His Asn Gly Met Glu Val Tyr Asn Thr Ser Lys Asp Phe Val Asn
   420 425 430
   Ala Gln Ile Ala Ser Thr Arg Asp Ile Lys Ala Gly Val Phe Asn Asp
   435 440 445
   Trp Phe Thr Gly Gly Leu Asn Arg Gln Ile Glu His His Leu Phe Pro
   450 455 460
   Thr Met Pro Arg His Asn Leu Asn Lys Ile Ser Pro His Val Glu Thr
   465 470 475 480
   Leu Cys Lys Lys His Gly Leu Val Tyr Glu Asp Val Ser Met Ala Ser
   485 490 495
   Gly Thr Tyr Arg Val Leu Lys Thr Leu Lys Asp Val Ala Asp Ala Ala
   500 505 510
   Ser His Gln Gln Leu Ala Ala Ser
   515 520
<210> 46
   <211> 1434
   <212> DNA
   <213> Phaeodactylum tricornutum
<220>
   <221> CDS
   <222> (1)..(1434)
   <223> delta-6-Desaturase
<400> 46
   atg ggc aaa gga ggg gac gct cgg gcc tcg aag ggc tca acg gcg gct        48
   Met Gly Lys Gly Gly Asp Ala Arg Ala Ser Lys Gly Ser Thr Ala Ala
   1               5                   10                  15
   cgc aag atc agt tgg cag gaa gtc aag acc cac gcg tct ccg gag gac        96
   Arg Lys Ile Ser Trp Gln Glu Val Lys Thr His Ala Ser Pro Glu Asp
               20                  25                  30
   gcc tgg atc att cac tcc aat aag gtc tac gac gtg tcc aac tgg cac        144
   Ala Trp Ile Ile His Ser Asn Lys Val Tyr Asp Val Ser Asn Trp His
           35                  40                  45
   gaa cat ccc gga ggc gcc gtc att ttc acg cac gcc ggt gac gac atg        192
   Glu His Pro Gly Gly Ala Val Ile Phe Thr His Ala Gly Asp Asp Met
       50                  55                  60
   acg gac att ttc gct gcc ttt cac gca ccc gga tcg cag tcg ctc atg        240
   Thr Asp Ile Phe Ala Ala Phe His Ala Pro Gly Ser Gln Ser Leu Met
   65                  70                  75                  80
   aag aag ttc tac att ggc gaa ttg ctc ccg gaa acc acc ggc aag gag        288
   Lys Lys Phe Tyr Ile Gly Glu Leu Leu Pro Glu Thr Thr Gly Lys Glu
                   85                  90                  95
   ccg cag caa atc gcc ttt gaa aag ggc tac cgc gat ctg cgc tcc aaa        336
   Pro Gln Gln Ile Ala Phe Glu Lys Gly Tyr Arg Asp Leu Arg Ser Lys
                   100                 105                 110
   ctc atc atg atg ggc atg ttc aag tcc aac aag tgg ttc tac gtc tac      384
   Leu Ile Met Met Gly Met Phe Lys Ser Asn Lys Trp Phe Tyr Val Tyr
           115                 120                 125
   aag tgc ctc agc aac atg gcc att tgg gcc gcc gcc tgt gct ctc gtc      432
   Lys Cys Leu Ser Asn Met Ala Ile Trp Ala Ala Ala Cys Ala Leu Val
       130                 135                 140
   ttt tac tcg gac cgc ttc tgg gta cac ctg gcc agc gcc gtc atg ctg      480
   Phe Tyr Ser Asp Arg Phe Trp Val His Leu Ala Ser Ala Val Met Leu
   145                 150                 155                 160
   gga aca ttc ttt cag cag tcg gga tgg ttg gca cac gac ttt ctg cac      528
   Gly Thr Phe Phe Gln Gln Ser Gly Trp Leu Ala His Asp Phe Leu His
                   165                 170                 175
   cac cag gtc ttc acc aag cgc aag cac ggg gat ctc gga gga ctc ttt      576
   His Gln Val Phe Thr Lys Arg Lys His Gly Asp Leu Gly Gly Leu Phe
               180                 185                 190
   tgg ggg aac ctc atg cag ggt tac tcc gta cag tgg tgg aaa aac aag      624
   Trp Gly Asn Leu Met Gln Gly Tyr Ser Val Gln Trp Trp Lys Asn Lys
           195                 200                 205
   cac aac gga cac cac gcc gtc ccc aac ctc cac tgc tcc tcc gca gtc      672
   His Asn Gly His His Ala Val Pro Asn Leu His Cys Ser Ser Ala Val
       210                 215                 220
   gcg caa gat ggg gac ccg gac atc gat acc atg ccc ctt ctc gcc tgg      720
   Ala Gln Asp Gly Asp Pro Asp Ile Asp Thr Met Pro Leu Leu Ala Trp
   225                 230                 235                 240
   tcc gtc cag caa gcc cag tct tac cgg gaa ctc caa gcc gac gga aag      768
   Ser Val Gln Gln Ala Gln Ser Tyr Arg Glu Leu Gln Ala Asp Gly Lys
                   245                 250                 255
   gat tcg ggt ttg gtc aag ttc atg atc cgt aac caa tcc tac ttt tac      816
   Asp Ser Gly Leu Val Lys Phe Met Ile Arg Asn Gln Ser Tyr Phe Tyr
               260                 265                 270
   ttt ccc atc ttg ttg ctc gcc cgc ctg tcg tgg ttg aac gag tcc ttc      864
   Phe Pro Ile Leu Leu Leu Ala Arg Leu Ser Trp Leu Asn Glu Ser Phe
           275                 280                 285
   aag tgc gcc ttt ggg ctt gga gct gcg tcg gag aac gct gct ctc gaa      912
   Lys Cys Ala Phe Gly Leu Gly Ala Ala Ser Glu Asn Ala Ala Leu Glu
       290                 295                 300
   ctc aag gcc aag ggt ctt cag tac ccc ctt ttg gaa aag gct ggc atc      960
   Leu Lys Ala Lys Gly Leu Gln Tyr Pro Leu Leu Glu Lys Ala Gly Ile
   305                 310                 315                 320
   ctg ctg cac tac gct tgg atg ctt aca gtt tcg tcc ggc ttt gga cgc      1008
   Leu Leu His Tyr Ala Trp Met Leu Thr Val Ser Ser Gly Phe Gly Arg
                   325                 330                 335
   ttc tcg ttc gcg tac acc gca ttt tac ttt cta acc gcg acc gcg tcc      1056
   Phe Ser Phe Ala Tyr Thr Ala Phe Tyr Phe Leu Thr Ala Thr Ala Ser
               340                 345                 350
   tgt gga ttc ttg ctc gcc att gtc ttt ggc ctc ggc cac aac ggc atg      1104
   Cys Gly Phe Leu Leu Ala Ile Val Phe Gly Leu Gly His Asn Gly Met
           355                 360                 365
   gcc acc tac aat gcc gac gcc cgt ccg gac ttc tgg aag ctc caa gtc      1152
   Ala Thr Tyr Asn Ala Asp Ala Arg Pro Asp Phe Trp Lys Leu Gln Val
   370                     375                     380
   acc acg act cgc aac gtc acg ggc gga cac ggt ttc ccc caa gcc ttt      1200
   Thr Thr Thr Arg Asn Val Thr Gly Gly His Gly Phe Pro Gln Ala Phe
   385                 390                     395                 400
   gtc gac tgg ttc tgt ggt ggc ctc cag tac caa gtc gac cac cac tta      1248
   Val Asp Trp Phe Cys Gly Gly Leu Gln Tyr Gln Val Asp His His Leu
                   405                     410                 415
   ttc ccc agc ctg ccc cga cac aat ctg gcc aag aca cac gca ctg gtc      1296
   Phe Pro Ser Leu Pro Arg His Asn Leu Ala Lys Thr His Ala Leu Val
               420                 425                     430
   gaa tcg ttc tgc aag gag tgg ggt gtc cag tac cac gaa gcc gac ctt      1344
   Glu Ser Phe Cys Lys Glu Trp Gly Val Gln Tyr His Glu Ala Asp Leu
           435                     440                 445
   gtg gac ggg acc atg gaa gtc ttg cac cat ttg ggc agc gtg gcc ggc      1392
   Val Asp Gly Thr Met Glu Val Leu His His Leu Gly Ser Val Ala Gly
       450                     455                 460
   gaa ttc gtc gtg gat ttt gta cgc gat gga ccc gcc atg taa              1434
   Glu Phe Val Val Asp Phe Val Arg Asp Gly Pro Ala Met
   465                 470                     475
<210> 47
   <211> 477
   <212> PRT
   <213> Phaeodactylum tricornutum
<400> 47
   Met Gly Lys Gly Gly Asp Ala Arg Ala Ser Lys Gly Ser Thr Ala Ala
   1               5                   10                  15
   Arg Lys Ile Ser Trp Gln Glu Val Lys Thr His Ala Ser Pro Glu Asp
               20                  25                  30
   Ala Trp Ile Ile His Ser Asn Lys Val Tyr Asp Val Ser Asn Trp His
           35                  40                  45
   Glu His Pro Gly Gly Ala Val Ile Phe Thr His Ala Gly Asp Asp Met
       50                  55                  60
   Thr Asp Ile Phe Ala Ala Phe His Ala Pro Gly Ser Gln Ser Leu Met
   65                  70                  75                  80
   Lys Lys Phe Tyr Ile Gly Glu Leu Leu Pro Glu Thr Thr Gly Lys Glu
                   85                  90                  95
   Pro Gln Gln Ile Ala Phe Glu Lys Gly Tyr Arg Asp Leu Arg Ser Lys
               100                 105                 110
   Leu Ile Met Met Gly Met Phe Lys Ser Asn Lys Trp Phe Tyr Val Tyr
           115                 120                 125
   Lys Cys Leu Ser Asn Met Ala Ile Trp Ala Ala Ala Cys Ala Leu Val
       130                 135                 140
   Phe Tyr Ser Asp Arg Phe Trp Val His Leu Ala Ser Ala Val Met Leu
   145 150 155 160
   Gly Thr Phe Phe Gln Gln Ser Gly Trp Leu Ala His Asp Phe Leu His
   165 170 175
   His Gln Val Phe Thr Lys Arg Lys His Gly Asp Leu Gly Gly Leu Phe
   180 185 190
   Trp Gly Asn Leu Met Gln Gly Tyr Ser Val Gln Trp Trp Lys Asn Lys
   195 200 205
   His Asn Gly His His Ala Val Pro Asn Leu His Cys Ser Ser Ala Val
   210 215 220
   Ala Gln Asp Gly Asp Pro Asp Ile Asp Thr Met Pro Leu Leu Ala Trp
   225 230 235 240
   Ser Val Gln Gln Ala Gln Ser Tyr Arg Glu Leu Gln Ala Asp Gly Lys
   245 250 255
   Asp Ser Gly Leu Val Lys Phe Met Ile Arg Asn Gln Ser Tyr Phe Tyr
   260 265 270
   Phe Pro Ile Leu Leu Leu Ala Arg Leu Ser Trp Leu Asn Glu Ser Phe
   275 280 285
   Lys Cys Ala Phe Gly Leu Gly Ala Ala Ser Glu Asn Ala Ala Leu Glu
   290 295 300
   Leu Lys Ala Lys Gly Leu Gln Tyr Pro Leu Leu Glu Lys Ala Gly Ile
   305 310 315 320
   Leu Leu His Tyr Ala Trp Met Leu Thr Val Ser Ser Gly Phe Gly Arg
   325 330 335
   Phe Ser Phe Ala Tyr Thr Ala Phe Tyr Phe Leu Thr Ala Thr Ala Ser
   340 345 350
   Cys Gly Phe Leu Leu Ala Ile Val Phe Gly Leu Gly His Asn Gly Met
   355 360 365
   Ala Thr Tyr Asn Ala Asp Ala Arg Pro Asp Phe Trp Lys Leu Gln Val
   370 375 380
   Thr Thr Thr Arg Asn Val Thr Gly Gly His Gly Phe Pro Gln Ala Phe
   385 390 395 400
   Val Asp Trp Phe Cys Gly Gly Leu Gln Tyr Gln Val Asp His His Leu
   405 410 415
   Phe Pro Ser Leu Pro Arg His Asn Leu Ala Lys Thr His Ala Leu Val
               420                 425                 430
   Glu Ser Phe Cys Lys Glu Trp Gly Val Gln Tyr His Glu Ala Asp Leu
           435                 440                 445
   Val Asp Gly Thr Met Glu Val Leu His His Leu Gly Ser Val Ala Gly
       450                 455                 460
   Glu Phe Val Val Asp Phe Val Arg Asp Gly Pro Ala Met
   465                 470                 475
<210> 48
   <211> 1578
   <212> DNA
   <213> Physcomitrellla patens
<220>
   <221> CDS
   <222> (1)..(1578)
   <223> delta-6-Desaturase
<400> 48
   atg gta ttc gcg ggc ggt gga ctt cag cag ggc tct ctc gaa gaa aac      48
   Met Val Phe Ala Gly Gly Gly Leu Gln Gln Gly Ser Leu Glu Glu Asn
   1               5                   10                  15
   atc gac gtc gag cac att gcc agt atg tct ctc ttc agc gac ttc ttc      96
   Ile Asp Val Glu His Ile Ala Ser Met Ser Leu Phe Ser Asp Phe Phe
               20                  25                  30
   agt tat gtg tct tca act gtt ggt tcg tgg agc gta cac agt ata caa     144
   Ser Tyr Val Ser Ser Thr Val Gly Ser Trp Ser Val His Ser Ile Gln
           35                  40                  45
   cct ttg aag cgc ctg acg agt aag aag cgt gtt tcg gaa agc gct gcc     192
   Pro Leu Lys Arg Leu Thr Ser Lys Lys Arg Val Ser Glu Ser Ala Ala
       50                  55                  60
   gtg caa tgt ata tca gct gaa gtt cag aga aat tcg agt acc cag gga     240
   Val Gln Cys Ile Ser Ala Glu Val Gln Arg Asn Ser Ser Thr Gln Gly
   65                  70                  75                  80
   act gcg gag gca ctc gca gaa tca gtc gtg aag ccc acg aga cga agg     288
   Thr Ala Glu Ala Leu Ala Glu Ser Val Val Lys Pro Thr Arg Arg Arg
                   85                  90                  95
   tca tct cag tgg aag aag tcg aca cac ccc cta tca gaa gta gca gta     336
   Ser Ser Gln Trp Lys Lys Ser Thr His Pro Leu Ser Glu Val Ala Val
               100                 105                 110
   cac aac aag cca agc gat tgc tgg att gtt gta aaa aac aag gtg tat     384
   His Asn Lys Pro Ser Asp Cys Trp Ile Val Val Lys Asn Lys Val Tyr
           115                 120                 125
   gat gtt tcc aat ttt gcg gac gag cat ccc gga gga tca gtt att agt     432
   Asp Val Ser Asn Phe Ala Asp Glu His Pro Gly Gly Ser Val Ile Ser
       130                 135                 140
   act tat ttt gga cga gac ggc aca gat gtt ttc tct agt ttt cat gca     480
   Thr Tyr Phe Gly Arg Asp Gly Thr Asp Val Phe Ser Ser Phe His Ala
   145 150 155 160
   gct tct aca tgg aaa att ctt caa gac ttt tac att ggt gac gtg gag 528
   Ala Ser Thr Trp Lys Ile Leu Gln Asp Phe Tyr Ile Gly Asp Val Glu
   165 170 175
   agg gtg gag ccg act cca gag ctg ctg aaa gat ttc cga gaa atg aga 576
   Arg Val Glu Pro Thr Pro Glu Leu Leu Lys Asp Phe Arg Glu Met Arg
   180 185 190
   gct ctt ttc ctg agg gag caa ctt ttc aaa agt tcg aaa ttg tac tat 624
   Ala Leu Phe Leu Arg Glu Gln Leu Phe Lys Ser Ser Lys Leu Tyr Tyr
   195 200 205
   gtt atg aag ctg ctc acg aat gtt gct att ttt gct gcg agc att gca 672
   Val Met Lys Leu Leu Thr Asn Val Ala Ile Phe Ala Ala Ser Ile Ala
   210 215 220
   ata ata tgt tgg agc aag act att tca gcg gtt ttg gct tca gct tgt 720
   Ile Ile Cys Trp Ser Lys Thr Ile Ser Ala Val Leu Ala Ser Ala Cys
   225 230 235 240
   atg atg gct ctg tgt ttc caa cag tgc gga tgg cta tcc cat gat ttt 768
   Met Met Ala Leu Cys Phe Gln Gln Cys Gly Trp Leu Ser His Asp Phe
   245 250 255
   ctc cac aat cag gtg ttt gag aca cgc tgg ctt aat gaa gtt gtc ggg 816
   Leu His Asn Gln Val Phe Glu Thr Arg Trp Leu Asn Glu Val Val Gly
   260 265 270
   tat gtg atc ggc aac gcc gtt ctg ggg ttt agt aca ggg tgg tgg aag 864
   Tyr Val Ile Gly Asn Ala Val Leu Gly Phe Ser Thr Gly Trp Trp Lys
   275 280 285
   gag aag cat aac ctt cat cat gct gct cca aat gaa tgc gat cag act 912
   Glu Lys His Asn Leu His His Ala Ala Pro Asn Glu Cys Asp Gln Thr
   290 295 300
   tac caa cca att gat gaa gat att gat act ctc ccc ctc att gcc tgg 960
   Tyr Gln Pro Ile Asp Glu Asp Ile Asp Thr Leu Pro Leu Ile Ala Trp
   305 310 315 320
   agc aag gac ata ctg gcc aca gtt gag aat aag aca ttc ttg cga atc 1008
   Ser Lys Asp Ile Leu Ala Thr Val Glu Asn Lys Thr Phe Leu Arg Ile
   325 330 335
   ctc caa tac cag cat ctg ttc ttc atg ggt ctg tta ttt ttc gcc cgt 1056
   Leu Gln Tyr Gln His Leu Phe Phe Met Gly Leu Leu Phe Phe Ala Arg
   340 345 350
   ggt agt tgg ctc ttt tgg agc tgg aga tat acc tct aca gca gtg ctc 1104
   Gly Ser Trp Leu Phe Trp Ser Trp Arg Tyr Thr Ser Thr Ala Val Leu
   355 360 365
   tca cct gtc gac agg ttg ttg gag aag gga act gtt ctg ttt cac tac 1152
   Ser Pro Val Asp Arg Leu Leu Glu Lys Gly Thr Val Leu Phe His Tyr
   370 375 380
   ttt tgg ttc gtc ggg aca gcg tgc tat ctt ctc cct ggt tgg aag cca 1200
   Phe Trp Phe Val Gly Thr Ala Cys Tyr Leu Leu Pro Gly Trp Lys Pro
   385 390 395 400
   tta gta tgg atg gcg gtg act gag ctc atg tcc ggc atg ctg ctg ggc 1248
   Leu Val Trp Met Ala Val Thr Glu Leu Met Ser Gly Met Leu Leu Gly
   405 410 415
   ttt gta ttt gta ctt agc cac aat ggg atg gag gtt tat aat tcg tct 1296
   Phe Val Phe Val Leu Ser His Asn Gly Met Glu Val Tyr Asn Ser Ser
               420                 425                 430
   aaa gaa ttc gtg agt gca cag atc gta tcc aca cgg gat atc aaa gga      1344
   Lys Glu Phe Val Ser Ala Gln Ile Val Ser Thr Arg Asp Ile Lys Gly
           435                 440                 445
   aac ata ttc aac gac tgg ttc act ggt ggc ctt aac agg caa ata gag      1392
   Asn Ile Phe Asn Asp Trp Phe Thr Gly Gly Leu Asn Arg Gln Ile Glu
       450                 455                 460
   cat cat ctt ttc cca aca atg ccc agg cat aat tta aac aaa ata gca      1440
   His His Leu Phe Pro Thr Met Pro Arg His Asn Leu Asn Lys Ile Ala
   465                 470                 475                 480
   cct aga gtg gag gtg ttc tgt aag aaa cac ggt ctg gtg tac gaa gac      1488
   Pro Arg Val Glu Val Phe Cys Lys Lys His Gly Leu Val Tyr Glu Asp
                   485                 490                 495
   gta tct att gct acc ggc act tgc aag gtt ttg aaa gca ttg aag gaa      1536
   Val Ser Ile Ala Thr Gly Thr Cys Lys Val Leu Lys Ala Leu Lys Glu
               500                 505                 510
   gtc gcg gag gct gcg gca gag cag cat gct acc acc agt taa              1578
   Val Ala Glu Ala Ala Ala Glu Gln His Ala Thr Thr Ser
           515                 520                 525
<210> 49
   <211> 525
   <212> PRT
   <213> Physcomitrellla patens
<400> 49
   Met Val Phe Ala Gly Gly Gly Leu Gln Gln Gly Ser Leu Glu Glu Asn
   1               5                   10                  15
   Ile Asp Val Glu His Ile Ala Ser Met Ser Leu Phe Ser Asp Phe Phe
               20                  25                  30
   Ser Tyr Val Ser Ser Thr Val Gly Ser Trp Ser Val His Ser Ile Gln
           35                  40                  45
   Pro Leu Lys Arg Leu Thr Ser Lys Lys Arg Val Ser Glu Ser Ala Ala
       50                  55                  60
   Val Gln Cys Ile Ser Ala Glu Val Gln Arg Asn Ser Ser Thr Gln Gly
   65                  70                  75                  80
   Thr Ala Glu Ala Leu Ala Glu Ser Val Val Lys Pro Thr Arg Arg Arg
                   85                  90                  95
   Ser Ser Gln Trp Lys Lys Ser Thr His Pro Leu Ser Glu Val Ala Val
               100                 105                 110
   His Asn Lys Pro Ser Asp Cys Trp Ile Val Val Lys Asn Lys Val Tyr
           115                 120                 125
   Asp Val Ser Asn Phe Ala Asp Glu His Pro Gly Gly Ser Val Ile Ser
       130                 135                 140
   Thr Tyr Phe Gly Arg Asp Gly Thr Asp Val Phe Ser Ser Phe His Ala
   145 150 155 160
   Ala Ser Thr Trp Lys Ile Leu Gln Asp Phe Tyr Ile Gly Asp Val Glu
   165 170 175
   Arg Val Glu Pro Thr Pro Glu Leu Leu Lys Asp Phe Arg Glu Met Arg
   180 185 190
   Ala Leu Phe Leu Arg Glu Gln Leu Phe Lys Ser Ser Lys Leu Tyr Tyr
   195 200 205
   Val Met Lys Leu Leu Thr Asn Val Ala Ile Phe Ala Ala Ser Ile Ala
   210 215 220
   Ile Ile Cys Trp Ser Lys Thr Ile Ser Ala Val Leu Ala Ser Ala Cys
   225 230 235 240
   Met Met Ala Leu Cys Phe Gln Gln Cys Gly Trp Leu Ser His Asp Phe
   245 250 255
   Leu His Asn Gln Val Phe Glu Thr Arg Trp Leu Asn Glu Val Val Gly
   260 265 270
   Tyr Val Ile Gly Asn Ala Val Leu Gly Phe Ser Thr Gly Trp Trp Lys
   275 280 285
   Glu Lys His Asn Leu His His Ala Ala Pro Asn Glu Cys Asp Gln Thr
   290 295 300
   Tyr Gln Pro Ile Asp Glu Asp Ile Asp Thr Leu Pro Leu Ile Ala Trp
   305 310 315 320
   Ser Lys Asp Ile Leu Ala Thr Val Glu Asn Lys Thr Phe Leu Arg Ile
   325 330 335
   Leu Gln Tyr Gln His Leu Phe Phe Met Gly Leu Leu Phe Phe Ala Arg
   340 345 350
   Gly Ser Trp Leu Phe Trp Ser Trp Arg Tyr Thr Ser Thr Ala Val Leu
   355 360 365
   Ser Pro Val Asp Arg Leu Leu Glu Lys Gly Thr Val Leu Phe His Tyr
   370 375 380
   Phe Trp Phe Val Gly Thr Ala Cys Tyr Leu Leu Pro Gly Trp Lys Pro
   385 390 395 400
   Leu Val Trp Met Ala Val Thr Glu Leu Met Ser Gly Met Leu Leu Gly
   405 410 415
   Phe Val Phe Val Leu Ser His Asn Gly Met Glu Val Tyr Asn Ser Ser
               420                 425                 430
   Lys Glu Phe Val Ser Ala Gln Ile Val Ser Thr Arg Asp Ile Lys Gly
           435                 440                 445
   Asn Ile Phe Asn Asp Trp Phe Thr Gly Gly Leu Asn Arg Gln Ile Glu
       450                 455                 460
   His His Leu Phe Pro Thr Met Pro Arg His Asn Leu Asn Lys Ile Ala
   465                 470                 475                 480
   Pro Arg Val Glu Val Phe Cys Lys Lys His Gly Leu Val Tyr Glu Asp
                   485                 490                 495
   Val Ser Ile Ala Thr Gly Thr Cys Lys Val Leu Lys Ala Leu Lys Glu
               500                 505                 510
   Val Ala Glu Ala Ala Ala Glu Gln His Ala Thr Thr Ser
           515                 520                 525
<210> 50
   <211> 1332
   <212> DNA
   <213> Caenorhabditis elegans
<220>
   <221> CDS
   <222> (1)..(1332)
   <223> delta-6-Desaturase
<400> 50
   atg gtc gtc gac aag aat gcc tcc ggg ctt cga atg aag gtc gat ggc      48
   Met Val Val Asp Lys Asn Ala Ser Gly Leu Arg Met Lys Val Asp Gly
   1               5                   10                  15
   aaa tgg ctc tac ctt agc gag gaa ttg gtg aag aaa cat cca gga gga      96
   Lys Trp Leu Tyr Leu Ser Glu Glu Leu Val Lys Lys His Pro Gly Gly
               20                  25                  30
   gct gtt att gaa caa tat aga aat tcg gat gct act cat att ttc cac     144
   Ala Val Ile Glu Gln Tyr Arg Asn Ser Asp Ala Thr His Ile Phe His
           35                  40                  45
   gct ttc cac gaa gga tct tct cag gct tat aag caa ctt gac ctt ctg     192
   Ala Phe His Glu Gly Ser Ser Gln Ala Tyr Lys Gln Leu Asp Leu Leu
       50                  55                  60
   aaa aag cac gga gag cac gat gaa ttc ctt gag aaa caa ttg gaa aag     240
   Lys Lys His Gly Glu His Asp Glu Phe Leu Glu Lys Gln Leu Glu Lys
   65                  70                  75                  80
   aga ctt gac aaa gtt gat atc aat gta tca gca tat gat gtc agt gtt     288
   Arg Leu Asp Lys Val Asp Ile Asn Val Ser Ala Tyr Asp Val Ser Val
                   85                  90                  95
   gca caa gaa aag aaa atg gtt gaa tca ttc gaa aaa cta cga cag aag     336
   Ala Gln Glu Lys Lys Met Val Glu Ser Phe Glu Lys Leu Arg Gln Lys
                   100                 105                 110
   ctt cat gat gat gga tta atg aaa gca aat gaa aca tat ttc ctg ttt      384
   Leu His Asp Asp Gly Leu Met Lys Ala Asn Glu Thr Tyr Phe Leu Phe
           115                 120                 125
   aaa gcg att tca aca ctt tca att atg gca ttt gca ttt tat ctt cag      432
   Lys Ala Ile Ser Thr Leu Ser Ile Met Ala Phe Ala Phe Tyr Leu Gln
       130                 135                 140
   tat ctt gga tgg tat att act tct gca tgt tta tta gca ctt gca tgg      480
   Tyr Leu Gly Trp Tyr Ile Thr Ser Ala Cys Leu Leu Ala Leu Ala Trp
   145                 150                 155                 160
   caa caa ttc gga tgg tta aca cat gag ttc tgc cat caa cag cca aca      528
   Gln Gln Phe Gly Trp Leu Thr His Glu Phe Cys His Gln Gln Pro Thr
                   165                 170                 175
   aag aac aga cct ttg aat gat act att tct ttg ttc ttt ggt aat ttc      576
   Lys Asn Arg Pro Leu Asn Asp Thr Ile Ser Leu Phe Phe Gly Asn Phe
               180                 185                 190
   tta caa gga ttt tca aga gat tgg tgg aag gac aag cat aac act cat      624
   Leu Gln Gly Phe Ser Arg Asp Trp Trp Lys Asp Lys His Asn Thr His
           195                 200                 205
   cac gct gcc aca aat gta att gat cat gac ggt gat atc gac ttg gca      672
   His Ala Ala Thr Asn Val Ile Asp His Asp Gly Asp Ile Asp Leu Ala
       210                 215                 220
   cca ctt ttc gca ttt att cca gga gat ttg tgc aag tat aag gcc agc      720
   Pro Leu Phe Ala Phe Ile Pro Gly Asp Leu Cys Lys Tyr Lys Ala Ser
   225                 230                 235                 240
   ttt gaa aaa gca att ctc aag att gta cca tat caa cat ctc tat ttc      768
   Phe Glu Lys Ala Ile Leu Lys Ile Val Pro Tyr Gln His Leu Tyr Phe
                   245                 250                 255
   acc gca atg ctt cca atg ctc cgt ttc tca tgg act ggt cag tca gtt      816
   Thr Ala Met Leu Pro Met Leu Arg Phe Ser Trp Thr Gly Gln Ser Val
               260                 265                 270
   caa tgg gta ttc aaa gag aat caa atg gag tac aag gtc tat caa aga      864
   Gln Trp Val Phe Lys Glu Asn Gln Met Glu Tyr Lys Val Tyr Gln Arg
           275                 280                 285
   aat gca ttc tgg gag caa gca aca att gtt gga cat tgg gct tgg gta      912
   Asn Ala Phe Trp Glu Gln Ala Thr Ile Val Gly His Trp Ala Trp Val
       290                 295                 300
   ttc tat caa ttg ttc tta tta cca aca tgg cca ctt cgg gtt gct tat      960
   Phe Tyr Gln Leu Phe Leu Leu Pro Thr Trp Pro Leu Arg Val Ala Tyr
   305                 310                 315                 320
   ttc att att tca caa atg gga gga ggc ctt ttg att gct cac gta gtc     1008
   Phe Ile Ile Ser Gln Met Gly Gly Gly Leu Leu Ile Ala His Val Val
                   325                 330                 335
   act ttc aac cat aac tct gtt gat aag tat cca gcc aat tct cga att     1056
   Thr Phe Asn His Asn Ser Val Asp Lys Tyr Pro Ala Asn Ser Arg Ile
               340                 345                 350
   tta aac aac ttc gcc gct ctt caa att ttg acc aca cgc aac atg act     1104
   Leu Asn Asn Phe Ala Ala Leu Gln Ile Leu Thr Thr Arg Asn Met Thr
           355                 360                 365
   cca tct cca ttc att gat tgg ctt tgg ggt gga ctc aat tat cag atc     1152
   Pro Ser Pro Phe Ile Asp Trp Leu Trp Gly Gly Leu Asn Tyr Gln Ile
   370 375 380
   gag cac cac ttg ttc cca aca atg cca cgt tgc aat ctg aat gct tgc 1200
   Glu His His Leu Phe Pro Thr Met Pro Arg Cys Asn Leu Asn Ala Cys
   385 390 395 400
   gtg aaa tat gtg aaa gaa tgg tgc aaa gag aat aat ctt cct tac ctc 1248
   Val Lys Tyr Val Lys Glu Trp Cys Lys Glu Asn Asn Leu Pro Tyr Leu
   405 410 415
   gtc gat gac tac ttt gac gga tat gca atg aat ttg caa caa ttg aaa 1296
   Val Asp Asp Tyr Phe Asp Gly Tyr Ala Met Asn Leu Gln Gln Leu Lys
   420 425 430
   aat atg gct gag cac att caa gct aaa gct gcc taa 1332
   Asn Met Ala Glu His Ile Gln Ala Lys Ala Ala
   435 440
<210> 51
   <211> 443
   <212> PRT
   <213> Caenorhabditis elegans
<400> 51
   Met Val Val Asp Lys Asn Ala Ser Gly Leu Arg Met Lys Val Asp Gly
   1               5                   10                  15
   Lys Trp Leu Tyr Leu Ser Glu Glu Leu Val Lys Lys His Pro Gly Gly
               20                  25                  30
   Ala Val Ile Glu Gln Tyr Arg Asn Ser Asp Ala Thr His Ile Phe His
           35                  40                  45
   Ala Phe His Glu Gly Ser Ser Gln Ala Tyr Lys Gln Leu Asp Leu Leu
       50                  55                  60
   Lys Lys His Gly Glu His Asp Glu Phe Leu Glu Lys Gln Leu Glu Lys
   65                  70                  75                  80
   Arg Leu Asp Lys Val Asp Ile Asn Val Ser Ala Tyr Asp Val Ser Val
                   85                  90                  95
   Ala Gln Glu Lys Lys Met Val Glu Ser Phe Glu Lys Leu Arg Gln Lys
               100                 105                 110
   Leu His Asp Asp Gly Leu Met Lys Ala Asn Glu Thr Tyr Phe Leu Phe
           115                 120                 125
   Lys Ala Ile Ser Thr Leu Ser Ile Met Ala Phe Ala Phe Tyr Leu Gln
       130                 135                 140
   Tyr Leu Gly Trp Tyr Ile Thr Ser Ala Cys Leu Leu Ala Leu Ala Trp
   145                 150                 155                 160
   Gln Gln Phe Gly Trp Leu Thr His Glu Phe Cys His Gln Gln Pro Thr
                   165                 170                 175
   Lys Asn Arg Pro Leu Asn Asp Thr Ile Ser Leu Phe Phe Gly Asn Phe
   180 185 190
   Leu Gln Gly Phe Ser Arg Asp Trp Trp Lys Asp Lys His Asn Thr His
   195 200 205
   His Ala Ala Thr Asn Val Ile Asp His Asp Gly Asp Ile Asp Leu Ala
   210 215 220
   Pro Leu Phe Ala Phe Ile Pro Gly Asp Leu Cys Lys Tyr Lys Ala Ser
   225 230 235 240
   Phe Glu Lys Ala Ile Leu Lys Ile Val Pro Tyr Gln His Leu Tyr Phe
   245 250 255
   Thr Ala Met Leu Pro Met Leu Arg Phe Ser Trp Thr Gly Gln Ser Val
   260 265 270
   Gln Trp Val Phe Lys Glu Asn Gln Met Glu Tyr Lys Val Tyr Gln Arg
   275 280 285
   Asn Ala Phe Trp Glu Gln Ala Thr Ile Val Gly His Trp Ala Trp Val
   290 295 300
   Phe Tyr Gln Leu Phe Leu Leu Pro Thr Trp Pro Leu Arg Val Ala Tyr
   305 310 315 320
   Phe Ile Ile Ser Gln Met Gly Gly Gly Leu Leu Ile Ala His Val Val
   325 330 335
   Thr Phe Asn His Asn Ser Val Asp Lys Tyr Pro Ala Asn Ser Arg Ile
   340 345 350
   Leu Asn Asn Phe Ala Ala Leu Gln Ile Leu Thr Thr Arg Asn Met Thr
   355 360 365
   Pro Ser Pro Phe Ile Asp Trp Leu Trp Gly Gly Leu Asn Tyr Gln Ile
   370 375 380
   Glu His His Leu Phe Pro Thr Met Pro Arg Cys Asn Leu Asn Ala Cys
   385 390 395 400
   Val Lys Tyr Val Lys Glu Trp Cys Lys Glu Asn Asn Leu Pro Tyr Leu
   405 410 415
   Val Asp Asp Tyr Phe Asp Gly Tyr Ala Met Asn Leu Gln Gln Leu Lys
   420 425 430
   Asn Met Ala Glu His Ile Gln Ala Lys Ala Ala
   435 440
<210> 52
   <211> 1362
   <212> DNA
   <213> Primula farinosa
<220>
   <221> CDS
   <222> (1)..(1362)
   <223> delta-6-Desaturase
<400> 52
   atg gct aac aaa tct cca cca aac ccc aaa aca ggt tac ata acc agc      48
   Met Ala Asn Lys Ser Pro Pro Asn Pro Lys Thr Gly Tyr Ile Thr Ser
   1               5                   10                  15
   tca gac ctg aaa tcc cac aac aag gca ggt gac cta tgg ata tca atc      96
   Ser Asp Leu Lys Ser His Asn Lys Ala Gly Asp Leu Trp Ile Ser Ile
               20                  25                  30
   cac ggc caa gtc tac gac gtg tcc tct tgg gcc gcc ctt cat ccg ggg     144
   His Gly Gln Val Tyr Asp Val Ser Ser Trp Ala Ala Leu His Pro Gly
           35                  40                  45
   ggc act gcc cct ctc atg gcc ctt gca gga cac gac gtg acc gat gct     192
   Gly Thr Ala Pro Leu Met Ala Leu Ala Gly His Asp Val Thr Asp Ala
       50                  55                  60
   ttc ctc gcg tac cat ccc cct tcc act gcc cgt ctc ctc cct cct ctc     240
   Phe Leu Ala Tyr His Pro Pro Ser Thr Ala Arg Leu Leu Pro Pro Leu
   65                  70                  75                  80
   tct acc aac ctc ctt ctt caa aac cac tcc gtc tcc ccc acc tcc tca     288
   Ser Thr Asn Leu Leu Leu Gln Asn His Ser Val Ser Pro Thr Ser Ser
                   85                  90                  95
   gac tac cgc aaa ctc ctc gac aac ttc cat aaa cat ggc ctt ttc cgc     336
   Asp Tyr Arg Lys Leu Leu Asp Asn Phe His Lys His Gly Leu Phe Arg
               100                 105                 110
   gcc agg ggc cac act gct tac gcc acc ttc gtc ttc atg ata gcg atg     384
   Ala Arg Gly His Thr Ala Tyr Ala Thr Phe Val Phe Met Ile Ala Met
           115                 120                 125
   ttt cta atg agc gtg act gga gtc ctt tgc agc gac agt gcg tgg gtc     432
   Phe Leu Met Ser Val Thr Gly Val Leu Cys Ser Asp Ser Ala Trp Val
       130                 135                 140
   cat ttg gct agc ggc gga gca atg ggg ttc gcc tgg atc caa tgc gga     480
   His Leu Ala Ser Gly Gly Ala Met Gly Phe Ala Trp Ile Gln Cys Gly
   145                 150                 155                 160
   tgg ata ggt cac gac tct ggg cat tac cgg att atg tct gac agg aaa     528
   Trp Ile Gly His Asp Ser Gly His Tyr Arg Ile Met Ser Asp Arg Lys
                   165                 170                 175
   tgg aac tgg ttc gcg caa atc cta agc aca aac tgc ctc cag ggg att     576
   Trp Asn Trp Phe Ala Gln Ile Leu Ser Thr Asn Cys Leu Gln Gly Ile
               180                 185                 190
   agt atc ggg tgg tgg aag tgg aac cat aat gcg cac cac atc gct tgc     624
   Ser Ile Gly Trp Trp Lys Trp Asn His Asn Ala His His Ile Ala Cys
           195                 200                 205
   aat agc ctg gat tac gac ccc gac ctc cag tat atc cct ttg ctc gtc     672
   Asn Ser Leu Asp Tyr Asp Pro Asp Leu Gln Tyr Ile Pro Leu Leu Val
       210                 215                 220
   gtc tcc ccc aag ttc ttc aac tcc ctt act tct cgt ttc tac gac aag      720
   Val Ser Pro Lys Phe Phe Asn Ser Leu Thr Ser Arg Phe Tyr Asp Lys
   225                 230                 235                 240
   aag ctg aac ttc gac ggc gtg tcg agg ttt ctg gtt tgc tac cag cac      768
   Lys Leu Asn Phe Asp Gly Val Ser Arg Phe Leu Val Cys Tyr Gln His
                   245                 250                 255
   tgg acg ttt tat ccg gtc atg tgt gtc gct agg ctg aac atg ctc gcg      816
   Trp Thr Phe Tyr Pro Val Met Cys Val Ala Arg Leu Asn Met Leu Ala
               260                 265                 270
   cag tca ttt ata acg ctt ttc tcg agt agg gag gtg tgc cat agg gcg      864
   Gln Ser Phe Ile Thr Leu Phe Ser Ser Arg Glu Val Cys His Arg Ala
           275                 280                 285
   caa gag gtt ttc gga ctt gcc gtg ttt tgg gtt tgg ttt ccg ctt tta      912
   Gln Glu Val Phe Gly Leu Ala Val Phe Trp Val Trp Phe Pro Leu Leu
       290                 295                 300
   ctt tct tgt tta cct aat tgg ggc gag agg att atg ttt ttg ctt gcg      960
   Leu Ser Cys Leu Pro Asn Trp Gly Glu Arg Ile Met Phe Leu Leu Ala
   305                 310                 315                 320
   agc tat tcc gtt acg ggg ata caa cac gtg cag ttc agc ttg aac cat     1008
   Ser Tyr Ser Val Thr Gly Ile Gln His Val Gln Phe Ser Leu Asn His
                   325                 330                 335
   ttt tct tcg gac gtc tat gtg ggc ccg cca gta ggt aat gac tgg ttc     1056
   Phe Ser Ser Asp Val Tyr Val Gly Pro Pro Val Gly Asn Asp Trp Phe
               340                 345                 350
   aag aaa cag act gcc ggg aca ctt aac ata tcg tgc ccg gcg tgg atg     1104
   Lys Lys Gln Thr Ala Gly Thr Leu Asn Ile Ser Cys Pro Ala Trp Met
           355                 360                 365
   gat tgg ttc cat ggc ggg tta cag ttt cag gtc gag cac cac ttg ttt     1152
   Asp Trp Phe His Gly Gly Leu Gln Phe Gln Val Glu His His Leu Phe
       370                 375                 380
   ccg cgg atg cct agg ggt cag ttt agg aag att tct cct ttt gtg agg     1200
   Pro Arg Met Pro Arg Gly Gln Phe Arg Lys Ile Ser Pro Phe Val Arg
   385                 390                 395                 400
   gat ttg tgt aag aaa cac aac ttg cct tac aat atc gcg tct ttt act     1248
   Asp Leu Cys Lys Lys His Asn Leu Pro Tyr Asn Ile Ala Ser Phe Thr
                   405                 410                 415
   aaa gcg aat gtg ttt acg ctt aag acg ctg aga aat acg gcc att gag     1296
   Lys Ala Asn Val Phe Thr Leu Lys Thr Leu Arg Asn Thr Ala Ile Glu
               420                 425                 430
   gct cgg gac ctc tct aat ccg ctc cca aag aat atg gtg tgg gaa gct     1344
   Ala Arg Asp Leu Ser Asn Pro Leu Pro Lys Asn Met Val Trp Glu Ala
           435                 440                 445
   ctt aaa act ctc ggg tga                                             1362
   Leu Lys Thr Leu Gly
       450
<210> 53
   <211> 453
   <212> PRT
   <213> Primula farinosa
<400> 53
   Met Ala Asn Lys Ser Pro Pro Asn Pro Lys Thr Gly Tyr Ile Thr Ser
   1               5                   10                  15
   Ser Asp Leu Lys Ser His Asn Lys Ala Gly Asp Leu Trp Ile Ser Ile
               20                  25                  30
   His Gly Gln Val Tyr Asp Val Ser Ser Trp Ala Ala Leu His Pro Gly
           35                  40                  45
   Gly Thr Ala Pro Leu Met Ala Leu Ala Gly His Asp Val Thr Asp Ala
       50                  55                  60
   Phe Leu Ala Tyr His Pro Pro Ser Thr Ala Arg Leu Leu Pro Pro Leu
   65                  70                  75                  80
   Ser Thr Asn Leu Leu Leu Gln Asn His Ser Val Ser Pro Thr Ser Ser
                   85                  90                  95
   Asp Tyr Arg Lys Leu Leu Asp Asn Phe His Lys His Gly Leu Phe Arg
               100                 105                 110
   Ala Arg Gly His Thr Ala Tyr Ala Thr Phe Val Phe Met Ile Ala Met
           115                 120                 125
   Phe Leu Met Ser Val Thr Gly Val Leu Cys Ser Asp Ser Ala Trp Val
       130                 135                 140
   His Leu Ala Ser Gly Gly Ala Met Gly Phe Ala Trp Ile Gln Cys Gly
   145                 150                 155                 160
   Trp Ile Gly His Asp Ser Gly His Tyr Arg Ile Met Ser Asp Arg Lys
                   165                 170                 175
   Trp Asn Trp Phe Ala Gln Ile Leu Ser Thr Asn Cys Leu Gln Gly Ile
               180                 185                 190
   Ser Ile Gly Trp Trp Lys Trp Asn His Asn Ala His His Ile Ala Cys
           195                 200                 205
   Asn Ser Leu Asp Tyr Asp Pro Asp Leu Gln Tyr Ile Pro Leu Leu Val
       210                 215                 220
   Val Ser Pro Lys Phe Phe Asn Ser Leu Thr Ser Arg Phe Tyr Asp Lys
   225                 230                 235                 240
   Lys Leu Asn Phe Asp Gly Val Ser Arg Phe Leu Val Cys Tyr Gln His
                   245                 250                 255
   Trp Thr Phe Tyr Pro Val Met Cys Val Ala Arg Leu Asn Met Leu Ala
               260                 265                 270
   Gln Ser Phe Ile Thr Leu Phe Ser Ser Arg Glu Val Cys His Arg Ala
   275 280 285
   Gln Glu Val Phe Gly Leu Ala Val Phe Trp Val Trp Phe Pro Leu Leu
   290 295 300
   Leu Ser Cys Leu Pro Asn Trp Gly Glu Arg Ile Met Phe Leu Leu Ala
   305 310 315 320
   Ser Tyr Ser Val Thr Gly Ile Gln His Val Gln Phe Ser Leu Asn His
   325 330 335
   Phe Ser Ser Asp Val Tyr Val Gly Pro Pro Val Gly Asn Asp Trp Phe
   340 345 350
   Lys Lys Gln Thr Ala Gly Thr Leu Asn Ile Ser Cys Pro Ala Trp Met
   355 360 365
   Asp Trp Phe His Gly Gly Leu Gln Phe Gln Val Glu His His Leu Phe
   370 375 380
   Pro Arg Met Pro Arg Gly Gln Phe Arg Lys Ile Ser Pro Phe Val Arg
   385 390 395 400
   Asp Leu Cys Lys Lys His Asn Leu Pro Tyr Asn Ile Ala Ser Phe Thr
   405 410 415
   Lys Ala Asn Val Phe Thr Leu Lys Thr Leu Arg Asn Thr Ala Ile Glu
   420 425 430
   Ala Arg Asp Leu Ser Asn Pro Leu Pro Lys Asn Met Val Trp Glu Ala
   435 440 445
   Leu Lys Thr Leu Gly
   450
<210> 54
   <211> 1362
   <212> DNA
   <213> Primula vialii
<220>
   <221> CDS
   <222> (1)..(1362)
   <223> delta-6-Desaturase
<400> 54
   atg gct aac aaa tct cca cca aac ccc aaa aca ggt tac att acc agc      48
   Met Ala Asn Lys Ser Pro Pro Asn Pro Lys Thr Gly Tyr Ile Thr Ser
   1               5                   10                  15
   tca gac ctg aaa ggg cac aac aaa gca gga gac cta tgg ata tca atc      96
   Ser Asp Leu Lys Gly His Asn Lys Ala Gly Asp Leu Trp Ile Ser Ile
                   20                  25                  30
   cac ggg gag gta tac gac gtg tcc tcg tgg gcc ggc ctt cac ccg ggg      144
   His Gly Glu Val Tyr Asp Val Ser Ser Trp Ala Gly Leu His Pro Gly
           35                  40                  45
   ggc agt gcc ccc ctc atg gcc ctc gca gga cac gac gta acc gac gct      192
   Gly Ser Ala Pro Leu Met Ala Leu Ala Gly His Asp Val Thr Asp Ala
       50                  55                  60
   ttt cta gcg tat cat cct cct tct acc gcc cgc ctc ctc cct ccc ctc      240
   Phe Leu Ala Tyr His Pro Pro Ser Thr Ala Arg Leu Leu Pro Pro Leu
   65                  70                  75                  80
   tcc acc aac ctc ctc ctt caa aac cac tcc gtc tcc ccc acc tcc tct      288
   Ser Thr Asn Leu Leu Leu Gln Asn His Ser Val Ser Pro Thr Ser Ser
                   85                  90                  95
   gac tac cgc aaa ctc ctc cac aac ttc cat aaa att ggt atg ttc cgc      336
   Asp Tyr Arg Lys Leu Leu His Asn Phe His Lys Ile Gly Met Phe Arg
               100                 105                 110
   gcc agg ggc cac act gct tac gcc acc ttc gtc atc atg ata gtg atg      384
   Ala Arg Gly His Thr Ala Tyr Ala Thr Phe Val Ile Met Ile Val Met
           115                 120                 125
   ttt cta acg agc gtg acc gga gtc ctt tgc agc gac agt gcg tgg gtc      432
   Phe Leu Thr Ser Val Thr Gly Val Leu Cys Ser Asp Ser Ala Trp Val
       130                 135                 140
   cat ctg gct agc ggc gca gca atg ggg ttc gcc tgg atc cag tgc gga      480
   His Leu Ala Ser Gly Ala Ala Met Gly Phe Ala Trp Ile Gln Cys Gly
   145                 150                 155                 160
   tgg ata ggt cac gac tct ggg cat tac cgg att atg tct gac agg aaa      528
   Trp Ile Gly His Asp Ser Gly His Tyr Arg Ile Met Ser Asp Arg Lys
                   165                 170                 175
   tgg aac tgg ttc gcg cag gtc ctg agc aca aac tgc ctc cag ggg atc      576
   Trp Asn Trp Phe Ala Gln Val Leu Ser Thr Asn Cys Leu Gln Gly Ile
               180                 185                 190
   agt atc ggg tgg tgg aag tgg aac cat aac gcc cac cac att gct tgc      624
   Ser Ile Gly Trp Trp Lys Trp Asn His Asn Ala His His Ile Ala Cys
           195                 200                 205
   aat agc ctg gac tac gac ccc gac ctc cag tat atc cct ttg ctc gtg      672
   Asn Ser Leu Asp Tyr Asp Pro Asp Leu Gln Tyr Ile Pro Leu Leu Val
       210                 215                 220
   gtc tcc ccc aag ttc ttc aac tcc ctt act tct cgt ttc tac gac aag      720
   Val Ser Pro Lys Phe Phe Asn Ser Leu Thr Ser Arg Phe Tyr Asp Lys
   225                 230                 235                 240
   aag ctg aat ttc gac ggc gtg tca agg ttt ctg gtt tgc tac cag cac      768
   Lys Leu Asn Phe Asp Gly Val Ser Arg Phe Leu Val Cys Tyr Gln His
                   245                 250                 255
   tgg acg ttt tat cca gtc atg tgt gtc gct agg cta aac atg atc gca      816
   Trp Thr Phe Tyr Pro Val Met Cys Val Ala Arg Leu Asn Met Ile Ala
               260                 265                 270
   cag tcg ttt ata acg ctt ttc tcg agc agg gag gtg ggt cat agg gcg      864
   Gln Ser Phe Ile Thr Leu Phe Ser Ser Arg Glu Val Gly His Arg Ala
           275                 280                 285
   caa gag att ttc gga ctt gct gtg ttt tgg gtt tgg ttt ccg ctc ctg      912
   Gln Glu Ile Phe Gly Leu Ala Val Phe Trp Val Trp Phe Pro Leu Leu
               290                 295                 300
   ctc tct tgc tta cct aat tgg agc gag agg att atg ttt ctg cta gcg      960
   Leu Ser Cys Leu Pro Asn Trp Ser Glu Arg Ile Met Phe Leu Leu Ala
   305                 310                 315                 320
   agc tat tcc gtt acg ggg ata cag cac gtg cag ttc agc ttg aac cat     1008
   Ser Tyr Ser Val Thr Gly Ile Gln His Val Gln Phe Ser Leu Asn His
                   325                 330                 335
   ttt tct tcg gac gtc tac gtg ggc ccg cca gta gct aac gac tgg ttc     1056
   Phe Ser Ser Asp Val Tyr Val Gly Pro Pro Val Ala Asn Asp Trp Phe
               340                 345                 350
   aag aaa cag act gct ggg aca ctt aac ata tcg tgc ccg gcg tgg atg     1104
   Lys Lys Gln Thr Ala Gly Thr Leu Asn Ile Ser Cys Pro Ala Trp Met
           355                 360                 365
   gac tgg ttc cat ggc ggg ttg cag ttt cag gtc gag cac cac ttg ttt     1152
   Asp Trp Phe His Gly Gly Leu Gln Phe Gln Val Glu His His Leu Phe
       370                 375                 380
   ccg cgg atg cct agg ggt cag ttt agg aag att tct cct ttt gtg agg     1200
   Pro Arg Met Pro Arg Gly Gln Phe Arg Lys Ile Ser Pro Phe Val Arg
   385                 390                 395                 400
   gat ttg tgt aag aaa cac aac ttg cct tac aat atc gcg tct ttt act     1248
   Asp Leu Cys Lys Lys His Asn Leu Pro Tyr Asn Ile Ala Ser Phe Thr
                   405                 410                 415
   aaa gca aac gtg ttg acg ctt aag acg ctg aga aat acg gcc att gag     1296
   Lys Ala Asn Val Leu Thr Leu Lys Thr Leu Arg Asn Thr Ala Ile Glu
               420                 425                 430
   gct cgg gac ctc tct aat ccg acc cca aag aat atg gtg tgg gaa gcc     1344
   Ala Arg Asp Leu Ser Asn Pro Thr Pro Lys Asn Met Val Trp Glu Ala
           435                 440                 445
   gtc cac aca cac ggc tag                                             1362
   Val His Thr His Gly
       450
<210> 55
   <211> 453
   <212> PRT
   <213> Primula vialii
<400> 55
   Met Ala Asn Lys Ser Pro Pro Asn Pro Lys Thr Gly Tyr Ile Thr Ser
   1               5                   10                  15
   Ser Asp Leu Lys Gly His Asn Lys Ala Gly Asp Leu Trp Ile Ser Ile
               20                  25                  30
   His Gly Glu Val Tyr Asp Val Ser Ser Trp Ala Gly Leu His Pro Gly
           35                  40                  45
   Gly Ser Ala Pro Leu Met Ala Leu Ala Gly His Asp Val Thr Asp Ala
       50                  55                  60
   Phe Leu Ala Tyr His Pro Pro Ser Thr Ala Arg Leu Leu Pro Pro Leu
   65                  70                  75                  80
   Ser Thr Asn Leu Leu Leu Gln Asn His Ser Val Ser Pro Thr Ser Ser
                   85                  90                  95
   Asp Tyr Arg Lys Leu Leu His Asn Phe His Lys Ile Gly Met Phe Arg
               100                 105                 110
   Ala Arg Gly His Thr Ala Tyr Ala Thr Phe Val Ile Met Ile Val Met
           115                 120                 125
   Phe Leu Thr Ser Val Thr Gly Val Leu Cys Ser Asp Ser Ala Trp Val
       130                 135                 140
   His Leu Ala Ser Gly Ala Ala Met Gly Phe Ala Trp Ile Gln Cys Gly
   145                 150                 155                 160
   Trp Ile Gly His Asp Ser Gly His Tyr Arg Ile Met Ser Asp Arg Lys
                   165                 170                 175
   Trp Asn Trp Phe Ala Gln Val Leu Ser Thr Asn Cys Leu Gln Gly Ile
               180                 185                 190
   Ser Ile Gly Trp Trp Lys Trp Asn His Asn Ala His His Ile Ala Cys
           195                 200                 205
   Asn Ser Leu Asp Tyr Asp Pro Asp Leu Gln Tyr Ile Pro Leu Leu Val
       210                 215                 220
   Val Ser Pro Lys Phe Phe Asn Ser Leu Thr Ser Arg Phe Tyr Asp Lys
   225                 230                 235                 240
   Lys Leu Asn Phe Asp Gly Val Ser Arg Phe Leu Val Cys Tyr Gln His
                   245                 250                 255
   Trp Thr Phe Tyr Pro Val Met Cys Val Ala Arg Leu Asn Met Ile Ala
               260                 265                 270
   Gln Ser Phe Ile Thr Leu Phe Ser Ser Arg Glu Val Gly His Arg Ala
           275                 280                 285
   Gln Glu Ile Phe Gly Leu Ala Val Phe Trp Val Trp Phe Pro Leu Leu
       290                 295                 300
   Leu Ser Cys Leu Pro Asn Trp Ser Glu Arg Ile Met Phe Leu Leu Ala
   305                 310                 315                 320
   Ser Tyr Ser Val Thr Gly Ile Gln His Val Gln Phe Ser Leu Asn His
                   325                 330                 335
   Phe Ser Ser Asp Val Tyr Val Gly Pro Pro Val Ala Asn Asp Trp Phe
               340                 345                 350
   Lys Lys Gln Thr Ala Gly Thr Leu Asn Ile Ser Cys Pro Ala Trp Met
   355 360 365
   Asp Trp Phe His Gly Gly Leu Gln Phe Gln Val Glu His His Leu Phe
   370 375 380
   Pro Arg Met Pro Arg Gly Gln Phe Arg Lys Ile Ser Pro Phe Val Arg
   385 390 395 400
   Asp Leu Cys Lys Lys His Asn Leu Pro Tyr Asn Ile Ala Ser Phe Thr
   405 410 415
   Lys Ala Asn Val Leu Thr Leu Lys Thr Leu Arg Asn Thr Ala Ile Glu
   420 425 430
   Ala Arg Asp Leu Ser Asn Pro Thr Pro Lys Asn Met Val Trp Glu Ala
   435 440 445
   Val His Thr His Gly
   450
<210> 56
   <211> 1371
   <212> DNA
   <213> Ostreococcus tauri
<220>
   <221> CDS
   <222> (1)..(1371)
   <223> delta-6-Desaturase
<400> 56
   atg tgc gtg gag acg gaa aat aac gat ggg atc ccc acg gtg gag atc      48
   Met Cys Val Glu Thr Glu Asn Asn Asp Gly Ile Pro Thr Val Glu Ile
   1               5                   10                  15
   gcg ttc gac ggt gag cgc gag cgg gcg gag gca aac gtg aag ctg tcc      96
   Ala Phe Asp Gly Glu Arg Glu Arg Ala Glu Ala Asn Val Lys Leu Ser
               20                  25                  30
   gcg gag aag atg gag ccg gcg gcg ctg gcg aag acg ttc gcg agg cgg     144
   Ala Glu Lys Met Glu Pro Ala Ala Leu Ala Lys Thr Phe Ala Arg Arg
           35                  40                  45
   tac gtc gtg atc gag ggg gtg gag tac gat gtg acg gat ttt aag cac     192
   Tyr Val Val Ile Glu Gly Val Glu Tyr Asp Val Thr Asp Phe Lys His
       50                  55                  60
   ccg gga gga acg gtt att ttc tat gcg ttg tca aac acc ggg gcg gac     240
   Pro Gly Gly Thr Val Ile Phe Tyr Ala Leu Ser Asn Thr Gly Ala Asp
   65                  70                  75                  80
   gcg acg gaa gcg ttc aag gag ttt cat cat cgg tcg aga aag gcg agg     288
   Ala Thr Glu Ala Phe Lys Glu Phe His His Arg Ser Arg Lys Ala Arg
                   85                  90                  95
   aaa gcc ttg gcg gcg ctc ccg tct cga ccg gcc aag acg gcc aag gtg     336
   Lys Ala Leu Ala Ala Leu Pro Ser Arg Pro Ala Lys Thr Ala Lys Val
                   100                 105                 110
   gac gac gcg gag atg ctc caa gat ttc gcc aag tgg cgg aaa gaa ttg      384
   Asp Asp Ala Glu Met Leu Gln Asp Phe Ala Lys Trp Arg Lys Glu Leu
           115                 120                 125
   gag aga gat gga ttc ttc aag ccc tct ccg gcg cac gtg gcg tat cgc      432
   Glu Arg Asp Gly Phe Phe Lys Pro Ser Pro Ala His Val Ala Tyr Arg
       130                 135                 140
   ttc gcc gag ctc gcg gcg atg tac gct ctc ggg acg tac ctg atg tac      480
   Phe Ala Glu Leu Ala Ala Met Tyr Ala Leu Gly Thr Tyr Leu Met Tyr
   145                 150                 155                 160
   gct cga tac gtc gtc tcc tcg gtg ctc gtg tac gct tgc ttt ttc ggc      528
   Ala Arg Tyr Val Val Ser Ser Val Leu Val Tyr Ala Cys Phe Phe Gly
                   165                 170                 175
   gcc cga tgc ggt tgg gtg cag cac gag ggc gga cac agc tcg ctg acg      576
   Ala Arg Cys Gly Trp Val Gln His Glu Gly Gly His Ser Ser Leu Thr
               180                 185                 190
   ggc aac att tgg tgg gac aag cgc atc cag gcc ttc aca gcc ggg ttc      624
   Gly Asn Ile Trp Trp Asp Lys Arg Ile Gln Ala Phe Thr Ala Gly Phe
           195                 200                 205
   ggt ctc gcc ggt agc ggc gac atg tgg aac tcg atg cac aac aag cat      672
   Gly Leu Ala Gly Ser Gly Asp Met Trp Asn Ser Met His Asn Lys His
       210                 215                 220
   cac gcg acg cct caa aag gtt cgt cac gac atg gat ctg gac acc acc      720
   His Ala Thr Pro Gln Lys Val Arg His Asp Met Asp Leu Asp Thr Thr
   225                 230                 235                 240
   ccc gcg gtg gcg ttc ttc aac acc gcg gtg gaa gac aat cgt ccc cgt      768
   Pro Ala Val Ala Phe Phe Asn Thr Ala Val Glu Asp Asn Arg Pro Arg
                   245                 250                 255
   ggc ttt agc aag tac tgg ttg cgc ctt cag gcg tgg acc ttc atc ccc      816
   Gly Phe Ser Lys Tyr Trp Leu Arg Leu Gln Ala Trp Thr Phe Ile Pro
               260                 265                 270
   gtg acg tcc ggc ttg gtg ctc ctt ttc tgg atg ttt ttc ctc cac ccc      864
   Val Thr Ser Gly Leu Val Leu Leu Phe Trp Met Phe Phe Leu His Pro
           275                 280                 285
   tcc aag gct ttg aag ggt ggc aag tac gaa gag ttg gtg tgg atg ctc      912
   Ser Lys Ala Leu Lys Gly Gly Lys Tyr Glu Glu Leu Val Trp Met Leu
       290                 295                 300
   gcc gcg cac gtc atc cgc acg tgg acg atc aag gcg gtg acc gga ttc      960
   Ala Ala His Val Ile Arg Thr Trp Thr Ile Lys Ala Val Thr Gly Phe
   305                 310                 315                 320
   acc gcg atg cag tcc tac ggc tta ttt ttg gcg acg agc tgg gtg agc     1008
   Thr Ala Met Gln Ser Tyr Gly Leu Phe Leu Ala Thr Ser Trp Val Ser
                   325                 330                 335
   ggc tgc tat ctg ttt gca cac ttc tcc acg tcg cac acg cac ctg gat     1056
   Gly Cys Tyr Leu Phe Ala His Phe Ser Thr Ser His Thr His Leu Asp
               340                 345                 350
   gtg gtg ccc gcg gac gag cat ctc tcc tgg gtt cga tac gcc gtc gat     1104
   Val Val Pro Ala Asp Glu His Leu Ser Trp Val Arg Tyr Ala Val Asp
           355                 360                 365
   cac acg atc gac atc gat ccg agt caa ggt tgg gtg aac tgg ttg atg     1152
   His Thr Ile Asp Ile Asp Pro Ser Gln Gly Trp Val Asn Trp Leu Met
       370                 375                 380
   ggc tac ctc aac tgc caa gtc atc cac cac ctc ttt ccg agc atg ccg        1200
   Gly Tyr Leu Asn Cys Gln Val Ile His His Leu Phe Pro Ser Met Pro
   385                 390                 395                 400
   cag ttc cgc cag ccc gag gta tct cgc cgc ttc gtc gcc ttt gcg aaa        1248
   Gln Phe Arg Gln Pro Glu Val Ser Arg Arg Phe Val Ala Phe Ala Lys
                   405                 410                 415
   aag tgg aac ctc aac tac aag gtc atg acc tac gcc ggt gcg tgg aag        1296
   Lys Trp Asn Leu Asn Tyr Lys Val Met Thr Tyr Ala Gly Ala Trp Lys
               420                 425                 430
   gca acg ctc gga aac ctc gac aac gtg ggt aag cac tac tac gtg cac        1344
   Ala Thr Leu Gly Asn Leu Asp Asn Val Gly Lys His Tyr Tyr Val His
           435                 440                 445
   ggc caa cac tcc gga aag acg gcg taa                                    1371
   Gly Gln His Ser Gly Lys Thr Ala
       450                 455
<210> 57
   <211> 456
   <212> PRT
   <213> Ostreococcus tauri
<400> 57
   Met Cys Val Glu Thr Glu Asn Asn Asp Gly Ile Pro Thr Val Glu Ile
   1               5                   10                  15
   Ala Phe Asp Gly Glu Arg Glu Arg Ala Glu Ala Asn Val Lys Leu Ser
               20                  25                  30
   Ala Glu Lys Met Glu Pro Ala Ala Leu Ala Lys Thr Phe Ala Arg Arg
           35                  40                  45
   Tyr Val Val Ile Glu Gly Val Glu Tyr Asp Val Thr Asp Phe Lys His
       50                  55                  60
   Pro Gly Gly Thr Val Ile Phe Tyr Ala Leu Ser Asn Thr Gly Ala Asp
   65                  70                  75                  80
   Ala Thr Glu Ala Phe Lys Glu Phe His His Arg Ser Arg Lys Ala Arg
                   85                  90                  95
   Lys Ala Leu Ala Ala Leu Pro Ser Arg Pro Ala Lys Thr Ala Lys Val
               100                 105                 110
   Asp Asp Ala Glu Met Leu Gln Asp Phe Ala Lys Trp Arg Lys Glu Leu
           115                 120                 125
   Glu Arg Asp Gly Phe Phe Lys Pro Ser Pro Ala His Val Ala Tyr Arg
       130                 135                 140
   Phe Ala Glu Leu Ala Ala Met Tyr Ala Leu Gly Thr Tyr Leu Met Tyr
   145                 150                 155                 160
   Ala Arg Tyr Val Val Ser Ser Val Leu Val Tyr Ala Cys Phe Phe Gly
                   165                 170                 175
   Ala Arg Cys Gly Trp Val Gln His Glu Gly Gly His Ser Ser Leu Thr
               180                 185                 190
   Gly Asn Ile Trp Trp Asp Lys Arg Ile Gln Ala Phe Thr Ala Gly Phe
           195                 200                 205
   Gly Leu Ala Gly Ser Gly Asp Met Trp Asn Ser Met His Asn Lys His
       210                 215                 220
   His Ala Thr Pro Gln Lys Val Arg His Asp Met Asp Leu Asp Thr Thr
   225                 230                 235                 240
   Pro Ala Val Ala Phe Phe Asn Thr Ala Val Glu Asp Asn Arg Pro Arg
                   245                 250                 255
   Gly Phe Ser Lys Tyr Trp Leu Arg Leu Gln Ala Trp Thr Phe Ile Pro
               260                 265                 270
   Val Thr Ser Gly Leu Val Leu Leu Phe Trp Met Phe Phe Leu His Pro
           275                 280                 285
   Ser Lys Ala Leu Lys Gly Gly Lys Tyr Glu Glu Leu Val Trp Met Leu
       290                 295                 300
   Ala Ala His Val Ile Arg Thr Trp Thr Ile Lys Ala Val Thr Gly Phe
   305                 310                 315                 320
   Thr Ala Met Gln Ser Tyr Gly Leu Phe Leu Ala Thr Ser Trp Val Ser
                   325                 330                 335
   Gly Cys Tyr Leu Phe Ala His Phe Ser Thr Ser His Thr His Leu Asp
               340                 345                 350
   Val Val Pro Ala Asp Glu His Leu Ser Trp Val Arg Tyr Ala Val Asp
           355                 360                 365
   His Thr Ile Asp Ile Asp Pro Ser Gln Gly Trp Val Asn Trp Leu Met
       370                 375                 380
   Gly Tyr Leu Asn Cys Gln Val Ile His His Leu Phe Pro Ser Met Pro
   385                 390                 395                 400
   Gln Phe Arg Gln Pro Glu Val Ser Arg Arg Phe Val Ala Phe Ala Lys
                   405                 410                 415
   Lys Trp Asn Leu Asn Tyr Lys Val Met Thr Tyr Ala Gly Ala Trp Lys
               420                 425                 430
   Ala Thr Leu Gly Asn Leu Asp Asn Val Gly Lys His Tyr Tyr Val His
           435                 440                 445
   Gly Gln His Ser Gly Lys Thr Ala
       450                 455
<210> 58
   <211> 1455
   <212> DNA
   <213> Thalassiosira pseudonana
<220>
   <221> CDS
   <222> (1)..(1455)
   <223> delta-6-Desaturase
<400> 58
   atg gga aaa gga gga gac gca gcc gca gct acc aag cgt agt gga gca    48
   Met Gly Lys Gly Gly Asp Ala Ala Ala Ala Thr Lys Arg Ser Gly Ala
   1               5                   10                  15
   ttg aaa ttg gcg gag aag ccg cag aag tac act tgg cag gag gtg aag    96
   Leu Lys Leu Ala Glu Lys Pro Gln Lys Tyr Thr Trp Gln Glu Val Lys
               20                  25                  30
   aag cac atc acc ccc gac gat gcc tgg gta gtc cac caa aac aaa gtc    144
   Lys His Ile Thr Pro Asp Asp Ala Trp Val Val His Gln Asn Lys Val
           35                  40                  45
   tac gac gtc tcc aac tgg tac gac cac ccc ggt gga gcc gtg gtg ttc    192
   Tyr Asp Val Ser Asn Trp Tyr Asp His Pro Gly Gly Ala Val Val Phe
       50                  55                  60
   acc cac gcc gga gac gac atg acg gac atc ttc gcc gcc ttc cac gcc    240
   Thr His Ala Gly Asp Asp Met Thr Asp Ile Phe Ala Ala Phe His Ala
   65                  70                  75                  80
   caa ggc tct cag gcc atg atg aag aag ttt tac att gga gat ttg att    288
   Gln Gly Ser Gln Ala Met Met Lys Lys Phe Tyr Ile Gly Asp Leu Ile
                   85                  90                  95
   ccg gag agt gtg gag cat aag gat caa aga cag ttg gat ttc gag aag    336
   Pro Glu Ser Val Glu His Lys Asp Gln Arg Gln Leu Asp Phe Glu Lys
               100                 105                 110
   gga tat cgt gat tta cgg gcc aag ctt gtc atg atg ggg atg ttc aag    384
   Gly Tyr Arg Asp Leu Arg Ala Lys Leu Val Met Met Gly Met Phe Lys
           115                 120                 125
   tcg agt aag atg tat tat gca tac aag tgc tcg ttc aat atg tgc atg    432
   Ser Ser Lys Met Tyr Tyr Ala Tyr Lys Cys Ser Phe Asn Met Cys Met
       130                 135                 140
   tgg ttg gtg gcg gtg gcc atg gtg tac tac tcg gac agt ttg gca atg    480
   Trp Leu Val Ala Val Ala Met Val Tyr Tyr Ser Asp Ser Leu Ala Met
   145                 150                 155                 160
   cac att gga tcg gct ctc ttg ttg gga ttg ttc tgg cag cag tgt gga    528
   His Ile Gly Ser Ala Leu Leu Leu Gly Leu Phe Trp Gln Gln Cys Gly
                   165                 170                 175
   tgg ctt gcg cac gac ttt ctt cac cac caa gtc ttt aag caa cga aag    576
   Trp Leu Ala His Asp Phe Leu His His Gln Val Phe Lys Gln Arg Lys
               180                     185                     190
   tac gga gat ctc gtt ggc atc ttt tgg gga gat ctc atg cag ggg ttc      624
   Tyr Gly Asp Leu Val Gly Ile Phe Trp Gly Asp Leu Met Gln Gly Phe
           195                 200                 205
   tcg atg cag tgg tgg aag aac aag cac aat ggc cac cat gct gtt ccc      672
   Ser Met Gln Trp Trp Lys Asn Lys His Asn Gly His His Ala Val Pro
       210                 215                 220
   aac ttg cac aac tct tcc ttg gac agt cag gat ggt gat ccc gat att      720
   Asn Leu His Asn Ser Ser Leu Asp Ser Gln Asp Gly Asp Pro Asp Ile
   225                 230                 235                 240
   gat acc atg cca ctc ctt gct tgg agt ctc aag cag gct cag agt ttc      768
   Asp Thr Met Pro Leu Leu Ala Trp Ser Leu Lys Gln Ala Gln Ser Phe
                   245                 250                 255
   aga gag atc aat aag gga aag gac agt acc ttc gtc aag tac gct atc      816
   Arg Glu Ile Asn Lys Gly Lys Asp Ser Thr Phe Val Lys Tyr Ala Ile
               260                 265                 270
   aaa ttc cag gca ttc aca tac ttc ccc atc ctc ctc ttg gct cgc atc      864
   Lys Phe Gln Ala Phe Thr Tyr Phe Pro Ile Leu Leu Leu Ala Arg Ile
           275                 280                 285
   tct tgg ttg aat gaa tcc ttc aaa act gca ttc gga ctc gga gct gcc      912
   Ser Trp Leu Asn Glu Ser Phe Lys Thr Ala Phe Gly Leu Gly Ala Ala
       290                 295                 300
   tcg gag aat gcc aag ttg gag ttg gag aag cgt gga ctt cag tac cca      960
   Ser Glu Asn Ala Lys Leu Glu Leu Glu Lys Arg Gly Leu Gln Tyr Pro
   305                 310                 315                 320
   ctt ttg gag aag ctt gga atc acc ctt cat tac act tgg atg ttc gtc     1008
   Leu Leu Glu Lys Leu Gly Ile Thr Leu His Tyr Thr Trp Met Phe Val
                   325                 330                 335
   ctc tct tcc gga ttt gga agg tgg tct ctt cca tat tcc atc atg tat     1056
   Leu Ser Ser Gly Phe Gly Arg Trp Ser Leu Pro Tyr Ser Ile Met Tyr
               340                 345                 350
   ttc ttc act gcc aca tgc tcc tcg gga ctt ttc ctc gca ttg gtc ttt     1104
   Phe Phe Thr Ala Thr Cys Ser Ser Gly Leu Phe Leu Ala Leu Val Phe
           355                 360                 365
   gga ttg gga cac aac ggt atg tca gtg tac gat gcc acc acc cga cct     1152
   Gly Leu Gly His Asn Gly Met Ser Val Tyr Asp Ala Thr Thr Arg Pro
       370                 375                 380
   gac ttc tgg caa ctc caa gtc acc act aca cgt aac atc att ggt gga     1200
   Asp Phe Trp Gln Leu Gln Val Thr Thr Thr Arg Asn Ile Ile Gly Gly
   385                 390                 395                 400
   cac ggc att ccc caa ttc ttt gtg gat tgg ttc tgc ggt gga ttg caa     1248
   His Gly Ile Pro Gln Phe Phe Val Asp Trp Phe Cys Gly Gly Leu Gln
                   405                 410                 415
   tac caa gtg gat cac cac ctc ttc ccc atg atg cct aga aac aat atc     1296
   Tyr Gln Val Asp His His Leu Phe Pro Met Met Pro Arg Asn Asn Ile
               420                 425                 430
   gcg aaa tgc cac aag ctt gtg gag tca ttc tgt aag gag tgg ggt gtg     1344
   Ala Lys Cys His Lys Leu Val Glu Ser Phe Cys Lys Glu Trp Gly Val
           435                 440                 445
   aag tac cat gag gcc gat atg tgg gat ggt acc gtg gaa gtg ttg caa     1392
   Lys Tyr His Glu Ala Asp Met Trp Asp Gly Thr Val Glu Val Leu Gln
       450                 455                 460
   cat ctc tcc aag gtg tcg gat gat ttc ctt gtg gag atg gtg aag gat      1440
   His Leu Ser Lys Val Ser Asp Asp Phe Leu Val Glu Met Val Lys Asp
   465                 470                 475                 480
   ttc cct gcc atg taa                                                  1455
   Phe Pro Ala Met
<210> 59
   <211> 484
   <212> PRT
   <213> Thalassiosira pseudonana
<400> 59
   Met Gly Lys Gly Gly Asp Ala Ala Ala Ala Thr Lys Arg Ser Gly Ala
   1               5                   10                  15
   Leu Lys Leu Ala Glu Lys Pro Gln Lys Tyr Thr Trp Gln Glu Val Lys
               20                  25                  30
   Lys His Ile Thr Pro Asp Asp Ala Trp Val Val His Gln Asn Lys Val
           35                  40                  45
   Tyr Asp Val Ser Asn Trp Tyr Asp His Pro Gly Gly Ala Val Val Phe
       50                  55                  60
   Thr His Ala Gly Asp Asp Met Thr Asp Ile Phe Ala Ala Phe His Ala
   65                  70                  75                  80
   Gln Gly Ser Gln Ala Met Met Lys Lys Phe Tyr Ile Gly Asp Leu Ile
                   85                  90                  95
   Pro Glu Ser Val Glu His Lys Asp Gln Arg Gln Leu Asp Phe Glu Lys
               100                 105                 110
   Gly Tyr Arg Asp Leu Arg Ala Lys Leu Val Met Met Gly Met Phe Lys
           115                 120                 125
   Ser Ser Lys Met Tyr Tyr Ala Tyr Lys Cys Ser Phe Asn Met Cys Met
       130                 135                 140
   Trp Leu Val Ala Val Ala Met Val Tyr Tyr Ser Asp Ser Leu Ala Met
   145                 150                 155                 160
   His Ile Gly Ser Ala Leu Leu Leu Gly Leu Phe Trp Gln Gln Cys Gly
                   165                 170                 175
   Trp Leu Ala His Asp Phe Leu His His Gln Val Phe Lys Gln Arg Lys
               180                 185                 190
   Tyr Gly Asp Leu Val Gly Ile Phe Trp Gly Asp Leu Met Gln Gly Phe
           195                 200                 205
   Ser Met Gln Trp Trp Lys Asn Lys His Asn Gly His His Ala Val Pro
   210 215 220
   Asn Leu His Asn Ser Ser Leu Asp Ser Gln Asp Gly Asp Pro Asp Ile
   225 230 235 240
   Asp Thr Met Pro Leu Leu Ala Trp Ser Leu Lys Gln Ala Gln Ser Phe
   245 250 255
   Arg Glu Ile Asn Lys Gly Lys Asp Ser Thr Phe Val Lys Tyr Ala Ile
   260 265 270
   Lys Phe Gln Ala Phe Thr Tyr Phe Pro Ile Leu Leu Leu Ala Arg Ile
   275 280 285
   Ser Trp Leu Asn Glu Ser Phe Lys Thr Ala Phe Gly Leu Gly Ala Ala
   290 295 300
   Ser Glu Asn Ala Lys Leu Glu Leu Glu Lys Arg Gly Leu Gln Tyr Pro
   305 310 315 320
   Leu Leu Glu Lys Leu Gly Ile Thr Leu His Tyr Thr Trp Met Phe Val
   325 330 335
   Leu Ser Ser Gly Phe Gly Arg Trp Ser Leu Pro Tyr Ser Ile Met Tyr
   340 345 350
   Phe Phe Thr Ala Thr Cys Ser Ser Gly Leu Phe Leu Ala Leu Val Phe
   355 360 365
   Gly Leu Gly His Asn Gly Met Ser Val Tyr Asp Ala Thr Thr Arg Pro
   370 375 380
   Asp Phe Trp Gln Leu Gln Val Thr Thr Thr Arg Asn Ile Ile Gly Gly
   385 390 395 400
   His Gly Ile Pro Gln Phe Phe Val Asp Trp Phe Cys Gly Gly Leu Gln
   405 410 415
   Tyr Gln Val Asp His His Leu Phe Pro Met Met Pro Arg Asn Asn Ile
   420 425 430
   Ala Lys Cys His Lys Leu Val Glu Ser Phe Cys Lys Glu Trp Gly Val
   435 440 445
   Lys Tyr His Glu Ala Asp Met Trp Asp Gly Thr Val Glu Val Leu Gln
   450 455 460
   His Leu Ser Lys Val Ser Asp Asp Phe Leu Val Glu Met Val Lys Asp
   465 470 475 480
   Phe Pro Ala Met
<210> 60
   <211> 873
   <212> DNA
   <213> Physcomitrella patens
<220>
   <221> CDS
   <222> (1)..(873)
   <223> delta-6-Elongase
<400> 60
   atg gag gtc gtg gag aga ttc tac ggt gag ttg gat ggg aag gtc tcg      48
   Met Glu Val Val Glu Arg Phe Tyr Gly Glu Leu Asp Gly Lys Val Ser
   1               5                   10                  15
   cag ggc gtg aat gca ttg ctg ggt agt ttt ggg gtg gag ttg acg gat      96
   Gln Gly Val Asn Ala Leu Leu Gly Ser Phe Gly Val Glu Leu Thr Asp
               20                  25                  30
   acg ccc act acc aaa ggc ttg ccc ctc gtt gac agt ccc aca ccc atc     144
   Thr Pro Thr Thr Lys Gly Leu Pro Leu Val Asp Ser Pro Thr Pro Ile
           35                  40                  45
   gtc ctc ggt gtt tct gta tac ttg act att gtc att gga ggg ctt ttg     192
   Val Leu Gly Val Ser Val Tyr Leu Thr Ile Val Ile Gly Gly Leu Leu
       50                  55                  60
   tgg ata aag gcc agg gat ctg aaa ccg cgc gcc tcg gag cca ttt ttg     240
   Trp Ile Lys Ala Arg Asp Leu Lys Pro Arg Ala Ser Glu Pro Phe Leu
   65                  70                  75                  80
   ctc caa gct ttg gtg ctt gtg cac aac ctg ttc tgt ttt gcg ctc agt     288
   Leu Gln Ala Leu Val Leu Val His Asn Leu Phe Cys Phe Ala Leu Ser
                   85                  90                  95
   ctg tat atg tgc gtg ggc atc gct tat cag gct att acc tgg cgg tac     336
   Leu Tyr Met Cys Val Gly Ile Ala Tyr Gln Ala Ile Thr Trp Arg Tyr
               100                 105                 110
   tct ctc tgg ggc aat gca tac aat cct aaa cat aaa gag atg gcg att     384
   Ser Leu Trp Gly Asn Ala Tyr Asn Pro Lys His Lys Glu Met Ala Ile
           115                 120                 125
   ctg gta tac ttg ttc tac atg tct aag tac gtg gaa ttc atg gat acc     432
   Leu Val Tyr Leu Phe Tyr Met Ser Lys Tyr Val Glu Phe Met Asp Thr
       130                 135                 140
   gtt atc atg ata ctg aag cgc agc acc agg caa ata agc ttc ctc cac     480
   Val Ile Met Ile Leu Lys Arg Ser Thr Arg Gln Ile Ser Phe Leu His
   145                 150                 155                 160
   gtt tat cat cat tct tca att tcc ctc att tgg tgg gct att gct cat     528
   Val Tyr His His Ser Ser Ile Ser Leu Ile Trp Trp Ala Ile Ala His
                   165                 170                 175
   cac gct cct ggc ggt gaa gca tat tgg tct gcg gct ctg aac tca gga     576
   His Ala Pro Gly Gly Glu Ala Tyr Trp Ser Ala Ala Leu Asn Ser Gly
               180                 185                 190
   gtg cat gtt ctc atg tat gcg tat tac ttc ttg gct gcc tgc ctt cga     624
   Val His Val Leu Met Tyr Ala Tyr Tyr Phe Leu Ala Ala Cys Leu Arg
           195                 200                 205
   agt agc cca aag tta aaa aat aag tac ctt ttt tgg ggc agg tac ttg        672
   Ser Ser Pro Lys Leu Lys Asn Lys Tyr Leu Phe Trp Gly Arg Tyr Leu
       210                 215                 220
   aca caa ttc caa atg ttc cag ttt atg ctg aac tta gtg cag gct tac        720
   Thr Gln Phe Gln Met Phe Gln Phe Met Leu Asn Leu Val Gln Ala Tyr
   225                 230                 235                     240
   tac gac atg aaa acg aat gcg cca tat cca caa tgg ctg atc aag att        768
   Tyr Asp Met Lys Thr Asn Ala Pro Tyr Pro Gln Trp Leu Ile Lys Ile
                   245                 250                 255
   ttg ttc tac tac atg atc tcg ttg ctg ttt ctt ttc ggc aat ttt tac        816
   Leu Phe Tyr Tyr Met Ile Ser Leu Leu Phe Leu Phe Gly Asn Phe Tyr
               260                 265                 270
   gta caa aaa tac atc aaa ccc tct gac gga aag caa aag gga gct aaa        864
   Val Gln Lys Tyr Ile Lys Pro Ser Asp Gly Lys Gln Lys Gly Ala Lys
           275                 280                 285
   act gag tga                                                            873
   Thr Glu
       290
<210> 61
   <211> 290
   <212> PRT
   <213> Physcomitrella patens
<400> 61
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145 150 155 160
   Val Tyr His His Ser Ser Ile Ser Leu Ile Trp Trp Ala Ile Ala His
   165 170 175
   His Ala Pro Gly Gly Glu Ala Tyr Trp Ser Ala Ala Leu Asn Ser Gly
   180 185 190
   Val His Val Leu Met Tyr Ala Tyr Tyr Phe Leu Ala Ala Cys Leu Arg
   195 200 205
   Ser Ser Pro Lys Leu Lys Asn Lys Tyr Leu Phe Trp Gly Arg Tyr Leu
   210 215 220
   Thr Gln Phe Gln Met Phe Gln Phe Met Leu Asn Leu Val Gln Ala Tyr
   225 230 235 240
   Tyr Asp Met Lys Thr Asn Ala Pro Tyr Pro Gln Trp Leu Ile Lys Ile
   245 250 255
   Leu Phe Tyr Tyr Met Ile Ser Leu Leu Phe Leu Phe Gly Asn Phe Tyr
   260 265 270
   Val Gln Lys Tyr Ile Lys Pro Ser Asp Gly Lys Gln Lys Gly Ala Lys
   275 280 285
   Thr Glu
   290
<210> 62
   <211> 1049
   <212> DNA
   <213> Thraustochytrium
<220>
   <221> CDS
   <222> (43)..(858)
   <223> delta-6-Elongase
<400> 62
   gaattcggca cgagagcgcg cggagcggag acctcggccg cg atg atg gag ccg         54
                                                  Met Met Glu Pro
                                                  1
   ctc gac agg tac agg gcg ctg gcg gag ctc gcc gcg agg tac gcc agc       102
   Leu Asp Arg Tyr Arg Ala Leu Ala Glu Leu Ala Ala Arg Tyr Ala Ser
   5                   10                  15                  20
   tcg gcg gcc ttc aag tgg caa gtc acg tac gac gcc aag gac agc ttc       150
   Ser Ala Ala Phe Lys Trp Gln Val Thr Tyr Asp Ala Lys Asp Ser Phe
                   25                  30                  35
   gtc ggg ccc ctg gga atc cgg gag ccg ctc ggg ctc ctg gtg ggc tcc       198
   Val Gly Pro Leu Gly Ile Arg Glu Pro Leu Gly Leu Leu Val Gly Ser
                   40                         45                        50
   gtg gtc ctc tac ctg agc ctg ctg gcc gtg gtc tac gcg ctg cgg aac      246
   Val Val Leu Tyr Leu Ser Leu Leu Ala Val Val Tyr Ala Leu Arg Asn
           55                  60                  65
   tac ctt ggc ggc ctc atg gcg ctc cgc agc gtg cat aac ctc ggg ctc      294
   Tyr Leu Gly Gly Leu Met Ala Leu Arg Ser Val His Asn Leu Gly Leu
       70                  75                  80
   tgc ctc ttc tcg ggc gcc gtg tgg atc tac acg agc tac ctc atg atc      342
   Cys Leu Phe Ser Gly Ala Val Trp Ile Tyr Thr Ser Tyr Leu Met Ile
   85                  90                  95                  100
   cag gat ggg cac ttt cgc agc ctc gag gcg gca acg tgc gag ccg ctc      390
   Gln Asp Gly His Phe Arg Ser Leu Glu Ala Ala Thr Cys Glu Pro Leu
                   105                 110                 115
   aag cat ccg cac ttc cag ctc atc agc ttg ctc ttt gcg ctg tcc aag      438
   Lys His Pro His Phe Gln Leu Ile Ser Leu Leu Phe Ala Leu Ser Lys
               120                 125                 130
   atc tgg gag tgg ttc gac acg gtg ctc ctc atc gtc aag ggc aac aag      486
   Ile Trp Glu Trp Phe Asp Thr Val Leu Leu Ile Val Lys Gly Asn Lys
           135                 140                 145
   ctc cgc ttc ctg cac gtc ttg cac cac gcc acg acc ttt tgg ctc tac      534
   Leu Arg Phe Leu His Val Leu His His Ala Thr Thr Phe Trp Leu Tyr
       150                 155                 160
   gcc atc gac cac atc ttt ctc tcg tcc atc aag tac ggc gtc gcg gtc      582
   Ala Ile Asp His Ile Phe Leu Ser Ser Ile Lys Tyr Gly Val Ala Val
   165                 170                 175                 180
   aat gct ttc atc cac acc gtc atg tac gcg cac tac ttc cgc cca ttc      630
   Asn Ala Phe Ile His Thr Val Met Tyr Ala His Tyr Phe Arg Pro Phe
                   185                 190                 195
   ccg aag ggc ttg cgc ccg ctt att acg cag ttg cag atc gtc cag ttc      678
   Pro Lys Gly Leu Arg Pro Leu Ile Thr Gln Leu Gln Ile Val Gln Phe
               200                 205                 210
   att ttc agc atc ggc atc cat acc gcc att tac tgg cac tac gac tgc      726
   Ile Phe Ser Ile Gly Ile His Thr Ala Ile Tyr Trp His Tyr Asp Cys
           215                 220                 225
   gag ccg ctc gtg cat acc cac ttt tgg gaa tac gtc acg ccc tac ctt      774
   Glu Pro Leu Val His Thr His Phe Trp Glu Tyr Val Thr Pro Tyr Leu
       230                 235                 240
   ttc gtc gtg ccc ttc ctc atc ctc ttt ttc aat ttt tac ctg cag cag      822
   Phe Val Val Pro Phe Leu Ile Leu Phe Phe Asn Phe Tyr Leu Gln Gln
   245                 250                 255                 260
   tac gtc ctc gcg ccc gca aaa acc aag aag gca tag ccacgtaaca           868
   Tyr Val Leu Ala Pro Ala Lys Thr Lys Lys Ala
                   265                 270
   gtagaccagc agcgccgagg acgcgtgccg cgttatcgcg aagcacgaaa taaagaagat    928
   catttgattc aacgaggcta cttgcggcca cgagaaaaaa aaaaaaaaaa aaaaaaaaaa    988
   aaaaaaaaaa aaaaaaaaaa aaaaaaaaaa aaaaaaaaaa aaaaaaaaaa aaaaaaaaaa   1048
   c                                                                   1049
<210> 63
   <211> 271
   <212> PRT
   <213> Thraustochytrium
<400> 63
   Met Met Glu Pro Leu Asp Arg Tyr Arg Ala Leu Ala Glu Leu Ala Ala
   1               5                   10                  15
   Arg Tyr Ala Ser Ser Ala Ala Phe Lys Trp Gln Val Thr Tyr Asp Ala
               20                  25                  30
   Lys Asp Ser Phe Val Gly Pro Leu Gly Ile Arg Glu Pro Leu Gly Leu
           35                  40                  45
   Leu Val Gly Ser Val Val Leu Tyr Leu Ser Leu Leu Ala Val Val Tyr
       50                  55                  60
   Ala Leu Arg Asn Tyr Leu Gly Gly Leu Met Ala Leu Arg Ser Val His
   65                  70                  75                  80
   Asn Leu Gly Leu Cys Leu Phe Ser Gly Ala Val Trp Ile Tyr Thr Ser
                   85                  90                  95
   Tyr Leu Met Ile Gln Asp Gly His Phe Arg Ser Leu Glu Ala Ala Thr
               100                 105                 110
   Cys Glu Pro Leu Lys His Pro His Phe Gln Leu Ile Ser Leu Leu Phe
           115                 120                 125
   Ala Leu Ser Lys Ile Trp Glu Trp Phe Asp Thr Val Leu Leu Ile Val
       130                 135                 140
   Lys Gly Asn Lys Leu Arg Phe Leu His Val Leu His His Ala Thr Thr
   145                 150                 155                 160
   Phe Trp Leu Tyr Ala Ile Asp His Ile Phe Leu Ser Ser Ile Lys Tyr
                   165                 170                 175
   Gly Val Ala Val Asn Ala Phe Ile His Thr Val Met Tyr Ala His Tyr
               180                 185                 190
   Phe Arg Pro Phe Pro Lys Gly Leu Arg Pro Leu Ile Thr Gln Leu Gln
           195                 200                 205
   Ile Val Gln Phe Ile Phe Ser Ile Gly Ile His Thr Ala Ile Tyr Trp
       210                 215                 220
   His Tyr Asp Cys Glu Pro Leu Val His Thr His Phe Trp Glu Tyr Val
   225                 230                 235                 240
   Thr Pro Tyr Leu Phe Val Val Pro Phe Leu Ile Leu Phe Phe Asn Phe
                   245                 250                 255
   Tyr Leu Gln Gln Tyr Val Leu Ala Pro Ala Lys Thr Lys Lys Ala
   260 265 270
<210> 64
   <211> 837
   <212> DNA
   <213> Phytophthora infestans
<220>
   <221> CDS
   <222> (1)..(837)
   <223> delta-6-Elongase
<400> 64
   atg tcg act gag cta ctg cag agc tac tac gcg tgg gcc aac gcc acg 48
   Met Ser Thr Glu Leu Leu Gln Ser Tyr Tyr Ala Trp Ala Asn Ala Thr
   1 5 10 15
   gag gcc aag ctg ctg gac tgg gtc gac cct gag ggc ggc tgg aag gtg 96
   Glu Ala Lys Leu Leu Asp Trp Val Asp Pro Glu Gly Gly Trp Lys Val
   20 25 30
   cat cct atg gca gac tac ccc cta gcc aac ttc tcc agc gtc tac gcc 144
   His Pro Met Ala Asp Tyr Pro Leu Ala Asn Phe Ser Ser Val Tyr Ala
   35 40 45
   atc tgc gtc gga tac ttg ctc ttc gta atc ttc ggc acg gcc ctg atg 192
   Ile Cys Val Gly Tyr Leu Leu Phe Val Ile Phe Gly Thr Ala Leu Met
   50 55 60
   aaa atg gga gtc ccc gcc atc aag acc agt cca tta cag ttt gtg tac 240
   Lys Met Gly Val Pro Ala Ile Lys Thr Ser Pro Leu Gln Phe Val Tyr
   65 70 75 80
   aac ccc atc caa gtc att gcc tgc tct tat atg tgc gtg gag gcc gcc 288
   Asn Pro Ile Gln Val Ile Ala Cys Ser Tyr Met Cys Val Glu Ala Ala
   85 90 95
   atc cag gcc tac cgc aac ggc tac acc gcc gcc ccg tgc aac gcc ttt 336
   Ile Gln Ala Tyr Arg Asn Gly Tyr Thr Ala Ala Pro Cys Asn Ala Phe
   100 105 110
   aag tcc gac gac ccc gtc atg ggc aac gtt ctg tac ctc ttc tat ctc 384
   Lys Ser Asp Asp Pro Val Met Gly Asn Val Leu Tyr Leu Phe Tyr Leu
   115 120 125
   tcc aag atg ctc gac ctg tgc gac aca gtc ttc att atc cta gga aag 432
   Ser Lys Met Leu Asp Leu Cys Asp Thr Val Phe Ile Ile Leu Gly Lys
   130 135 140
   aag tgg aaa cag ctt tcc atc ttg cac gtg tac cac cac ctt acc gtg 480
   Lys Trp Lys Gln Leu Ser Ile Leu His Val Tyr His His Leu Thr Val
   145 150 155 160
   ctt ttc gtc tac tat gtg acg ttc cgc gcc gct cag gac ggg gac tca 528
   Leu Phe Val Tyr Tyr Val Thr Phe Arg Ala Ala Gln Asp Gly Asp Ser
   165 170 175
   tat gct acc atc gtg ctc aac ggc ttc gtg cac acc atc atg tac act 576
   Tyr Ala Thr Ile Val Leu Asn Gly Phe Val His Thr Ile Met Tyr Thr
   180 185 190
   tac tac ttc gtc agc gcc cac acg cgc aac att tgg tgg aag aag tac 624
   Tyr Tyr Phe Val Ser Ala His Thr Arg Asn Ile Trp Trp Lys Lys Tyr
           195                 200                 205
   ctc acg cgc att cag ctt atc cag ttc gtg acc atg aac gtg cag ggc      672
   Leu Thr Arg Ile Gln Leu Ile Gln Phe Val Thr Met Asn Val Gln Gly
       210                 215                 220
   tac ctg acc tac tct cga cag tgc cca ggc atg cct cct aag gtg ccg      720
   Tyr Leu Thr Tyr Ser Arg Gln Cys Pro Gly Met Pro Pro Lys Val Pro
   225                 230                 235                 240
   ctc atg tac ctt gtg tac gtg cag tca ctc ttc tgg ctc ttc atg aat      768
   Leu Met Tyr Leu Val Tyr Val Gln Ser Leu Phe Trp Leu Phe Met Asn
                   245                 250                 255
   ttc tac att cgc gcg tac gtg ttc ggc ccc aag aaa ccg gcc gtg gag      816
   Phe Tyr Ile Arg Ala Tyr Val Phe Gly Pro Lys Lys Pro Ala Val Glu
               260                 265                 270
   gaa tcg aag aag aag ttg taa                                          837
   Glu Ser Lys Lys Lys Leu
           275
<210> 65
   <211> 278
   <212> PRT
   <213> Phytophthora infestans
<400> 65 *
<210> 66
   <211> 954
   <212> DNA
   <213> Mortierella alpina
<220>
   <221> CDS
   <222> (1)..(954)
   <223> delta-6-Elongase
<400> 66
   atg gcc gcc gca atc ttg gac aag gtc aac ttc ggc att gat cag ccc       48
   Met Ala Ala Ala Ile Leu Asp Lys Val Asn Phe Gly Ile Asp Gln Pro
   1               5                   10                  15
   ttc gga atc aag ctc gac acc tac ttt gct cag gcc tat gaa ctc gtc       96
   Phe Gly Ile Lys Leu Asp Thr Tyr Phe Ala Gln Ala Tyr Glu Leu Val
               20                  25                  30
   acc gga aag tcc atc gac tcc ttc gtc ttc cag gag ggc gtc acg cct      144
   Thr Gly Lys Ser Ile Asp Ser Phe Val Phe Gln Glu Gly Val Thr Pro
           35                  40                  45
   ctc tcg acc cag aga gag gtc gcc atg tgg act atc act tac ttc gtc      192
   Leu Ser Thr Gln Arg Glu Val Ala Met Trp Thr Ile Thr Tyr Phe Val
       50                  55                  60
   gtc atc ttt ggt ggt cgc cag atc atg aag agc cag gac gcc ttc aag      240
   Val Ile Phe Gly Gly Arg Gln Ile Met Lys Ser Gln Asp Ala Phe Lys
   65                  70                  75                  80
   ctc aag ccc ctc ttc atc ctc cac aac ttc ctc ctg acg atc gcg tcc      288
   Leu Lys Pro Leu Phe Ile Leu His Asn Phe Leu Leu Thr Ile Ala Ser
   85 90 95
   gga tcg ctg ttg ctc ctg ttc atc gag aac ctg gtc ccc atc ctc gcc 336
   Gly Ser Leu Leu Leu Leu Phe Ile Glu Asn Leu Val Pro Ile Leu Ala
   100 105 110
   aga aac gga ctt ttc tac gcc atc tgc gac gac ggt gcc tgg acc cag 384
   Arg Asn Gly Leu Phe Tyr Ala Ile Cys Asp Asp Gly Ala Trp Thr Gln
   115 120 125
   cgc ctc gag ctc ctc tac tac ctc aac tac ctg gtc aag tac tgg gag 432
   Arg Leu Glu Leu Leu Tyr Tyr Leu Asn Tyr Leu Val Lys Tyr Trp Glu
   130 135 140
   ttg gcc gac acc gtc ttt ttg gtc ctc aag aag aag cct ctt gag ttc 480
   Leu Ala Asp Thr Val Phe Leu Val Leu Lys Lys Lys Pro Leu Glu Phe
   145 150 155 160
   ctg cac tac ttc cac cac tcg atg acc atg gtt ctc tgc ttt gtc cag 528
   Leu His Tyr Phe His His Ser Met Thr Met Val Leu Cys Phe Val Gln
   165 170 175
   ctt gga gga tac act tca gtg tcc tgg gtc cct att acc ctc aac ttg 576
   Leu Gly Gly Tyr Thr Ser Val Ser Trp Val Pro Ile Thr Leu Asn Leu
   180 185 190
   act gtc cac gtc ttc atg tac tac tac tac atg cgc tcc gct gcc ggt 624
   Thr Val His Val Phe Met Tyr Tyr Tyr Tyr Met Arg Ser Ala Ala Gly
   195 200 205
   gtt cgc atc tgg tgg aag cag tac ttg acc act ctc cag atc gtc cag 672
   Val Arg Ile Trp Trp Lys Gln Tyr Leu Thr Thr Leu Gln Ile Val Gln
   210 215 220
   ttc gtt ctt gac ctc gga ttc atc tac ttc tgc gcc tac acc tac ttc 720
   Phe Val Leu Asp Leu Gly Phe Ile Tyr Phe Cys Ala Tyr Thr Tyr Phe
   225 230 235 240
   gcc ttc acc tac ttc ccc tgg gct ccc aac gtc ggc aag tgc gcc ggt 768
   Ala Phe Thr Tyr Phe Pro Trp Ala Pro Asn Val Gly Lys Cys Ala Gly
   245 250 255
   acc gag ggt gct gct ctc ttt ggc tgc gga ctc ctc tcc agc tat ctc 816
   Thr Glu Gly Ala Ala Leu Phe Gly Cys Gly Leu Leu Ser Ser Tyr Leu
   260 265 270
   ttg ctc ttt atc aac ttc tac cgc att acc tac aat gcc aag gcc aag 864
   Leu Leu Phe Ile Asn Phe Tyr Arg Ile Thr Tyr Asn Ala Lys Ala Lys
   275 280 285
   gca gcc aag gag cgt gga agc aac ttt acc ccc aag act gtc aag tcc 912
   Ala Ala Lys Glu Arg Gly Ser Asn Phe Thr Pro Lys Thr Val Lys Ser
   290 295 300
   ggc gga tcg ccc aag aag ccc tcc aag agc aag cac atc taa 954
   Gly Gly Ser Pro Lys Lys Pro Ser Lys Ser Lys His Ile
   305 310 315
<210> 67
   <211> 317
   <212> PRT
   <213> Mortierella alpina
<400> 67
   Met Ala Ala Ala Ile Leu Asp Lys Val Asn Phe Gly Ile Asp Gln Pro
   1               5                   10                  15
   Phe Gly Ile Lys Leu Asp Thr Tyr Phe Ala Gln Ala Tyr Glu Leu Val
   20 25 30
   Thr Gly Lys Ser Ile Asp Ser Phe Val Phe Gln Glu Gly Val Thr Pro
   35 40 45
   Leu Ser Thr Gln Arg Glu Val Ala Met Trp Thr Ile Thr Tyr Phe Val
   50 55 60
   Val Ile Phe Gly Gly Arg Gln Ile Met Lys Ser Gln Asp Ala Phe Lys
   65 70 75 80
   Leu Lys Pro Leu Phe Ile Leu His Asn Phe Leu Leu Thr Ile Ala Ser
   85 90 95
   Gly Ser Leu Leu Leu Leu Phe Ile Glu Asn Leu Val Pro Ile Leu Ala
   100 105 110
   Arg Asn Gly Leu Phe Tyr Ala Ile Cys Asp Asp Gly Ala Trp Thr Gln
   115 120 125
   Arg Leu Glu Leu Leu Tyr Tyr Leu Asn Tyr Leu Val Lys Tyr Trp Glu
   130 135 140
   Leu Ala Asp Thr Val Phe Leu Val Leu Lys Lys Lys Pro Leu Glu Phe
   145 150 155 160
   Leu His Tyr Phe His His Ser Met Thr Met Val Leu Cys Phe Val Gln
   165 170 175
   Leu Gly Gly Tyr Thr Ser Val Ser Trp Val Pro Ile Thr Leu Asn Leu
   180 185 190
   Thr Val His Val Phe Met Tyr Tyr Tyr Tyr Met Arg Ser Ala Ala Gly
   195 200 205
   Val Arg Ile Trp Trp Lys Gln Tyr Leu Thr Thr Leu Gln Ile Val Gln
   210 215 220
   Phe Val Leu Asp Leu Gly Phe Ile Tyr Phe Cys Ala Tyr Thr Tyr Phe
   225 230 235 240
   Ala Phe Thr Tyr Phe Pro Trp Ala Pro Asn Val Gly Lys Cys Ala Gly
   245 250 255
   Thr Glu Gly Ala Ala Leu Phe Gly Cys Gly Leu Leu Ser Ser Tyr Leu
   260 265 270
   Leu Leu Phe Ile Asn Phe Tyr Arg Ile Thr Tyr Asn Ala Lys Ala Lys
   275 280 285
   Ala Ala Lys Glu Arg Gly Ser Asn Phe Thr Pro Lys Thr Val Lys Ser
       290                 295                 300
   Gly Gly Ser Pro Lys Lys Pro Ser Lys Ser Lys His Ile
   305                 310                 315
<210> 68
   <211> 957
   <212> DNA
   <213> Mortierella alpina
<220>
   <221> CDS
   <222> (1)..(957)
   <223> delta-6-Elongase
<400> 68
   atg gag tcg att gcg cca ttc ctc cca tca aag atg ccg caa gat ctg      48
   Met Glu Ser Ile Ala Pro Phe Leu Pro Ser Lys Met Pro Gln Asp Leu
   1               5                   10                  15
   ttt atg gac ctt gcc acc gct atc ggt gtc cgg gcc gcg ccc tat gtc      96
   Phe Met Asp Leu Ala Thr Ala Ile Gly Val Arg Ala Ala Pro Tyr Val
               20                  25                  30
   gat cct ctc gag gcc gcg ctg gtg gcc cag gcc gag aag tac atc ccc     144
   Asp Pro Leu Glu Ala Ala Leu Val Ala Gln Ala Glu Lys Tyr Ile Pro
           35                  40                  45
   acg att gtc cat cac acg cgt ggg ttc ctg gtc gcg gtg gag tcg cct     192
   Thr Ile Val His His Thr Arg Gly Phe Leu Val Ala Val Glu Ser Pro
       50                  55                  60
   ttg gcc cgt gag ctg ccg ttg atg aac ccg ttc cac gtg ctg ttg atc     240
   Leu Ala Arg Glu Leu Pro Leu Met Asn Pro Phe His Val Leu Leu Ile
   65                  70                  75                  80
   gtg ctc gct tat ttg gtc acg gtc ttt gtg ggc atg cag atc atg aag     288
   Val Leu Ala Tyr Leu Val Thr Val Phe Val Gly Met Gln Ile Met Lys
                   85                  90                  95
   aac ttt gag cgg ttc gag gtc aag acg ttt tcg ctc ctg cac aac ttt     336
   Asn Phe Glu Arg Phe Glu Val Lys Thr Phe Ser Leu Leu His Asn Phe
               100                 105                 110
   tgt ctg gtc tcg atc agc gcc tac atg tgc ggt ggg atc ctg tac gag     384
   Cys Leu Val Ser Ile Ser Ala Tyr Met Cys Gly Gly Ile Leu Tyr Glu
           115                 120                 125
   gct tat cag gcc aac tat gga ctg ttt gag aac gct gct gat cat acc     432
   Ala Tyr Gln Ala Asn Tyr Gly Leu Phe Glu Asn Ala Ala Asp His Thr
       130                 135                 140
   ttc aag ggt ctt cct atg gcc aag atg atc tgg ctc ttc tac ttc tcc     480
   Phe Lys Gly Leu Pro Met Ala Lys Met Ile Trp Leu Phe Tyr Phe Ser
   145                 150                 155                 160
   aag atc atg gag ttt gtc gac acc atg atc atg gtc ctc aag aag aac     528
   Lys Ile Met Glu Phe Val Asp Thr Met Ile Met Val Leu Lys Lys Asn
                   165                 170                 175
   aac cgc cag atc tcc ttc ttg cac gtt tac cac cac agc tcc atc ttc     576
   Asn Arg Gln Ile Ser Phe Leu His Val Tyr His His Ser Ser Ile Phe
                   180                 185                 190
   acc atc tgg tgg ttg gtc acc ttt gtt gca ccc aac ggt gaa gcc tac     624
   Thr Ile Trp Trp Leu Val Thr Phe Val Ala Pro Asn Gly Glu Ala Tyr
           195                 200                 205
   ttc tct gct gcg ttg aac tcg ttc atc cat gtg atc atg tac ggc tac     672
   Phe Ser Ala Ala Leu Asn Ser Phe Ile His Val Ile Met Tyr Gly Tyr
       210                 215                 220
   tac ttc ttg tcg gcc ttg ggc ttc aag cag gtg tcg ttc atc aag ttc     720
   Tyr Phe Leu Ser Ala Leu Gly Phe Lys Gln Val Ser Phe Ile Lys Phe
   225                 230                 235                 240
   tac atc acg cgc tcg cag atg aca cag ttc tgc atg atg tcg gtc cag     768
   Tyr Ile Thr Arg Ser Gln Met Thr Gln Phe Cys Met Met Ser Val Gln
                   245                 250                 255
   tct tcc tgg gac atg tac gcc atg aag gtc ctt ggc cgc ccc gga tac     816
   Ser Ser Trp Asp Met Tyr Ala Met Lys Val Leu Gly Arg Pro Gly Tyr
               260                 265                 270
   ccc ttc ttc atc acg gct ctg ctt tgg ttc tac atg tgg acc atg ctc     864
   Pro Phe Phe Ile Thr Ala Leu Leu Trp Phe Tyr Met Trp Thr Met Leu
           275                 280                 285
   ggt ctc ttc tac aac ttt tac aga aag aac gcc aag ttg gcc aag cag     912
   Gly Leu Phe Tyr Asn Phe Tyr Arg Lys Asn Ala Lys Leu Ala Lys Gln
       290                 295                 300
   gcc aag gcc gac gct gcc aag gag aag gca agg aag ttg cag taa         957
   Ala Lys Ala Asp Ala Ala Lys Glu Lys Ala Arg Lys Leu Gln
   305                 310                 315
<210> 69
   <211> 318
   <212> PRT
   <213> Mortierella alpina
<400> 69
   Met Glu Ser Ile Ala Pro Phe Leu Pro Ser Lys Met Pro Gln Asp Leu
   1               5                   10                  15
   Phe Met Asp Leu Ala Thr Ala Ile Gly Val Arg Ala Ala Pro Tyr Val
               20                  25                  30
   Asp Pro Leu Glu Ala Ala Leu Val Ala Gln Ala Glu Lys Tyr Ile Pro
           35                  40                  45
   Thr Ile Val His His Thr Arg Gly Phe Leu Val Ala Val Glu Ser Pro
       50                  55                  60
   Leu Ala Arg Glu Leu Pro Leu Met Asn Pro Phe His Val Leu Leu Ile
   65                  70                  75                  80
   Val Leu Ala Tyr Leu Val Thr Val Phe Val Gly Met Gln Ile Met Lys
                   85                  90                  95
   Asn Phe Glu Arg Phe Glu Val Lys Thr Phe Ser Leu Leu His Asn Phe
               100                 105                 110
   Cys Leu Val Ser Ile Ser Ala Tyr Met Cys Gly Gly Ile Leu Tyr Glu
           115                 120                 125
   Ala Tyr Gln Ala Asn Tyr Gly Leu Phe Glu Asn Ala Ala Asp His Thr
       130                 135                 140
   Phe Lys Gly Leu Pro Met Ala Lys Met Ile Trp Leu Phe Tyr Phe Ser
   145                 150                 155                 160
   Lys Ile Met Glu Phe Val Asp Thr Met Ile Met Val Leu Lys Lys Asn
                   165                 170                 175
   Asn Arg Gln Ile Ser Phe Leu His Val Tyr His His Ser Ser Ile Phe
               180                 185                 190
   Thr Ile Trp Trp Leu Val Thr Phe Val Ala Pro Asn Gly Glu Ala Tyr
           195                 200                 205
   Phe Ser Ala Ala Leu Asn Ser Phe Ile His Val Ile Met Tyr Gly Tyr
       210                 215                 220
   Tyr Phe Leu Ser Ala Leu Gly Phe Lys Gln Val Ser Phe Ile Lys Phe
   225                 230                 235                 240
   Tyr Ile Thr Arg Ser Gln Met Thr Gln Phe Cys Met Met Ser Val Gln
                   245                 250                 255
   Ser Ser Trp Asp Met Tyr Ala Met Lys Val Leu Gly Arg Pro Gly Tyr
               260                 265                 270
   Pro Phe Phe Ile Thr Ala Leu Leu Trp Phe Tyr Met Trp Thr Met Leu
           275                 280                 285
   Gly Leu Phe Tyr Asn Phe Tyr Arg Lys Asn Ala Lys Leu Ala Lys Gln
       290                 295                 300
   Ala Lys Ala Asp Ala Ala Lys Glu Lys Ala Arg Lys Leu Gln
   305                 310                 315
<210> 70
   <211> 867
   <212> DNA
   <213> Caenorhabditis elegans
<220>
   <221> CDS
   <222> (1)..(867)
   <223> delta-6-Elongase
<400> 70
   atg gct cag cat ccg ctc gtt caa cgg ctt ctc gat gtc aaa ttc gac 48
   Met Ala Gln His Pro Leu Val Gln Arg Leu Leu Asp Val Lys Phe Asp
   1               5                   10                  15
   acg aaa cga ttt gtg gct att gct act cat ggg cca aag aat ttc cct      96
   Thr Lys Arg Phe Val Ala Ile Ala Thr His Gly Pro Lys Asn Phe Pro
               20                  25                  30
   gac gca gaa ggt cgc aag ttc ttt gct gat cac ttt gat gtt act att     144
   Asp Ala Glu Gly Arg Lys Phe Phe Ala Asp His Phe Asp Val Thr Ile
           35                  40                  45
   cag gct tca atc ctg tac atg gtc gtt gtg ttc gga aca aaa tgg ttc     192
   Gln Ala Ser Ile Leu Tyr Met Val Val Val Phe Gly Thr Lys Trp Phe
       50                  55                  60
   atg cgt aat cgt caa cca ttc caa ttg act att cca ctc aac atc tgg     240
   Met Arg Asn Arg Gln Pro Phe Gln Leu Thr Ile Pro Leu Asn Ile Trp
   65                  70                  75                  80
   aat ttc atc ctc gcc gca ttt tcc atc gca gga gct gtc aaa atg acc     288
   Asn Phe Ile Leu Ala Ala Phe Ser Ile Ala Gly Ala Val Lys Met Thr
                   85                  90                  95
   cca gag ttc ttt gga acc att gcc aac aaa gga att gtc gca tcc tac     336
   Pro Glu Phe Phe Gly Thr Ile Ala Asn Lys Gly Ile Val Ala Ser Tyr
               100                 105                 110
   tgc aaa gtg ttt gat ttc acg aaa gga gag aat gga tac tgg gtg tgg     384
   Cys Lys Val Phe Asp Phe Thr Lys Gly Glu Asn Gly Tyr Trp Val Trp
           115                 120                 125
   ctc ttc atg gct tcc aaa ctt ttc gaa ctt gtt gac acc atc ttc ttg     432
   Leu Phe Met Ala Ser Lys Leu Phe Glu Leu Val Asp Thr Ile Phe Leu
       130                 135                 140
   gtt ctc cgt aaa cgt cca ctc atg ttc ctt cac tgg tat cac cat att     480
   Val Leu Arg Lys Arg Pro Leu Met Phe Leu His Trp Tyr His His Ile
   145                 150                 155                 160
   ctc acc atg atc tac gcc tgg tac tct cat cca ttg acc cca gga ttc     528
   Leu Thr Met Ile Tyr Ala Trp Tyr Ser His Pro Leu Thr Pro Gly Phe
                   165                 170                 175
   aac aga tac gga att tat ctt aac ttt gtc gtc cac gcc ttc atg tac     576
   Asn Arg Tyr Gly Ile Tyr Leu Asn Phe Val Val His Ala Phe Met Tyr
               180                 185                 190
   tct tac tac ttc ctt cgc tcg atg aag att cgc gtg cca gga ttc atc     624
   Ser Tyr Tyr Phe Leu Arg Ser Met Lys Ile Arg Val Pro Gly Phe Ile
           195                 200                 205
   gcc caa gct atc aca tct ctt caa atc gtt caa ttc atc atc tct tgc     672
   Ala Gln Ala Ile Thr Ser Leu Gln Ile Val Gln Phe Ile Ile Ser Cys
       210                 215                 220
   gcc gtt ctt gct cat ctt ggt tat ctc atg cac ttc acc aat gcc aac     720
   Ala Val Leu Ala His Leu Gly Tyr Leu Met His Phe Thr Asn Ala Asn
   225                 230                 235                 240
   tgt gat ttc gag cca tca gta ttc aag ctc gca gtt ttc atg gac aca     768
   Cys Asp Phe Glu Pro Ser Val Phe Lys Leu Ala Val Phe Met Asp Thr
                   245                 250                 255
   aca tac ttg gct ctt ttc gtc aac ttc ttc ctc caa tca tat gtt ctc     816
   Thr Tyr Leu Ala Leu Phe Val Asn Phe Phe Leu Gln Ser Tyr Val Leu
               260                 265                 270
   cgc gga gga aaa gac aag tac aag gca gtg cca aag aag aag aac aac     864
   Arg Gly Gly Lys Asp Lys Tyr Lys Ala Val Pro Lys Lys Lys Asn Asn
   275 280 285
   taa 867
<210> 71
   <211> 288
   <212> PRT
   <213> Caenorhabditis elegans
<400> 71
   Met Ala Gln His Pro Leu Val Gln Arg Leu Leu Asp Val Lys Phe Asp
   1               5                   10                  15
   Thr Lys Arg Phe Val Ala Ile Ala Thr His Gly Pro Lys Asn Phe Pro
               20                  25                  30
   Asp Ala Glu Gly Arg Lys Phe Phe Ala Asp His Phe Asp Val Thr Ile
           35                  40                  45
   Gln Ala Ser Ile Leu Tyr Met Val Val Val Phe Gly Thr Lys Trp Phe
       50                  55                  60
   Met Arg Asn Arg Gln Pro Phe Gln Leu Thr Ile Pro Leu Asn Ile Trp
   65                  70                  75                  80
   Asn Phe Ile Leu Ala Ala Phe Ser Ile Ala Gly Ala Val Lys Met Thr
                   85                  90                  95
   Pro Glu Phe Phe Gly Thr Ile Ala Asn Lys Gly Ile Val Ala Ser Tyr
               100                 105                 110
   Cys Lys Val Phe Asp Phe Thr Lys Gly Glu Asn Gly Tyr Trp Val Trp
           115                 120                 125
   Leu Phe Met Ala Ser Lys Leu Phe Glu Leu Val Asp Thr Ile Phe Leu
       130                 135                 140
   Val Leu Arg Lys Arg Pro Leu Met Phe Leu His Trp Tyr His His Ile
   145                 150                 155                 160
   Leu Thr Met Ile Tyr Ala Trp Tyr Ser His Pro Leu Thr Pro Gly Phe
                   165                 170                 175
   Asn Arg Tyr Gly Ile Tyr Leu Asn Phe Val Val His Ala Phe Met Tyr
               180                 185                 190
   Ser Tyr Tyr Phe Leu Arg Ser Met Lys Ile Arg Val Pro Gly Phe Ile
           195                 200                 205
   Ala Gln Ala Ile Thr Ser Leu Gln Ile Val Gln Phe Ile Ile Ser Cys
       210                 215                 220
   Ala Val Leu Ala His Leu Gly Tyr Leu Met His Phe Thr Asn Ala Asn
   225 230 235 240
   Cys Asp Phe Glu Pro Ser Val Phe Lys Leu Ala Val Phe Met Asp Thr
   245 250 255
   Thr Tyr Leu Ala Leu Phe Val Asn Phe Phe Leu Gln Ser Tyr Val Leu
   260 265 270
   Arg Gly Gly Lys Asp Lys Tyr Lys Ala Val Pro Lys Lys Lys Asn Asn
   275 280 285
<210> 72
   <211> 879
   <212> DNA
   <213> Ostreococcus tauri
<220>
   <221> CDS
   <222> (1)..(879)
   <223> delta-6-Elongase
<400> 72
   atg agt ggc tta cgt gca ccc aac ttt tta cac aga ttc tgg aca aag      48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt      96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc      144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc      192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa      240
   Thr Val Phe Leu Trp Tyr Gly Arg Leu Thr Arg Ser Ser Asp Lys Lys
   65                  70                  75                  80
   att aga gag cct acg tgg tta aga aga ttc ata ata tgt cat aat gcg      288
   Ile Arg Glu Pro Thr Trp Leu Arg Arg Phe Ile Ile Cys His Asn Ala
                   85                  90                  95
   ttc ttg ata gtc ctc agt ctt tac atg tgc ctt ggt tgt gtg gcc caa      336
   Phe Leu Ile Val Leu Ser Leu Tyr Met Cys Leu Gly Cys Val Ala Gln
               100                 105                 110
   gcg tat cag aat gga tat act tta tgg ggt aat gaa ttc aag gcc acg      384
   Ala Tyr Gln Asn Gly Tyr Thr Leu Trp Gly Asn Glu Phe Lys Ala Thr
           115                 120                 125
   gaa act cag ctt gct ctc tac att tac att ttt tac gta agt aaa ata      432
   Glu Thr Gln Leu Ala Leu Tyr Ile Tyr Ile Phe Tyr Val Ser Lys Ile
       130                 135                 140
   tac gag ttt gta gat act tac att atg ctt ctc aag aat aac ttg cgg      480
   Tyr Glu Phe Val Asp Thr Tyr Ile Met Leu Leu Lys Asn Asn Leu Arg
   145                 150                 155                 160
   caa gta agt ttc cta cac att tat cac cac agc acg att tcc ttt att      528
   Gln Val Ser Phe Leu His Ile Tyr His His Ser Thr Ile Ser Phe Ile
                   165                 170                 175
   tgg tgg atc att gct cgg agg gct ccg ggt ggt gat gct tac ttc agc      576
   Trp Trp Ile Ile Ala Arg Arg Ala Pro Gly Gly Asp Ala Tyr Phe Ser
               180                 185                 190
   gcg gcc ttg aac tca tgg gta cac gtg tgc atg tac acc tat tat cta      624
   Ala Ala Leu Asn Ser Trp Val His Val Cys Met Tyr Thr Tyr Tyr Leu
           195                 200                 205
   tta tca acc ctt att gga aaa gaa gat cct aag cgt tcc aac tac ctt      672
   Leu Ser Thr Leu Ile Gly Lys Glu Asp Pro Lys Arg Ser Asn Tyr Leu
       210                 215                 220
   tgg tgg ggt cgc cac cta acg caa atg cag atg ctt cag ttt ttc ttc      720
   Trp Trp Gly Arg His Leu Thr Gln Met Gln Met Leu Gln Phe Phe Phe
   225                 230                 235                 240
   aac gta ctt caa gcg ttg tac tgc gct tcg ttc tct acg tat ccc aag      768
   Asn Val Leu Gln Ala Leu Tyr Cys Ala Ser Phe Ser Thr Tyr Pro Lys
                   245                 250                 255
   ttt ttg tcc aaa att ctg ctc gtc tat atg atg agc ctt ctc ggc ttg      816
   Phe Leu Ser Lys Ile Leu Leu Val Tyr Met Met Ser Leu Leu Gly Leu
               260                 265                 270
   ttt ggg cat ttc tac tat tcc aag cac ata gca gca gct aag ctc cag      864
   Phe Gly His Phe Tyr Tyr Ser Lys His Ile Ala Ala Ala Lys Leu Gln
           275                 280                 285
   aaa aaa cag cag tga                                                  879
   Lys Lys Gln Gln
       290
<210> 73
   <211> 292
   <212> PRT
   <213> Ostreococcus tauri
<400> 73
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Ser Phe Leu His Ile Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
           275                 280                 285
   Lys Lys Gln Gln
       290
<210> 74
   <211> 879
   <212> DNA
   <213> Ostreococcus tauri
<220>
   <221> CDS
   <222> (1)..(879)
   <223> delta-6-Elongase
<400> 74
   atg agt ggc tta cgt gca ccc aac ttt tta cac aga ttc tgg aca aag     48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt      96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc     144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc     192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa     240
   Thr Val Phe Leu Trp Tyr Gly Arg Leu Thr Arg Ser Ser Asp Lys Lys
   65                  70                  75                  80
   att aga gag cct acg tgg tta aga aga ttc ata ata tgt cat aat gcg     288
   Ile Arg Glu Pro Thr Trp Leu Arg Arg Phe Ile Ile Cys His Asn Ala
                   85                  90                  95
   ttc ttg ata gtc ctc agt ctt tac atg tgc ctt ggt tgt gtg gcc caa     336
   Phe Leu Ile Val Leu Ser Leu Tyr Met Cys Leu Gly Cys Val Ala Gln
               100                 105                 110
   gcg tat cag aat gga tat act tta tgg ggt aat gaa ttc aag gcc acg     384
   Ala Tyr Gln Asn Gly Tyr Thr Leu Trp Gly Asn Glu Phe Lys Ala Thr
           115                 120                 125
   gaa act cag ctt gct ctc tac att tac att ttt tac gta agt aaa ata     432
   Glu Thr Gln Leu Ala Leu Tyr Ile Tyr Ile Phe Tyr Val Ser Lys Ile
       130                 135                 140
   tac gag ttt gta gat act tac att atg ctt ctc aag aat aac ttg cgg     480
   Tyr Glu Phe Val Asp Thr Tyr Ile Met Leu Leu Lys Asn Asn Leu Arg
   145                 150                 155                 160
   caa gta aga ttc cta cac act tat cac cac agc acg att tcc ttt att     528
   Gln Val Arg Phe Leu His Thr Tyr His His Ser Thr Ile Ser Phe Ile
                   165                 170                 175
   tgg tgg atc att gct cgg agg gct ccg ggt ggt gat gct tac ttc agc     576
   Trp Trp Ile Ile Ala Arg Arg Ala Pro Gly Gly Asp Ala Tyr Phe Ser
               180                 185                 190
   gcg gcc ttg aac tca tgg gta cac gtg tgc atg tac acc tat tat cta     624
   Ala Ala Leu Asn Ser Trp Val His Val Cys Met Tyr Thr Tyr Tyr Leu
           195                 200                 205
   tta tca acc ctt att gga aaa gaa gat cct aag cgt tcc aac tac ctt     672
   Leu Ser Thr Leu Ile Gly Lys Glu Asp Pro Lys Arg Ser Asn Tyr Leu
       210                 215                 220
   tgg tgg ggt cgc cac cta acg caa atg cag atg ctt cag ttt ttc ttc     720
   Trp Trp Gly Arg His Leu Thr Gln Met Gln Met Leu Gln Phe Phe Phe
   225                 230                 235                 240
   aac gta ctt caa gcg ttg tac tgc gct tcg ttc tct acg tat ccc aag     768
   Asn Val Leu Gln Ala Leu Tyr Cys Ala Ser Phe Ser Thr Tyr Pro Lys
                   245                 250                 255
   ttt ttg tcc aaa att ctg ctc gtc tat atg atg agc ctt ctc ggc ttg     816
   Phe Leu Ser Lys Ile Leu Leu Val Tyr Met Met Ser Leu Leu Gly Leu
               260                 265                 270
   ttt ggg cat ttc tac tat tcc aag cac ata gca gca gct aag ctc cag     864
   Phe Gly His Phe Tyr Tyr Ser Lys His Ile Ala Ala Ala Lys Leu Gln
           275                 280                 285
   aaa aaa cag cag tga 879
   Lys Lys Gln Gln
   290
<210> 75
   <211> 292
   <212> PRT
   <213> Ostreococcus tauri
<400> 75
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Arg Phe Leu His Thr Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225 230 235 240
   Asn Val Leu Gln Ala Leu Tyr Cys Ala Ser Phe Ser Thr Tyr Pro Lys
   245 250 255
   Phe Leu Ser Lys Ile Leu Leu Val Tyr Met Met Ser Leu Leu Gly Leu
   260 265 270
   Phe Gly His Phe Tyr Tyr Ser Lys His Ile Ala Ala Ala Lys Leu Gln
   275 280 285
   Lys Lys Gln Gln
   290
<210> 76
   <211> 993
   <212> DNA
   <213> Phaeodactylum tricornutum
<220>
   <221> CDS
   <222> (103)..(939)
   <223> delta-6-Elongase
<400> 76
   ggtcttttgt ggtagctatc gtcatcacac gcaggtcgtt gctcactatc gtgatccgta      60
   tattgaccgt gcacttgtgt aaaacagaga tatttcaaga gt atg atg gta cct        114
                                                  Met Met Val Pro
                                                  1
   tca agt tat gac gag tat atc gtc atg gtc aac gac ctt ggc gac tct      162
   Ser Ser Tyr Asp Glu Tyr Ile Val Met Val Asn Asp Leu Gly Asp Ser
   5                   10                  15                  20
   att ctg agc tgg gcc gac cct gat cac tat cgt gga cat acc gag gga      210
   Ile Leu Ser Trp Ala Asp Pro Asp His Tyr Arg Gly His Thr Glu Gly
                   25                  30                  35
   tgg gag ttc act gac ttt tct gct gct ttt agc att gcc gtc gcg tac      258
   Trp Glu Phe Thr Asp Phe Ser Ala Ala Phe Ser Ile Ala Val Ala Tyr
               40                  45                  50
   ctc ctg ttt gtc ttt gtt gga tct ctc att atg agt atg gga gtc ccc      306
   Leu Leu Phe Val Phe Val Gly Ser Leu Ile Met Ser Met Gly Val Pro
           55                  60                  65
   gca att gac cct tat ccg ctc aag ttt gtc tac aat gtt tca cag att      354
   Ala Ile Asp Pro Tyr Pro Leu Lys Phe Val Tyr Asn Val Ser Gln Ile
       70                  75                  80
   atg ctt tgt gct tac atg acc att gaa gcc agt ctt cta gct tat cgt      402
   Met Leu Cys Ala Tyr Met Thr Ile Glu Ala Ser Leu Leu Ala Tyr Arg
   85                  90                  95                  100
   aac ggc tac aca ttc tgg cct tgc aac gat tgg gac ttt gaa aag ccg      450
   Asn Gly Tyr Thr Phe Trp Pro Cys Asn Asp Trp Asp Phe Glu Lys Pro
                   105                 110                 115
   cct atc gct aag ctc ctc tgg ctc ttt tac gtt tcc aaa att tgg gat      498
   Pro Ile Ala Lys Leu Leu Trp Leu Phe Tyr Val Ser Lys Ile Trp Asp
                   120                     125                     130
   ttt tgg gac acc atc ttt att gtt ctc ggg aag aag tgg cgt caa ctt       546
   Phe Trp Asp Thr Ile Phe Ile Val Leu Gly Lys Lys Trp Arg Gln Leu
           135                     140                     145
   tcc ttc ctg cac gtc tac cat cac acc acc atc ttt ctc ttc tac tgg       594
   Ser Phe Leu His Val Tyr His His Thr Thr Ile Phe Leu Phe Tyr Trp
       150                     155                     160
   ttg aat gca cat gta aac ttt gat ggt gat att ttc ctc acc atc gtc       642
   Leu Asn Ala His Val Asn Phe Asp Gly Asp Ile Phe Leu Thr Ile Val
   165                     170                     175                 180
   ttg aac ggt ttc atc cac acc gtc atg tac acg tac tac ttc att tgc       690
   Leu Asn Gly Phe Ile His Thr Val Met Tyr Thr Tyr Tyr Phe Ile Cys
                   185                     190                     195
   atg cac acc aag gtc cca gag acc ggc aaa tcc ttg ccc att tgg tgg       738
   Met His Thr Lys Val Pro Glu Thr Gly Lys Ser Leu Pro Ile Trp Trp
               200                     205                     210
   aaa tct agt ttg aca agc atg cag ctg gtg cag ttc atc acg atg atg       786
   Lys Ser Ser Leu Thr Ser Met Gln Leu Val Gln Phe Ile Thr Met Met
           215                     220                     225
   acg cag gct atc atg atc ttg tac aag ggc tgt gct gct ccc cat agc       834
   Thr Gln Ala Ile Met Ile Leu Tyr Lys Gly Cys Ala Ala Pro His Ser
       230                     235                     240
   cgg gtg gtg aca tcg tac ttg gtt tac att ttg tcg ctc ttt att ttg       882
   Arg Val Val Thr Ser Tyr Leu Val Tyr Ile Leu Ser Leu Phe Ile Leu
   245                 250                     255                     260
   ttc gcc cag ttc ttt gtc agc tca tac ctc aag ccg aag aag aag aag       930
   Phe Ala Gln Phe Phe Val Ser Ser Tyr Leu Lys Pro Lys Lys Lys Lys
                   265                     270                     275
   aca gct taa gcgaaatttg ggtctacgtt aaaacaatta cgttacaaaa               979
   Thr Ala
   aaaaaaaaaa aaaa                                                        993
<210> 77
   <211> 278
   <212> PRT
   <213> Phaeodactylum tricornutum
<400> 77
   Met Met Val Pro Ser Ser Tyr Asp Glu Tyr Ile Val Met Val Asn Asp
   1               5                   10                  15
   Leu Gly Asp Ser Ile Leu Ser Trp Ala Asp Pro Asp His Tyr Arg Gly
               20                  25                  30
   His Thr Glu Gly Trp Glu Phe Thr Asp Phe Ser Ala Ala Phe Ser Ile
           35                  40                  45
   Ala Val Ala Tyr Leu Leu Phe Val Phe Val Gly Ser Leu Ile Met Ser
       50                  55                  60
   Met Gly Val Pro Ala Ile Asp Pro Tyr Pro Leu Lys Phe Val Tyr Asn
   65 70 75 80
   Val Ser Gln Ile Met Leu Cys Ala Tyr Met Thr Ile Glu Ala Ser Leu
   85 90 95
   Leu Ala Tyr Arg Asn Gly Tyr Thr Phe Trp Pro Cys Asn Asp Trp Asp
   100 105 110
   Phe Glu Lys Pro Pro Ile Ala Lys Leu Leu Trp Leu Phe Tyr Val Ser
   115 120 125
   Lys Ile Trp Asp Phe Trp Asp Thr Ile Phe Ile Val Leu Gly Lys Lys
   130 135 140
   Trp Arg Gln Leu Ser Phe Leu His Val Tyr His His Thr Thr Ile Phe
   145 150 155 160
   Leu Phe Tyr Trp Leu Asn Ala His Val Asn Phe Asp Gly Asp Ile Phe
   165 170 175
   Leu Thr Ile Val Leu Asn Gly Phe Ile His Thr Val Met Tyr Thr Tyr
   180 185 190
   Tyr Phe Ile Cys Met His Thr Lys Val Pro Glu Thr Gly Lys Ser Leu
   195 200 205
   Pro Ile Trp Trp Lys Ser Ser Leu Thr Ser Met Gln Leu Val Gln Phe
   210 215 220
   Ile Thr Met Met Thr Gln Ala Ile Met Ile Leu Tyr Lys Gly Cys Ala
   225 230 235 240
   Ala Pro His Ser Arg Val Val Thr Ser Tyr Leu Val Tyr Ile Leu Ser
   245 250 255
   Leu Phe Ile Leu Phe Ala Gln Phe Phe Val Ser Ser Tyr Leu Lys Pro
   260 265 270
   Lys Lys Lys Lys Thr Ala
   275
<210> 78
   <211> 1512
   <212> DNA
   <213> Thalassiosira pseudonana
<220>
   <221> CDS
   <222> (1)..(1512)
   <223> delta-4-Desaturase
<400> 78
   atg tgc aac ggc aac ctc cca gca tcc acc gca cag ctc aag tcc acc 48
   Met Cys Asn Gly Asn Leu Pro Ala Ser Thr Ala Gln Leu Lys Ser Thr
   1 5 10 15
   tcg aag ccc cag cag caa cat gag cat cgc acc atc tcc aag tcc gag 96
   Ser Lys Pro Gln Gln Gln His Glu His Arg Thr Ile Ser Lys Ser Glu
   20 25 30
   ctc gcc caa cac aac acg ccc aaa tca gca tgg tgt gcc gtc cac tcc 144
   Leu Ala Gln His Asn Thr Pro Lys Ser Ala Trp Cys Ala Val His Ser
   35 40 45
   act ccc gcc acc gac cca tcc cac tcc aac aac aaa caa cac gca cac 192
   Thr Pro Ala Thr Asp Pro Ser His Ser Asn Asn Lys Gln His Ala His
   50 55 60
   cta gtc ctc gac att acc gac ttt gcg tcc cgc cat cca ggg gga gac 240
   Leu Val Leu Asp Ile Thr Asp Phe Ala Ser Arg His Pro Gly Gly Asp
   65 70 75 80
   ctc atc ctc ctc gct tcc ggc aaa gac gcc tcg gtg ctg ttt gaa aca 288
   Leu Ile Leu Leu Ala Ser Gly Lys Asp Ala Ser Val Leu Phe Glu Thr
   85 90 95
   tac cat cca cgt gga gtt ccg acg tct ctc att caa aag ctg cag att 336
   Tyr His Pro Arg Gly Val Pro Thr Ser Leu Ile Gln Lys Leu Gln Ile
   100 105 110
   gga gtg atg gag gag gag gcg ttt cgg gat tcg ttt tac agt tgg act 384
   Gly Val Met Glu Glu Glu Ala Phe Arg Asp Ser Phe Tyr Ser Trp Thr
   115 120 125
   gat tct gac ttt tat act gtg ttg aag agg agg gtt gtg gag cgg ttg 432
   Asp Ser Asp Phe Tyr Thr Val Leu Lys Arg Arg Val Val Glu Arg Leu
   130 135 140
   gag gag agg ggg ttg gac agg agg gga tcg aaa gag att tgg atc aag 480
   Glu Glu Arg Gly Leu Asp Arg Arg Gly Ser Lys Glu Ile Trp Ile Lys
   145 150 155 160
   gct ttg ttc ttg ttg gtt gga ttt tgg tac tgt ttg tac aag atg tat 528
   Ala Leu Phe Leu Leu Val Gly Phe Trp Tyr Cys Leu Tyr Lys Met Tyr
   165 170 175
   act acg tcg gat atc gat cag tac ggt att gcc att gcc tat tct att 576
   Thr Thr Ser Asp Ile Asp Gln Tyr Gly Ile Ala Ile Ala Tyr Ser Ile
   180 185 190
   gga atg gga acc ttt gcg gca ttc atc ggc acg tgt att caa cac gat 624
   Gly Met Gly Thr Phe Ala Ala Phe Ile Gly Thr Cys Ile Gln His Asp
   195 200 205
   gga aat cac ggt gca ttc gct cag aac aag tta ctc aac aag ttg gct 672
   Gly Asn His Gly Ala Phe Ala Gln Asn Lys Leu Leu Asn Lys Leu Ala
   210 215 220
   ggg tgg acg ttg gat atg att ggt gcg agt gcg ttt acg tgg gag ctt 720
   Gly Trp Thr Leu Asp Met Ile Gly Ala Ser Ala Phe Thr Trp Glu Leu
   225 230 235 240
   cag cac atg ctg ggg cat cat cca tat acg aat gtg ttg gat ggg gtg 768
   Gln His Met Leu Gly His His Pro Tyr Thr Asn Val Leu Asp Gly Val
   245 250 255
   gag gag gag agg aag gag agg ggg gag gat gtt gct ttg gaa gaa aag 816
   Glu Glu Glu Arg Lys Glu Arg Gly Glu Asp Val Ala Leu Glu Glu Lys
   260 265 270
   gat cag gat ttt gaa gtt gcc aca tcc gga cga tta tat cat att gat      864
   Asp Gln Asp Phe Glu Val Ala Thr Ser Gly Arg Leu Tyr His Ile Asp
           275                 280                 285
   gcc aat gta cgt tat ggt tcg gta tgg aat gtc atg agg ttt tgg gct      912
   Ala Asn Val Arg Tyr Gly Ser Val Trp Asn Val Met Arg Phe Trp Ala
       290                 295                 300
   atg aag gtc att acg atg gga tat atg atg gga tta cca atc tac ttt      960
   Met Lys Val Ile Thr Met Gly Tyr Met Met Gly Leu Pro Ile Tyr Phe
   305                 310                 315                 320
   cat gga gta ctg agg gga gtt gga ttg ttt gtt att ggg cat ttg gcg     1008
   His Gly Val Leu Arg Gly Val Gly Leu Phe Val Ile Gly His Leu Ala
                   325                 330                 335
   tgt gga gag ttg ttg gcg acg atg ttt att gtg aat cac gtc att gag     1056
   Cys Gly Glu Leu Leu Ala Thr Met Phe Ile Val Asn His Val Ile Glu
               340                 345                 350
   ggt gtg agt tat gga acg aag gat ttg gtt ggt ggt gcg agt cat gta     1104
   Gly Val Ser Tyr Gly Thr Lys Asp Leu Val Gly Gly Ala Ser His Val
           355                 360                 365
   gat gag aag aag att gtc aag cca acg act gta ttg gga gat aca cca     1152
   Asp Glu Lys Lys Ile Val Lys Pro Thr Thr Val Leu Gly Asp Thr Pro
       370                 375                 380
   atg gta aag act cgc gag gag gca ttg aaa agc aac agc aat aac aac     1200
   Met Val Lys Thr Arg Glu Glu Ala Leu Lys Ser Asn Ser Asn Asn Asn
   385                 390                 395                 400
   aag aag aag gga gag aag aac tcg gta cca tcc gtt cca ttc aac gac     1248
   Lys Lys Lys Gly Glu Lys Asn Ser Val Pro Ser Val Pro Phe Asn Asp
                   405                 410                 415
   tgg gca gca gtc caa tgc cag acc tcc gtg aat tgg tct cca ggc tca     1296
   Trp Ala Ala Val Gln Cys Gln Thr Ser Val Asn Trp Ser Pro Gly Ser
               420                 425                 430
   tgg ttc tgg aat cac ttt tct ggg gga ctc tct cat cag att gag cat     1344
   Trp Phe Trp Asn His Phe Ser Gly Gly Leu Ser His Gln Ile Glu His
           435                 440                 445
   cac ttg ttc ccc agc att tgt cat aca aac tac tgt cat atc cag gat     1392
   His Leu Phe Pro Ser Ile Cys His Thr Asn Tyr Cys His Ile Gln Asp
       450                 455                 460
   gtt gtg gag agt acg tgt gct gag tac gga gtt ccg tat cag agt gag     1440
   Val Val Glu Ser Thr Cys Ala Glu Tyr Gly Val Pro Tyr Gln Ser Glu
   465                 470                 475                 480
   agt aat ttg ttt gtt gct tat gga aag atg att agt cat ttg aag ttt     1488
   Ser Asn Leu Phe Val Ala Tyr Gly Lys Met Ile Ser His Leu Lys Phe
                   485                 490                 495
   ttg ggt aaa gcc aag tgt gag tag                                     1512
   Leu Gly Lys Ala Lys Cys Glu
               500
<210> 79
   <211> 503
   <212> PRT
   <213> Thalassiosira pseudonana
<400> 79
   Met Cys Asn Gly Asn Leu Pro Ala Ser Thr Ala Gln Leu Lys Ser Thr
   1               5                   10                  15
   Ser Lys Pro Gln Gln Gln His Glu His Arg Thr Ile Ser Lys Ser Glu
               20                  25                  30
   Leu Ala Gln His Asn Thr Pro Lys Ser Ala Trp Cys Ala Val His Ser
           35                  40                  45
   Thr Pro Ala Thr Asp Pro Ser His Ser Asn Asn Lys Gln His Ala His
       50                  55                  60
   Leu Val Leu Asp Ile Thr Asp Phe Ala Ser Arg His Pro Gly Gly Asp
   65                  70                  75                  80
   Leu Ile Leu Leu Ala Ser Gly Lys Asp Ala Ser Val Leu Phe Glu Thr
                   85                  90                  95
   Tyr His Pro Arg Gly Val Pro Thr Ser Leu Ile Gln Lys Leu Gln Ile
               100                 105                 110
   Gly Val Met Glu Glu Glu Ala Phe Arg Asp Ser Phe Tyr Ser Trp Thr
           115                 120                 125
   Asp Ser Asp Phe Tyr Thr Val Leu Lys Arg Arg Val Val Glu Arg Leu
       130                 135                 140
   Glu Glu Arg Gly Leu Asp Arg Arg Gly Ser Lys Glu Ile Trp Ile Lys
   145                 150                 155                 160
   Ala Leu Phe Leu Leu Val Gly Phe Trp Tyr Cys Leu Tyr Lys Met Tyr
                   165                 170                 175
   Thr Thr Ser Asp Ile Asp Gln Tyr Gly Ile Ala Ile Ala Tyr Ser Ile
               180                 185                 190
   Gly Met Gly Thr Phe Ala Ala Phe Ile Gly Thr Cys Ile Gln His Asp
           195                 200                 205
   Gly Asn His Gly Ala Phe Ala Gln Asn Lys Leu Leu Asn Lys Leu Ala
       210                 215                 220
   Gly Trp Thr Leu Asp Met Ile Gly Ala Ser Ala Phe Thr Trp Glu Leu
   225                 230                 235                 240
   Gln His Met Leu Gly His His Pro Tyr Thr Asn Val Leu Asp Gly Val
                   245                 250                 255
   Glu Glu Glu Arg Lys Glu Arg Gly Glu Asp Val Ala Leu Glu Glu Lys
               260                 265                 270
   Asp Gln Asp Phe Glu Val Ala Thr Ser Gly Arg Leu Tyr His Ile Asp
   275 280 285
   Ala Asn Val Arg Tyr Gly Ser Val Trp Asn Val Met Arg Phe Trp Ala
   290 295 300
   Met Lys Val Ile Thr Met Gly Tyr Met Met Gly Leu Pro Ile Tyr Phe
   305 310 315 320
   His Gly Val Leu Arg Gly Val Gly Leu Phe Val Ile Gly His Leu Ala
   325 330 335
   Cys Gly Glu Leu Leu Ala Thr Met Phe Ile Val Asn His Val Ile Glu
   340 345 350
   Gly Val Ser Tyr Gly Thr Lys Asp Leu Val Gly Gly Ala Ser His Val
   355 360 365
   Asp Glu Lys Lys Ile Val Lys Pro Thr Thr Val Leu Gly Asp Thr Pro
   370 375 380
   Met Val Lys Thr Arg Glu Glu Ala Leu Lys Ser Asn Ser Asn Asn Asn
   385 390 395 400
   Lys Lys Lys Gly Glu Lys Asn Ser Val Pro Ser Val Pro Phe Asn Asp
   405 410 415
   Trp Ala Ala Val Gln Cys Gln Thr Ser Val Asn Trp Ser Pro Gly Ser
   420 425 430
   Trp Phe Trp Asn His Phe Ser Gly Gly Leu Ser His Gln Ile Glu His
   435 440 445
   His Leu Phe Pro Ser Ile Cys His Thr Asn Tyr Cys His Ile Gln Asp
   450 455 460
   Val Val Glu Ser Thr Cys Ala Glu Tyr Gly Val Pro Tyr Gln Ser Glu
   465 470 475 480
   Ser Asn Leu Phe Val Ala Tyr Gly Lys Met Ile Ser His Leu Lys Phe
   485 490 495
   Leu Gly Lys Ala Lys Cys Glu
   500
<210> 80
   <211> 1626
   <212> DNA
   <213> Euglena gracilis
<220>
   <221> CDS
   <222> (1)..(1626)
   <223> delta-4-Desaturase
<400> 80
   atg ttg gtg ctg ttt ggc aat ttc tat gtc aag caa tac tcc caa aag 48
   Met Leu Val Leu Phe Gly Asn Phe Tyr Val Lys Gln Tyr Ser Gln Lys
   1 5 10 15
   aac ggc aag ccg gag aac gga gcc acc cct gag aac gga gcg aag ccg 96
   Asn Gly Lys Pro Glu Asn Gly Ala Thr Pro Glu Asn Gly Ala Lys Pro
   20 25 30
   caa cct tgc gag aac ggc acg gtg gaa aag cga gag aat gac acc gcc 144
   Gln Pro Cys Glu Asn Gly Thr Val Glu Lys Arg Glu Asn Asp Thr Ala
   35 40 45
   aac gtt cgg ccc acc cgt cca gct gga ccc ccg ccg gcc acg tac tac 192
   Asn Val Arg Pro Thr Arg Pro Ala Gly Pro Pro Pro Ala Thr Tyr Tyr
   50 55 60
   gac tcc ctg gca gtg tcg ggg cag ggc aag gag cgg ctg ttc acc acc 240
   Asp Ser Leu Ala Val Ser Gly Gln Gly Lys Glu Arg Leu Phe Thr Thr
   65 70 75 80
   gat gag gtg agg cgg cac atc ctc ccc acc gat ggc tgg ctg acg tgc 288
   Asp Glu Val Arg Arg His Ile Leu Pro Thr Asp Gly Trp Leu Thr Cys
   85 90 95
   cac gaa gga gtc tac gat gtc act gat ttc ctt gcc aag cac cct ggt 336
   His Glu Gly Val Tyr Asp Val Thr Asp Phe Leu Ala Lys His Pro Gly
   100 105 110
   ggc ggt gtc atc acg ctg ggc ctt gga agg gac tgc aca atc ctc atc 384
   Gly Gly Val Ile Thr Leu Gly Leu Gly Arg Asp Cys Thr Ile Leu Ile
   115 120 125
   gag tca tac cac cct gct ggg cgc ccg gac aag gtg atg gag aag tac 432
   Glu Ser Tyr His Pro Ala Gly Arg Pro Asp Lys Val Met Glu Lys Tyr
   130 135 140
   cgc att ggt acg ctg cag gac ccc aag acg ttc tat gct tgg gga gag 480
   Arg Ile Gly Thr Leu Gln Asp Pro Lys Thr Phe Tyr Ala Trp Gly Glu
   145 150 155 160
   tcc gat ttc tac cct gag ttg aag cgc cgg gcc ctt gca agg ctg aag 528
   Ser Asp Phe Tyr Pro Glu Leu Lys Arg Arg Ala Leu Ala Arg Leu Lys
   165 170 175
   gag gct ggt cag gcg cgg cgc ggc ggc ctt ggg gtg aag gcc ctc ctg 576
   Glu Ala Gly Gln Ala Arg Arg Gly Gly Leu Gly Val Lys Ala Leu Leu
   180 185 190
   gtg ctc acc ctc ttc ttc gtg tcg tgg tac atg tgg gtg gcc cac aag 624
   Val Leu Thr Leu Phe Phe Val Ser Trp Tyr Met Trp Val Ala His Lys
   195 200 205
   tcc ttc ctc tgg gcc gcc gtc tgg ggc ttc gcc ggc tcc cac gtc ggg 672
   Ser Phe Leu Trp Ala Ala Val Trp Gly Phe Ala Gly Ser His Val Gly
   210 215 220
   ctg agc atc cag cac gat ggc aac cac ggc gcg ttc agc cgc aac aca 720
   Leu Ser Ile Gln His Asp Gly Asn His Gly Ala Phe Ser Arg Asn Thr
   225 230 235 240
   ctg gtg aac cgc ctg gcg ggg tgg ggc atg gac ttg atc ggc gcg tcg 768
   Leu Val Asn Arg Leu Ala Gly Trp Gly Met Asp Leu Ile Gly Ala Ser
   245 250 255
   tcc acg gtg tgg gag tac cag cac gtc atc ggc cac cac cag tac acc      816
   Ser Thr Val Trp Glu Tyr Gln His Val Ile Gly His His Gln Tyr Thr
               260                 265                 270
   aac ctc gtg tcg gac acg cta ttc agt ctg cct gag aac gat ccg gac      864
   Asn Leu Val Ser Asp Thr Leu Phe Ser Leu Pro Glu Asn Asp Pro Asp
           275                 280                 285
   gtc ttc tcc agc tac ccg ctg atg cgc atg cac ccg gat acg gcg tgg      912
   Val Phe Ser Ser Tyr Pro Leu Met Arg Met His Pro Asp Thr Ala Trp
       290                 295                 300
   cag ccg cac cac cgc ttc cag cac ctg ttc gcg ttc cca ctg ttc gcc      960
   Gln Pro His His Arg Phe Gln His Leu Phe Ala Phe Pro Leu Phe Ala
   305                 310                 315                 320
   ctg atg aca atc agc aag gtg ctg acc agc gat ttc gct gtc tgc ctc     1008
   Leu Met Thr Ile Ser Lys Val Leu Thr Ser Asp Phe Ala Val Cys Leu
                   325                 330                 335
   agc atg aag aag ggg tcc atc gac tgc tcc tcc agg ctc gtc cca ctg     1056
   Ser Met Lys Lys Gly Ser Ile Asp Cys Ser Ser Arg Leu Val Pro Leu
               340                 345                 350
   gag ggg cag ctg ctg ttc tgg ggg gcc aag ctg gcg aac ttc ctg ttg     1104
   Glu Gly Gln Leu Leu Phe Trp Gly Ala Lys Leu Ala Asn Phe Leu Leu
           355                 360                 365
   cag att gtg ttg cca tgc tac ctc cac ggg aca gct atg ggc ctg gcc     1152
   Gln Ile Val Leu Pro Cys Tyr Leu His Gly Thr Ala Met Gly Leu Ala
       370                 375                 380
   ctc ttc tct gtt gct cac ctt gtg tcg ggg gag tac ctc gcg atc tgc     1200
   Leu Phe Ser Val Ala His Leu Val Ser Gly Glu Tyr Leu Ala Ile Cys
   385                 390                 395                 400
   ttc atc atc aac cac atc agc gag tct tgt gag ttt atg aat aca agc     1248
   Phe Ile Ile Asn His Ile Ser Glu Ser Cys Glu Phe Met Asn Thr Ser
                   405                 410                 415
   ttt caa acc gcc gcc cgg agg aca gag atg ctt cag gca gca cat cag     1296
   Phe Gln Thr Ala Ala Arg Arg Thr Glu Met Leu Gln Ala Ala His Gln
               420                 425                 430
   gca gcg gag gcc aag aag gtg aag ccc acc cct cca ccg aac gat tgg     1344
   Ala Ala Glu Ala Lys Lys Val Lys Pro Thr Pro Pro Pro Asn Asp Trp
           435                 440                 445
   gct gtg aca cag gtc caa tgc tgc gtg aat tgg aga tca ggt ggc gtg     1392
   Ala Val Thr Gln Val Gln Cys Cys Val Asn Trp Arg Ser Gly Gly Val
       450                 455                 460
   ttg gcc aat cac ctc tct gga ggc ttg aac cac cag atc gag cat cat     1440
   Leu Ala Asn His Leu Ser Gly Gly Leu Asn His Gln Ile Glu His His
   465                 470                 475                 480
   ctg ttc ccc agc atc tcg cat gcc aac tac ccc acc atc gcc cct gtt     1488
   Leu Phe Pro Ser Ile Ser His Ala Asn Tyr Pro Thr Ile Ala Pro Val
                   485                 490                 495
   gtg aag gag gtg tgc gag gag tac ggg ttg ccg tac aag aat tac gtc     1536
   Val Lys Glu Val Cys Glu Glu Tyr Gly Leu Pro Tyr Lys Asn Tyr Val
               500                 505                 510
   acg ttc tgg gat gca gtc tgt ggc atg gtt cag cac ctc cgg ttg atg     1584
   Thr Phe Trp Asp Ala Val Cys Gly Met Val Gln His Leu Arg Leu Met
           515                 520                 525
   ggt gct cca ccg gtg cca acg aac ggg gac aaa aag tca taa                 1626
   Gly Ala Pro Pro Val Pro Thr Asn Gly Asp Lys Lys Ser
       530                 535                 540
<210> 81
   <211> 541
   <212> PRT
   <213> Euglena gracilis
<400> 81
   Met Leu Val Leu Phe Gly Asn Phe Tyr Val Lys Gln Tyr Ser Gln Lys
   1               5                   10                  15
   Asn Gly Lys Pro Glu Asn Gly Ala Thr Pro Glu Asn Gly Ala Lys Pro
               20                  25                  30
   Gln Pro Cys Glu Asn Gly Thr Val Glu Lys Arg Glu Asn Asp Thr Ala
           35                  40                  45
   Asn Val Arg Pro Thr Arg Pro Ala Gly Pro Pro Pro Ala Thr Tyr Tyr
       50                  55                  60
   Asp Ser Leu Ala Val Ser Gly Gln Gly Lys Glu Arg Leu Phe Thr Thr
   65                  70                  75                  80
   Asp Glu Val Arg Arg His Ile Leu Pro Thr Asp Gly Trp Leu Thr Cys
                   85                  90                  95
   His Glu Gly Val Tyr Asp Val Thr Asp Phe Leu Ala Lys His Pro Gly
               100                 105                 110
   Gly Gly Val Ile Thr Leu Gly Leu Gly Arg Asp Cys Thr Ile Leu Ile
           115                 120                 125
   Glu Ser Tyr His Pro Ala Gly Arg Pro Asp Lys Val Met Glu Lys Tyr
       130                 135                 140
   Arg Ile Gly Thr Leu Gln Asp Pro Lys Thr Phe Tyr Ala Trp Gly Glu
   145                 150                 155                 160
   Ser Asp Phe Tyr Pro Glu Leu Lys Arg Arg Ala Leu Ala Arg Leu Lys
                   165                 170                 175
   Glu Ala Gly Gln Ala Arg Arg Gly Gly Leu Gly Val Lys Ala Leu Leu
               180                 185                 190
   Val Leu Thr Leu Phe Phe Val Ser Trp Tyr Met Trp Val Ala His Lys
           195                 200                 205
   Ser Phe Leu Trp Ala Ala Val Trp Gly Phe Ala Gly Ser His Val Gly
       210                 215                 220
   Leu Ser Ile Gln His Asp Gly Asn His Gly Ala Phe Ser Arg Asn Thr
   225 230 235 240
   Leu Val Asn Arg Leu Ala Gly Trp Gly Met Asp Leu Ile Gly Ala Ser
   245 250 255
   Ser Thr Val Trp Glu Tyr Gln His Val Ile Gly His His Gln Tyr Thr
   260 265 270
   Asn Leu Val Ser Asp Thr Leu Phe Ser Leu Pro Glu Asn Asp Pro Asp
   275 280 285
   Val Phe Ser Ser Tyr Pro Leu Met Arg Met His Pro Asp Thr Ala Trp
   290 295 300
   Gln Pro His His Arg Phe Gln His Leu Phe Ala Phe Pro Leu Phe Ala
   305 310 315 320
   Leu Met Thr Ile Ser Lys Val Leu Thr Ser Asp Phe Ala Val Cys Leu
   325 330 335
   Ser Met Lys Lys Gly Ser Ile Asp Cys Ser Ser Arg Leu Val Pro Leu
   340 345 350
   Glu Gly Gln Leu Leu Phe Trp Gly Ala Lys Leu Ala Asn Phe Leu Leu
   355 360 365
   Gln Ile Val Leu Pro Cys Tyr Leu His Gly Thr Ala Met Gly Leu Ala
   370 375 380
   Leu Phe Ser Val Ala His Leu Val Ser Gly Glu Tyr Leu Ala Ile Cys
   385 390 395 400
   Phe Ile Ile Asn His Ile Ser Glu Ser Cys Glu Phe Met Asn Thr Ser
   405 410 415
   Phe Gln Thr Ala Ala Arg Arg Thr Glu Met Leu Gln Ala Ala His Gln
   420 425 430
   Ala Ala Glu Ala Lys Lys Val Lys Pro Thr Pro Pro Pro Asn Asp Trp
   435 440 445
   Ala Val Thr Gln Val Gln Cys Cys Val Asn Trp Arg Ser Gly Gly Val
   450 455 460
   Leu Ala Asn His Leu Ser Gly Gly Leu Asn His Gln Ile Glu His His
   465 470 475 480
   Leu Phe Pro Ser Ile Ser His Ala Asn Tyr Pro Thr Ile Ala Pro Val
   485 490 495
   Val Lys Glu Val Cys Glu Glu Tyr Gly Leu Pro Tyr Lys Asn Tyr Val
   500 505 510
   Thr Phe Trp Asp Ala Val Cys Gly Met Val Gln His Leu Arg Leu Met
   515 520 525
   Gly Ala Pro Pro Val Pro Thr Asn Gly Asp Lys Lys Ser
   530 535 540
<210> 82
   <211> 1548
   <212> DNA
   <213> Thraustochytrium
<220>
   <221> CDS
   <222> (1)..(1548)
   <223> delta-4-Desaturase
<400> 82
   atg acg gtc ggg ttt gac gaa acg gtg act atg gac acg gtc cgc aac      48
   Met Thr Val Gly Phe Asp Glu Thr Val Thr Met Asp Thr Val Arg Asn
   1               5                   10                  15
   cac aac atg ccg gac gac gcc tgg tgc gcg atc cac ggc acc gtg tac      96
   His Asn Met Pro Asp Asp Ala Trp Cys Ala Ile His Gly Thr Val Tyr
               20                  25                  30
   gac atc acc aag ttc agc aag gtg cac ccc ggc ggg gac atc atc atg     144
   Asp Ile Thr Lys Phe Ser Lys Val His Pro Gly Gly Asp Ile Ile Met
           35                  40                  45
   ctg gcc gct ggc aag gag gcc acc atc ctg ttc gag acc tac cac atc     192
   Leu Ala Ala Gly Lys Glu Ala Thr Ile Leu Phe Glu Thr Tyr His Ile
       50                  55                  60
   aag ggc gtc ccg gac gcg gtg ctg cgc aag tac aag gtc ggc aag ctc     240
   Lys Gly Val Pro Asp Ala Val Leu Arg Lys Tyr Lys Val Gly Lys Leu
   65                  70                  75                  80
   ccc cag ggc aag aag ggc gaa acg agc cac atg ccc acc ggg ctc gac     288
   Pro Gln Gly Lys Lys Gly Glu Thr Ser His Met Pro Thr Gly Leu Asp
                   85                  90                  95
   tcg gcc tcc tac tac tcg tgg gac agc gag ttt tac agg gtg ctc cgc     336
   Ser Ala Ser Tyr Tyr Ser Trp Asp Ser Glu Phe Tyr Arg Val Leu Arg
               100                 105                 110
   gag cgc gtc gcc aag aag ctg gcc gag ccc ggc ctc atg cag cgc gcg     384
   Glu Arg Val Ala Lys Lys Leu Ala Glu Pro Gly Leu Met Gln Arg Ala
           115                 120                 125
   cgc atg gag ctc tgg gcc aag gcg atc ttc ctc ctg gca ggt ttc tgg     432
   Arg Met Glu Leu Trp Ala Lys Ala Ile Phe Leu Leu Ala Gly Phe Trp
       130                 135                 140
   ggc tcc ctt tac gcc atg tgc gtg cta gac ccg cac ggc ggt gcc atg     480
   Gly Ser Leu Tyr Ala Met Cys Val Leu Asp Pro His Gly Gly Ala Met
   145                 150                 155                 160
   gta gcc gcc gtt acg ctc ggc gtg ttc gct gcc ttt gtc gga act tgc     528
   Val Ala Ala Val Thr Leu Gly Val Phe Ala Ala Phe Val Gly Thr Cys
                   165                 170                 175
   atc cag cac gac ggc agc cac ggc gcc ttc tcc aag tcg cga ttc atg      576
   Ile Gln His Asp Gly Ser His Gly Ala Phe Ser Lys Ser Arg Phe Met
               180                 185                 190
   aac aag gcg gcg ggc tgg acc ctc gac atg atc ggc gcg agt gcg atg      624
   Asn Lys Ala Ala Gly Trp Thr Leu Asp Met Ile Gly Ala Ser Ala Met
           195                 200                 205
   acc tgg gag atg cag cac gtt ctt ggc cac cac ccg tac acc aac ctc      672
   Thr Trp Glu Met Gln His Val Leu Gly His His Pro Tyr Thr Asn Leu
       210                 215                 220
   atc gag atg gag aac ggt ttg gcc aag gtc aag ggc gcc gac gtc gac      720
   Ile Glu Met Glu Asn Gly Leu Ala Lys Val Lys Gly Ala Asp Val Asp
   225                 230                 235                 240
   ccg aag aag gtc gac cag gag agc gac ccg gac gtc ttc agt acg tac      768
   Pro Lys Lys Val Asp Gln Glu Ser Asp Pro Asp Val Phe Ser Thr Tyr
                   245                 250                 255
   ccg atg ctt cgc ctg cac ccg tgg cac cgc cag cgg ttt tac cac aag      816
   Pro Met Leu Arg Leu His Pro Trp His Arg Gln Arg Phe Tyr His Lys
               260                 265                 270
   ttc cag cac ctg tac gcc ccg ttt atc ttt ggg tct atg acg att aac      864
   Phe Gln His Leu Tyr Ala Pro Phe Ile Phe Gly Ser Met Thr Ile Asn
           275                 280                 285
   aag gtg att tcc cag gat gtc ggg gtt gtg ctg cgc aag cgc ctg ttc      912
   Lys Val Ile Ser Gln Asp Val Gly Val Val Leu Arg Lys Arg Leu Phe
       290                 295                 300
   cag atc gac gcc aac tgc cgg tat ggc agc ccc tgg tac gtg gcc cgc      960
   Gln Ile Asp Ala Asn Cys Arg Tyr Gly Ser Pro Trp Tyr Val Ala Arg
   305                 310                 315                 320
   ttc tgg atc atg aag ctc ctc acc acg ctc tac atg gtg gcg ctt ccc     1008
   Phe Trp Ile Met Lys Leu Leu Thr Thr Leu Tyr Met Val Ala Leu Pro
                   325                 330                 335
   atg tac atg cag ggg cct gct cag ggc ttg aag ctt ttc ttc atg gcc     1056
   Met Tyr Met Gln Gly Pro Ala Gln Gly Leu Lys Leu Phe Phe Met Ala
               340                 345                 350
   cac ttc acc tgc gga gag gtc ctc gcc acc atg ttt att gtc aac cac     1104
   His Phe Thr Cys Gly Glu Val Leu Ala Thr Met Phe Ile Val Asn His
           355                 360                 365
   atc atc gag ggc gtc agc tac gct tcc aag gac gcg gtc aag ggc gtc     1152
   Ile Ile Glu Gly Val Ser Tyr Ala Ser Lys Asp Ala Val Lys Gly Val
       370                 375                 380
   atg gct ccg ccg cgc act gtg cac ggt gtc acc ccg atg cag gtg acg     1200
   Met Ala Pro Pro Arg Thr Val His Gly Val Thr Pro Met Gln Val Thr
   385                 390                 395                 400
   caa aag gcg ctc agt gcg gcc gag tcg gcc aag tcg gac gcc gac aag     1248
   Gln Lys Ala Leu Ser Ala Ala Glu Ser Ala Lys Ser Asp Ala Asp Lys
                   405                 410                 415
   acg acc atg atc ccc ctc aac gac tgg gcc gct gtg cag tgc cag acc     1296
   Thr Thr Met Ile Pro Leu Asn Asp Trp Ala Ala Val Gln Cys Gln Thr
               420                 425                 430
   tct gtg aac tgg gct gtc ggg tcg tgg ttt tgg aac cac ttt tcg ggc     1344
   Ser Val Asn Trp Ala Val Gly Ser Trp Phe Trp Asn His Phe Ser Gly
           435                 440                 445
   ggc ctc aac cac cag att gag cac cac tgc ttc ccc caa aac ccc cac      1392
   Gly Leu Asn His Gln Ile Glu His His Cys Phe Pro Gln Asn Pro His
       450                 455                 460
   acg gtc aac gtc tac atc tcg ggc atc gtc aag gag acc tgc gaa gaa      1440
   Thr Val Asn Val Tyr Ile Ser Gly Ile Val Lys Glu Thr Cys Glu Glu
   465                 470                 475                     480
   tac ggc gtg ccg tac cag gct gag atc agc ctc ttc tct gcc tat ttc      1488
   Tyr Gly Val Pro Tyr Gln Ala Glu Ile Ser Leu Phe Ser Ala Tyr Phe
                   485                 490                 495
   aag atg ctg tcg cac ctc cgc acg ctc ggc aac gag gac ctc acg gcc      1536
   Lys Met Leu Ser His Leu Arg Thr Leu Gly Asn Glu Asp Leu Thr Ala
               500                 505                 510
   tgg tcc acg tga                                                      1548
   Trp Ser Thr
           515
<210> 83
   <211> 515
   <212> PRT
   <213> Thraustochytrium
<400> 83
   Met Thr Val Gly Phe Asp Glu Thr Val Thr Met Asp Thr Val Arg Asn
   1 5 10 15
   His Asn Met Pro Asp Asp Ala Trp Cys Ala Ile His Gly Thr Val Tyr
   20 25 30
   Asp Ile Thr Lys Phe Ser Lys Val His Pro Gly Gly Asp Ile Ile Met
   35 40 45
   Leu Ala Ala Gly Lys Glu Ala Thr Ile Leu Phe Glu Thr Tyr His Ile
   50 55 60
   Lys Gly Val Pro Asp Ala Val Leu Arg Lys Tyr Lys Val Gly Lys Leu
   65 70 75 80
   Pro Gln Gly Lys Lys Gly Glu Thr Ser His Met Pro Thr Gly Leu Asp
   85 90 95
   Ser Ala Ser Tyr Tyr Ser Trp Asp Ser Glu Phe Tyr Arg Val Leu Arg
   100 105 110
   Glu Arg Val Ala Lys Lys Leu Ala Glu Pro Gly Leu Met Gln Arg Ala
   115 120 125
   Arg Met Glu Leu Trp Ala Lys Ala Ile Phe Leu Leu Ala Gly Phe Trp
   130 135 140
   Gly Ser Leu Tyr Ala Met Cys Val Leu Asp Pro His Gly Gly Ala Met
   145 150 155 160
   Val Ala Ala Val Thr Leu Gly Val Phe Ala Ala Phe Val Gly Thr Cys
   165 170 175
   Ile Gln His Asp Gly Ser His Gly Ala Phe Ser Lys Ser Arg Phe Met
   180 185 190
   Asn Lys Ala Ala Gly Trp Thr Leu Asp Met Ile Gly Ala Ser Ala Met
   195 200 205
   Thr Trp Glu Met Gln His Val Leu Gly His His Pro Tyr Thr Asn Leu
   210 215 220
   Ile Glu Met Glu Asn Gly Leu Ala Lys Val Lys Gly Ala Asp Val Asp
   225 230 235 240
   Pro Lys Lys Val Asp Gln Glu Ser Asp Pro Asp Val Phe Ser Thr Tyr
   245 250 255
   Pro Met Leu Arg Leu His Pro Trp His Arg Gln Arg Phe Tyr His Lys
   260 265 270
   Phe Gln His Leu Tyr Ala Pro Phe Ile Phe Gly Ser Met Thr Ile Asn
   275 280 285
   Lys Val Ile Ser Gln Asp Val Gly Val Val Leu Arg Lys Arg Leu Phe
   290 295 300
   Gln Ile Asp Ala Asn Cys Arg Tyr Gly Ser Pro Trp Tyr Val Ala Arg
   305 310 315 320
   Phe Trp Ile Met Lys Leu Leu Thr Thr Leu Tyr Met Val Ala Leu Pro
   325 330 335
   Met Tyr Met Gln Gly Pro Ala Gln Gly Leu Lys Leu Phe Phe Met Ala
   340 345 350
   His Phe Thr Cys Gly Glu Val Leu Ala Thr Met Phe Ile Val Asn His
   355 360 365
   Ile Ile Glu Gly Val Ser Tyr Ala Ser Lys Asp Ala Val Lys Gly Val
   370 375 380
   Met Ala Pro Pro Arg Thr Val His Gly Val Thr Pro Met Gln Val Thr
   385 390 395 400
   Gln Lys Ala Leu Ser Ala Ala Glu Ser Ala Lys Ser Asp Ala Asp Lys
   405 410 415
   Thr Thr Met Ile Pro Leu Asn Asp Trp Ala Ala Val Gln Cys Gln Thr
   420 425 430
   Ser Val Asn Trp Ala Val Gly Ser Trp Phe Trp Asn His Phe Ser Gly
   435 440 445
   Gly Leu Asn His Gln Ile Glu His His Cys Phe Pro Gln Asn Pro His
   450 455 460
   Thr Val Asn Val Tyr Ile Ser Gly Ile Val Lys Glu Thr Cys Glu Glu
   465 470 475 480
   Tyr Gly Val Pro Tyr Gln Ala Glu Ile Ser Leu Phe Ser Ala Tyr Phe
   485 490 495
   Lys Met Leu Ser His Leu Arg Thr Leu Gly Asn Glu Asp Leu Thr Ala
   500 505 510
   Trp Ser Thr
   515
<210> 84
   <211> 1611
   <212> DNA
   <213> Ostreococcus tauri
<220>
   <221> CDS
   <222> (1)..(1611)
   <223> delta-4-Desaturase
<400> 84
   atg tac ctc gga cgc ggc cgt ctc gag agc ggg acg acg cga ggg atg      48
   Met Tyr Leu Gly Arg Gly Arg Leu Glu Ser Gly Thr Thr Arg Gly Met
   1               5                   10                  15
   atg cgg acg cac gcg cgg cga ccg tcg acg acg tcg aat ccg tgc gcg      96
   Met Arg Thr His Ala Arg Arg Pro Ser Thr Thr Ser Asn Pro Cys Ala
               20                  25                  30
   cgg tca cgc gtg cgt aag acg acg gag cga tcg ctc gcg cga gtg cga     144
   Arg Ser Arg Val Arg Lys Thr Thr Glu Arg Ser Leu Ala Arg Val Arg
           35                  40                  45
   cga tcg acg agt gag aag gga agc gcg ctc gtg ctc gag cga gag agc     192
   Arg Ser Thr Ser Glu Lys Gly Ser Ala Leu Val Leu Glu Arg Glu Ser
       50                  55                  60
   gaa cgg gag aag gag gag gga ggg aaa gcg cga gcg gag gga ttg cga     240
   Glu Arg Glu Lys Glu Glu Gly Gly Lys Ala Arg Ala Glu Gly Leu Arg
   65                  70                  75                  80
   ttc caa cgc ccg gac gtc gcc gcg ccg ggg gga gcg gat cct tgg aac     288
   Phe Gln Arg Pro Asp Val Ala Ala Pro Gly Gly Ala Asp Pro Trp Asn
                   85                  90                  95
   gac gag aag tgg aca aag acc aag tgg acg gta ttc aga gac gtc gcg     336
   Asp Glu Lys Trp Thr Lys Thr Lys Trp Thr Val Phe Arg Asp Val Ala
               100                 105                 110
   tac gat ctc gat cct ttc ttc gct cga cac ccc gga gga gac tgg ctc     384
   Tyr Asp Leu Asp Pro Phe Phe Ala Arg His Pro Gly Gly Asp Trp Leu
           115                 120                 125
   ctg aac ttg gcc gtg gga cga gac tgc acc gcg ctc atc gaa tcc tat 432
   Leu Asn Leu Ala Val Gly Arg Asp Cys Thr Ala Leu Ile Glu Ser Tyr
       130                 135                 140
   cac ttg cga cca gag gtg gcg acg gct cgt ttc aga atg ctg ccc aaa 480
   His Leu Arg Pro Glu Val Ala Thr Ala Arg Phe Arg Met Leu Pro Lys
   145                 150                 155                 160
   ctc gag gat ttt ccc gtc gag gcc gtg ccc aag tcc ccg aga ccg aac 528
   Leu Glu Asp Phe Pro Val Glu Ala Val Pro Lys Ser Pro Arg Pro Asn
                   165                 170                 175
   gat tcg ccg tta tac aac aac att cgc aac cga gtc cgc gaa gag ctc 576
   Asp Ser Pro Leu Tyr Asn Asn Ile Arg Asn Arg Val Arg Glu Glu Leu
               180                 185                 190
   ttc cca gag gag gga aag aat atg cac aga cag ggc ggc gac cac ggc 624
   Phe Pro Glu Glu Gly Lys Asn Met His Arg Gln Gly Gly Asp His Gly
           195                 200                 205
   gac ggt gac gat tct ggg ttt cgc cgc ctt ttg ctt atg ccg tgt acc 672
   Asp Gly Asp Asp Ser Gly Phe Arg Arg Leu Leu Leu Met Pro Cys Thr
       210                 215                 220
   tat tcc ctt ccg ggg gtt cct ttc cgg ctg cct cct cgg gtc tcg cgg 720
   Tyr Ser Leu Pro Gly Val Pro Phe Arg Leu Pro Pro Arg Val Ser Arg
   225                 230                 235                 240
   ggg cgt gga ttg gtc tca cga ttc agg cac tgc gcc aac cac ggc gcg 768
   Gly Arg Gly Leu Val Ser Arg Phe Arg His Cys Ala Asn His Gly Ala
                   245                 250                 255
   atg tct cct tcg ccg gcc gtt aac ggc gtc ctc ggt ttg acg aac gat 816
   Met Ser Pro Ser Pro Ala Val Asn Gly Val Leu Gly Leu Thr Asn Asp
               260                 265                 270
   ctc atc ggc ggc tcg tcc ttg atg tgg aga tat cac cac caa gtc agc 864
   Leu Ile Gly Gly Ser Ser Leu Met Trp Arg Tyr His His Gln Val Ser
           275                 280                 285
   cac cac att cat tgc aac gac aac gcc atg gat caa gac gtg tac acg 912
   His His Ile His Cys Asn Asp Asn Ala Met Asp Gln Asp Val Tyr Thr
       290                 295                 300
   gcg atg cca tta ttg cgt ttc gac gct cgc cgg ccc aag tcc tgg tac 960
   Ala Met Pro Leu Leu Arg Phe Asp Ala Arg Arg Pro Lys Ser Trp Tyr
   305                 310                 315                 320
   cat cgc ttc cag cag tgg tac atg ttt tta gcg ttc ccg ttg ttg cag 1008
   His Arg Phe Gln Gln Trp Tyr Met Phe Leu Ala Phe Pro Leu Leu Gln
                   325                 330                 335
   gtt gcc ttc caa gtc gga gac att gcc gca ctg ttc acg cgt gat acc 1056
   Val Ala Phe Gln Val Gly Asp Ile Ala Ala Leu Phe Thr Arg Asp Thr
               340                 345                 350
   gaa ggc gct aag ctt cac ggg gcg acg acg tgg gag ctt acc acg gtt 1104
   Glu Gly Ala Lys Leu His Gly Ala Thr Thr Trp Glu Leu Thr Thr Val
           355                 360                 365
   gtc ctc ggt aag att gtg cac ttc ggt ctt ttg ttg ggg ccg ttg atg 1152
   Val Leu Gly Lys Ile Val His Phe Gly Leu Leu Leu Gly Pro Leu Met
       370                 375                 380
   aac cac gcg gtg agt tct gtt ttg ctg ggg atc gtc ggt ttc atg gcg 1200
   Asn His Ala Val Ser Ser Val Leu Leu Gly Ile Val Gly Phe Met Ala
   385                 390                 395                 400
   tgc caa ggt ata gtt ctg gcg tgc acg ttt gct gtg agt cac aat gtc      1248
   Cys Gln Gly Ile Val Leu Ala Cys Thr Phe Ala Val Ser His Asn Val
                   405                 410                 415
   gcg gag gcg aag ata cct gag gac acc gga gga gaa gcc tgg gag aga      1296
   Ala Glu Ala Lys Ile Pro Glu Asp Thr Gly Gly Glu Ala Trp Glu Arg
               420                 425                 430
   gat tgg ggt gtc cag cag ttg gtg act agc gcc gac tgg ggt gga aag      1344
   Asp Trp Gly Val Gln Gln Leu Val Thr Ser Ala Asp Trp Gly Gly Lys
           435                 440                 445
   ata ggt aac ttc ttc acg ggt ggc ctc aac ttg caa gtt gag cac cac      1392
   Ile Gly Asn Phe Phe Thr Gly Gly Leu Asn Leu Gln Val Glu His His
       450                 455                 460
   ttg ttt ccg gcg att tgc ttc gtc cac tac ccg gac atc gcg aag atc      1440
   Leu Phe Pro Ala Ile Cys Phe Val His Tyr Pro Asp Ile Ala Lys Ile
   465                 470                 475                 480
   gtg aag gaa gaa gcg gcc aag ctc aac atc cct tac gcg tct tac agg      1488
   Val Lys Glu Glu Ala Ala Lys Leu Asn Ile Pro Tyr Ala Ser Tyr Arg
                   485                 490                 495
   act ctt cct ggt att ttc gtc caa ttc tgg aga ttt atg aag gac atg      1536
   Thr Leu Pro Gly Ile Phe Val Gln Phe Trp Arg Phe Met Lys Asp Met
               500                 505                 510
   ggc acg gct gag caa att ggt gaa gtt cca ttg ccg aag att ccc aac      1584
   Gly Thr Ala Glu Gln Ile Gly Glu Val Pro Leu Pro Lys Ile Pro Asn
           515                 520                 525
   ccg cag ctc gcg ccg aag ctc gct tag                                  1611
   Pro Gln Leu Ala Pro Lys Leu Ala
       530                 535
<210> 85
   <211> 536
   <212> PRT
   <213> Ostreococcus tauri
<400> 85
   Met Tyr Leu Gly Arg Gly Arg Leu Glu Ser Gly Thr Thr Arg Gly Met
   1 5 10 15
   Met Arg Thr His Ala Arg Arg Pro Ser Thr Thr Ser Asn Pro Cys Ala
   20 25 30
   Arg Ser Arg Val Arg Lys Thr Thr Glu Arg Ser Leu Ala Arg Val Arg
   35 40 45
   Arg Ser Thr Ser Glu Lys Gly Ser Ala Leu Val Leu Glu Arg Glu Ser
   50 55 60
   Glu Arg Glu Lys Glu Glu Gly Gly Lys Ala Arg Ala Glu Gly Leu Arg
   65 70 75 80
   Phe Gln Arg Pro Asp Val Ala Ala Pro Gly Gly Ala Asp Pro Trp Asn
   85 90 95
   Asp Glu Lys Trp Thr Lys Thr Lys Trp Thr Val Phe Arg Asp Val Ala
               100                 105                 110
   Tyr Asp Leu Asp Pro Phe Phe Ala Arg His Pro Gly Gly Asp Trp Leu
           115                 120                 125
   Leu Asn Leu Ala Val Gly Arg Asp Cys Thr Ala Leu Ile Glu Ser Tyr
       130                 135                 140
   His Leu Arg Pro Glu Val Ala Thr Ala Arg Phe Arg Met Leu Pro Lys
   145                 150                 155                 160
   Leu Glu Asp Phe Pro Val Glu Ala Val Pro Lys Ser Pro Arg Pro Asn
                   165                 170                 175
   Asp Ser Pro Leu Tyr Asn Asn Ile Arg Asn Arg Val Arg Glu Glu Leu
               180                 185                 190
   Phe Pro Glu Glu Gly Lys Asn Met His Arg Gln Gly Gly Asp His Gly
           195                 200                 205
   Asp Gly Asp Asp Ser Gly Phe Arg Arg Leu Leu Leu Met Pro Cys Thr
       210                 215                 220
   Tyr Ser Leu Pro Gly Val Pro Phe Arg Leu Pro Pro Arg Val Ser Arg
   225                 230                 235                 240
   Gly Arg Gly Leu Val Ser Arg Phe Arg His Cys Ala Asn His Gly Ala
                   245                 250                 255
   Met Ser Pro Ser Pro Ala Val Asn Gly Val Leu Gly Leu Thr Asn Asp
               260                 265                 270
   Leu Ile Gly Gly Ser Ser Leu Met Trp Arg Tyr His His Gln Val Ser
           275                 280                 285
   His His Ile His Cys Asn Asp Asn Ala Met Asp Gln Asp Val Tyr Thr
       290                 295                 300
   Ala Met Pro Leu Leu Arg Phe Asp Ala Arg Arg Pro Lys Ser Trp Tyr
   305                 310                 315                 320
   His Arg Phe Gln Gln Trp Tyr Met Phe Leu Ala Phe Pro Leu Leu Gln
                   325                 330                 335
   Val Ala Phe Gln Val Gly Asp Ile Ala Ala Leu Phe Thr Arg Asp Thr
               340                 345                 350
   Glu Gly Ala Lys Leu His Gly Ala Thr Thr Trp Glu Leu Thr Thr Val
           355                 360                 365
   Val Leu Gly Lys Ile Val His Phe Gly Leu Leu Leu Gly Pro Leu Met
   370 375 380
   Asn His Ala Val Ser Ser Val Leu Leu Gly Ile Val Gly Phe Met Ala
   385 390 395 400
   Cys Gln Gly Ile Val Leu Ala Cys Thr Phe Ala Val Ser His Asn Val
   405 410 415
   Ala Glu Ala Lys Ile Pro Glu Asp Thr Gly Gly Glu Ala Trp Glu Arg
   420 425 430
   Asp Trp Gly Val Gln Gln Leu Val Thr Ser Ala Asp Trp Gly Gly Lys
   435 440 445
   Ile Gly Asn Phe Phe Thr Gly Gly Leu Asn Leu Gln Val Glu His His
   450 455 460
   Leu Phe Pro Ala Ile Cys Phe Val His Tyr Pro Asp Ile Ala Lys Ile
   465 470 475 480
   Val Lys Glu Glu Ala Ala Lys Leu Asn Ile Pro Tyr Ala Ser Tyr Arg
   485 490 495
   Thr Leu Pro Gly Ile Phe Val Gln Phe Trp Arg Phe Met Lys Asp Met
   500 505 510
   Gly Thr Ala Glu Gln Ile Gly Glu Val Pro Leu Pro Lys Ile Pro Asn
   515 520 525
   Pro Gln Leu Ala Pro Lys Leu Ala
   530 535
<210> 86
   <211> 960
   <212> DNA
   <213> Thalassiosira pseudonana
<220>
   <221> CDS
   <222> (1)..(960)
   <223> delta-5-Elongase
<400> 86
   atg gtg ttg tac aat gtg gcg caa gtg ctg ctc aat ggg tgg acg gtg      48
   Met Val Leu Tyr Asn Val Ala Gln Val Leu Leu Asn Gly Trp Thr Val
   1               5                   10                  15
   tat gcg att gtg gat gcg gtg atg aat aga gac cat ccg ttt att gga      96
   Tyr Ala Ile Val Asp Ala Val Met Asn Arg Asp His Pro Phe Ile Gly
               20                  25                  30
   agt aga agt ttg gtt ggg gcg gcg ttg cat agt ggg agc tcg tat gcg      144
   Ser Arg Ser Leu Val Gly Ala Ala Leu His Ser Gly Ser Ser Tyr Ala
           35                  40                  45
   gtg tgg gtt cat tat tgt gat aag tat ttg gag ttc ttt gat acg tat      192
   Val Trp Val His Tyr Cys Asp Lys Tyr Leu Glu Phe Phe Asp Thr Tyr
       50                  55                  60
   ttt atg gtg ttg agg ggg aaa atg gac cag atg gta ctt ggt gaa gtt      240
   Phe Met Val Leu Arg Gly Lys Met Asp Gln Met Val Leu Gly Glu Val
   65                  70                  75                  80
   ggt ggc agt gtg tgg tgt ggc gtt gga tat atg gat atg gag aag atg      288
   Gly Gly Ser Val Trp Cys Gly Val Gly Tyr Met Asp Met Glu Lys Met
                   85                  90                  95
   ata cta ctc agc ttt gga gtg cat cgg tct gct cag gga acg ggg aag      336
   Ile Leu Leu Ser Phe Gly Val His Arg Ser Ala Gln Gly Thr Gly Lys
               100                 105                 110
   gct ttc acc aac aac gtt acc aat cca cat ctc acg ctt cca cct cat      384
   Ala Phe Thr Asn Asn Val Thr Asn Pro His Leu Thr Leu Pro Pro His
           115                 120                 125
   tct aca aaa aca aaa aaa cag gtc tcc ttc ctc cac atc tac cac cac      432
   Ser Thr Lys Thr Lys Lys Gln Val Ser Phe Leu His Ile Tyr His His
       130                 135                 140
   acg acc ata gcg tgg gca tgg tgg atc gcc ctc cgc ttc tcc ccc ggt      480
   Thr Thr Ile Ala Trp Ala Trp Trp Ile Ala Leu Arg Phe Ser Pro Gly
   145                 150                 155                 160
   gga gac att tac ttc ggg gca ctc ctc aac tcc atc atc cac gtc ctc      528
   Gly Asp Ile Tyr Phe Gly Ala Leu Leu Asn Ser Ile Ile His Val Leu
                   165                 170                 175
   atg tat tcc tac tac gcc ctt gcc cta ctc aag gtc agt tgt cca tgg      576
   Met Tyr Ser Tyr Tyr Ala Leu Ala Leu Leu Lys Val Ser Cys Pro Trp
               180                 185                 190
   aaa cga tac ctg act caa gct caa tta ttg caa ttc aca agt gtg gtg      624
   Lys Arg Tyr Leu Thr Gln Ala Gln Leu Leu Gln Phe Thr Ser Val Val
           195                 200                 205
   gtt tat acg ggg tgt acg ggt tat act cat tac tat cat acg aag cat      672
   Val Tyr Thr Gly Cys Thr Gly Tyr Thr His Tyr Tyr His Thr Lys His
       210                 215                 220
   gga gcg gat gag aca cag cct agt tta gga acg tat tat ttc tgt tgt      720
   Gly Ala Asp Glu Thr Gln Pro Ser Leu Gly Thr Tyr Tyr Phe Cys Cys
   225                 230                 235                 240
   gga gtg cag gtg ttt gag atg gtt agt ttg ttt gta ctc ttt tcc atc      768
   Gly Val Gln Val Phe Glu Met Val Ser Leu Phe Val Leu Phe Ser Ile
                   245                 250                 255
   ttt tat aaa cga tcc tat tcg aag aag aac aag tca gga gga aag gat      816
   Phe Tyr Lys Arg Ser Tyr Ser Lys Lys Asn Lys Ser Gly Gly Lys Asp
               260                 265                 270
   agc aag aag aat gat gat ggg aat aat gag gat caa tgt cac aag gct      864
   Ser Lys Lys Asn Asp Asp Gly Asn Asn Glu Asp Gln Cys His Lys Ala
           275                 280                 285
   atg aag gat ata tcg gag ggt gcg aag gag gtt gtg ggg cat gca gcg      912
   Met Lys Asp Ile Ser Glu Gly Ala Lys Glu Val Val Gly His Ala Ala
       290                 295                 300
   aag gat gct gga aag ttg gtg gct acg aga gta agg tgt aag gtg taa      960
   Lys Asp Ala Gly Lys Leu Val Ala Thr Arg Val Arg Cys Lys Val
   305                 310                 315
<210> 87
   <211> 319
   <212> PRT
   <213> Thalassiosira pseudonana
<400> 87
   Met Val Leu Tyr Asn Val Ala Gln Val Leu Leu Asn Gly Trp Thr Val
   1 5 10 15
   Tyr Ala Ile Val Asp Ala Val Met Asn Arg Asp His Pro Phe Ile Gly
   20 25 30
   Ser Arg Ser Leu Val Gly Ala Ala Leu His Ser Gly Ser Ser Tyr Ala
   35 40 45
   Val Trp Val His Tyr Cys Asp Lys Tyr Leu Glu Phe Phe Asp Thr Tyr
   50 55 60
   Phe Met Val Leu Arg Gly Lys Met Asp Gln Met Val Leu Gly Glu Val
   65 70 75 80
   Gly Gly Ser Val Trp Cys Gly Val Gly Tyr Met Asp Met Glu Lys Met
   85 90 95
   Ile Leu Leu Ser Phe Gly Val His Arg Ser Ala Gln Gly Thr Gly Lys
   100 105 110
   Ala Phe Thr Asn Asn Val Thr Asn Pro His Leu Thr Leu Pro Pro His
   115 120 125
   Ser Thr Lys Thr Lys Lys Gln Val Ser Phe Leu His Ile Tyr His His
   130 135 140
   Thr Thr Ile Ala Trp Ala Trp Trp Ile Ala Leu Arg Phe Ser Pro Gly
   145 150 155 160
   Gly Asp Ile Tyr Phe Gly Ala Leu Leu Asn Ser Ile Ile His Val Leu
   165 170 175
   Met Tyr Ser Tyr Tyr Ala Leu Ala Leu Leu Lys Val Ser Cys Pro Trp
   180 185 190
   Lys Arg Tyr Leu Thr Gln Ala Gln Leu Leu Gln Phe Thr Ser Val Val
   195 200 205
   Val Tyr Thr Gly Cys Thr Gly Tyr Thr His Tyr Tyr His Thr Lys His
   210 215 220
   Gly Ala Asp Glu Thr Gln Pro Ser Leu Gly Thr Tyr Tyr Phe Cys Cys
   225 230 235 240
   Gly Val Gln Val Phe Glu Met Val Ser Leu Phe Val Leu Phe Ser Ile
                   245                 250                 255
   Phe Tyr Lys Arg Ser Tyr Ser Lys Lys Asn Lys Ser Gly Gly Lys Asp
               260                 265                 270
   Ser Lys Lys Asn Asp Asp Gly Asn Asn Glu Asp Gln Cys His Lys Ala
           275                 280                 285
   Met Lys Asp Ile Ser Glu Gly Ala Lys Glu Val Val Gly His Ala Ala
       290                 295                 300
   Lys Asp Ala Gly Lys Leu Val Ala Thr Arg Val Arg Cys Lys Val
   305                 310                 315
<210> 88
   <211> 819
   <212> DNA
   <213> Thalassiosira pseudonana
<220>
   <221> CDS
   <222> (1)..(819)
   <223> delta-5-Elongase
<400> 88
   atg gac gcc tac aac gct gca atg gat aag atc ggt gcc gcc atc atc      48
   Met Asp Ala Tyr Asn Ala Ala Met Asp Lys Ile Gly Ala Ala Ile Ile
   1               5                   10                  15
   gat tgg tct gat ccc gat gga aag ttc cgt gcc gat aga gag gac tgg      96
   Asp Trp Ser Asp Pro Asp Gly Lys Phe Arg Ala Asp Arg Glu Asp Trp
               20                  25                  30
   tgg ctc tgc gac ttc cgt agc gcc atc acc atc gcc ctc atc tac atc      144
   Trp Leu Cys Asp Phe Arg Ser Ala Ile Thr Ile Ala Leu Ile Tyr Ile
           35                  40                  45
   gcc ttc gtc atc ctc ggt tcc gcc gtc atg caa tcc ctc ccc gca atg      192
   Ala Phe Val Ile Leu Gly Ser Ala Val Met Gln Ser Leu Pro Ala Met
       50                  55                  60
   gat ccc tac ccc atc aaa ttc ctc tac aac gtc tcc caa atc ttc ctt      240
   Asp Pro Tyr Pro Ile Lys Phe Leu Tyr Asn Val Ser Gln Ile Phe Leu
   65                  70                  75                  80
   tgt gcc tac atg act gtc gag gcg gga ttt ttg gcc tac cgc aat gga      288
   Cys Ala Tyr Met Thr Val Glu Ala Gly Phe Leu Ala Tyr Arg Asn Gly
                   85                  90                  95
   tat acc gtc atg cct tgc aat cat ttc aat gtg aat gat cct ccc gtg      336
   Tyr Thr Val Met Pro Cys Asn His Phe Asn Val Asn Asp Pro Pro Val
               100                 105                 110
   gcg aat ctt ctt tgg ttg ttt tat att tcc aag gtg tgg gac ttt tgg      384
   Ala Asn Leu Leu Trp Leu Phe Tyr Ile Ser Lys Val Trp Asp Phe Trp
           115                 120                 125
   gat acc att ttc att gtg ttg ggg aag aag tgg cgt caa tta tct ttc      432
   Asp Thr Ile Phe Ile Val Leu Gly Lys Lys Trp Arg Gln Leu Ser Phe
       130                 135                 140
   ttg cat gta tac cat cac acc acc atc ttt cta ttc tat tgg ctg aat      480
   Leu His Val Tyr His His Thr Thr Ile Phe Leu Phe Tyr Trp Leu Asn
   145                 150                 155                 160
   gcc aat gtc ttg tac gat ggt gac atc ttc ctt acc atc ttg ctc aat      528
   Ala Asn Val Leu Tyr Asp Gly Asp Ile Phe Leu Thr Ile Leu Leu Asn
                   165                 170                 175
   gga ttc atc cac acg gtg atg tac acg tat tac ttc atc tgt atg cat      576
   Gly Phe Ile His Thr Val Met Tyr Thr Tyr Tyr Phe Ile Cys Met His
               180                 185                 190
   acc aaa gat tcc aag acg ggc aag agt ctt cct ata tgg tgg aag tcg      624
   Thr Lys Asp Ser Lys Thr Gly Lys Ser Leu Pro Ile Trp Trp Lys Ser
           195                 200                 205
   agt ttg acg gcg ttt cag ttg ttg caa ttc act atc atg atg agt cag      672
   Ser Leu Thr Ala Phe Gln Leu Leu Gln Phe Thr Ile Met Met Ser Gln
       210                 215                 220
   gct acc tac ctt gtc ttc cac ggg tgt gat aag gtg tcg ctt cgt atc      720
   Ala Thr Tyr Leu Val Phe His Gly Cys Asp Lys Val Ser Leu Arg Ile
   225                 230                 235                 240
   acg att gtg tac ttt gtg tcc ctt ttg agt ttg ttc ttc ctt ttt gct      768
   Thr Ile Val Tyr Phe Val Ser Leu Leu Ser Leu Phe Phe Leu Phe Ala
                   245                 250                 255
   cag ttc ttt gtg caa tca tac atg gca ccc aaa aag aag aag agt gct      816
   Gln Phe Phe Val Gln Ser Tyr Met Ala Pro Lys Lys Lys Lys Ser Ala
               260                 265                 270
   tag                                                                  819
<210> 89
   <211> 272
   <212> PRT
   <213> Thalassiosira pseudonana
<400> 89
   Met Asp Ala Tyr Asn Ala Ala Met Asp Lys Ile Gly Ala Ala Ile Ile
   1 5 10 15
   Asp Trp Ser Asp Pro Asp Gly Lys Phe Arg Ala Asp Arg Glu Asp Trp
   20 25 30
   Trp Leu Cys Asp Phe Arg Ser Ala Ile Thr Ile Ala Leu Ile Tyr Ile
   35 40 45
   Ala Phe Val Ile Leu Gly Ser Ala Val Met Gln Ser Leu Pro Ala Met
   50 55 60
   Asp Pro Tyr Pro Ile Lys Phe Leu Tyr Asn Val Ser Gln Ile Phe Leu
   65 70 75 80
   Cys Ala Tyr Met Thr Val Glu Ala Gly Phe Leu Ala Tyr Arg Asn Gly
   85 90 95
   Tyr Thr Val Met Pro Cys Asn His Phe Asn Val Asn Asp Pro Pro Val
   100 105 110
   Ala Asn Leu Leu Trp Leu Phe Tyr Ile Ser Lys Val Trp Asp Phe Trp
           115                 120                 125
   Asp Thr Ile Phe Ile Val Leu Gly Lys Lys Trp Arg Gln Leu Ser Phe
       130                 135                 140
   Leu His Val Tyr His His Thr Thr Ile Phe Leu Phe Tyr Trp Leu Asn
   145                 150                 155                 160
   Ala Asn Val Leu Tyr Asp Gly Asp Ile Phe Leu Thr Ile Leu Leu Asn
                   165                 170                 175
   Gly Phe Ile His Thr Val Met Tyr Thr Tyr Tyr Phe Ile Cys Met His
               180                 185                 190
   Thr Lys Asp Ser Lys Thr Gly Lys Ser Leu Pro Ile Trp Trp Lys Ser
           195                 200                 205
   Ser Leu Thr Ala Phe Gln Leu Leu Gln Phe Thr Ile Met Met Ser Gln
       210                 215                 220
   Ala Thr Tyr Leu Val Phe His Gly Cys Asp Lys Val Ser Leu Arg Ile
   225                 230                 235                 240
   Thr Ile Val Tyr Phe Val Ser Leu Leu Ser Leu Phe Phe Leu Phe Ala
                   245                 250                 255
   Gln Phe Phe Val Gln Ser Tyr Met Ala Pro Lys Lys Lys Lys Ser Ala
               260                 265                 270
<210> 90
   <211> 936
   <212> DNA
   <213> Crypthecodinium cohnii
<220>
   <221> CDS
   <222> (1)..(936)
   <223> delta-5-Elongase
<400> 90
   atg tct gcc ttc atg act ctc cca cag gct ctc tcc gat gtg acc tcg      48
   Met Ser Ala Phe Met Thr Leu Pro Gln Ala Leu Ser Asp Val Thr Ser
   1               5                   10                  15
   gcc ttg gtc acg ctg gga aag gat gtc tcc agc cct tca gct ttt caa      96
   Ala Leu Val Thr Leu Gly Lys Asp Val Ser Ser Pro Ser Ala Phe Gln
               20                  25                  30
   gct gtc act ggc ttc tgc agg gag cag tgg ggg att ccg aca gta ttc      144
   Ala Val Thr Gly Phe Cys Arg Glu Gln Trp Gly Ile Pro Thr Val Phe
           35                  40                  45
   tgc ctg ggc tac ttg gcc atg gtc tac gcg gcc aga aga ccc ctc ccg      192
   Cys Leu Gly Tyr Leu Ala Met Val Tyr Ala Ala Arg Arg Pro Leu Pro
       50                  55                  60
   cag cac ggc tac atg gtt gcg gtg gac cgt tgc ttc gct gct tgg aac     240
   Gln His Gly Tyr Met Val Ala Val Asp Arg Cys Phe Ala Ala Trp Asn
   65                  70                  75                  80
   ttg gct ctc tct gtc ttc agc act tgg ggc ttc tac cac atg gct gtc     288
   Leu Ala Leu Ser Val Phe Ser Thr Trp Gly Phe Tyr His Met Ala Val
                   85                  90                  95
   ggg ctc tac aac atg aca gag acg agg ggc ttg caa ttc acc atc tgc     336
   Gly Leu Tyr Asn Met Thr Glu Thr Arg Gly Leu Gln Phe Thr Ile Cys
               100                 105                 110
   ggt tcg act ggg gag ctc gtg cag aac ctt cag act ggc cca acc gct     384
   Gly Ser Thr Gly Glu Leu Val Gln Asn Leu Gln Thr Gly Pro Thr Ala
           115                 120                 125
   ctg gcg ctc tgc ctc ttc tgc ttc agc aag atc ccc gag ttg atg gac     432
   Leu Ala Leu Cys Leu Phe Cys Phe Ser Lys Ile Pro Glu Leu Met Asp
       130                 135                 140
   acg gtg ttt ctc atc ctg aag gcc aag aag gtc cgc ttc ttg cag tgg     480
   Thr Val Phe Leu Ile Leu Lys Ala Lys Lys Val Arg Phe Leu Gln Trp
   145                 150                 155                 160
   tac cac cat gcc aca gtc atg ctc ttc tgt tgg ctc gcc ctc gcg acg     528
   Tyr His His Ala Thr Val Met Leu Phe Cys Trp Leu Ala Leu Ala Thr
                   165                 170                 175
   gag tac act cct ggc ttg tgg ttt gcg gcg acg aac tac ttc gtg cac     576
   Glu Tyr Thr Pro Gly Leu Trp Phe Ala Ala Thr Asn Tyr Phe Val His
               180                 185                 190
   tcc atc atg tac atg tac ttc ttc ctc atg acc ttc aag tcg gcc gcg     624
   Ser Ile Met Tyr Met Tyr Phe Phe Leu Met Thr Phe Lys Ser Ala Ala
           195                 200                 205
   aag gtg gtg aag ccc atc gcc cct ctc atc aca gtt atc cag att gct     672
   Lys Val Val Lys Pro Ile Ala Pro Leu Ile Thr Val Ile Gln Ile Ala
       210                 215                 220
   cag atg gtc tgg ggc ctc atc gtc aac ggc atc gcc atc acc acc ttc     720
   Gln Met Val Trp Gly Leu Ile Val Asn Gly Ile Ala Ile Thr Thr Phe
   225                 230                 235                 240
   ttc acg act ggt gcc tgc cag atc cag tct gtg act gtg tat tcg gcc     768
   Phe Thr Thr Gly Ala Cys Gln Ile Gln Ser Val Thr Val Tyr Ser Ala
                   245                 250                 255
   atc atc atg tac gct tcg tac ttc tac ctg ttc tcc cag ctc ttc ttc     816
   Ile Ile Met Tyr Ala Ser Tyr Phe Tyr Leu Phe Ser Gln Leu Phe Phe
               260                 265                 270
   gag gcc cat ggt gcc gct ggc aag aac aag aag aag ttg acc cgc gag     864
   Glu Ala His Gly Ala Ala Gly Lys Asn Lys Lys Lys Leu Thr Arg Glu
           275                 280                 285
   ctc tct cga aaa atc tcg gag gct ctc ctg aac acc ggt gac gag gtt     912
   Leu Ser Arg Lys Ile Ser Glu Ala Leu Leu Asn Thr Gly Asp Glu Val
       290                 295                 300
   tcc aag cac ctg aag gtg aat tga                                     936
   Ser Lys His Leu Lys Val Asn
   305                 310
<210> 91
   <211> 311
   <212> PRT
   <213> Crypthecodimum cohnii
<400> 91
   Met Ser Ala Phe Met Thr Leu Pro Gln Ala Leu Ser Asp Val Thr Ser
   1               5                   10                  15
   Ala Leu Val Thr Leu Gly Lys Asp Val Ser Ser Pro Ser Ala Phe Gln
               20                  25                  30
   Ala Val Thr Gly Phe Cys Arg Glu Gln Trp Gly Ile Pro Thr Val Phe
           35                  40                  45
   Cys Leu Gly Tyr Leu Ala Met Val Tyr Ala Ala Arg Arg Pro Leu Pro
       50                  55                  60
   Gln His Gly Tyr Met Val Ala Val Asp Arg Cys Phe Ala Ala Trp Asn
   65                  70                  75                  80
   Leu Ala Leu Ser Val Phe Ser Thr Trp Gly Phe Tyr His Met Ala Val
                   85                  90                  95
   Gly Leu Tyr Asn Met Thr Glu Thr Arg Gly Leu Gln Phe Thr Ile Cys
               100                 105                 110
   Gly Ser Thr Gly Glu Leu Val Gln Asn Leu Gln Thr Gly Pro Thr Ala
           115                 120                 125
   Leu Ala Leu Cys Leu Phe Cys Phe Ser Lys Ile Pro Glu Leu Met Asp
       130                 135                 140
   Thr Val Phe Leu Ile Leu Lys Ala Lys Lys Val Arg Phe Leu Gln Trp
   145                 150                 155                 160
   Tyr His His Ala Thr Val Met Leu Phe Cys Trp Leu Ala Leu Ala Thr
                   165                 170                 175
   Glu Tyr Thr Pro Gly Leu Trp Phe Ala Ala Thr Asn Tyr Phe Val His
               180                 185                 190
   Ser Ile Met Tyr Met Tyr Phe Phe Leu Met Thr Phe Lys Ser Ala Ala
           195                 200                 205
   Lys Val Val Lys Pro Ile Ala Pro Leu Ile Thr Val Ile Gln Ile Ala
       210                 215                 220
   Gln Met Val Trp Gly Leu Ile Val Asn Gly Ile Ala Ile Thr Thr Phe
   225                 230                 235                 240
   Phe Thr Thr Gly Ala Cys Gln Ile Gln Ser Val Thr Val Tyr Ser Ala
                   245                 250                 255
   Ile Ile Met Tyr Ala Ser Tyr Phe Tyr Leu Phe Ser Gln Leu Phe Phe
               260                 265                 270
   Glu Ala His Gly Ala Ala Gly Lys Asn Lys Lys Lys Leu Thr Arg Glu
           275                 280                 285
   Leu Ser Arg Lys Ile Ser Glu Ala Leu Leu Asn Thr Gly Asp Glu Val
       290                 295                 300
   Ser Lys His Leu Lys Val Asn
   305                 310
<210> 92
   <211> 927
   <212> DNA
   <213> Crypthecodimum cohnii
<220>
   <221> CDS
   <222> (1)..(927)
   <223> delta-5-Elongase
<400> 92
   atg gct tcc tac caa caa gca ttc tcc gaa ttg gct aga gct ttg tcc      48
   Met Ala Ser Tyr Gln Gln Ala Phe Ser Glu Leu Ala Arg Ala Leu Ser
   1               5                   10                  15
   act ttg aac cac gac ttc tcc agc gtc gag cca ttc aaa gtc gtg acg      96
   Thr Leu Asn His Asp Phe Ser Ser Val Glu Pro Phe Lys Val Val Thr
               20                  25                  30
   cag ttc tgc agg gac cag tgg gcg atc ccg aca gtc ttt tgc atc ggt      144
   Gln Phe Cys Arg Asp Gln Trp Ala Ile Pro Thr Val Phe Cys Ile Gly
           35                  40                  45
   tac ttg gca atg gtc tac gcc acg cga aga cct atc gcg aag cac ccc      192
   Tyr Leu Ala Met Val Tyr Ala Thr Arg Arg Pro Ile Ala Lys His Pro
       50                  55                  60
   tac atg tct ctc gtg gat cgc tgc ttt gcg gcc tgg aac ttg ggc ctc      240
   Tyr Met Ser Leu Val Asp Arg Cys Phe Ala Ala Trp Asn Leu Gly Leu
   65                  70                  75                  80
   tcg ctc ttc agt tgc tgg ggc ttc tac cac atg gca gtg gga ctc tcc      288
   Ser Leu Phe Ser Cys Trp Gly Phe Tyr His Met Ala Val Gly Leu Ser
                   85                  90                  95
   cac acc act tgg aat ttc ggg ctc cag ttc acc atc tgc ggc agc acc      336
   His Thr Thr Trp Asn Phe Gly Leu Gln Phe Thr Ile Cys Gly Ser Thr
               100                 105                 110
   acg gag ctt gtg aat ggc ttc cag aag ggc ccg gcg gcc ctc gcc ctc      384
   Thr Glu Leu Val Asn Gly Phe Gln Lys Gly Pro Ala Ala Leu Ala Leu
           115                 120                 125
   atc ctg ttc tgc ttc tcc aag atc ccg gag ttg ggc gac acc gtc ttc      432
   Ile Leu Phe Cys Phe Ser Lys Ile Pro Glu Leu Gly Asp Thr Val Phe
       130                 135                 140
   ttg atc ttg aag gga aag aag gtc cgc ttc ttg cag tgg tac cac cac      480
   Leu Ile Leu Lys Gly Lys Lys Val Arg Phe Leu Gln Trp Tyr His His
   145                 150                 155                 160
   acg acc gtg atg ctc ttc tgt tgg atg gcc ttg gcg act gag tac act      528
   Thr Thr Val Met Leu Phe Cys Trp Met Ala Leu Ala Thr Glu Tyr Thr
                   165                 170                 175
   cct gga ttg tgg ttc gcg gcc acg aac tac ttc gtg cac tcc atc atg      576
   Pro Gly Leu Trp Phe Ala Ala Thr Asn Tyr Phe Val His Ser Ile Met
               180                 185                 190
   tac atg tac ttc ttc ctc atg acc ttc aag acg gcc gcc ggc atc atc      624
   Tyr Met Tyr Phe Phe Leu Met Thr Phe Lys Thr Ala Ala Gly Ile Ile
           195                 200                 205
   aag ccc atc gcg cct ctc atc acc atc atc cag atc tcc cag atg gtc      672
   Lys Pro Ile Ala Pro Leu Ile Thr Ile Ile Gln Ile Ser Gln Met Val
       210                 215                 220
   tgg ggc ttg gtc gtg aac gcc atc gcc gtc ggc acc ttc ttc acc aca      720
   Trp Gly Leu Val Val Asn Ala Ile Ala Val Gly Thr Phe Phe Thr Thr
   225                 230                 235                 240
   ggc aac tgc cag atc cag gca gtg aca gtc tac tcc gcc atc gtg atg      768
   Gly Asn Cys Gln Ile Gln Ala Val Thr Val Tyr Ser Ala Ile Val Met
                   245                 250                 255
   tac gcc tcc tac ttc tac ctc ttc ggc cag ctc ttc ttc gag gcc cag      816
   Tyr Ala Ser Tyr Phe Tyr Leu Phe Gly Gln Leu Phe Phe Glu Ala Gln
               260                 265                 270
   ggt tcg gct gga aag gac aag aag aag ttg gcc cga gag ctg agc cga      864
   Gly Ser Ala Gly Lys Asp Lys Lys Lys Leu Ala Arg Glu Leu Ser Arg
           275                 280                 285
   aag gtc tcg cgg gct ctc aca gca acg ggc gaa gag gtg tcg aag cac      912
   Lys Val Ser Arg Ala Leu Thr Ala Thr Gly Glu Glu Val Ser Lys His
       290                 295                 300
   atg aag gtg aat tga                                                  927
   Met Lys Val Asn
   305
<210> 93
   <211> 308
   <212> PRT
   <213> Crypthecodimum cohnii
<400> 93
   Met Ala Ser Tyr Gln Gln Ala Phe Ser Glu Leu Ala Arg Ala Leu Ser
   1               5                   10                  15
   Thr Leu Asn His Asp Phe Ser Ser Val Glu Pro Phe Lys Val Val Thr
               20                  25                  30
   Gln Phe Cys Arg Asp Gln Trp Ala Ile Pro Thr Val Phe Cys Ile Gly
           35                  40                  45
   Tyr Leu Ala Met Val Tyr Ala Thr Arg Arg Pro Ile Ala Lys His Pro
       50                  55                  60
   Tyr Met Ser Leu Val Asp Arg Cys Phe Ala Ala Trp Asn Leu Gly Leu
   65                  70                  75                  80
   Ser Leu Phe Ser Cys Trp Gly Phe Tyr His Met Ala Val Gly Leu Ser
   85 90 95
   His Thr Thr Trp Asn Phe Gly Leu Gln Phe Thr Ile Cys Gly Ser Thr
   100 105 110
   Thr Glu Leu Val Asn Gly Phe Gln Lys Gly Pro Ala Ala Leu Ala Leu
   115 120 125
   Ile Leu Phe Cys Phe Ser Lys Ile Pro Glu Leu Gly Asp Thr Val Phe
   130 135 140
   Leu Ile Leu Lys Gly Lys Lys Val Arg Phe Leu Gln Trp Tyr His His
   145 150 155 160
   Thr Thr Val Met Leu Phe Cys Trp Met Ala Leu Ala Thr Glu Tyr Thr
   165 170 175
   Pro Gly Leu Trp Phe Ala Ala Thr Asn Tyr Phe Val His Ser Ile Met
   180 185 190
   Tyr Met Tyr Phe Phe Leu Met Thr Phe Lys Thr Ala Ala Gly Ile Ile
   195 200 205
   Lys Pro Ile Ala Pro Leu Ile Thr Ile Ile Gln Ile Ser Gln Met Val
   210 215 220
   Trp Gly Leu Val Val Asn Ala Ile Ala Val Gly Thr Phe Phe Thr Thr
   225 230 235 240
   Gly Asn Cys Gln Ile Gln Ala Val Thr Val Tyr Ser Ala Ile Val Met
   245 250 255
   Tyr Ala Ser Tyr Phe Tyr Leu Phe Gly Gln Leu Phe Phe Glu Ala Gln
   260 265 270
   Gly Ser Ala Gly Lys Asp Lys Lys Lys Leu Ala Arg Glu Leu Ser Arg
   275 280 285
   Lys Val Ser Arg Ala Leu Thr Ala Thr Gly Glu Glu Val Ser Lys His
   290 295 300
   Met Lys Val Asn
   305
<210> 94
   <211> 795
   <212> DNA
   <213> Oncorhynchus mykiss
<220>
   <221> CDS
   <222> (1)..(795)
   <223> delta-5-Elongase
<400> 94
   atg gct tca aca tgg caa agc gtt cag tcc atg cgc cag tgg att tta      48
   Met Ala Ser Thr Trp Gln Ser Val Gln Ser Met Arg Gln Trp Ile Leu
   1               5                   10                  15
   gag aat gga gat aaa agg aca gac cca tgg cta ctg gtc tac tcc cct      96
   Glu Asn Gly Asp Lys Arg Thr Asp Pro Trp Leu Leu Val Tyr Ser Pro
               20                  25                  30
   atg cca gtg gcc att ata ttc ctc ctc tat ctt ggt gtg gtc tgg gct     144
   Met Pro Val Ala Ile Ile Phe Leu Leu Tyr Leu Gly Val Val Trp Ala
           35                  40                  45
   ggg ccc aag ctg atg aaa cgc agg gaa cca gtt gat ctc aag gct gta     192
   Gly Pro Lys Leu Met Lys Arg Arg Glu Pro Val Asp Leu Lys Ala Val
       50                  55                  60
   ctc att gtc tac aac ttc gcc atg gtc tgc ctg tct gtc tac atg ttc     240
   Leu Ile Val Tyr Asn Phe Ala Met Val Cys Leu Ser Val Tyr Met Phe
   65                  70                  75                  80
   cat gag ttc ttg gtc acg tcc ttg ctg tct aac tac agt tac ctg tgt     288
   His Glu Phe Leu Val Thr Ser Leu Leu Ser Asn Tyr Ser Tyr Leu Cys
                   85                  90                  95
   caa cct gtg gat tac agc act agt cca ctg gcg atg agg atg gcc aaa     336
   Gln Pro Val Asp Tyr Ser Thr Ser Pro Leu Ala Met Arg Met Ala Lys
               100                 105                 110
   gta tgc tgg tgg ttt ttc ttc tcc aag gtc ata gaa ttg gct gac acg     384
   Val Cys Trp Trp Phe Phe Phe Ser Lys Val Ile Glu Leu Ala Asp Thr
           115                 120                 125
   gtg ttc ttc atc ctg agg aag aag aac agt cag ctg act ttc ctg cat     432
   Val Phe Phe Ile Leu Arg Lys Lys Asn Ser Gln Leu Thr Phe Leu His
       130                 135                 140
   gtc tat cac cat ggc acc atg atc ttc aac tgg tgg gca ggg gtc aag     480
   Val Tyr His His Gly Thr Met Ile Phe Asn Trp Trp Ala Gly Val Lys
   145                 150                 155                 160
   tat ctg gct gga ggc caa tcg ttc ttc atc ggc ctg ctc aat acc ttt     528
   Tyr Leu Ala Gly Gly Gln Ser Phe Phe Ile Gly Leu Leu Asn Thr Phe
                   165                 170                 175
   gtg cac atc gtg atg tac tct tac tac gga ctg gct gcc ctg ggg cct     576
   Val His Ile Val Met Tyr Ser Tyr Tyr Gly Leu Ala Ala Leu Gly Pro
               180                 185                 190
   cac acg cag aag tac tta tgg tgg aag cgc tat ctg acc tca ctg cag     624
   His Thr Gln Lys Tyr Leu Trp Trp Lys Arg Tyr Leu Thr Ser Leu Gln
           195                 200                 205
   ctg ctc cag ttt gtc ctg ttg acc act cac act ggc tac aac ctc ttc     672
   Leu Leu Gln Phe Val Leu Leu Thr Thr His Thr Gly Tyr Asn Leu Phe
       210                 215                 220
   act gag tgt gac ttc ccg gac tcc atg aac gct gtg gtg ttt gcc tac     720
   Thr Glu Cys Asp Phe Pro Asp Ser Met Asn Ala Val Val Phe Ala Tyr
   225                 230                 235                 240
   tgt gtc agt ctc att gct ctc ttc agc aac ttc tac tat cag agc tac     768
   Cys Val Ser Leu Ile Ala Leu Phe Ser Asn Phe Tyr Tyr Gln Ser Tyr
   245 250 255
   ctc aac agg aag agc aag aag aca taa   795
   Leu Asn Arg Lys Ser Lys Lys Thr
   260
<210> 95
   <211> 264
   <212> PRT
   <213> Oncorhynchus mykiss
<400> 95
   Met Ala Ser Thr Trp Gln Ser Val Gln Ser Met Arg Gln Trp Ile Leu
   1               5                   10                  15
   Glu Asn Gly Asp Lys Arg Thr Asp Pro Trp Leu Leu Val Tyr Ser Pro
               20                  25                  30
   Met Pro Val Ala Ile Ile Phe Leu Leu Tyr Leu Gly Val Val Trp Ala
           35                  40                  45
   Gly Pro Lys Leu Met Lys Arg Arg Glu Pro Val Asp Leu Lys Ala Val
       50                  55                  60
   Leu Ile Val Tyr Asn Phe Ala Met Val Cys Leu Ser Val Tyr Met Phe
   65                  70                  75                  80
   His Glu Phe Leu Val Thr Ser Leu Leu Ser Asn Tyr Ser Tyr Leu Cys
                   85                  90                  95
   Gln Pro Val Asp Tyr Ser Thr Ser Pro Leu Ala Met Arg Met Ala Lys
               100                 105                 110
   Val Cys Trp Trp Phe Phe Phe Ser Lys Val Ile Glu Leu Ala Asp Thr
           115                 120                 125
   Val Phe Phe Ile Leu Arg Lys Lys Asn Ser Gln Leu Thr Phe Leu His
       130                 135                 140
   Val Tyr His His Gly Thr Met Ile Phe Asn Trp Trp Ala Gly Val Lys
   145                 150                 155                 160
   Tyr Leu Ala Gly Gly Gln Ser Phe Phe Ile Gly Leu Leu Asn Thr Phe
                   165                 170                 175
   Val His Ile Val Met Tyr Ser Tyr Tyr Gly Leu Ala Ala Leu Gly Pro
               180                 185                 190
   His Thr Gln Lys Tyr Leu Trp Trp Lys Arg Tyr Leu Thr Ser Leu Gln
           195                 200                 205
   Leu Leu Gln Phe Val Leu Leu Thr Thr His Thr Gly Tyr Asn Leu Phe
       210                 215                 220
   Thr Glu Cys Asp Phe Pro Asp Ser Met Asn Ala Val Val Phe Ala Tyr
   225                 230                 235                 240
   Cys Val Ser Leu Ile Ala Leu Phe Ser Asn Phe Tyr Tyr Gln Ser Tyr
                   245                 250                 255
   Leu Asn Arg Lys Ser Lys Lys Thr
               260
<210> 96
   <211> 885
   <212> DNA
   <213> Oncorhynchus mykiss
<220>
   <221> CDS
   <222> (1)..(885)
   <223> delta-5-Elongase
<400> 96
   atg gag act ttt aat tat aaa cta aac atg tac ata gac tca tgg atg      48
   Met Glu Thr Phe Asn Tyr Lys Leu Asn Met Tyr Ile Asp Ser Trp Met
   1               5                   10                  15
   ggt ccc aga gat gag cgg gta cag gga tgg ctg ctt ctg gac aac tac      96
   Gly Pro Arg Asp Glu Arg Val Gln Gly Trp Leu Leu Leu Asp Asn Tyr
               20                  25                  30
   cct cca acc ttt gca cta aca gtc atg tac ctg ctg atc gta tgg atg     144
   Pro Pro Thr Phe Ala Leu Thr Val Met Tyr Leu Leu Ile Val Trp Met
           35                  40                  45
   ggg ccc aag tac atg aga cac aga cag ccg gtg tct tgc cgg ggt ctc     192
   Gly Pro Lys Tyr Met Arg His Arg Gln Pro Val Ser Cys Arg Gly Leu
       50                  55                  60
   ctc ttg gtc tac aat ctg ggc ctc acg atc ttg tcc ttc tat atg ttc     240
   Leu Leu Val Tyr Asn Leu Gly Leu Thr Ile Leu Ser Phe Tyr Met Phe
   65                  70                  75                  80
   tat gag atg gtg tct gct gtg tgg cac ggg gat tat aac ttc ttt tgc     288
   Tyr Glu Met Val Ser Ala Val Trp His Gly Asp Tyr Asn Phe Phe Cys
                   85                  90                  95
   caa gac aca cac agt gca gga gaa acc gat acc aag atc ata aat gtg     336
   Gln Asp Thr His Ser Ala Gly Glu Thr Asp Thr Lys Ile Ile Asn Val
               100                 105                 110
   ctg tgg tgg tac tac ttc tcc aag ctc ata gag ttt atg gat acc ttc     384
   Leu Trp Trp Tyr Tyr Phe Ser Lys Leu Ile Glu Phe Met Asp Thr Phe
           115                 120                 125
   ttc ttc atc ctg cgg aag aac aac cat caa atc acg ttt ctg cac atc     432
   Phe Phe Ile Leu Arg Lys Asn Asn His Gln Ile Thr Phe Leu His Ile
       130                 135                 140
   tac cac cat gct agc atg ctc aac atc tgg tgg ttc gtc atg aac tgg     480
   Tyr His His Ala Ser Met Leu Asn Ile Trp Trp Phe Val Met Asn Trp
   145                 150                 155                 160
   gtg ccc tgt ggt cac tcc tac ttt ggt gcc tcc ctg aac agc ttc atc     528
   Val Pro Cys Gly His Ser Tyr Phe Gly Ala Ser Leu Asn Ser Phe Ile
                       165                     170                     175
   cat gtc ctg atg tac tct tac tat ggg ctc tct gct gtc ccg gcc ttg        576
   His Val Leu Met Tyr Ser Tyr Tyr Gly Leu Ser Ala Val Pro Ala Leu
               180                 185                     190
   cgg ccc tat cta tgg tgg aag aaa tac atc aca caa gta cag ctg att        624
   Arg Pro Tyr Leu Trp Trp Lys Lys Tyr Ile Thr Gln Val Gln Leu Ile
           195                 200                 205
   cag ttc ttt ttg acc atg tcc cag acg ata tgt gca gtc att tgg cca        672
   Gln Phe Phe Leu Thr Met Ser Gln Thr Ile Cys Ala Val Ile Trp Pro
       210                 215                 220
   tgt gat ttc ccc aga ggg tgg ctg tat ttc cag ata ttc tat gtc atc        720
   Cys Asp Phe Pro Arg Gly Trp Leu Tyr Phe Gln Ile Phe Tyr Val Ile
   225                 230                 235                     240
   aca ctt att gcc ctt ttc tca aac ttc tac att cag act tac aag aaa        768
   Thr Leu Ile Ala Leu Phe Ser Asn Phe Tyr Ile Gln Thr Tyr Lys Lys
                   245                 250                     255
   cac ctt gtt tca caa aag aag gag tat cat cag aat ggc tct gtt gct        816
   His Leu Val Ser Gln Lys Lys Glu Tyr His Gln Asn Gly Ser Val Ala
               260                 265                 270
   tca ttg aat ggc cat gtg aat ggg gtg aca ccc acg gaa acc att aca        864
   Ser Leu Asn Gly His Val Asn Gly Val Thr Pro Thr Glu Thr Ile Thr
           275                 280                 285
   cac agg aaa gtg agg ggg gac                                            885
   His Arg Lys Val Arg Gly Asp
       290                 295
<210> 97
   <211> 295
   <212> PRT
   <213> Oncorhynchus mykiss
<400> 97
   Met Glu Thr Phe Asn Tyr Lys Leu Asn Met Tyr Ile Asp Ser Trp Met
   1               5                   10                  15
   Gly Pro Arg Asp Glu Arg Val Gln Gly Trp Leu Leu Leu Asp Asn Tyr
               20                  25                  30
   Pro Pro Thr Phe Ala Leu Thr Val Met Tyr Leu Leu Ile Val Trp Met
           35                  40                  45
   Gly Pro Lys Tyr Met Arg His Arg Gln Pro Val Ser Cys Arg Gly Leu
       50                  55                  60
   Leu Leu Val Tyr Asn Leu Gly Leu Thr Ile Leu Ser Phe Tyr Met Phe
   65                  70                  75                  80
   Tyr Glu Met Val Ser Ala Val Trp His Gly Asp Tyr Asn Phe Phe Cys
                   85                  90                  95
   Gln Asp Thr His Ser Ala Gly Glu Thr Asp Thr Lys Ile Ile Asn Val
               100                 105                 110
   Leu Trp Trp Tyr Tyr Phe Ser Lys Leu Ile Glu Phe Met Asp Thr Phe
           115                 120                 125
   Phe Phe Ile Leu Arg Lys Asn Asn His Gln Ile Thr Phe Leu His Ile
       130                 135                 140
   Tyr His His Ala Ser Met Leu Asn Ile Trp Trp Phe Val Met Asn Trp
   145                 150                 155                 160
   Val Pro Cys Gly His Ser Tyr Phe Gly Ala Ser Leu Asn Ser Phe Ile
                   165                 170                 175
   His Val Leu Met Tyr Ser Tyr Tyr Gly Leu Ser Ala Val Pro Ala Leu
               180                 185                 190
   Arg Pro Tyr Leu Trp Trp Lys Lys Tyr Ile Thr Gln Val Gln Leu Ile
           195                 200                 205
   Gln Phe Phe Leu Thr Met Ser Gln Thr Ile Cys Ala Val Ile Trp Pro
       210                 215                 220
   Cys Asp Phe Pro Arg Gly Trp Leu Tyr Phe Gln Ile Phe Tyr Val Ile
   225                 230                 235                 240
   Thr Leu Ile Ala Leu Phe Ser Asn Phe Tyr Ile Gln Thr Tyr Lys Lys
                   245                 250                 255
   His Leu Val Ser Gln Lys Lys Glu Tyr His Gln Asn Gly Ser Val Ala
               260                 265                 270
   Ser Leu Asn Gly His Val Asn Gly Val Thr Pro Thr Glu Thr Ile Thr
           275                 280                 285
   His Arg Lys Val Arg Gly Asp
       290                 295
<210> 98
   <211> 6753
   <212> DNA
   <213> Oncorhynchus mykiss
<220>
   <221> CDS
   <222> (513)..(1397)
   <223> delta-5-Elongase
<400> 98
   acggattaga agccgccgag cgggtgacag ccctccgaag gaagactctc ctccgtgcgt 60
   cctcgtcctc accggtcgcg ttcctgaaac gcagatgtgc ctcgcgccgc actgctccga 120
   acaataaaga ttctacaata ctagctttta tggttatgaa gaggaaaaat tggcagtaac 180
   ctggccccac aaaccttcaa atgaacgaat caaattaaca accataggat gataatgcga     240
   ttagtttttt agccttattt ctggggtaat taatcagcga agcgatgatt tttgatctat     300
   taacagatat ataaatgcaa aaactgcatt aaccacttta actaatactt tcaacatttt     360
   cggtttgtat tacttcttat tcaaatgtaa taaaagtatc aacaaaaaat tgttaatata     420
   cctctatact ttaacgtcaa ggagaaaaaa ccccggatcg gactactagc agctgtaata     480
   cgactcacta tagggaatat taagcttaca ta atg gag act ttt aat tat aaa       533
                                       Met Glu Thr Phe Asn Tyr Lys
                                       1               5
   cta aac atg tac ata gac tca tgg atg ggt ccc aga gat gag cgg gta      581
   Leu Asn Met Tyr Ile Asp Ser Trp Met Gly Pro Arg Asp Glu Arg Val
           10                  15                  20
   cag gga tgg ctg ctt ctg gac aac tac cct cca acc ttt gca cta aca      629
   Gln Gly Trp Leu Leu Leu Asp Asn Tyr Pro Pro Thr Phe Ala Leu Thr
       25                  30                  35
   gtc atg tac ctg ctg atc gta tgg atg ggg ccc aag tac atg aga cac      677
   Val Met Tyr Leu Leu Ile Val Trp Met Gly Pro Lys Tyr Met Arg His
   40                  45                  50                  55
   aga cag ccg gtg tct tgc cgg ggt ctc ctc ttg gtc tac aat ctg ggc      725
   Arg Gln Pro Val Ser Cys Arg Gly Leu Leu Leu Val Tyr Asn Leu Gly
                   60                  65                  70
   ctc acg atc ttg tcc ttc tat atg ttc tat gag atg gtg tct gct gtg      773
   Leu Thr Ile Leu Ser Phe Tyr Met Phe Tyr Glu Met Val Ser Ala Val
               75                  80                  85
   tgg cac ggg gat tat aac ttc ttt tgc caa gac aca cac agt gca gga      821
   Trp His Gly Asp Tyr Asn Phe Phe Cys Gln Asp Thr His Ser Ala Gly
           90                  95                  100
   gaa acc gat acc aag atc ata aat gtg ctg tgg tgg tac tac ttc tcc      869
   Glu Thr Asp Thr Lys Ile Ile Asn Val Leu Trp Trp Tyr Tyr Phe Ser
       105                 110                 115
   aag ctc ata gag ttt atg gat acc ttc ttc ttc atc ctg cgg aag aac      917
   Lys Leu Ile Glu Phe Met Asp Thr Phe Phe Phe Ile Leu Arg Lys Asn
   120                 125                 130                 135
   aac cat caa atc acg ttt ctg cac atc tac cac cat gct agc atg ctc      965
   Asn His Gln Ile Thr Phe Leu His Ile Tyr His His Ala Ser Met Leu
                   140                 145                 150
   aac atc tgg tgg ttc gtc atg aac tgg gtg ccc tgt ggt cac tcc tac     1013
   Asn Ile Trp Trp Phe Val Met Asn Trp Val Pro Cys Gly His Ser Tyr
               155                 160                 165
   ttt ggt gcc tcc ctg aac agc ttc atc cat gtc ctg atg tac tct tac     1061
   Phe Gly Ala Ser Leu Asn Ser Phe Ile His Val Leu Met Tyr Ser Tyr
           170                 175                 180
   tat ggg ctc tct gct gtc ccg gcc ttg cgg ccc tat cta tgg tgg aag     1109
   Tyr Gly Leu Ser Ala Val Pro Ala Leu Arg Pro Tyr Leu Trp Trp Lys
       185                 190                 195
   aaa tac atc aca caa gta cag ctg att cag ttc ttt ttg acc atg tcc     1157
   Lys Tyr Ile Thr Gln Val Gln Leu Ile Gln Phe Phe Leu Thr Met Ser
   200                 205                 210                 215
   cag acg ata tgt gca gtc att tgg cca tgt gat ttc ccc aga ggg tgg     1205
   Gln Thr Ile Cys Ala Val Ile Trp Pro Cys Asp Phe Pro Arg Gly Trp
                   220                 225                 230
   ctg tat ttc cag ata ttc tat gtc atc aca ctt att gcc ctt ttc tca    1253
   Leu Tyr Phe Gln Ile Phe Tyr Val Ile Thr Leu Ile Ala Leu Phe Ser
               235                 240                 245
   aac ttc tac att cag act tac aag aaa cac ctt gtt tca caa aag aag    1301
   Asn Phe Tyr Ile Gln Thr Tyr Lys Lys His Leu Val Ser Gln Lys Lys
           250                 255                 260
   gag tat cat cag aat ggc tct gtt gct tca ttg aat ggc cat gtg aat    1349
   Glu Tyr His Gln Asn Gly Ser Val Ala Ser Leu Asn Gly His Val Asn
       265                 270                 275
   ggg gtg aca ccc acg gaa acc att aca cac agg aaa gtg agg ggg gac    1397
   Gly Val Thr Pro Thr Glu Thr Ile Thr His Arg Lys Val Arg Gly Asp
   280                 285                 290                 295
   tgaaggatcc actagtaacg gccgccagtg tgctggaatt ctgcagatat ccagcacagt   1457
   ggcggccgct cgagtctaga gggcccttcg aaggtaagcc tatccctaac cctctcctcg   1517
   gtctcgattc tacgcgtacc ggtcatcatc accatcacca ttgagtttaa acccgctgat   1577
   cctagagggc cgcatcatgt aattagttat gtcacgctta cattcacgcc ctccccccac   1637
   atccgctcta accgaaaagg aaggagttag acaacctgaa gtctaggtcc ctatttattt   1697
   ttttatagtt atgttagtat taagaacgtt atttatattt caaatttttc ttttttttct   1757
   gtacagacgc gtgtacgcat gtaacattat actgaaaacc ttgcttgaga aggttttggg   1817
   acgctcgaag gctttaattt gcaagctgcg gccctgcatt aatgaatcgg ccaacgcgcg   1877
   gggagaggcg gtttgcgtat tgggcgctct tccgcttcct cgctcactga ctcgctgcgc   1937
   tcggtcgttc ggctgcggcg agcggtatca gctcactcaa aggcggtaat acggttatcc   1997
   acagaatcag gggataacgc aggaaagaac atgtgagcaa aaggccagca aaagcccagg   2057
   aaccgtaaaa aggccgcgtt gctggcgttt ttccataggc tccgcccccc tgacgagcat   2117
   cacaaaaatc gacgctcaag tcagaggtgg cgaaacccga caggactata aagataccag   2177
   gcgtttcccc ctggaagctc cctcgtgcgc tctcctgttc cgaccctgcc gcttaccgga   2237
   tacctgtccg cctttctccc ttcgggaagc gtggcgcttt ctcatagctc acgctgtagg   2297
   tatctcagtt cggtgtaggt cgttcgctcc aagctgggct gtgtgcacga accccccgtt   2357
   cagcccgacc gctgcgcctt atccggtaac tatcgtcttg agtccaaccc ggtaagacac   2417
   gacttatcgc cactggcagc agccactggt aacaggatta gcagagcgag gtatgtaggc   2477
   ggtgctacag agttcttgaa gtggtggcct aactacggct acactagaag gacagtattt   2537
   ggtatctgcg ctctgctgaa gccagttacc ttcggaaaaa gagttggtag ctcttgatcc   2597
   ggcaaacaaa ccaccgctgg tagcggtggt ttttttgttt gcaagcagca gattacgcgc   2657
   agaaaaaaag gatctcaaga agatcctttg atcttttcta cggggtctga cgctcagtgg   2717
   aacgaaaact cacgttaagg gattttggtc atgagattat caaaaaggat cttcacctag   2777
   atccttttaa attaaaaatg aagttttaaa tcaatctaaa gtatatatga gtaaacttgg   2837
   tctgacagtt accaatgctt aatcagtgag gcacctatct cagcgatctg tctatttcgt   2897
   tcatccatag ttgcctgact ccccgtcgtg tagataacta cgatacggga gcgcttacca 2957
   tctggcccca gtgctgcaat gataccgcga gacccacgct caccggctcc agatttatca 3017
   gcaataaacc agccagccgg aagggccgag cgcagaagtg gtcctgcaac tttatccgcc 3077
   tccatccagt ctattaattg ttgccgggaa gctagagtaa gtagttcgcc agttaatagt 3137
   ttgcgcaacg ttgttgccat tgctacaggc atcgtggtgt cacgctcgtc gtttggtatg 3197
   gcttcattca gctccggttc ccaacgatca aggcgagtta catgatcccc catgttgtgc 3257
   aaaaaagcgg ttagctcctt cggtcctccg atcgttgtca gaagtaagtt ggccgcagtg 3317
   ttatcactca tggttatggc agcactgcat aattctctta ctgtcatgcc atccgtaaga 3377
   tgcttttctg tgactggtga gtactcaacc aagtcattct gagaatagtg tatgcggcga 3437
   ccgagttgct cttgcccggc gtcaacacgg gataataccg cgccacatag cagaacttta 3497
   aaagtgctca tcattggaaa acgttcttcg gggcgaaaac tctcaaggat cttaccgctg 3557
   ttgagatcca gttcgatgta acccactcgt gcacccaact gatcttcagc atcttttact 3617
   ttcaccagcg tttctgggtg agcaaaaaca ggaaggcaaa atgccgcaaa aaagggaata 3677
   agggcgacac ggaaatgttg aatactcata ctcttccttt ttcaatatta ttgaagcatt 3737
   tatcagggtt attgtctcat gagcggatac atatttgaat gtatttagaa aaataaacaa 3797
   ataggggttc cgcgcacatt tccccgaaaa gtgccacctg acgtctaaga aaccattatt 3857
   atcatgacat taacctataa aaataggcgt atcacgaggc cctttcgtct tcaagaaatt 3917
   cggtcgaaaa aagaaaagga gagggccaag agggagggca ttggtgacta ttgagcacgt 3977
   gagtatacgt gattaagcac acaaaggcag cttggagtat gtctgttatt aatttcacag 4037
   gtagttctgg tccattggtg aaagtttgcg gcttgcagag cacagaggcc gcagaatgtg 4097
   ctctagattc cgatgctgac ttgctgggta ttatatgtgt gcccaataga aagagaacaa 4157
   ttgacccggt tattgcaagg aaaatttcaa gtcttgtaaa agcatataaa aatagttcag 4217
   gcactccgaa atacttggtt ggcgtgtttc gtaatcaacc taaggaggat gttttggctc 4277
   tggtcaatga ttacggcatt gatatcgtcc aactgcacgg agatgagtcg tggcaagaat 4337
   accaagagtt cctcggtttg ccagttatta aaagactcgt atttccaaaa gactgcaaca 4397
   tactactcag tgcagcttca cagaaacctc attcgtttat tcccttgttt gattcagaag 4457
   caggtgggac aggtgaactt ttggattgga actcgatttc tgactgggtt ggaaggcaag 4517
   agagccccga gagcttacat tttatgttag ctggtggact gacgccagaa aatgttggtg 4577
   atgcgcttag attaaatggc gttattggtg ttgatgtaag cggaggtgtg gagacaaatg 4637
   gtgtaaaaga ctctaacaaa atagcaaatt tcgtcaaaaa tgctaagaaa taggttatta 4697
   ctgagtagta tttatttaag tattgtttgt gcacttgccc tagcttatcg atgataagct 4757
   gtcaaagatg agaattaatt ccacggacta tagactatac tagatactcc gtctactgta 4817
   cgatacactt ccgctcaggt ccttgtcctt taacgaggcc ttaccactct tttgttactc 4877
   tattgatcca gctcagcaaa ggcagtgtga tctaagattc tatcttcgcg atgtagtaaa 4937
   actagctaga ccgagaaaga gactagaaat gcaaaaggca cttctacaat ggctgccatc 4997
   attattatcc gatgtgacgc tgcagcttct caatgatatt cgaatacgct ttgaggagat 5057
   acagcctaat atccgacaaa ctgttttaca gatttacgat cgtacttgtt acccatcatt 5117
   gaattttgaa catccgaacc tgggagtttt ccctgaaaca gatagtatat ttgaacctgt 5177
   ataataatat atagtctagc gctttacgga agacaatgta tgtatttcgg ttcctggaga 5237
   aactattgca tctattgcat aggtaatctt gcacgtcgca tccccggttc attttctgcg 5297
   tttccatctt gcacttcaat agcatatctt tgttaacgaa gcatctgtgc ttcattttgt 5357
   agaacaaaaa tgcaacgcga gagcgctaat ttttcaaaca aagaatctga gctgcatttt 5417
   tacagaacag aaatgcaacg cgaaagcgct attttaccaa cgaagaatct gtgcttcatt 5477
   tttgtaaaac aaaaatgcaa cgcgacgaga gcgctaattt ttcaaacaaa gaatctgagc 5537
   tgcattttta cagaacagaa atgcaacgcg agagcgctat tttaccaaca aagaatctat 5597
   acttcttttt tgttctacaa aaatgcatcc cgagagcgct atttttctaa caaagcatct 5657
   tagattactt tttttctcct ttgtgcgctc tataatgcag tctcttgata actttttgca 5717
   ctgtaggtcc gttaaggtta gaagaaggct actttggtgt ctattttctc ttccataaaa 5777
   aaagcctgac tccacttccc gcgtttactg attactagcg aagctgcggg tgcatttttt 5837
   caagataaag gcatccccga ttatattcta taccgatgtg gattgcgcat actttgtgaa 5897
   cagaaagtga tagcgttgat gattcttcat tggtcagaaa attatgaacg gtttcttcta 5957
   ttttgtctct atatactacg tataggaaat gtttacattt tcgtattgtt ttcgattcac 6017
   tctatgaata gttcttacta caattttttt gtctaaagag taatactaga gataaacata 6077
   aaaaatgtag aggtcgagtt tagatgcaag ttcaaggagc gaaaggtgga tgggtaggtt 6137
   atatagggat atagcacaga gatatatagc aaagagatac ttttgagcaa tgtttgtgga 6197
   agcggtattc gcaatgggaa gctccacccc ggttgataat cagaaaagcc ccaaaaacag 6257
   gaagattgta taagcaaata tttaaattgt aaacgttaat attttgttaa aattcgcgtt 6317
   aaatttttgt taaatcagct cattttttaa cgaatagccc gaaatcggca aaatccctta 6377
   taaatcaaaa gaatagaccg agatagggtt gagtgttgtt ccagtttcca acaagagtcc 6437
   actattaaag aacgtggact ccaacgtcaa agggcgaaaa agggtctatc agggcgatgg 6497
   cccactacgt gaaccatcac cctaatcaag ttttttgggg tcgaggtgcc gtaaagcagt 6557
   aaatcggaag ggtaaacgga tgcccccatt tagagcttga cggggaaagc cggcgaacgt 6617
   ggcgagaaag gaagggaaga aagcgaaagg agcgggggct agggcggtgg gaagtgtagg 6677
   ggtcacgctg ggcgtaacca ccacacccgc cgcgcttaat ggggcgctac agggcgcgtg 6737
   gggatgatcc actagt 6753
<210> 99
   <211> 295
   <212> PRT
   <213> Oncorhynchus mykiss
<400> 99
   Met Glu Thr Phe Asn Tyr Lys Leu Asn Met Tyr Ile Asp Ser Trp Met
   1 5 10 15
   Gly Pro Arg Asp Glu Arg Val Gln Gly Trp Leu Leu Leu Asp Asn Tyr
   20 25 30
   Pro Pro Thr Phe Ala Leu Thr Val Met Tyr Leu Leu Ile Val Trp Met
   35 40 45
   Gly Pro Lys Tyr Met Arg His Arg Gln Pro Val Ser Cys Arg Gly Leu
   50 55 60
   Leu Leu Val Tyr Asn Leu Gly Leu Thr Ile Leu Ser Phe Tyr Met Phe
   65 70 75 80
   Tyr Glu Met Val Ser Ala Val Trp His Gly Asp Tyr Asn Phe Phe Cys
   85 90 95
   Gln Asp Thr His Ser Ala Gly Glu Thr Asp Thr Lys Ile Ile Asn Val
   100 105 110
   Leu Trp Trp Tyr Tyr Phe Ser Lys Leu Ile Glu Phe Met Asp Thr Phe
   115 120 125
   Phe Phe Ile Leu Arg Lys Asn Asn His Gln Ile Thr Phe Leu His Ile
   130 135 140
   Tyr His His Ala Ser Met Leu Asn Ile Trp Trp Phe Val Met Asn Trp
   145 150 155 160
   Val Pro Cys Gly His Ser Tyr Phe Gly Ala Ser Leu Asn Ser Phe Ile
   165 170 175
   His Val Leu Met Tyr Ser Tyr Tyr Gly Leu Ser Ala Val Pro Ala Leu
   180 185 190
   Arg Pro Tyr Leu Trp Trp Lys Lys Tyr Ile Thr Gln Val Gln Leu Ile
   195 200 205
   Gln Phe Phe Leu Thr Met Ser Gln Thr Ile Cys Ala Val Ile Trp Pro
   210 215 220
   Cys Asp Phe Pro Arg Gly Trp Leu Tyr Phe Gln Ile Phe Tyr Val Ile
   225 230 235 240
   Thr Leu Ile Ala Leu Phe Ser Asn Phe Tyr Ile Gln Thr Tyr Lys Lys
   245 250 255
   His Leu Val Ser Gln Lys Lys Glu Tyr His Gln Asn Gly Ser Val Ala
   260 265 270
   Ser Leu Asn Gly His Val Asn Gly Val Thr Pro Thr Glu Thr Ile Thr
   275 280 285
   His Arg Lys Val Arg Gly Asp
   290 295
<210> 100
   <211> 6645
   <212> DNA
   <213> Oncorhynchus mykiss
<220>
   <221> CDS
   <222> (513)..(1304)
   <223> delta-5-Elongase
<400> 100
   acggattaga agccgccgag cgggtgacag ccctccgaag gaagactctc ctccgtgcgt      60
   cctcgtcctc accggtcgcg ttcctgaaac gcagatgtgc ctcgcgccgc actgctccga     120
   acaataaaga ttctacaata ctagctttta tggttatgaa gaggaaaaat tggcagtaac     180
   ctggccccac aaaccttcaa atgaacgaat caaattaaca accataggat gataatgcga     240
   ttagtttttt agccttattt ctggggtaat taatcagcga agcgatgatt tttgatctat     300
   taacagatat ataaatgcaa aaactgcatt aaccacttta actaatactt tcaacatttt     360
   cggtttgtat tacttcttat tcaaatgtaa taaaagtatc aacaaaaaat tgttaatata     420
   cctctatact ttaacgtcaa ggagaaaaaa ccccggatcg gactactagc agctgtaata     480
   cgactcacta tagggaatat taagcttaca ta atg gct tca aca tgg caa agc       533
                                       Met Ala Ser Thr Trp Gln Ser
                                       1               5
   gtt cag tcc atg cgc cag tgg att tta gag aat gga gat aaa agg aca       581
   Val Gln Ser Met Arg Gln Trp Ile Leu Glu Asn Gly Asp Lys Arg Thr
           10                  15                  20
   gac cca tgg cta ctg gtc tac tcc cct atg cca gtg gcc att ata ttc       629
   Asp Pro Trp Leu Leu Val Tyr Ser Pro Met Pro Val Ala Ile Ile Phe
       25                  30                  35
   ctc ctc tat ctt ggt gtg gtc tgg gct ggg ccc aag ctg atg aaa cgc       677
   Leu Leu Tyr Leu Gly Val Val Trp Ala Gly Pro Lys Leu Met Lys Arg
   40                  45                  50                  55
   agg gaa cca gtt gat ctc aag gct gta ctc att gtc tac aac ttc gcc       725
   Arg Glu Pro Val Asp Leu Lys Ala Val Leu Ile Val Tyr Asn Phe Ala
                   60                  65                  70
   atg gtc tgc ctg tct gtc tac atg ttc cat gag ttc ttg gtc acg tcc       773
   Met Val Cys Leu Ser Val Tyr Met Phe His Glu Phe Leu Val Thr Ser
               75                  80                  85
   ttg ctg tct aac tac agt tac ctg tgt caa cct gtg gat tac agc act       821
   Leu Leu Ser Asn Tyr Ser Tyr Leu Cys Gln Pro Val Asp Tyr Ser Thr
           90                  95                  100
   agt cca ctg gcg atg agg atg gcc aaa gta tgc tgg tgg ttt ttc ttc       869
   Ser Pro Leu Ala Met Arg Met Ala Lys Val Cys Trp Trp Phe Phe Phe
       105                 110                 115
   tcc aag gtc ata gaa ttg gct gac acg gtg ttc ttc atc ctg agg aag       917
   Ser Lys Val Ile Glu Leu Ala Asp Thr Val Phe Phe Ile Leu Arg Lys
   120                 125                 130                 135
   aag aac agt cag ctg act ttc ctg cat gtc tat cac cat ggc acc atg       965
   Lys Asn Ser Gln Leu Thr Phe Leu His Val Tyr His His Gly Thr Met
                   140                 145                 150
   atc ttc aac tgg tgg gca ggg gtc aag tat ctg gct gga ggc caa tcg      1013
   Ile Phe Asn Trp Trp Ala Gly Val Lys Tyr Leu Ala Gly Gly Gln Ser
               155                 160                 165
   ttc ttc atc ggc ctg ctc aat acc ttt gtg cac atc gtg atg tac tct      1061
   Phe Phe Ile Gly Leu Leu Asn Thr Phe Val His Ile Val Met Tyr Ser
           170                 175                 180
   tac tac gga ctg gct gcc ctg ggg cct cac acg cag aag tac tta tgg      1109
   Tyr Tyr Gly Leu Ala Ala Leu Gly Pro His Thr Gln Lys Tyr Leu Trp
       185                 190                 195
   tgg aag cgc tat ctg acc tca ctg cag ctg ctc cag ttt gtc ctg ttg      1157
   Trp Lys Arg Tyr Leu Thr Ser Leu Gln Leu Leu Gln Phe Val Leu Leu
   200                 205                 210                 215
   acc act cac act ggc tac aac ctc ttc act gag tgt gac ttc ccg gac      1205
   Thr Thr His Thr Gly Tyr Asn Leu Phe Thr Glu Cys Asp Phe Pro Asp
                   220                 225                 230
   tcc atg aac gct gtg gtg ttt gcc tac tgt gtc agt ctc att gct ctc      1253
   Ser Met Asn Ala Val Val Phe Ala Tyr Cys Val Ser Leu Ile Ala Leu
               235                 240                 245
   ttc agc aac ttc tac tat cag agc tac ctc aac agg aag agc aag aag      1301
   Phe Ser Asn Phe Tyr Tyr Gln Ser Tyr Leu Asn Arg Lys Ser Lys Lys
           250                 255                 260
   aca taaggatcca ctagtaacgg ccgccagtgt gctggaattc tgcagatatc           1354
   Thr
   catcacactg gcggccgctc gagcatgcat ctagagggcc gcatcatgta attagttatg    1414
   tcacgcttac attcacgccc tccccccaca tccgctctaa ccgaaaagga aggagttaga    1474
   caacctgaag tctaggtccc tatttatttt tttatagtta tgttagtatt aagaacgtta    1534
   tttatatttc aaatttttct tttttttctg tacagacgcg tgtacgcatg taacattata    1594
   ctgaaaacct tgcttgagaa ggttttggga cgctcgaagg ctttaatttg cggccctgca    1654
   ttaatgaatc ggccaacgcg cggggagagg cggtttgcgt attgggcgct cttccgcttc    1714
   ctcgctcact gactcgctgc gctcggtcgt tcggctgcgg cgagcggtat cagctcactc    1774
   aaaggcggta atacggttat ccacagaatc aggggataac gcaggaaaga acatgtgagc    1834
   aaaaggccag caaaagccca ggaaccgtaa aaaggccgcg ttgctggcgt ttttccatag    1894
   gctccgcccc cctgacgagc atcacaaaaa tcgacgctca agtcagaggt ggcgaaaccc    1954
   gacaggacta taaagatacc aggcgtttcc ccctggaagc tccctcgtgc gctctcctgt    2014
   tccgaccctg ccgcttaccg gatacctgtc cgcctttctc ccttcgggaa gcgtggcgct    2074
   ttctcatagc tcacgctgta ggtatctcag ttcggtgtag gtcgttcgct ccaagctggg    2134
   ctgtgtgcac gaaccccccg ttcagcccga ccgctgcgcc ttatccggta actatcgtct 2194
   tgagtccaac ccggtaagac acgacttatc gccactggca gcagccactg gtaacaggat 2254
   tagcagagcg aggtatgtag gcggtgctac agagttcttg aagtggtggc ctaactacgg 2314
   ctacactaga aggacagtat ttggtatctg cgctctgctg aagccagtta ccttcggaaa 2374
   aagagttggt agctcttgat ccggcaaaca aaccaccgct ggtagcggtg gtttttttgt 2434
   ttgcaagcag cagattacgc gcagaaaaaa aggatctcaa gaagatcctt tgatcttttc 2494
   tacggggtct gacgctcagt ggaacgaaaa ctcacgttaa gggattttgg tcatgagatt 2554
   atcaaaaagg atcttcacct agatcctttt aaattaaaaa tgaagtttta aatcaatcta 2614
   aagtatatat gagtaaactt ggtctgacag ttaccaatgc ttaatcagtg aggcacctat 2674
   ctcagcgatc tgtctatttc gttcatccat agttgcctga ctccccgtcg tgtagataac 2734
   tacgatacgg gagcgcttac catctggccc cagtgctgca atgataccgc gagacccacg 2794
   ctcaccggct ccagatttat cagcaataaa ccagccagcc ggaagggccg agcgcagaag 2854
   tggtcctgca actttatccg cctccattca gtctattaat tgttgccggg aagctagagt 2914
   aagtagttcg ccagttaata gtttgcgcaa cgttgttggc attgctacag gcatcgtggt 2974
   gtcactctcg tcgtttggta tggcttcatt cagctccggt tcccaacgat caaggcgagt 3034
   tacatgatcc cccatgttgt gcaaaaaagc ggttagctcc ttcggtcctc cgatcgttgt 3094
   cagaagtaag ttggccgcag tgttatcact catggttatg gcagcactgc ataattctct 3154
   tactgtcatg ccatccgtaa gatgcttttc tgtgactggt gagtactcaa ccaagtcatt 3214
   ctgagaatag tgtatgcggc gaccgagttg ctcttgcccg gcgtcaatac gggataatag 3274
   tgtatcacat agcagaactt taaaagtgct catcattgga aaacgttctt cggggcgaaa 3334
   actctcaagg atcttaccgc tgttgagatc cagttcgatg taacccactc gtgcacccaa 3394
   ctgatcttca gcatctttta ctttcaccag cgtttctggg tgagcaaaaa caggaaggca 3454
   aaatgccgca aaaaagggaa taagggcgac acggaaatgt tgaatactca tactcttcct 3514
   ttttcaatgg gtaataactg atataattaa attgaagctc taatttgtga gtttagtata 3574
   catgcattta cttataatac agttttttag ttttgctggc cgcatcttct caaatatgct 3634
   tcccagcctg cttttctgta acgttcaccc tctaccttag catcccttcc ctttgcaaat 3694
   agtcctcttc caacaataat aatgtcagat cctgtagaga ccacatcatc cacggttcta 3754
   tactgttgac ccaatgcgtc tcccttgtca tctaaaccca caccgggtgt cataatcaac 3814
   caatcgtaac cttcatctct tccacccatg tctctttgag caataaagcc gataacaaaa 3874
   tctttgtcgc tcttcgcaat gtcaacagta cccttagtat attctccagt agatagggag 3934
   cccttgcatg acaattctgc taacatcaaa aggcctctag gttcctttgt tacttcttct 3994
   gccgcctgct tcaaaccgct aacaatacct gggcccacca caccgtgtgc attcgtaatg 4054
   tctgcccatt ctgctattct gtatacaccc gcagagtact gcaatttgac tgtattacca 4114
   atgtcagcaa attttctgtc ttcgaagagt aaaaaattgt acttggcgga taatgccttt 4174
   agcggcttaa ctgtgccctc catggaaaaa tcagtcaaga tatccacatg tgtttttagt 4234
   aaacaaattt tgggacctaa tgcttcaact aactccagta attccttggt ggtacgaaca 4294
   tccaatgaag cacacaagtt tgtttgcttt tcgtgcatga tattaaatag cttggcagca 4354
   acaggactag gatgagtagc agcacgttcc ttatatgtag ctttcgacat gatttatctt 4414
   cgtttcctgc aggtttttgt tctgtgcagt tgggttaaga atactgggca atttcatgtt 4474
   tcttcaacac tacatatgcg tatatatacc aatctaagtc tgtgctcctt ccttcgttct 4534
   tccttctgtt cggagattac cgaatcaaaa aaatttcaaa gaaaccgaaa tcaaaaaaaa 4594
   gaataaaaaa aaaatgatga attgaattga aaagctagct tatcgatgat aagctgtcaa 4654
   agatgagaat taattccacg gactatagac tatactagat actccgtcta ctgtacgata 4714
   cacttccgct caggtccttg tcctttaacg aggccttacc actcttttgt tactctattg 4774
   atccagctca gcaaaggcag tgtgatctaa gattctatct tcgcgatgta gtaaaactag 4834
   ctagaccgag aaagagacta gaaatgcaaa aggcacttct acaatggctg ccatcattat 4894
   tatccgatgt gacgctgcag cttctcaatg atattcgaat acgctttgag gagatacagc 4954
   ctaatatccg acaaactgtt ttacagattt acgatcgtac ttgttaccca tcattgaatt 5014
   ttgaacatcc gaacctggga gttttccctg aaacagatag tatatttgaa cctgtataat 5074
   aatatatagt ctagcgcttt acggaagaca atgtatgtat ttcggttcct ggagaaacta 5134
   ttgcatctat tgcataggta atcttgcacg tcgcatcccc ggttcatttt ctgcgtttcc 5194
   atcttgcact tcaatagcat atctttgtta acgaagcatc tgtgcttcat tttgtagaac 5254
   aaaaatgcaa cgcgagagcg ctaatttttc aaacaaagaa tctgagctgc atttttacag 5314
   aacagaaatg caacgcgaaa gcgctatttt accaacgaag aatctgtgct tcatttttgt 5374
   aaaacaaaaa tgcaacgcga cgagagcgct aatttttcaa acaaagaatc tgagctgcat 5434
   ttttacagaa cagaaatgca acgcgagagc gctattttac caacaaagaa tctatacttc 5494
   ttttttgttc tacaaaaatg catcccgaga gcgctatttt tctaacaaag catcttagat 5554
   tacttttttt ctcctttgtg cgctctataa tgcagtctct tgataacttt ttgcactgta 5614
   ggtccgttaa ggttagaaga aggctacttt ggtgtctatt ttctcttcca taaaaaaagc 5674
   ctgactccac ttcccgcgtt tactgattac tagcgaagct gcgggtgcat tttttcaaga 5734
   taaaggcatc cccgattata ttctataccg atgtggattg cgcatacttt gtgaacagaa 5794
   agtgatagcg ttgatgattc ttcattggtc agaaaattat gaacggtttc ttctattttg 5854
   tctctatata ctacgtatag gaaatgttta cattttcgta ttgttttcga ttcactctat 5914
   gaatagttct tactacaatt tttttgtcta aagagtaata ctagagataa acataaaaaa 5974
   tgtagaggtc gagtttagat gcaagttcaa ggagcgaaag gtggatgggt aggttatata 6034
   gggatatagc acagagatat atagcaaaga gatacttttg agcaatgttt gtggaagcgg 6094
   tattcgcaat gggaagctcc accccggttg ataatcagaa aagccccaaa aacaggaaga 6154
   ttgtataagc aaatatttaa attgtaaacg ttaatatttt gttaaaattc gcgttaaatt 6214
   tttgttaaat cagctcattt tttaacgaat agcccgaaat cggcaaaatc ccttataaat   6274
   caaaagaata gaccgagata gggttgagtg ttgttccagt ttccaacaag agtccactat   6334
   taaagaacgt ggactccaac gtcaaagggc gaaaaagggt ctatcagggc gatggcccac   6394
   tacgtgaacc atcaccctaa tcaagttttt tggggtcgag gtgccgtaaa gcagtaaatc   6454
   ggaagggtaa acggatgccc ccatttagag cttgacgggg aaagccggcg aacgtggcga   6514
   gaaaggaagg gaagaaagcg aaaggagcgg gggctagggc ggtgggaagt gtaggggtca   6574
   cgctgggcgt aaccaccaca cccgccgcgc ttaatggggc gctacagggc gcgtggggat   6634
   gatccactag t                                                        6645
<210> 101
   <211> 264
   <212> PRT
   <213> Oncorhynchus mykiss
<400> 101
   Met Ala Ser Thr Trp Gln Ser Val Gln Ser Met Arg Gln Trp Ile Leu
   1 5 10 15
   Glu Asn Gly Asp Lys Arg Thr Asp Pro Trp Leu Leu Val Tyr Ser Pro
   20 25 30
   Met Pro Val Ala Ile Ile Phe Leu Leu Tyr Leu Gly Val Val Trp Ala
   35 40 45
   Gly Pro Lys Leu Met Lys Arg Arg Glu Pro Val Asp Leu Lys Ala Val
   50 55 60
   Leu Ile Val Tyr Asn Phe Ala Met Val Cys Leu Ser Val Tyr Met Phe
   65 70 75 80
   His Glu Phe Leu Val Thr Ser Leu Leu Ser Asn Tyr Ser Tyr Leu Cys
   85 90 95
   Gln Pro Val Asp Tyr Ser Thr Ser Pro Leu Ala Met Arg Met Ala Lys
   100 105 110
   Val Cys Trp Trp Phe Phe Phe Ser Lys Val Ile Glu Leu Ala Asp Thr
   115 120 125
   Val Phe Phe Ile Leu Arg Lys Lys Asn Ser Gln Leu Thr Phe Leu His
   130 135 140
   Val Tyr His His Gly Thr Met Ile Phe Asn Trp Trp Ala Gly Val Lys
   145 150 155 160
   Tyr Leu Ala Gly Gly Gln Ser Phe Phe Ile Gly Leu Leu Asn Thr Phe
   165 170 175
   Val His Ile Val Met Tyr Ser Tyr Tyr Gly Leu Ala Ala Leu Gly Pro
   180 185 190
   His Thr Gln Lys Tyr Leu Trp Trp Lys Arg Tyr Leu Thr Ser Leu Gln
   195 200 205
   Leu Leu Gln Phe Val Leu Leu Thr Thr His Thr Gly Tyr Asn Leu Phe
   210 215 220
   Thr Glu Cys Asp Phe Pro Asp Ser Met Asn Ala Val Val Phe Ala Tyr
   225 230 235 240
   Cys Val Ser Leu Ile Ala Leu Phe Ser Asn Phe Tyr Tyr Gln Ser Tyr
   245 250 255
   Leu Asn Arg Lys Ser Lys Lys Thr
   260
<210> 102
   <211> 1077
   <212> DNA
   <213> Thalassiosira pseudonana
<220>
   <221> CDS
   <222> (1)..(1077)
   <223> delta-5-Elongase
<400> 102
   atg tgc tca tca ccg ccg tca caa tcc aaa aca aca tcc ctc cta gca      48
   Met Cys Ser Ser Pro Pro Ser Gln Ser Lys Thr Thr Ser Leu Leu Ala
   1               5                   10                  15
   cgg tac acc acc gcc gcc ctc ctc ctc ctc acc ctc aca aca tgg tgc      96
   Arg Tyr Thr Thr Ala Ala Leu Leu Leu Leu Thr Leu Thr Thr Trp Cys
               20                  25                  30
   cac ttc gcc ttc cca gcc gcc acc gcc aca ccc ggc ctc acc gcc gaa     144
   His Phe Ala Phe Pro Ala Ala Thr Ala Thr Pro Gly Leu Thr Ala Glu
           35                  40                  45
   atg cac tcc tac aaa gtc cca ctc ggt ctc acc gta ttc tac ctg ctg     192
   Met His Ser Tyr Lys Val Pro Leu Gly Leu Thr Val Phe Tyr Leu Leu
       50                  55                  60
   agt cta ccg tca cta aag tac gtt acg gac aac tac ctt gcc aaa aag     240
   Ser Leu Pro Ser Leu Lys Tyr Val Thr Asp Asn Tyr Leu Ala Lys Lys
   65                  70                  75                  80
   tat gat atg aag tca ctc cta acg gaa tca atg gtg ttg tac aat gtg     288
   Tyr Asp Met Lys Ser Leu Leu Thr Glu Ser Met Val Leu Tyr Asn Val
                   85                  90                  95
   gcg caa gtg ctg ctc aat ggg tgg acg gtg tat gcg att gtg gat gcg     336
   Ala Gln Val Leu Leu Asn Gly Trp Thr Val Tyr Ala Ile Val Asp Ala
               100                 105                 110
   gtg atg aat aga gac cat ccg ttt att gga agt aga agt ttg gtt ggg     384
   Val Met Asn Arg Asp His Pro Phe Ile Gly Ser Arg Ser Leu Val Gly
           115                 120                 125
   gcg gcg ttg cat agt ggg agc tcg tat gcg gtg tgg gtt cat tat tgt 432
   Ala Ala Leu His Ser Gly Ser Ser Tyr Ala Val Trp Val His Tyr Cys
   130 135 140
   gat aag tat ttg gag ttc ttt gat acg tat ttt atg gtg ttg agg ggg 480
   Asp Lys Tyr Leu Glu Phe Phe Asp Thr Tyr Phe Met Val Leu Arg Gly
   145 150 155 160
   aaa atg gac cag gtc tcc ttc ctc cac atc tac cac cac acg acc ata 528
   Lys Met Asp Gln Val Ser Phe Leu His Ile Tyr His His Thr Thr Ile
   165 170 175
   gcg tgg gca tgg tgg atc gcc ctc cgc ttc tcc ccc ggt gga gac att 576
   Ala Trp Ala Trp Trp Ile Ala Leu Arg Phe Ser Pro Gly Gly Asp Ile
   180 185 190
   tac ttc ggg gca ctc ctc aac tcc atc atc cac gtc ctc atg tat tcc 624
   Tyr Phe Gly Ala Leu Leu Asn Ser Ile Ile His Val Leu Met Tyr Ser
   195 200 205
   tac tac gcc ctt gcc cta ctc aag gtc agt tgt cca tgg aaa cga tac 672
   Tyr Tyr Ala Leu Ala Leu Leu Lys Val Ser Cys Pro Trp Lys Arg Tyr
   210 215 220
   ctg act caa gct caa tta ttg caa ttc aca agt gtg gtg gtt tat acg 720
   Leu Thr Gln Ala Gln Leu Leu Gln Phe Thr Ser Val Val Val Tyr Thr
   225 230 235 240
   ggg tgt acg ggt tat act cat tac tat cat acg aag cat gga gcg gat 768
   Gly Cys Thr Gly Tyr Thr His Tyr Tyr His Thr Lys His Gly Ala Asp
   245 250 255
   gag aca cag cct agt tta gga acg tat tat ttc tgt tgt gga gtg cag 816
   Glu Thr Gln Pro Ser Leu Gly Thr Tyr Tyr Phe Cys Cys Gly Val Gln
   260 265 270
   gtg ttt gag atg gtt agt ttg ttt gta ctc ttt tcc atc ttt tat aaa 864
   Val Phe Glu Met Val Ser Leu Phe Val Leu Phe Ser Ile Phe Tyr Lys
   275 280 285
   cga tcc tat tcg aag aag aac aag tca gga gga aag gat agc aag aag 912
   Arg Ser Tyr Ser Lys Lys Asn Lys Ser Gly Gly Lys Asp Ser Lys Lys
   290 295 300
   aat gat gat ggg aat aat gag gat caa tgt cac aag gct atg aag gat 960
   Asn Asp Asp Gly Asn Asn Glu Asp Gln Cys His Lys Ala Met Lys Asp
   305 310 315 320
   ata tcg gag ggt gcg aag gag gtt gtg ggg cat gca gcg aag gat gct 1008
   Ile Ser Glu Gly Ala Lys Glu Val Val Gly His Ala Ala Lys Asp Ala
   325 330 335
   gga aag ttg gtg gct acg gcg agt aag gct gta aag agg aag gga act 1056
   Gly Lys Leu Val Ala Thr Ala Ser Lys Ala Val Lys Arg Lys Gly Thr
   340 345 350
   cgt gtt act ggt gcc atg tag 1077
   Arg Val Thr Gly Ala Met
   355
<210> 103
   <211> 358
   <212> PRT
   <213> Thalassiosira pseudonana
<400> 103
   Met Cys Ser Ser Pro Pro Ser Gln Ser Lys Thr Thr Ser Leu Leu Ala
   1 5 10 15
   Arg Tyr Thr Thr Ala Ala Leu Leu Leu Leu Thr Leu Thr Thr Trp Cys
   20 25 30
   His Phe Ala Phe Pro Ala Ala Thr Ala Thr Pro Gly Leu Thr Ala Glu
   35 40 45
   Met His Ser Tyr Lys Val Pro Leu Gly Leu Thr Val Phe Tyr Leu Leu
   50 55 60
   Ser Leu Pro Ser Leu Lys Tyr Val Thr Asp Asn Tyr Leu Ala Lys Lys
   65 70 75 80
   Tyr Asp Met Lys Ser Leu Leu Thr Glu Ser Met Val Leu Tyr Asn Val
   85 90 95
   Ala Gln Val Leu Leu Asn Gly Trp Thr Val Tyr Ala Ile Val Asp Ala
   100 105 110
   Val Met Asn Arg Asp His Pro Phe Ile Gly Ser Arg Ser Leu Val Gly
   115 120 125
   Ala Ala Leu His Ser Gly Ser Ser Tyr Ala Val Trp Val His Tyr Cys
   130 135 140
   Asp Lys Tyr Leu Glu Phe Phe Asp Thr Tyr Phe Met Val Leu Arg Gly
   145 150 155 160
   Lys Met Asp Gln Val Ser Phe Leu His Ile Tyr His His Thr Thr Ile
   165 170 175
   Ala Trp Ala Trp Trp Ile Ala Leu Arg Phe Ser Pro Gly Gly Asp Ile
   180 185 190
   Tyr Phe Gly Ala Leu Leu Asn Ser Ile Ile His Val Leu Met Tyr Ser
   195 200 205
   Tyr Tyr Ala Leu Ala Leu Leu Lys Val Ser Cys Pro Trp Lys Arg Tyr
   210 215 220
   Leu Thr Gln Ala Gln Leu Leu Gln Phe Thr Ser Val Val Val Tyr Thr
   225 230 235 240
   Gly Cys Thr Gly Tyr Thr His Tyr Tyr His Thr Lys His Gly Ala Asp
   245 250 255
   Glu Thr Gln Pro Ser Leu Gly Thr Tyr Tyr Phe Cys Cys Gly Val Gln
   260 265 270
   Val Phe Glu Met Val Ser Leu Phe Val Leu Phe Ser Ile Phe Tyr Lys
           275                 280                 285
   Arg Ser Tyr Ser Lys Lys Asn Lys Ser Gly Gly Lys Asp Ser Lys Lys
       290                 295                 300
   Asn Asp Asp Gly Asn Asn Glu Asp Gln Cys His Lys Ala Met Lys Asp
   305                 310                 315                 320
   Ile Ser Glu Gly Ala Lys Glu Val Val Gly His Ala Ala Lys Asp Ala
                   325                 330                 335
   Gly Lys Leu Val Ala Thr Ala Ser Lys Ala Val Lys Arg Lys Gly Thr
               340                 345                 350
   Arg Val Thr Gly Ala Met
           355
<210> 104
   <211> 933
   <212> DNA
   <213> Thalassiosira pseudonana
<220>
   <221> CDS
   <222> (1)..(933)
   <223> delta-5-Elongase
<400> 104
   atg cac tcc tac aaa gtc cca ctc ggt ctc acc gta ttc tac ctg ctg      48
   Met His Ser Tyr Lys Val Pro Leu Gly Leu Thr Val Phe Tyr Leu Leu
   1               5                   10                  15
   agt cta ccg tca cta aag tac gtt acg gac aac tac ctt gcc aaa aag      96
   Ser Leu Pro Ser Leu Lys Tyr Val Thr Asp Asn Tyr Leu Ala Lys Lys
               20                  25                  30
   tat gat atg aag tca ctc cta acg gaa tca atg gtg ttg tac aat gtg     144
   Tyr Asp Met Lys Ser Leu Leu Thr Glu Ser Met Val Leu Tyr Asn Val
           35                  40                  45
   gcg caa gtg ctg ctc aat ggg tgg acg gtg tat gcg att gtg gat gcg     192
   Ala Gln Val Leu Leu Asn Gly Trp Thr Val Tyr Ala Ile Val Asp Ala
       50                  55                  60
   gtg atg aat aga gac cat ccg ttt att gga agt aga agt ttg gtt ggg     240
   Val Met Asn Arg Asp His Pro Phe Ile Gly Ser Arg Ser Leu Val Gly
   65                  70                  75                  80
   gcg gcg ttg cat agt ggg agc tcg tat gcg gtg tgg gtt cat tat tgt     288
   Ala Ala Leu His Ser Gly Ser Ser Tyr Ala Val Trp Val His Tyr Cys
                   85                  90                  95
   gat aag tat ttg gag ttc ttt gat acg tat ttt atg gtg ttg agg ggg     336
   Asp Lys Tyr Leu Glu Phe Phe Asp Thr Tyr Phe Met Val Leu Arg Gly
               100                 105                 110
   aaa atg gac cag gtc tcc ttc ctc cac atc tac cac cac acg acc ata     384
   Lys Met Asp Gln Val Ser Phe Leu His Ile Tyr His His Thr Thr Ile
           115                 120                 125
   gcg tgg gca tgg tgg atc gcc ctc cgc ttc tcc ccc ggt gga gac att      432
   Ala Trp Ala Trp Trp Ile Ala Leu Arg Phe Ser Pro Gly Gly Asp Ile
       130                 135                 140
   tac ttc ggg gca ctc ctc aac tcc atc atc cac gtc ctc atg tat tcc      480
   Tyr Phe Gly Ala Leu Leu Asn Ser Ile Ile His Val Leu Met Tyr Ser
   145                 150                 155                 160
   tac tac gcc ctt gcc cta ctc aag gtc agt tgt cca tgg aaa cga tac      528
   Tyr Tyr Ala Leu Ala Leu Leu Lys Val Ser Cys Pro Trp Lys Arg Tyr
                   165                 170                 175
   ctg act caa gct caa tta ttg caa ttc aca agt gtg gtg gtt tat acg      576
   Leu Thr Gln Ala Gln Leu Leu Gln Phe Thr Ser Val Val Val Tyr Thr
               180                 185                 190
   ggg tgt acg ggt tat act cat tac tat cat acg aag cat gga gcg gat      624
   Gly Cys Thr Gly Tyr Thr His Tyr Tyr His Thr Lys His Gly Ala Asp
           195                 200                 205
   gag aca cag cct agt tta gga acg tat tat ttc tgt tgt gga gtg cag      672
   Glu Thr Gln Pro Ser Leu Gly Thr Tyr Tyr Phe Cys Cys Gly Val Gln
       210                 215                 220
   gtg ttt gag atg gtt agt ttg ttt gta ctc ttt tcc atc ttt tat aaa      720
   Val Phe Glu Met Val Ser Leu Phe Val Leu Phe Ser Ile Phe Tyr Lys
   225                 230                 235                 240
   cga tcc tat tcg aag aag aac aag tca gga gga aag gat agc aag aag      768
   Arg Ser Tyr Ser Lys Lys Asn Lys Ser Gly Gly Lys Asp Ser Lys Lys
                   245                 250                 255
   aat gat gat ggg aat aat gag gat caa tgt cac aag gct atg aag gat      816
   Asn Asp Asp Gly Asn Asn Glu Asp Gln Cys His Lys Ala Met Lys Asp
               260                 265                 270
   ata tcg gag ggt gcg aag gag gtt gtg ggg cat gca gcg aag gat gct      864
   Ile Ser Glu Gly Ala Lys Glu Val Val Gly His Ala Ala Lys Asp Ala
           275                 280                 285
   gga aag ttg gtg gct acg gcg agt aag gct gta aag agg aag gga act      912
   Gly Lys Leu Val Ala Thr Ala Ser Lys Ala Val Lys Arg Lys Gly Thr
       290                 295                 300
   cgt gtt act ggt gcc atg tag                                          933
   Arg Val Thr Gly Ala Met
   305                 310
<210> 105
   <211> 310
   <212> PRT
   <213> Thalassiosira pseudonana
<400> 105
   Met His Ser Tyr Lys Val Pro Leu Gly Leu Thr Val Phe Tyr Leu Leu
   1 5 10 15
   Ser Leu Pro Ser Leu Lys Tyr Val Thr Asp Asn Tyr Leu Ala Lys Lys
   20 25 30
   Tyr Asp Met Lys Ser Leu Leu Thr Glu Ser Met Val Leu Tyr Asn Val
   35 40 45
   Ala Gln Val Leu Leu Asn Gly Trp Thr Val Tyr Ala Ile Val Asp Ala
       50                  55                  60
   Val Met Asn Arg Asp His Pro Phe Ile Gly Ser Arg Ser Leu Val Gly
   65                  70                  75                  80
   Ala Ala Leu His Ser Gly Ser Ser Tyr Ala Val Trp Val His Tyr Cys
                   85                  90                  95
   Asp Lys Tyr Leu Glu Phe Phe Asp Thr Tyr Phe Met Val Leu Arg Gly
               100                 105                 110
   Lys Met Asp Gln Val Ser Phe Leu His Ile Tyr His His Thr Thr Ile
           115                 120                 125
   Ala Trp Ala Trp Trp Ile Ala Leu Arg Phe Ser Pro Gly Gly Asp Ile
       130                 135                 140
   Tyr Phe Gly Ala Leu Leu Asn Ser Ile Ile His Val Leu Met Tyr Ser
   145                 150                 155                 160
   Tyr Tyr Ala Leu Ala Leu Leu Lys Val Ser Cys Pro Trp Lys Arg Tyr
                   165                 170                 175
   Leu Thr Gln Ala Gln Leu Leu Gln Phe Thr Ser Val Val Val Tyr Thr
               180                 185                 190
   Gly Cys Thr Gly Tyr Thr His Tyr Tyr His Thr Lys His Gly Ala Asp
           195                 200                 205
   Glu Thr Gln Pro Ser Leu Gly Thr Tyr Tyr Phe Cys Cys Gly Val Gln
       210                 215                 220
   Val Phe Glu Met Val Ser Leu Phe Val Leu Phe Ser Ile Phe Tyr Lys
   225                 230                 235                 240
   Arg Ser Tyr Ser Lys Lys Asn Lys Ser Gly Gly Lys Asp Ser Lys Lys
                   245                 250                 255
   Asn Asp Asp Gly Asn Asn Glu Asp Gln Cys His Lys Ala Met Lys Asp
               260                 265                 270
   Ile Ser Glu Gly Ala Lys Glu Val Val Gly His Ala Ala Lys Asp Ala
           275                 280                 285
   Gly Lys Leu Val Ala Thr Ala Ser Lys Ala Val Lys Arg Lys Gly Thr
       290                 295                 300
   Arg Val Thr Gly Ala Met
   305                 310
<210> 106
   <211> 933
   <212> DNA
   <213> Thalassiosira pseudonana
<220>
   <221> CDS
   <222> (1)..(933)
   <223> delta-5-Elongase
<400> 106
   atg cac tcc tac aaa gtc cca ctc ggt ctc acc gta ttc tac ctg ctg      48
   Met His Ser Tyr Lys Val Pro Leu Gly Leu Thr Val Phe Tyr Leu Leu
   1               5                   10                  15
   agt cta ccg tca cta aag tac gtt acg gac aac tac ctt gcc aaa aag      96
   Ser Leu Pro Ser Leu Lys Tyr Val Thr Asp Asn Tyr Leu Ala Lys Lys
               20                  25                  30
   tat gat atg aag tca ctc cta acg gaa tca atg gtg ttg tac aat gtg     144
   Tyr Asp Met Lys Ser Leu Leu Thr Glu Ser Met Val Leu Tyr Asn Val
           35                  40                  45
   gcg caa gtg ctg ctc aat ggg tgg acg gtg tat gcg att gtg gat gcg     192
   Ala Gln Val Leu Leu Asn Gly Trp Thr Val Tyr Ala Ile Val Asp Ala
       50                  55                  60
   gtg atg aat aga gac cat ccg ttt att gga agt aga agt ttg gtt ggg     240
   Val Met Asn Arg Asp His Pro Phe Ile Gly Ser Arg Ser Leu Val Gly
   65                  70                  75                  80
   gcg gcg ttg cat agt ggg agc tcg tat gcg gtg tgg gtt cat tat tgt     288
   Ala Ala Leu His Ser Gly Ser Ser Tyr Ala Val Trp Val His Tyr Cys
                   85                  90                  95
   gat aag tat ttg gag ttc ttt gat acg tat ttt atg gtg ttg agg ggg     336
   Asp Lys Tyr Leu Glu Phe Phe Asp Thr Tyr Phe Met Val Leu Arg Gly
               100                 105                 110
   aaa atg gac cag gtc tcc ttc ctc cac atc tac cac cac acg acc ata     384
   Lys Met Asp Gln Val Ser Phe Leu His Ile Tyr His His Thr Thr Ile
           115                 120                 125
   gcg tgg gca tgg tgg atc gcc ctc cgc ttc tcc ccc ggt gga gac att     432
   Ala Trp Ala Trp Trp Ile Ala Leu Arg Phe Ser Pro Gly Gly Asp Ile
       130                 135                 140
   tac ttc ggg gca ctc ctc aac tcc atc atc cac gtc ctc atg tat tcc     480
   Tyr Phe Gly Ala Leu Leu Asn Ser Ile Ile His Val Leu Met Tyr Ser
   145                 150                 155                 160
   tac tac gcc ctt gcc cta ctc aag gtc agt tgt cca tgg aaa cga tac     528
   Tyr Tyr Ala Leu Ala Leu Leu Lys Val Ser Cys Pro Trp Lys Arg Tyr
                   165                 170                 175
   ctg act caa gct caa tta ttg caa ttc aca agt gtg gtg gtt tat acg     576
   Leu Thr Gln Ala Gln Leu Leu Gln Phe Thr Ser Val Val Val Tyr Thr
               180                 185                 190
   ggg tgt acg ggt tat act cat tac tat cat acg aag cat gga gcg gat     624
   Gly Cys Thr Gly Tyr Thr His Tyr Tyr His Thr Lys His Gly Ala Asp
           195                 200                 205
   gag aca cag cct agt tta gga acg tat tat ttc tgt tgt gga gtg cag     672
   Glu Thr Gln Pro Ser Leu Gly Thr Tyr Tyr Phe Cys Cys Gly Val Gln
       210                 215                 220
   gtg ttt gag atg gtt agt ttg ttt gta ctc ttt tcc atc ttt tat aaa     720
   Val Phe Glu Met Val Ser Leu Phe Val Leu Phe Ser Ile Phe Tyr Lys
   225                 230                 235                 240
   cga tcc tat tcg aag aag aac aag tca gga gga aag gat agc aag aag     768
   Arg Ser Tyr Ser Lys Lys Asn Lys Ser Gly Gly Lys Asp Ser Lys Lys
                   245                 250                 255
   aat gat gat ggg aat aat gag gat caa tgt cac aag gct atg aag gat     816
   Asn Asp Asp Gly Asn Asn Glu Asp Gln Cys His Lys Ala Met Lys Asp
               260                 265                 270
   ata tcg gag ggt gcg aag gag gtt gtg ggg cat gca gcg aag gat gct     864
   Ile Ser Glu Gly Ala Lys Glu Val Val Gly His Ala Ala Lys Asp Ala
           275                 280                 285
   gga aag ttg gtg gct acg gcg agt aag gct gta aag agg aag gga act     912
   Gly Lys Leu Val Ala Thr Ala Ser Lys Ala Val Lys Arg Lys Gly Thr
       290                 295                 300
   cgt gtt act ggt gcc atg tag                                         933
   Arg Val Thr Gly Ala Met
   305                 310
<210> 107
   <211> 310
   <212> PRT
   <213> Thalassiosira pseudonana
<400> 107
   Met His Ser Tyr Lys Val Pro Leu Gly Leu Thr Val Phe Tyr Leu Leu
   1               5                   10                  15
   Ser Leu Pro Ser Leu Lys Tyr Val Thr Asp Asn Tyr Leu Ala Lys Lys
               20                  25                  30
   Tyr Asp Met Lys Ser Leu Leu Thr Glu Ser Met Val Leu Tyr Asn Val
           35                  40                  45
   Ala Gln Val Leu Leu Asn Gly Trp Thr Val Tyr Ala Ile Val Asp Ala
       50                  55                  60
   Val Met Asn Arg Asp His Pro Phe Ile Gly Ser Arg Ser Leu Val Gly
   65                  70                  75                  80
   Ala Ala Leu His Ser Gly Ser Ser Tyr Ala Val Trp Val His Tyr Cys
                   85                  90                  95
   Asp Lys Tyr Leu Glu Phe Phe Asp Thr Tyr Phe Met Val Leu Arg Gly
               100                 105                 110
   Lys Met Asp Gln Val Ser Phe Leu His Ile Tyr His His Thr Thr Ile
           115                 120                 125
   Ala Trp Ala Trp Trp Ile Ala Leu Arg Phe Ser Pro Gly Gly Asp Ile
       130                 135                 140
   Tyr Phe Gly Ala Leu Leu Asn Ser Ile Ile His Val Leu Met Tyr Ser
   145                 150                 155                 160
   Tyr Tyr Ala Leu Ala Leu Leu Lys Val Ser Cys Pro Trp Lys Arg Tyr
                   165                 170                 175
   Leu Thr Gln Ala Gln Leu Leu Gln Phe Thr Ser Val Val Val Tyr Thr
               180                 185                 190
   Gly Cys Thr Gly Tyr Thr His Tyr Tyr His Thr Lys His Gly Ala Asp
           195                 200                 205
   Glu Thr Gln Pro Ser Leu Gly Thr Tyr Tyr Phe Cys Cys Gly Val Gln
       210                 215                 220
   Val Phe Glu Met Val Ser Leu Phe Val Leu Phe Ser Ile Phe Tyr Lys
   225                 230                 235                 240
   Arg Ser Tyr Ser Lys Lys Asn Lys Ser Gly Gly Lys Asp Ser Lys Lys
                   245                 250                 255
   Asn Asp Asp Gly Asn Asn Glu Asp Gln Cys His Lys Ala Met Lys Asp
               260                 265                 270
   Ile Ser Glu Gly Ala Lys Glu Val Val Gly His Ala Ala Lys Asp Ala
           275                 280                 285
   Gly Lys Leu Val Ala Thr Ala Ser Lys Ala Val Lys Arg Lys Gly Thr
       290                 295                 300
   Arg Val Thr Gly Ala Met
   305                 310
<210> 108
   <211> 825
   <212> DNA
   <213> Thraustochytrium aureum
<220>
   <221> CDS
   <222> (1)..(825)
   <223> delta-5-Elongase
<400> 108
   atg acg agc aac atg agc gcg tgg ggc gtc gcc gtc gac cag acg cag      48
   Met Thr Ser Asn Met Ser Ala Trp Gly Val Ala Val Asp Gln Thr Gln
   1               5                   10                  15
   cag gtc gtc gac cag atc atg ggc ggc gcc gag ccg tac aag ctg aca      96
   Gln Val Val Asp Gln Ile Met Gly Gly Ala Glu Pro Tyr Lys Leu Thr
               20                  25                  30
   gaa ggg cgc atg acg aac gtc gag acg atg ctg gcg atc gag tgc ggc      144
   Glu Gly Arg Met Thr Asn Val Glu Thr Met Leu Ala Ile Glu Cys Gly
           35                  40                  45
   tac gcc gcc atg ctg ctg ttc ctg acc ccg atc atg aag cag gcc gag      192
   Tyr Ala Ala Met Leu Leu Phe Leu Thr Pro Ile Met Lys Gln Ala Glu
       50                  55                  60
   aag ccc ttc gag ctc aag tcc ttc aag ctc gcc cac aac ctg ttc ctg      240
   Lys Pro Phe Glu Leu Lys Ser Phe Lys Leu Ala His Asn Leu Phe Leu
   65                  70                  75                  80
   ttc gtc ctg tcc gcc tac atg tgc ctc gag acc gtc cgc cag gcc tac      288
   Phe Val Leu Ser Ala Tyr Met Cys Leu Glu Thr Val Arg Gln Ala Tyr
                   85                  90                  95
   ctt gcg ggc tac tcg gtg ttc ggc aac gac atg gag aag ggc agc gag      336
   Leu Ala Gly Tyr Ser Val Phe Gly Asn Asp Met Glu Lys Gly Ser Glu
               100                 105                 110
   ccg cac gcg cac ggc atg gcc caa atc gtg tgg atc ttt tac gtg tcc      384
   Pro His Ala His Gly Met Ala Gln Ile Val Trp Ile Phe Tyr Val Ser
           115                 120                 125
   aag gcg tac gag ttc gtg gac acg ctg atc atg atc ctg tgc aaa aag      432
   Lys Ala Tyr Glu Phe Val Asp Thr Leu Ile Met Ile Leu Cys Lys Lys
       130                 135                 140
   ttc aac cag gtc tcc gtc ctg cac gtg tac cac cac gcc acc atc ttt      480
   Phe Asn Gln Val Ser Val Leu His Val Tyr His His Ala Thr Ile Phe
   145                 150                 155                 160
   gct atc tgg ttt atg atc gcc aag tac gcc ccg ggc ggc gac gca tac      528
   Ala Ile Trp Phe Met Ile Ala Lys Tyr Ala Pro Gly Gly Asp Ala Tyr
                   165                 170                 175
   ttt agc gtc atc ctg aac tcg ttc gtg cac acc gtc atg tac gcg tac      576
   Phe Ser Val Ile Leu Asn Ser Phe Val His Thr Val Met Tyr Ala Tyr
               180                 185                 190
   tac ttc ttc tcg tcg cag ggc ttc ggg ttc gtc aag ccg atc aag ccg      624
   Tyr Phe Phe Ser Ser Gln Gly Phe Gly Phe Val Lys Pro Ile Lys Pro
           195                 200                 205
   tac atc acc tcg ctg cag atg acg cag ttc atg gcg atg ctc gtg cag      672
   Tyr Ile Thr Ser Leu Gln Met Thr Gln Phe Met Ala Met Leu Val Gln
       210                 215                 220
   tcg ctg tac gac tac ctt tac ccg tgc gac tac ccg cag ggg ctc gtc      720
   Ser Leu Tyr Asp Tyr Leu Tyr Pro Cys Asp Tyr Pro Gln Gly Leu Val
   225                 230                 235                 240
   aag ctc ctc ggc gtg tac atg ctc acc ctg ctt gcg ctc ttc ggc aac      768
   Lys Leu Leu Gly Val Tyr Met Leu Thr Leu Leu Ala Leu Phe Gly Asn
                   245                 250                 255
   ttt ttc gtg cag agc tac ctc aag aag tcg aac aag ccc aag gcc aag      816
   Phe Phe Val Gln Ser Tyr Leu Lys Lys Ser Asn Lys Pro Lys Ala Lys
               260                 265                 270
   tcg gcc taa                                                          825
   Ser Ala
<210> 109
   <211> 274
   <212> PRT
   <213> Thraustochytrium aureum
<400> 109
   Met Thr Ser Asn Met Ser Ala Trp Gly Val Ala Val Asp Gln Thr Gln
   1               5                   10                  15
   Gln Val Val Asp Gln Ile Met Gly Gly Ala Glu Pro Tyr Lys Leu Thr
               20                  25                  30
   Glu Gly Arg Met Thr Asn Val Glu Thr Met Leu Ala Ile Glu Cys Gly
           35                  40                  45
   Tyr Ala Ala Met Leu Leu Phe Leu Thr Pro Ile Met Lys Gln Ala Glu
       50                  55                  60
   Lys Pro Phe Glu Leu Lys Ser Phe Lys Leu Ala His Asn Leu Phe Leu
   65                  70                  75                  80
   Phe Val Leu Ser Ala Tyr Met Cys Leu Glu Thr Val Arg Gln Ala Tyr
                   85                  90                  95
   Leu Ala Gly Tyr Ser Val Phe Gly Asn Asp Met Glu Lys Gly Ser Glu
               100                 105                 110
   Pro His Ala His Gly Met Ala Gln Ile Val Trp Ile Phe Tyr Val Ser
           115                 120                 125
   Lys Ala Tyr Glu Phe Val Asp Thr Leu Ile Met Ile Leu Cys Lys Lys
       130                 135                 140
   Phe Asn Gln Val Ser Val Leu His Val Tyr His His Ala Thr Ile Phe
   145                 150                 155                 160
   Ala Ile Trp Phe Met Ile Ala Lys Tyr Ala Pro Gly Gly Asp Ala Tyr
                   165                 170                 175
   Phe Ser Val Ile Leu Asn Ser Phe Val His Thr Val Met Tyr Ala Tyr
               180                 185                 190
   Tyr Phe Phe Ser Ser Gln Gly Phe Gly Phe Val Lys Pro Ile Lys Pro
           195                 200                 205
   Tyr Ile Thr Ser Leu Gln Met Thr Gln Phe Met Ala Met Leu Val Gln
       210                 215                 220
   Ser Leu Tyr Asp Tyr Leu Tyr Pro Cys Asp Tyr Pro Gln Gly Leu Val
   225                 230                 235                 240
   Lys Leu Leu Gly Val Tyr Met Leu Thr Leu Leu Ala Leu Phe Gly Asn
                   245                 250                 255
   Phe Phe Val Gln Ser Tyr Leu Lys Lys Ser Asn Lys Pro Lys Ala Lys
               260                 265                 270
   Ser Ala
<210> 110
   <211> 903
   <212> DNA
   <213> Ostreococcus tauri
<220>
   <221> CDS
   <222> (1)..(903)
   <223> delta-5-Elongase
<400> 110
   atg agc gcc tcc ggt gcg ctg ctg ccc gcg atc gcg ttc gcc gcg tac     48
   Met Ser Ala Ser Gly Ala Leu Leu Pro Ala Ile Ala Phe Ala Ala Tyr
   1               5                   10                  15
   gcg tac gcg acg tac gcc tac gcc ttt gag tgg tcg cac gcg aat ggc     96
   Ala Tyr Ala Thr Tyr Ala Tyr Ala Phe Glu Trp Ser His Ala Asn Gly
               20                  25                  30
   atc gac aac gtc gac gcg cgc gag tgg atc ggt gcg ctg tcg ttg agg    144
   Ile Asp Asn Val Asp Ala Arg Glu Trp Ile Gly Ala Leu Ser Leu Arg
           35                  40                  45
   ctc ccg gcg atc gcg acg acg atg tac ctg ttg ttc tgc ctg gtc gga    192
   Leu Pro Ala Ile Ala Thr Thr Met Tyr Leu Leu Phe Cys Leu Val Gly
       50                  55                  60
   ccg agg ttg atg gcg aag cgc gag gcg ttc gac ccg aag ggg ttc atg    240
   Pro Arg Leu Met Ala Lys Arg Glu Ala Phe Asp Pro Lys Gly Phe Met
   65                  70                  75                  80
   ctg gcg tac aat gcg tat cag acg gcg ttc aac gtc gtc gtg ctc ggg    288
   Leu Ala Tyr Asn Ala Tyr Gln Thr Ala Phe Asn Val Val Val Leu Gly
                   85                  90                  95
   atg ttc gcg cga gag atc tcg ggg ctg ggg cag ccc gtg tgg ggg tca    336
   Met Phe Ala Arg Glu Ile Ser Gly Leu Gly Gln Pro Val Trp Gly Ser
               100                 105                 110
   acc atg ccg tgg agc gat aga aaa tcg ttt aag atc ctc ctc ggg gtg    384
   Thr Met Pro Trp Ser Asp Arg Lys Ser Phe Lys Ile Leu Leu Gly Val
           115                 120                 125
   tgg ttg cac tac aac aac caa tat ttg gag cta ttg gac act gtg ttc    432
   Trp Leu His Tyr Asn Asn Gln Tyr Leu Glu Leu Leu Asp Thr Val Phe
       130                 135                 140
   atg gtt gcg cgc aag aag acg aag cag ttg agc ttc ttg cac gtt tat    480
   Met Val Ala Arg Lys Lys Thr Lys Gln Leu Ser Phe Leu His Val Tyr
   145                 150                 155                 160
   cat cac gcc ctg ttg atc tgg gcg tgg tgg ttg gtg tgt cac ttg atg    528
   His His Ala Leu Leu Ile Trp Ala Trp Trp Leu Val Cys His Leu Met
                   165                 170                 175
   gcc acg aac gat tgt atc gat gcc tac ttc ggc gcg gcg tgc aac tcg    576
   Ala Thr Asn Asp Cys Ile Asp Ala Tyr Phe Gly Ala Ala Cys Asn Ser
               180                 185                 190
   ttc att cac atc gtg atg tac tcg tat tat ctc atg tcg gcg ctc ggc    624
   Phe Ile His Ile Val Met Tyr Ser Tyr Tyr Leu Met Ser Ala Leu Gly
           195                 200                 205
   att cga tgc ccg tgg aag cga tac atc acc cag gct caa atg ctc caa      672
   Ile Arg Cys Pro Trp Lys Arg Tyr Ile Thr Gln Ala Gln Met Leu Gln
       210                 215                 220
   ttc gtc att gtc ttc gcg cac gcc gtg ttc gtg ctg cgt cag aag cac      720
   Phe Val Ile Val Phe Ala His Ala Val Phe Val Leu Arg Gln Lys His
   225                 230                 235                 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg      816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg      864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                  903
   Pro Ser Val Arg Arg Thr Arg Ser Arg Lys Ile Asp
       290                 295                 300
<210> 111
   <211> 300
   <212> PRT
   <213> Ostreococcus tauri
<400> 111
   Met Ser Ala Ser Gly Ala Leu Leu Pro Ala Ile Ala Phe Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Gln Tyr Leu Glu Leu Leu Asp Thr Val Phe
       130                 135                 140
   Met Val Ala Arg Lys Lys Thr Lys Gln Leu Ser Phe Leu His Val Tyr
   145                 150                 155                 160
   His His Ala Leu Leu Ile Trp Ala Trp Trp Leu Val Cys His Leu Met
                   165                 170                 175
   Ala Thr Asn Asp Cys Ile Asp Ala Tyr Phe Gly Ala Ala Cys Asn Ser
               180                 185                 190
   Phe Ile His Ile Val Met Tyr Ser Tyr Tyr Leu Met Ser Ala Leu Gly
           195                 200                 205
   Ile Arg Cys Pro Trp Lys Arg Tyr Ile Thr Gln Ala Gln Met Leu Gln
       210                 215                 220
   Phe Val Ile Val Phe Ala His Ala Val Phe Val Leu Arg Gln Lys His
   225                 230                 235                 240
   Cys Pro Val Thr Leu Pro Trp Ala Gln Met Phe Val Met Thr Asn Met
                   245                 250                 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 112
   <211> 879
   <212> DNA
   <213> ostreococcus tauri
<220>
   <221> CDS
   <222> (1)..(879)
   <223> delta-6-Elongase
<400> 112
   atg agt ggc tta cgt gca ccc aac ttt tta cac aga ttc tgg aca aag      48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt      96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc      144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc      192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa      240
   Thr Val Phe Leu Trp Tyr Gly Arg Leu Thr Arg Ser Ser Asp Lys Lys
   65                  70                  75                  80
   att aga gag cct acg tgg tta aga aga ttc ata ata tgt cat aat gcg      288
   Ile Arg Glu Pro Thr Trp Leu Arg Arg Phe Ile Ile Cys His Asn Ala
                   85                  90                  95
   ttc ttg ata gtc ctc agt ctt tac atg tgc ctt ggt tgt gtg gcc caa      336
   Phe Leu Ile Val Leu Ser Leu Tyr Met Cys Leu Gly Cys Val Ala Gln
               100                 105                 110
   gcg tat cag aat gga tat act tta tgg ggt aat gaa ttc aag gcc acg      384
   Ala Tyr Gln Asn Gly Tyr Thr Leu Trp Gly Asn Glu Phe Lys Ala Thr
           115                 120                 125
   gaa act cag ctt gct ctc tac att tac att ttt tac gta agt aaa ata      432
   Glu Thr Gln Leu Ala Leu Tyr Ile Tyr Ile Phe Tyr Val Ser Lys Ile
       130                 135                 140
   tac gag ttt gta gat act tac att atg ctt ctc aag aat aac ttg cgg      480
   Tyr Glu Phe Val Asp Thr Tyr Ile Met Leu Leu Lys Asn Asn Leu Arg
   145                 150                 155                 160
   caa gta agt ttc cta cac att tat cac cac agc acg att tcc ttt att      528
   Gln Val Ser Phe Leu His Ile Tyr His His Ser Thr Ile Ser Phe Ile
                   165                 170                 175
   tgg tgg atc att gct cgg agg gct ccg ggt ggt gat gct tac ttc agc      576
   Trp Trp Ile Ile Ala Arg Arg Ala Pro Gly Gly Asp Ala Tyr Phe Ser
               180                 185                 190
   gcg gcc ttg aac tca tgg gta cac gtg tgc atg tac acc tat tat cta      624
   Ala Ala Leu Asn Ser Trp Val His Val Cys Met Tyr Thr Tyr Tyr Leu
           195                 200                 205
   tta tca acc ctt att gga aaa gaa gat cct aag cgt tcc aac tac ctt      672
   Leu Ser Thr Leu Ile Gly Lys Glu Asp Pro Lys Arg Ser Asn Tyr Leu
       210                 215                 220
   tgg tgg ggt cgc cac cta acg caa atg cag atg ctt cag ttt ttc ttc      720
   Trp Trp Gly Arg His Leu Thr Gln Met Gln Met Leu Gln Phe Phe Phe
   225                 230                 235                 240
   aac gta ctt caa gcg ttg tac tgc gct tcg ttc tct acg tat ccc aag      768
   Asn Val Leu Gln Ala Leu Tyr Cys Ala Ser Phe Ser Thr Tyr Pro Lys
                   245                 250                 255
   ttt ttg tcc aaa att ctg ctc gtc tat atg atg agc ctt ctc ggc ttg      816
   Phe Leu Ser Lys Ile Leu Leu Val Tyr Met Met Ser Leu Leu Gly Leu
               260                 265                 270
   ttt ggg cat ttc tac tat tcc aag cac ata gca gca gct aag ctc cag      864
   Phe Gly His Phe Tyr Tyr Ser Lys His Ile Ala Ala Ala Lys Leu Gln
           275                 280                 285
   aaa aaa cag cag tga                                                  879
   Lys Lys Gln Gln
       290
<210> 113
   <211> 292
   <212> PRT
   <213> ostreococcus tauri
<400> 113
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Ser Phe Leu His Ile Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
   275 280 285
   Lys Lys Gln Gln
   290
<210> 114
   <211> 903
   <212> DNA
   <213> Ostreococcus tauri
<220>
   <221> CDS
   <222> (1)..(903)
   <223> delta-5-Elongase
<400> 114
   atg agc gcc tcc ggt gcg ctg ctg ccc gcg atc gcg tcc gcc gcg tac       48
   Met Ser Ala Ser Gly Ala Leu Leu Pro Ala Ile Ala Ser Ala Ala Tyr
   1               5                   10                  15
   gcg tac gcg acg tac gcc tac gcc ttt gag tgg tcg cac gcg aat ggc       96
   Ala Tyr Ala Thr Tyr Ala Tyr Ala Phe Glu Trp Ser His Ala Asn Gly
               20                  25                  30
   atc gac aac gtc gac gcg cgc gag tgg atc ggt gcg ctg tcg ttg agg      144
   Ile Asp Asn Val Asp Ala Arg Glu Trp Ile Gly Ala Leu Ser Leu Arg
           35                  40                  45
   ctc ccg gcg atc gcg acg acg atg tac ctg ttg ttc tgc ctg gtc gga      192
   Leu Pro Ala Ile Ala Thr Thr Met Tyr Leu Leu Phe Cys Leu Val Gly
       50                  55                  60
   ccg agg ttg atg gcg aag cgc gag gcg ttc gac ccg aag ggg ttc atg      240
   Pro Arg Leu Met Ala Lys Arg Glu Ala Phe Asp Pro Lys Gly Phe Met
   65                  70                  75                  80
   ctg gcg tac aat gcg tat cag acg gcg ttc aac gtc gtc gtg ctc ggg      288
   Leu Ala Tyr Asn Ala Tyr Gln Thr Ala Phe Asn Val Val Val Leu Gly
                   85                  90                  95
   atg ttc gcg cga gag atc tcg ggg ctg ggg cag ccc gtg tgg ggg tca      336
   Met Phe Ala Arg Glu Ile Ser Gly Leu Gly Gln Pro Val Trp Gly Ser
               100                 105                 110
   acc atg ccg tgg agc gat aga aaa tcg ttt aag atc ctc ctc ggg gtg      384
   Thr Met Pro Trp Ser Asp Arg Lys Ser Phe Lys Ile Leu Leu Gly Val
           115                 120                 125
   tgg ttg cac tac aac aac aaa tat ttg gag cta ttg gac act gtg ttc      432
   Trp Leu His Tyr Asn Asn Lys Tyr Leu Glu Leu Leu Asp Thr Val Phe
       130                 135                 140
   atg gtt gcg cgc aag aag acg aag cag ttg agc ttc ttg cac gtt tat      480
   Met Val Ala Arg Lys Lys Thr Lys Gln Leu Ser Phe Leu His Val Tyr
   145                 150                 155                 160
   cat cac gcc ctg ttg atc tgg gcg tgg tgg ttg gtg tgt cac ttg atg      528
   His His Ala Leu Leu Ile Trp Ala Trp Trp Leu Val Cys His Leu Met
                   165                 170                 175
   gcc acg aac gat tgt atc gat gcc tac ttc ggc gcg gcg tgc aac tcg      576
   Ala Thr Asn Asp Cys Ile Asp Ala Tyr Phe Gly Ala Ala Cys Asn Ser
               180                 185                 190
   ttc att cac atc gtg atg tac tcg tat tat ctc atg tcg gcg ctc ggc      624
   Phe Ile His Ile Val Met Tyr Ser Tyr Tyr Leu Met Ser Ala Leu Gly
           195                 200                 205
   att cga tgc ccg tgg aag cga tac atc acc cag gct caa atg ctc caa      672
   Ile Arg Cys Pro Trp Lys Arg Tyr Ile Thr Gln Ala Gln Met Leu Gln
       210                 215                 220
   ttc gtc att gtc ttc gcg cac gcc gtg ttc gtg ctg cgt cag aag cac      720
   Phe Val Ile Val Phe Ala His Ala Val Phe Val Leu Arg Gln Lys His
   225                 230                 235                 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg      816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg      864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                  903
   Pro Ser Val Arg Arg Thr Arg Ser Arg Lys Ile Asp
       290                 295                 300
<210> 115
   <211> 300
   <212> PRT
   <213> Ostreococcus tauri
<400> 115
   Met Ser Ala Ser Gly Ala Leu Leu Pro Ala Ile Ala Ser Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Lys Tyr Leu Glu Leu Leu Asp Thr Val Phe
   130 135 140
   Met Val Ala Arg Lys Lys Thr Lys Gln Leu Ser Phe Leu His Val Tyr
   145 150 155 160
   His His Ala Leu Leu Ile Trp Ala Trp Trp Leu Val Cys His Leu Met
   165 170 175
   Ala Thr Asn Asp Cys Ile Asp Ala Tyr Phe Gly Ala Ala Cys Asn Ser
   180 185 190
   Phe Ile His Ile Val Met Tyr Ser Tyr Tyr Leu Met Ser Ala Leu Gly
   195 200 205
   Ile Arg Cys Pro Trp Lys Arg Tyr Ile Thr Gln Ala Gln Met Leu Gln
   210 215 220
   Phe Val Ile Val Phe Ala His Ala Val Phe Val Leu Arg Gln Lys His
   225 230 235 240
   Cys Pro Val Thr Leu Pro Trp Ala Gln Met Phe Val Met Thr Asn Met
   245 250 255
   Leu Val Leu Phe Gly Asn Phe Tyr Leu Lys Ala Tyr Ser Asn Lys Ser
   260 265 270
   Arg Gly Asp Gly Ala Ser Ser Val Lys Pro Ala Glu Thr Thr Arg Ala
   275 280 285
   Pro Ser Val Arg Arg Thr Arg Ser Arg Lys Ile Asp
   290 295 300
<210> 116
   <211> 903
   <212> DNA
   <213> Ostreococcus tauri
<220>
   <221> CDS
   <222> (1)..(903)
   <223> delta-5-Elongase
<400> 116
   atg agc gcc tcc ggt gcg ctg ctg ccc gcg atc gcg ttc gcc gcg tac      48
   Met Ser Ala Ser Gly Ala Leu Leu Pro Ala Ile Ala Phe Ala Ala Tyr
   1               5                   10                  15
   gcg tac gcg acg tac gcc tac gcc ttt gag tgg tcg cac gcg aat ggc      96
   Ala Tyr Ala Thr Tyr Ala Tyr Ala Phe Glu Trp Ser His Ala Asn Gly
               20                  25                  30
   atc gac aac gtc gac gcg cgc gag tgg atc ggt gcg ctg tcg ttg agg     144
   Ile Asp Asn Val Asp Ala Arg Glu Trp Ile Gly Ala Leu Ser Leu Arg
           35                  40                  45
   ctc ccg gcg atc gcg acg acg atg tac ctg ttg ttc tgc ctg gtc gga     192
   Leu Pro Ala Ile Ala Thr Thr Met Tyr Leu Leu Phe Cys Leu Val Gly
       50                  55                  60
   ccg agg ttg atg gcg aag cgc gag gcg ttc gac ccg aag ggg ttc atg     240
   Pro Arg Leu Met Ala Lys Arg Glu Ala Phe Asp Pro Lys Gly Phe Met
   65                  70                  75                  80
   ctg gcg tac aat gcg tat cag acg gcg ttc aac gtc gtc gtg ctc ggg     288
   Leu Ala Tyr Asn Ala Tyr Gln Thr Ala Phe Asn Val Val Val Leu Gly
                   85                  90                  95
   atg ttc gcg cga gag atc tcg ggg ctg ggg cag ccc gtg tgg ggg tca     336
   Met Phe Ala Arg Glu Ile Ser Gly Leu Gly Gln Pro Val Trp Gly Ser
               100                 105                 110
   acc atg ccg tgg agc gat aga aaa tcg ttt aag atc ctc ctc ggg gtg     384
   Thr Met Pro Trp Ser Asp Arg Lys Ser Phe Lys Ile Leu Leu Gly Val
           115                 120                 125
   tgg ttg cac tac aac aac aaa tat ttg gag cta ttg gac act gtg ttc     432
   Trp Leu His Tyr Asn Asn Lys Tyr Leu Glu Leu Leu Asp Thr Val Phe
       130                 135                 140
   atg gtt gcg cgc aag aag acg aag cag ttg agc ttc ttg cac gtt tat     480
   Met Val Ala Arg Lys Lys Thr Lys Gln Leu Ser Phe Leu His Val Tyr
   145                 150                 155                 160
   cat cac gcc ctg ttg atc tgg gcg tgg tgg ttg gtg tgt cac ttg atg     528
   His His Ala Leu Leu Ile Trp Ala Trp Trp Leu Val Cys His Leu Met
                   165                 170                 175
   gcc acg aac gat tgt atc gat gcc tac ttc ggc gcg gcg tgc aac tcg     576
   Ala Thr Asn Asp Cys Ile Asp Ala Tyr Phe Gly Ala Ala Cys Asn Ser
               180                 185                 190
   ttc att cac atc gtg atg tac tcg tat tat ctc atg tcg gcg ctc ggc     624
   Phe Ile His Ile Val Met Tyr Ser Tyr Tyr Leu Met Ser Ala Leu Gly
           195                 200                 205
   att cga tgc ccg tgg aag cga tac atc acc cag gct caa atg ctc caa     672
   Ile Arg Cys Pro Trp Lys Arg Tyr Ile Thr Gln Ala Gln Met Leu Gln
       210                 215                 220
   ttc gtc att gtc ttc gcg cac gcc gtg ttc gtg ctg cgt cag aag cac     720
   Phe Val Ile Val Phe Ala His Ala Val Phe Val Leu Arg Gln Lys His
   225                 230                 235                 240
   tgc ccg gtc acc ctt cct tgg gcg caa atg ttc gtc atg acg aac atg     768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg     816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg     864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                 903
   Pro Ser Val Arg Arg Thr Arg Ser Arg Lys Ile Asp
       290                 295                 300
<210> 117
   <211> 300
   <212> PRT
   <213> Ostreococcus tauri
<400> 117
   Met Ser Ala Ser Gly Ala Leu Leu Pro Ala Ile Ala Phe Ala Ala Tyr
   1 5 10 15
   Ala Tyr Ala Thr Tyr Ala Tyr Ala Phe Glu Trp Ser His Ala Asn Gly
   20 25 30
   Ile Asp Asn Val Asp Ala Arg Glu Trp Ile Gly Ala Leu Ser Leu Arg
   35 40 45
   Leu Pro Ala Ile Ala Thr Thr Met Tyr Leu Leu Phe Cys Leu Val Gly
   50 55 60
   Pro Arg Leu Met Ala Lys Arg Glu Ala Phe Asp Pro Lys Gly Phe Met
   65 70 75 80
   Leu Ala Tyr Asn Ala Tyr Gln Thr Ala Phe Asn Val Val Val Leu Gly
   85 90 95
   Met Phe Ala Arg Glu Ile Ser Gly Leu Gly Gln Pro Val Trp Gly Ser
   100 105 110
   Thr Met Pro Trp Ser Asp Arg Lys Ser Phe Lys Ile Leu Leu Gly Val
   115 120 125
   Trp Leu His Tyr Asn Asn Lys Tyr Leu Glu Leu Leu Asp Thr Val Phe
   130 135 140
   Met Val Ala Arg Lys Lys Thr Lys Gln Leu Ser Phe Leu His Val Tyr
   145 150 155 160
   His His Ala Leu Leu Ile Trp Ala Trp Trp Leu Val Cys His Leu Met
   165 170 175
   Ala Thr Asn Asp Cys Ile Asp Ala Tyr Phe Gly Ala Ala Cys Asn Ser
   180 185 190
   Phe Ile His Ile Val Met Tyr Ser Tyr Tyr Leu Met Ser Ala Leu Gly
   195 200 205
   Ile Arg Cys Pro Trp Lys Arg Tyr Ile Thr Gln Ala Gln Met Leu Gln
   210 215 220
   Phe Val Ile Val Phe Ala His Ala Val Phe Val Leu Arg Gln Lys His
   225 230 235 240
   Cys Pro Val Thr Leu Pro Trp Ala Gln Met Phe Val Met Thr Asn Met
   245 250 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 118
   <211> 903
   <212> DNA
   <213> Ostreococcus tauri
<220>
   <221> CDS
   <222> (1)..(903)
   <223> delta-5-Elongase
<400> 118
   atg agc gcc tcc ggt gcg ctg ctg ccc gcg atc gcg tcc gcc gcg tac      48
   Met Ser Ala Ser Gly Ala Leu Leu Pro Ala Ile Ala Ser Ala Ala Tyr
   1               5                   10                  15
   gcg tac gcg acg tac gcc tac gcc ttt gag tgg tcg cac gcg aat ggc      96
   Ala Tyr Ala Thr Tyr Ala Tyr Ala Phe Glu Trp Ser His Ala Asn Gly
               20                  25                  30
   atc gac aac gtc gac gcg cgc gag tgg atc ggt gcg ctg tcg ttg agg     144
   Ile Asp Asn Val Asp Ala Arg Glu Trp Ile Gly Ala Leu Ser Leu Arg
           35                  40                  45
   ctc ccg gcg atc gcg acg acg atg tac ctg ttg ttc tgc ctg gtc gga     192
   Leu Pro Ala Ile Ala Thr Thr Met Tyr Leu Leu Phe Cys Leu Val Gly
       50                  55                  60
   ccg agg ttg atg gcg aag cgc gag gcg ttc gac ccg aag ggg ttc atg     240
   Pro Arg Leu Met Ala Lys Arg Glu Ala Phe Asp Pro Lys Gly Phe Met
   65                  70                  75                  80
   ctg gcg tac aat gcg tat cag acg gcg ttc aac gtc gtc gtg ctc ggg     288
   Leu Ala Tyr Asn Ala Tyr Gln Thr Ala Phe Asn Val Val Val Leu Gly
                   85                  90                  95
   atg ttc gcg cga gag atc tcg ggg ctg ggg cag ccc gtg tgg ggg tca     336
   Met Phe Ala Arg Glu Ile Ser Gly Leu Gly Gln Pro Val Trp Gly Ser
               100                 105                 110
   acc atg ccg tgg agc gat aga aaa tcg ttt aag atc ctc ctc ggg gtg     384
   Thr Met Pro Trp Ser Asp Arg Lys Ser Phe Lys Ile Leu Leu Gly Val
           115                 120                 125
   tgg ttg cac tac aac aac caa tat ttg gag cta ttg gac act gtg ttc     432
   Trp Leu His Tyr Asn Asn Gln Tyr Leu Glu Leu Leu Asp Thr Val Phe
       130                 135                 140
   atg gtt gcg cgc aag aag acg aag cag ttg agc ttc ttg cac gtt tat     480
   Met Val Ala Arg Lys Lys Thr Lys Gln Leu Ser Phe Leu His Val Tyr
   145                 150                 155                 160
   cat cac gcc ctg ttg atc tgg gcg tgg tgg ttg gtg tgt cac ttg atg     528
   His His Ala Leu Leu Ile Trp Ala Trp Trp Leu Val Cys His Leu Met
                   165                 170                 175
   gcc acg aac gat tgt atc gat gcc tac ttc ggc gcg gcg tgc aac tcg      576
   Ala Thr Asn Asp Cys Ile Asp Ala Tyr Phe Gly Ala Ala Cys Asn Ser
               180                 185                 190
   ttc att cac atc gtg atg tac tcg tat tat ctc atg tcg gcg ctc ggc      624
   Phe Ile His Ile Val Met Tyr Ser Tyr Tyr Leu Met Ser Ala Leu Gly
           195                 200                 205
   att cga tgc ccg tgg aag cga tac atc acc cag gct caa atg ctc caa      672
   Ile Arg Cys Pro Trp Lys Arg Tyr Ile Thr Gln Ala Gln Met Leu Gln
       210                 215                 220
   ttc gtc att gtc ttc gcg cac gcc gtg ttc gtg ctg cgt cag aag cac      720
   Phe Val Ile Val Phe Ala His Ala Val Phe Val Leu Arg Gln Lys His
   225                 230                 235                 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg      816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg      864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                  903
   Pro Ser Val Arg Arg Thr Arg Ser Arg Lys Ile Asp
       290                 295                 300
<210> 119
   <211> 300
   <212> PRT
   <213> Ostreococcus tauri
<400> 119
   Met Ser Ala Ser Gly Ala Leu Leu Pro Ala Ile Ala Ser Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
   115 120 125
   Trp Leu His Tyr Asn Asn Gln Tyr Leu Glu Leu Leu Asp Thr Val Phe
   130 135 140
   Met Val Ala Arg Lys Lys Thr Lys Gln Leu Ser Phe Leu His Val Tyr
   145 150 155 160
   His His Ala Leu Leu Ile Trp Ala Trp Trp Leu Val Cys His Leu Met
   165 170 175
   Ala Thr Asn Asp Cys Ile Asp Ala Tyr Phe Gly Ala Ala Cys Asn Ser
   180 185 190
   Phe Ile His Ile Val Met Tyr Ser Tyr Tyr Leu Met Ser Ala Leu Gly
   195 200 205
   Ile Arg Cys Pro Trp Lys Arg Tyr Ile Thr Gln Ala Gln Met Leu Gln
   210 215 220
   Phe Val Ile Val Phe Ala His Ala Val Phe Val Leu Arg Gln Lys His
   225 230 235 240
   Cys Pro Val Thr Leu Pro Trp Ala Gln Met Phe Val Met Thr Asn Met
   245 250 255
   Leu Val Leu Phe Gly Asn Phe Tyr Leu Lys Ala Tyr Ser Asn Lys Ser
   260 265 270
   Arg Gly Asp Gly Ala Ser Ser Val Lys Pro Ala Glu Thr Thr Arg Ala
   275 280 285
   Pro Ser Val Arg Arg Thr Arg Ser Arg Lys Ile Asp
   290 295 300
<210> 120
   <211> 831
   <212> DNA
   <213> Thraustochytrium sp.
<220>
   <221> CDS
   <222> (1)..(831)
   <223> delta-5-Elongase
<400> 120
   atg gac gtc gtc gag cag caa tgg cgc cgc ttc gtg gac gcc gtg gac      48
   Met Asp Val Val Glu Gln Gln Trp Arg Arg Phe Val Asp Ala Val Asp
   1               5                   10                  15
   aac gga atc gtg gag ttc atg gag cat gag aag ccc aac aag ctg aac      96
   Asn Gly Ile Val Glu Phe Met Glu His Glu Lys Pro Asn Lys Leu Asn
               20                  25                  30
   gag ggc aag ctc ttc acc tcg acc gag gag atg atg gcg ctt atc gtc      144
   Glu Gly Lys Leu Phe Thr Ser Thr Glu Glu Met Met Ala Leu Ile Val
           35                  40                  45
   ggc tac ctg gcg ttc gtg gtc ctc ggg tcc gcc ttc atg aag gcc ttt      192
   Gly Tyr Leu Ala Phe Val Val Leu Gly Ser Ala Phe Met Lys Ala Phe
       50                  55                  60
   gtc gat aag cct ttc gag ctc aag ttc ctc aag ctc gtg cac aac atc      240
   Val Asp Lys Pro Phe Glu Leu Lys Phe Leu Lys Leu Val His Asn Ile
   65                  70                  75                  80
   ttc ctc acc ggt ctg tcc atg tac atg gcc acc gag tgc gcg cgc cag      288
   Phe Leu Thr Gly Leu Ser Met Tyr Met Ala Thr Glu Cys Ala Arg Gln
                   85                  90                  95
   gca tac ctc ggc ggc tac aag ctc ttt ggc aac ccg atg gag aag ggc      336
   Ala Tyr Leu Gly Gly Tyr Lys Leu Phe Gly Asn Pro Met Glu Lys Gly
               100                 105                 110
   acc gag tcg cac gcc ccg ggc atg gcc aac atc atc tac atc ttc tac      384
   Thr Glu Ser His Ala Pro Gly Met Ala Asn Ile Ile Tyr Ile Phe Tyr
           115                 120                 125
   gtg agc aag ttc ctc gaa ttc ctc gac acc gtc ttc atg atc ctc ggc      432
   Val Ser Lys Phe Leu Glu Phe Leu Asp Thr Val Phe Met Ile Leu Gly
       130                 135                 140
   aag aag tgg aag cag ctc agc ttt ctc cac gtc tac cac cac gcg agc      480
   Lys Lys Trp Lys Gln Leu Ser Phe Leu His Val Tyr His His Ala Ser
   145                 150                 155                 160
   atc agc ttc atc tgg ggc atc atc gcc cgc ttc gcg ccc ggt ggc gac      528
   Ile Ser Phe Ile Trp Gly Ile Ile Ala Arg Phe Ala Pro Gly Gly Asp
                   165                 170                 175
   gcc tac ttc tct acc atc ctc aac agc agc gtg cat gtc gtg ctc tac      576
   Ala Tyr Phe Ser Thr Ile Leu Asn Ser Ser Val His Val Val Leu Tyr
               180                 185                 190
   ggc tac tac gcc tcg acc acc ctc ggc tac acc ttc atg cgc ccg ctg      624
   Gly Tyr Tyr Ala Ser Thr Thr Leu Gly Tyr Thr Phe Met Arg Pro Leu
           195                 200                 205
   cgc ccg tac att acc acc att cag ctc acg cag ttc atg gcc atg gtc      672
   Arg Pro Tyr Ile Thr Thr Ile Gln Leu Thr Gln Phe Met Ala Met Val
       210                 215                 220
   gtc cag tcc gtc tat gac tac tac aac ccc tgc gac tac ccg cag ccc      720
   Val Gln Ser Val Tyr Asp Tyr Tyr Asn Pro Cys Asp Tyr Pro Gln Pro
   225                 230                 235                 240
   ctc gtc aag ctg ctc ttc tgg tac atg ctc acc atg ctc ggc ctc ttc      768
   Leu Val Lys Leu Leu Phe Trp Tyr Met Leu Thr Met Leu Gly Leu Phe
                   245                 250                 255
   ggc aac ttc ttc gtg cag cag tac ctc aag ccc aag gcg ccc aag aag      816
   Gly Asn Phe Phe Val Gln Gln Tyr Leu Lys Pro Lys Ala Pro Lys Lys
               260                 265                 270
   cag aag acc atc taa                                                  831
   Gln Lys Thr Ile
           275
<210> 121
   <211> 276
   <212> PRT
   <213> Thraustochytrium sp.
<400> 121
   Met Asp Val Val Glu Gln Gln Trp Arg Arg Phe Val Asp Ala Val Asp
   1               5                   10                  15
   Asn Gly Ile Val Glu Phe Met Glu His Glu Lys Pro Asn Lys Leu Asn
               20                  25                  30
   Glu Gly Lys Leu Phe Thr Ser Thr Glu Glu Met Met Ala Leu Ile Val
           35                  40                  45
   Gly Tyr Leu Ala Phe Val Val Leu Gly Ser Ala Phe Met Lys Ala Phe
       50                  55                  60
   Val Asp Lys Pro Phe Glu Leu Lys Phe Leu Lys Leu Val His Asn Ile
   65                  70                  75                  80
   Phe Leu Thr Gly Leu Ser Met Tyr Met Ala Thr Glu Cys Ala Arg Gln
                   85                  90                  95
   Ala Tyr Leu Gly Gly Tyr Lys Leu Phe Gly Asn Pro Met Glu Lys Gly
               100                 105                 110
   Thr Glu Ser His Ala Pro Gly Met Ala Asn Ile Ile Tyr Ile Phe Tyr
           115                 120                 125
   Val Ser Lys Phe Leu Glu Phe Leu Asp Thr Val Phe Met Ile Leu Gly
       130                 135                 140
   Lys Lys Trp Lys Gln Leu Ser Phe Leu His Val Tyr His His Ala Ser
   145                 150                 155                 160
   Ile Ser Phe Ile Trp Gly Ile Ile Ala Arg Phe Ala Pro Gly Gly Asp
                   165                 170                 175
   Ala Tyr Phe Ser Thr Ile Leu Asn Ser Ser Val His Val Val Leu Tyr
               180                 185                 190
   Gly Tyr Tyr Ala Ser Thr Thr Leu Gly Tyr Thr Phe Met Arg Pro Leu
           195                 200                 205
   Arg Pro Tyr Ile Thr Thr Ile Gln Leu Thr Gln Phe Met Ala Met Val
       210                 215                 220
   Val Gln Ser Val Tyr Asp Tyr Tyr Asn Pro Cys Asp Tyr Pro Gln Pro
   225                 230                 235                 240
   Leu Val Lys Leu Leu Phe Trp Tyr Met Leu Thr Met Leu Gly Leu Phe
                   245                 250                 255
   Gly Asn Phe Phe Val Gln Gln Tyr Leu Lys Pro Lys Ala Pro Lys Lys
   260 265 270
   Gln Lys Thr Ile
   275
<210> 122
   <211> 1077
   <212> DNA
   <213> Thalassiosira pseudonana
<220>
   <221> CDS
   <222> (1)..(1077)
   <223> delta-5-Elongase
<400> 122
   atg tgc tca cca ccg ccg tca caa tcc aaa aca aca tcc ctc cta gca      48
   Met Cys Ser Pro Pro Pro Ser Gln Ser Lys Thr Thr Ser Leu Leu Ala
   1               5                   10                  15
   cgg tac acc acc gcc gcc ctc ctc ctc ctc acc ctc aca acg tgg tgc      96
   Arg Tyr Thr Thr Ala Ala Leu Leu Leu Leu Thr Leu Thr Thr Trp Cys
               20                  25                  30
   cac ttc gcc ttc cca gcc gcc acc gcc aca ccc ggc ctc acc gcc gaa      144
   His Phe Ala Phe Pro Ala Ala Thr Ala Thr Pro Gly Leu Thr Ala Glu
           35                  40                  45
   atg cac tcc tac aaa gtc cca ctc ggt ctc acc gta ttc tac ctg ctg      192
   Met His Ser Tyr Lys Val Pro Leu Gly Leu Thr Val Phe Tyr Leu Leu
       50                  55                  60
   agt cta ccg tca cta aag tac gtt acg gac aac tac ctt gcc aaa aag      240
   Ser Leu Pro Ser Leu Lys Tyr Val Thr Asp Asn Tyr Leu Ala Lys Lys
   65                  70                  75                  80
   tat gat atg aag tca ctc ctg acg gaa tca atg gtg ttg tac aat gtg      288
   Tyr Asp Met Lys Ser Leu Leu Thr Glu Ser Met Val Leu Tyr Asn Val
                   85                  90                  95
   gcg caa gtg ctg ctc aat ggg tgg acg gtg tat gcg att gtg gat gcg      336
   Ala Gln Val Leu Leu Asn Gly Trp Thr Val Tyr Ala Ile Val Asp Ala
               100                 105                 110
   gtg atg aat aga gac cat cct ttt att gga agt aga agt ttg gtt ggg      384
   Val Met Asn Arg Asp His Pro Phe Ile Gly Ser Arg Ser Leu Val Gly
           115                 120                 125
   gcg gcg ttg cat agt ggg agc tcg tat gcg gtg tgg gtt cat tat tgt      432
   Ala Ala Leu His Ser Gly Ser Ser Tyr Ala Val Trp Val His Tyr Cys
       130                 135                 140
   gat aag tat ttg gag ttc ttt gat acg tat ttt atg gtg ttg agg ggg      480
   Asp Lys Tyr Leu Glu Phe Phe Asp Thr Tyr Phe Met Val Leu Arg Gly
   145                 150                 155                 160
   aaa atg gac cag gtc tcc ttc ctc cac atc tac cac cac acg acc ata      528
   Lys Met Asp Gln Val Ser Phe Leu His Ile Tyr His His Thr Thr Ile
                   165                 170                 175
   gcg tgg gca tgg tgg atc gcc ctc cgc ttc tcc ccc ggc gga gac att      576
   Ala Trp Ala Trp Trp Ile Ala Leu Arg Phe Ser Pro Gly Gly Asp Ile
               180                 185                 190
   tac ttc ggg gca ctc ctc aac tcc atc atc cac gtc ctc atg tat tcc      624
   Tyr Phe Gly Ala Leu Leu Asn Ser Ile Ile His Val Leu Met Tyr Ser
           195                 200                 205
   tac tac gcc ctt gcc cta ctc aag gtc agt tgt cca tgg aaa cga tac      672
   Tyr Tyr Ala Leu Ala Leu Leu Lys Val Ser Cys Pro Trp Lys Arg Tyr
       210                 215                 220
   ttg act caa gct caa tta ttg caa ttc aca agt gtg gtg gtt tat acg      720
   Leu Thr Gln Ala Gln Leu Leu Gln Phe Thr Ser Val Val Val Tyr Thr
   225                 230                 235                 240
   ggg tgt acg ggt tat act cat tac tat cat acg aag cat gga gcg gat      768
   Gly Cys Thr Gly Tyr Thr His Tyr Tyr His Thr Lys His Gly Ala Asp
                   245                 250                 255
   gag aca cag cct agt tta gga acg tat tat ttc tgt tgt gga gtg cag      816
   Glu Thr Gln Pro Ser Leu Gly Thr Tyr Tyr Phe Cys Cys Gly Val Gln
               260                 265                 270
   gtg ttt gag atg gtt agt ttg ttt gta ctc ttt tcc atc ttt tat aaa      864
   Val Phe Glu Met Val Ser Leu Phe Val Leu Phe Ser Ile Phe Tyr Lys
           275                 280                 285
   cga tcc tat tcg aag aag aac aag tca gga gga aag gat agc aag aag      912
   Arg Ser Tyr Ser Lys Lys Asn Lys Ser Gly Gly Lys Asp Ser Lys Lys
       290                 295                 300
   aat gat gat ggg aat aat gag gat caa tgt cac aag gct atg aag gat      960
   Asn Asp Asp Gly Asn Asn Glu Asp Gln Cys His Lys Ala Met Lys Asp
   305                 310                 315                 320
   ata tcg gag ggt gcg aag gag gtt gtg ggg cat gca gcg aag gat gct     1008
   Ile Ser Glu Gly Ala Lys Glu Val Val Gly His Ala Ala Lys Asp Ala
                   325                 330                 335
   gga aag ttg gtg gct acg gcg agt aag gct gta aag agg aag gga act     1056
   Gly Lys Leu Val Ala Thr Ala Ser Lys Ala Val Lys Arg Lys Gly Thr
               340                 345                 350
   cgt gtt act ggt gcc atg tag                                         1077
   Arg Val Thr Gly Ala Met
           355
<210> 123
   <211> 358
   <212> PRT
   <213> Thalassiosira pseudonana
<400> 123
   Met Cys Ser Pro Pro Pro Ser Gln Ser Lys Thr Thr Ser Leu Leu Ala
   1               5                   10                  15
   Arg Tyr Thr Thr Ala Ala Leu Leu Leu Leu Thr Leu Thr Thr Trp Cys
               20                  25                  30
   His Phe Ala Phe Pro Ala Ala Thr Ala Thr Pro Gly Leu Thr Ala Glu
           35                  40                  45
   Met His Ser Tyr Lys Val Pro Leu Gly Leu Thr Val Phe Tyr Leu Leu
       50                  55                  60
   Ser Leu Pro Ser Leu Lys Tyr Val Thr Asp Asn Tyr Leu Ala Lys Lys
   65 70 75 80
   Tyr Asp Met Lys Ser Leu Leu Thr Glu Ser Met Val Leu Tyr Asn Val
   85 90 95
   Ala Gln Val Leu Leu Asn Gly Trp Thr Val Tyr Ala Ile Val Asp Ala
   100 105 110
   Val Met Asn Arg Asp His Pro Phe Ile Gly Ser Arg Ser Leu Val Gly
   115 120 125
   Ala Ala Leu His Ser Gly Ser Ser Tyr Ala Val Trp Val His Tyr Cys
   130 135 140
   Asp Lys Tyr Leu Glu Phe Phe Asp Thr Tyr Phe Met Val Leu Arg Gly
   145 150 155 160
   Lys Met Asp Gln Val Ser Phe Leu His Ile Tyr His His Thr Thr Ile
   165 170 175
   Ala Trp Ala Trp Trp Ile Ala Leu Arg Phe Ser Pro Gly Gly Asp Ile
   180 185 190
   Tyr Phe Gly Ala Leu Leu Asn Ser Ile Ile His Val Leu Met Tyr Ser
   195 200 205
   Tyr Tyr Ala Leu Ala Leu Leu Lys Val Ser Cys Pro Trp Lys Arg Tyr
   210 215 220
   Leu Thr Gln Ala Gln Leu Leu Gln Phe Thr Ser Val Val Val Tyr Thr
   225 230 235 240
   Gly Cys Thr Gly Tyr Thr His Tyr Tyr His Thr Lys His Gly Ala Asp
   245 250 255
   Glu Thr Gln Pro Ser Leu Gly Thr Tyr Tyr Phe Cys Cys Gly Val Gln
   260 265 270
   Val Phe Glu Met Val Ser Leu Phe Val Leu Phe Ser Ile Phe Tyr Lys
   275 280 285
   Arg Ser Tyr Ser Lys Lys Asn Lys Ser Gly Gly Lys Asp Ser Lys Lys
   290 295 300
   Asn Asp Asp Gly Asn Asn Glu Asp Gln Cys His Lys Ala Met Lys Asp
   305 310 315 320
   Ile Ser Glu Gly Ala Lys Glu Val Val Gly His Ala Ala Lys Asp Ala
   325 330 335
   Gly Lys Leu Val Ala Thr Ala Ser Lys Ala Val Lys Arg Lys Gly Thr
   340 345 350
   Arg Val Thr Gly Ala Met
   355
<210> 124
   <211> 903
   <212> DNA
   <213> Ostreococcus tauri
<220>
   <221> CDS
   <222> (1)..(903)
   <223> delta-5-Elongase
<400> 124
   atg agc gcc tcc ggt gcg ctg ctg ccc gcg atc gcg ttc gcc gcg tac 48
   Met Ser Ala Ser Gly Ala Leu Leu Pro Ala Ile Ala Phe Ala Ala Tyr
   1 5 10 15
   gcg tac gcg acg tac gcc tac gcc ttt gag tgg tcg cac gcg aat ggc 96
   Ala Tyr Ala Thr Tyr Ala Tyr Ala Phe Glu Trp Ser His Ala Asn Gly
   20 25 30
   atc gac aac gtc gac gcg cgc gag tgg atc ggt gcg ctg tcg ttg agg 144
   Ile Asp Asn Val Asp Ala Arg Glu Trp Ile Gly Ala Leu Ser Leu Arg
   35 40 45
   ctc ccg gcg atc gcg acg acg atg tac ctg ttg ttc tgc ctg gtc gga 192
   Leu Pro Ala Ile Ala Thr Thr Met Tyr Leu Leu Phe Cys Leu Val Gly
   50 55 60
   ccg agg ttg atg gcg aag cgc gag gcg ttc gac ccg aag ggg ttc atg 240
   Pro Arg Leu Met Ala Lys Arg Glu Ala Phe Asp Pro Lys Gly Phe Met
   65 70 75 80
   ctg gcg tac aat gcg tat cag acg gcg ttc aac gtc gtc gtg ctc ggg 288
   Leu Ala Tyr Asn Ala Tyr Gln Thr Ala Phe Asn Val Val Val Leu Gly
   85 90 95
   atg ttc gcg cga gag atc tcg ggg ctg ggg cag ccc gtg tgg ggg tca 336
   Met Phe Ala Arg Glu Ile Ser Gly Leu Gly Gln Pro Val Trp Gly Ser
   100 105 110
   acc atg ccg tgg agc gat aga aaa tcg ttt aag atc ctc ctc ggg gtg 384
   Thr Met Pro Trp Ser Asp Arg Lys Ser Phe Lys Ile Leu Leu Gly Val
   115 120 125
   tgg ttg cac tac aac aac aaa tat ttg gag cta ttg gac act gtg ttc 432
   Trp Leu His Tyr Asn Asn Lys Tyr Leu Glu Leu Leu Asp Thr Val Phe
   130 135 140
   atg gtt gcg cgc aag aag acg aag cag ttg agc ttc ttg cac gtt tat 480
   Met Val Ala Arg Lys Lys Thr Lys Gln Leu Ser Phe Leu His Val Tyr
   145 150 155 160
   cat cac gcc ctg ttg atc tgg gcg tgg tgg ttg gtg tgt cac ttg atg 528
   His His Ala Leu Leu Ile Trp Ala Trp Trp Leu Val Cys His Leu Met
   165 170 175
   gcc acg aac gat tgt atc gat gcc tac ttc ggc gcg gcg tgc aac tcg 576
   Ala Thr Asn Asp Cys Ile Asp Ala Tyr Phe Gly Ala Ala Cys Asn Ser
   180 185 190
   ttc att cac atc gtg atg tac tcg tat tat ctc atg tcg gcg ctc ggc      624
   Phe Ile His Ile Val Met Tyr Ser Tyr Tyr Leu Met Ser Ala Leu Gly
           195                 200                     205
   att cga tgc ccg tgg aag cga tac atc acc cag gct caa atg ctc caa      672
   Ile Arg Cys Pro Trp Lys Arg Tyr Ile Thr Gln Ala Gln Met Leu Gln
       210                 215                     220
   ttc gtc att gtc ttc gcg cac gcc gtg ttc gtg ctg cgt cag aag cac      720
   Phe Val Ile Val Phe Ala His Ala Val Phe Val Leu Arg Gln Lys His
   225                     230                     235                 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                     250                     255
   ctc gtg ctc ttc ggg aac ttc tac ctc aag gcg tac tcg aac aag tcg      816
   Leu Val Leu Phe Gly Asn Phe Tyr Leu Lys Ala Tyr Ser Asn Lys Ser
               260                     265                     270
   cgc ggc gac ggc gcg agt tcc gtg aaa cca gcc gag acc acg cgc gcg      864
   Arg Gly Asp Gly Ala Ser Ser Val Lys Pro Ala Glu Thr Thr Arg Ala
           275                     280                     285
   ccc agc gtg cga cgc acg cga tct cga aaa att gac taa                  903
   Pro Ser Val Arg Arg Thr Arg Ser Arg Lys Ile Asp
       290                     295                     300
<210> 125
   <211> 300
   <212> PRT
   <213> Ostreococcus tauri
<400> 125
   Met Ser Ala Ser Gly Ala Leu Leu Pro Ala Ile Ala Phe Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Lys Tyr Leu Glu Leu Leu Asp Thr Val Phe
   130 135 140
   Met Val Ala Arg Lys Lys Thr Lys Gln Leu Ser Phe Leu His Val Tyr
   145 150 155 160
   His His Ala Leu Leu Ile Trp Ala Trp Trp Leu Val Cys His Leu Met
   165 170 175
   Ala Thr Asn Asp Cys Ile Asp Ala Tyr Phe Gly Ala Ala Cys Asn Ser
   180 185 190
   Phe Ile His Ile Val Met Tyr Ser Tyr Tyr Leu Met Ser Ala Leu Gly
   195 200 205
   Ile Arg Cys Pro Trp Lys Arg Tyr Ile Thr Gln Ala Gln Met Leu Gln
   210 215 220
   Phe Val Ile Val Phe Ala His Ala Val Phe Val Leu Arg Gln Lys His
   225 230 235 240
   Cys Pro Val Thr Leu Pro Trp Ala Gln Met Phe Val Met Thr Asn Met
   245 250 255
   Leu Val Leu Phe Gly Asn Phe Tyr Leu Lys Ala Tyr Ser Asn Lys Ser
   260 265 270
   Arg Gly Asp Gly Ala Ser Ser Val Lys Pro Ala Glu Thr Thr Arg Ala
   275 280 285
   Pro Ser Val Arg Arg Thr Arg Ser Arg Lys Ile Asp
   290 295 300
<210> 126
   <211> 909
   <212> DNA
   <213> Xenopus laevis
<220>
   <221> CDS
   <222> (1)..(909)
   <223> delta-5-Elongase
<400> 126
   atg gcc ttc aag gag ctc aca tca agg gca gtg ctc ctg tat gat gaa      48
   Met Ala Phe Lys Glu Leu Thr Ser Arg Ala Val Leu Leu Tyr Asp Glu
   1               5                   10                  15
   tgg att aaa gat gct gat cct agg gtt gaa gac tgg cca ctc atg tcc      96
   Trp Ile Lys Asp Ala Asp Pro Arg Val Glu Asp Trp Pro Leu Met Ser
               20                  25                  30
   tct cct atc cta caa acc atc atc atc ggc gct tac atc tac ttt gtc     144
   Ser Pro Ile Leu Gln Thr Ile Ile Ile Gly Ala Tyr Ile Tyr Phe Val
           35                  40                  45
   aca tca ttg ggc cca agg atc atg gag aac agg aag ccg ttt gct ctg      192
   Thr Ser Leu Gly Pro Arg Ile Met Glu Asn Arg Lys Pro Phe Ala Leu
       50                  55                  60
   aag gag atc atg gca tgt tac aac tta ttc atg gtt ctg ttt tct gtg      240
   Lys Glu Ile Met Ala Cys Tyr Asn Leu Phe Met Val Leu Phe Ser Val
   65                  70                  75                  80
   tac atg tgc tat gag ttt ctc atg tcg ggc tgg gct act gga tat tcc      288
   Tyr Met Cys Tyr Glu Phe Leu Met Ser Gly Trp Ala Thr Gly Tyr Ser
                   85                  90                  95
   ttt aga tgt gac att gtt gac tac tct cag tca cct cag gcg tta cgg      336
   Phe Arg Cys Asp Ile Val Asp Tyr Ser Gln Ser Pro Gln Ala Leu Arg
               100                 105                 110
   atg gcc tgg acc tgc tgg ctc ttc tat ttt tca aag ttc att gaa tta      384
   Met Ala Trp Thr Cys Trp Leu Phe Tyr Phe Ser Lys Phe Ile Glu Leu
           115                 120                 125
   tta gac act gtt ttc ttt gtg ctg cgt aag aag aac agc cag att aca      432
   Leu Asp Thr Val Phe Phe Val Leu Arg Lys Lys Asn Ser Gln Ile Thr
       130                 135                 140
   ttc ctg cac gtc tat cac cac tcc att atg cct tgg acg tgg tgg ttt      480
   Phe Leu His Val Tyr His His Ser Ile Met Pro Trp Thr Trp Trp Phe
   145                 150                 155                 160
   gga gtc aaa ttt gct cca ggt ggt ttg ggc aca ttc cat gca ctg gtg      528
   Gly Val Lys Phe Ala Pro Gly Gly Leu Gly Thr Phe His Ala Leu Val
                   165                 170                 175
   aac tgt gtg gtc cat gtt atc atg tac agc tac tac ggc ctg tca gcc      576
   Asn Cys Val Val His Val Ile Met Tyr Ser Tyr Tyr Gly Leu Ser Ala
               180                 185                 190
   ttg ggg cct gcc tac cag aag tac ctg tgg tgg aaa aag tac atg acg      624
   Leu Gly Pro Ala Tyr Gln Lys Tyr Leu Trp Trp Lys Lys Tyr Met Thr
           195                 200                 205
   tct atc caa ctg acc cag ttc ttg atg gtt act ttt cac atc ggc cag      672
   Ser Ile Gln Leu Thr Gln Phe Leu Met Val Thr Phe His Ile Gly Gln
       210                 215                 220
   ttc ttc ttc atg gag aat tgc ccg tac cag tat ccc gtc ttc ttg tat      720
   Phe Phe Phe Met Glu Asn Cys Pro Tyr Gln Tyr Pro Val Phe Leu Tyr
   225                 230                 235                 240
   gtc att tgg ctg tac ggg ttc gtt ttc tta atc ttg ttc ctc aac ttc      768
   Val Ile Trp Leu Tyr Gly Phe Val Phe Leu Ile Leu Phe Leu Asn Phe
                   245                 250                 255
   tgg ttc cac gct tac atc aaa gga cag agg ctg ccg aaa gcc gtc caa      816
   Trp Phe His Ala Tyr Ile Lys Gly Gln Arg Leu Pro Lys Ala Val Gln
               260                 265                 270
   aat ggc cac tgc aag aac aac aac aac caa gaa aac act tgg tgc aag      864
   Asn Gly His Cys Lys Asn Asn Asn Asn Gln Glu Asn Thr Trp Cys Lys
           275                 280                 285
   aac aaa aac cag aaa aac ggt gca ttg aaa agc aaa aac cat tga          909
   Asn Lys Asn Gln Lys Asn Gly Ala Leu Lys Ser Lys Asn His
       290                 295                 300
<210> 127
   <211> 302
   <212> PRT
   <213> Xenopus laevis
<400> 127
   Met Ala Phe Lys Glu Leu Thr Ser Arg Ala Val Leu Leu Tyr Asp Glu
   1               5                   10                  15
   Trp Ile Lys Asp Ala Asp Pro Arg Val Glu Asp Trp Pro Leu Met Ser
               20                  25                  30
   Ser Pro Ile Leu Gln Thr Ile Ile Ile Gly Ala Tyr Ile Tyr Phe Val
           35                  40                  45
   Thr Ser Leu Gly Pro Arg Ile Met Glu Asn Arg Lys Pro Phe Ala Leu
       50                  55                  60
   Lys Glu Ile Met Ala Cys Tyr Asn Leu Phe Met Val Leu Phe Ser Val
   65                  70                  75                  80
   Tyr Met Cys Tyr Glu Phe Leu Met Ser Gly Trp Ala Thr Gly Tyr Ser
                   85                  90                  95
   Phe Arg Cys Asp Ile Val Asp Tyr Ser Gln Ser Pro Gln Ala Leu Arg
               100                 105                 110
   Met Ala Trp Thr Cys Trp Leu Phe Tyr Phe Ser Lys Phe Ile Glu Leu
           115                 120                 125
   Leu Asp Thr Val Phe Phe Val Leu Arg Lys Lys Asn Ser Gln Ile Thr
       130                 135                 140
   Phe Leu His Val Tyr His His Ser Ile Met Pro Trp Thr Trp Trp Phe
   145                 150                 155                 160
   Gly Val Lys Phe Ala Pro Gly Gly Leu Gly Thr Phe His Ala Leu Val
                   165                 170                 175
   Asn Cys Val Val His Val Ile Met Tyr Ser Tyr Tyr Gly Leu Ser Ala
               180                 185                 190
   Leu Gly Pro Ala Tyr Gln Lys Tyr Leu Trp Trp Lys Lys Tyr Met Thr
           195                 200                 205
   Ser Ile Gln Leu Thr Gln Phe Leu Met Val Thr Phe His Ile Gly Gln
       210                 215                 220
   Phe Phe Phe Met Glu Asn Cys Pro Tyr Gln Tyr Pro Val Phe Leu Tyr
   225                 230                 235                 240
   Val Ile Trp Leu Tyr Gly Phe Val Phe Leu Ile Leu Phe Leu Asn Phe
                   245                 250                 255
   Trp Phe His Ala Tyr Ile Lys Gly Gln Arg Leu Pro Lys Ala Val Gln
               260                 265                 270
   Asn Gly His Cys Lys Asn Asn Asn Asn Gln Glu Asn Thr Trp Cys Lys
           275                 280                 285
   Asn Lys Asn Gln Lys Asn Gly Ala Leu Lys Ser Lys Asn His
       290                 295                 300
<210> 128
   <211> 870
   <212> DNA
   <213> Ciona intestinalis
<220>
   <221> CDS
   <222> (1)..(870)
   <223> delta-5-Elongase
<400> 128
   atg gac gta ctt cat cgt ttc tta gga ttc tac gaa tgg acg ctg act      48
   Met Asp Val Leu His Arg Phe Leu Gly Phe Tyr Glu Trp Thr Leu Thr
   1               5                   10                  15
   ttc gcg gac ccc cga gtg gca aaa tgg cct tta ata gaa aac ccc ctt      96
   Phe Ala Asp Pro Arg Val Ala Lys Trp Pro Leu Ile Glu Asn Pro Leu
               20                  25                  30
   cct aca att gct att gtg ttg ctg tac ctg gcg ttt gtt ctg tat att      144
   Pro Thr Ile Ala Ile Val Leu Leu Tyr Leu Ala Phe Val Leu Tyr Ile
           35                  40                  45
   ggg ccg cgt ttt atg cga aaa aga gca cca gtt gac ttt ggt tta ttc      192
   Gly Pro Arg Phe Met Arg Lys Arg Ala Pro Val Asp Phe Gly Leu Phe
       50                  55                  60
   ctc cct gga tat aac ttt gct ttg gtt gca tta aat tat tat atc ctg      240
   Leu Pro Gly Tyr Asn Phe Ala Leu Val Ala Leu Asn Tyr Tyr Ile Leu
   65                  70                  75                  80
   caa gaa gtg gtc act ggg agt tat ggg gct ggg tat gat ttg gtt tgc      288
   Gln Glu Val Val Thr Gly Ser Tyr Gly Ala Gly Tyr Asp Leu Val Cys
                   85                  90                  95
   aca cca ctt cga agt gat tcc tac gat ccc aat gaa atg aag gtt gca      336
   Thr Pro Leu Arg Ser Asp Ser Tyr Asp Pro Asn Glu Met Lys Val Ala
               100                 105                 110
   aac gct gta tgg tgg tat tat gta tcc aag ata ata gag ttg ttt gat      384
   Asn Ala Val Trp Trp Tyr Tyr Val Ser Lys Ile Ile Glu Leu Phe Asp
           115                 120                 125
   act gtg ttg ttc act cta cgc aaa cga gac cga caa gta act ttc ctt      432
   Thr Val Leu Phe Thr Leu Arg Lys Arg Asp Arg Gln Val Thr Phe Leu
       130                 135                 140
   cat gtt tat cac cat tct acc atg ccc ctg ttg tgg tgg att ggg gca      480
   His Val Tyr His His Ser Thr Met Pro Leu Leu Trp Trp Ile Gly Ala
   145                 150                 155                 160
   aag tgg gtg cct ggt ggg caa tca ttt gtt ggc atc ata ctg aac tcc      528
   Lys Trp Val Pro Gly Gly Gln Ser Phe Val Gly Ile Ile Leu Asn Ser
                   165                 170                 175
   agt gtt cat gtt atc atg tat acg tac tat gga ttg tca gcc ttg ggg      576
   Ser Val His Val Ile Met Tyr Thr Tyr Tyr Gly Leu Ser Ala Leu Gly
               180                 185                 190
   cct cac atg cag aag ttt cta tgg tgg aag aaa tat atc aca atg ttg      624
   Pro His Met Gln Lys Phe Leu Trp Trp Lys Lys Tyr Ile Thr Met Leu
           195                 200                 205
   caa ctg gtt caa ttt gtt ctt gcc atc tac cat act gct cga tca ttg      672
   Gln Leu Val Gln Phe Val Leu Ala Ile Tyr His Thr Ala Arg Ser Leu
       210                 215                 220
   tac gtt aaa tgt ccc tcg cct gtt tgg atg cac tgg gca ctt atc ttg      720
   Tyr Val Lys Cys Pro Ser Pro Val Trp Met His Trp Ala Leu Ile Leu
   225                 230                 235                 240
   tac gct ttc tca ttc att ttg ctt ttc tca aac ttc tac atg cat gcc      768
   Tyr Ala Phe Ser Phe Ile Leu Leu Phe Ser Asn Phe Tyr Met His Ala
                   245                 250                 255
   tat atc aag aaa tca aga aaa ggg aaa gag aat ggc agt cga gga aaa      816
   Tyr Ile Lys Lys Ser Arg Lys Gly Lys Glu Asn Gly Ser Arg Gly Lys
               260                 265                 270
   ggt ggt gta agt aat gga aag gaa aag ctg cac gct aat ggt aaa acc      864
   Gly Gly Val Ser Asn Gly Lys Glu Lys Leu His Ala Asn Gly Lys Thr
           275                 280                 285
   gat taa                                                              870
   Asp
<210> 129
   <211> 289
   <212> PRT
   <213> Ciona intestinalis
<400> 129
   Met Asp Val Leu His Arg Phe Leu Gly Phe Tyr Glu Trp Thr Leu Thr
   1               5                   10                  15
   Phe Ala Asp Pro Arg Val Ala Lys Trp Pro Leu Ile Glu Asn Pro Leu
               20                  25                  30
   Pro Thr Ile Ala Ile Val Leu Leu Tyr Leu Ala Phe Val Leu Tyr Ile
           35                  40                  45
   Gly Pro Arg Phe Met Arg Lys Arg Ala Pro Val Asp Phe Gly Leu Phe
       50                  55                  60
   Leu Pro Gly Tyr Asn Phe Ala Leu Val Ala Leu Asn Tyr Tyr Ile Leu
   65                  70                  75                  80
   Gln Glu Val Val Thr Gly Ser Tyr Gly Ala Gly Tyr Asp Leu Val Cys
                   85                  90                  95
   Thr Pro Leu Arg Ser Asp Ser Tyr Asp Pro Asn Glu Met Lys Val Ala
               100                 105                 110
   Asn Ala Val Trp Trp Tyr Tyr Val Ser Lys Ile Ile Glu Leu Phe Asp
           115                 120                 125
   Thr Val Leu Phe Thr Leu Arg Lys Arg Asp Arg Gln Val Thr Phe Leu
       130                 135                 140
   His Val Tyr His His Ser Thr Met Pro Leu Leu Trp Trp Ile Gly Ala
   145                 150                 155                 160
   Lys Trp Val Pro Gly Gly Gln Ser Phe Val Gly Ile Ile Leu Asn Ser
                   165                 170                 175
   Ser Val His Val Ile Met Tyr Thr Tyr Tyr Gly Leu Ser Ala Leu Gly
               180                 185                 190
   Pro His Met Gln Lys Phe Leu Trp Trp Lys Lys Tyr Ile Thr Met Leu
           195                 200                 205
   Gln Leu Val Gln Phe Val Leu Ala Ile Tyr His Thr Ala Arg Ser Leu
       210                 215                 220
   Tyr Val Lys Cys Pro Ser Pro Val Trp Met His Trp Ala Leu Ile Leu
   225                 230                 235                 240
   Tyr Ala Phe Ser Phe Ile Leu Leu Phe Ser Asn Phe Tyr Met His Ala
                   245                 250                 255
   Tyr Ile Lys Lys Ser Arg Lys Gly Lys Glu Asn Gly Ser Arg Gly Lys
               260                 265                 270
   Gly Gly Val Ser Asn Gly Lys Glu Lys Leu His Ala Asn Gly Lys Thr
           275                 280                 285
   Asp
<210> 130
   <211> 789
   <212> DNA
   <213> Euglena gracilis
<220>
   <221> CDS
   <222> (1)..(789)
   <223> delta-5-Elongase
<400> 130
   atg ctg ggg gcc atc gcg gac gtc gtg ctc cgg ggg ccc gcc gca ttc      48
   Met Leu Gly Ala Ile Ala Asp Val Val Leu Arg Gly Pro Ala Ala Phe
   1               5                   10                  15
   cac tgg gac cct gcc acc acc ccg ctc gca tcg atc gtc agc ccc tgt      96
   His Trp Asp Pro Ala Thr Thr Pro Leu Ala Ser Ile Val Ser Pro Cys
               20                  25                  30
   gtg gcc tcc gtg gcg tac ctg ggg gcc atc ggg ctg ctg aag cgc cgc      144
   Val Ala Ser Val Ala Tyr Leu Gly Ala Ile Gly Leu Leu Lys Arg Arg
           35                  40                  45
   act gga ccg gag gtc cgc tcc aag ccc ttc gag ctg cta cac aac ggg      192
   Thr Gly Pro Glu Val Arg Ser Lys Pro Phe Glu Leu Leu His Asn Gly
       50                  55                  60
   ctg ctg gtg ggc tgg tcc ctc gtg gtg ctg ctc ggg acg ctg tac ggc      240
   Leu Leu Val Gly Trp Ser Leu Val Val Leu Leu Gly Thr Leu Tyr Gly
   65                  70                  75                  80
   gcg ttc cag cgc gtg cag gag gac ggc cgg ggg gtg cag gcc ctc ctg      288
   Ala Phe Gln Arg Val Gln Glu Asp Gly Arg Gly Val Gln Ala Leu Leu
                   85                  90                  95
   tgc acc cag cgg cca cca tct cag atc tgg gac ggc ccg gtg ggg tac      336
   Cys Thr Gln Arg Pro Pro Ser Gln Ile Trp Asp Gly Pro Val Gly Tyr
               100                 105                 110
   ttc acg tac ctc ttc tac ctc gcg aag tac tgg gag ctg gcg gac act      384
   Phe Thr Tyr Leu Phe Tyr Leu Ala Lys Tyr Trp Glu Leu Ala Asp Thr
           115                 120                 125
   gtc atc ctc gcc ctc cgc cag aag ccc acc atc ccc ctc cac gtc tac      432
   Val Ile Leu Ala Leu Arg Gln Lys Pro Thr Ile Pro Leu His Val Tyr
       130                 135                 140
   cat cac gcc gtc atg ctg ttc atc gtg tgg tcg tgg ttc gcg cac ccc      480
   His His Ala Val Met Leu Phe Ile Val Trp Ser Trp Phe Ala His Pro
   145                 150                 155                 160
   tgg ctc gag ggg agc tgg tgg tgc tcc ctg gtc aac tct ttc atc cac      528
   Trp Leu Glu Gly Ser Trp Trp Cys Ser Leu Val Asn Ser Phe Ile His
                   165                 170                 175
   acg gtg atg tac tcg tac tac acc ctg acg gtg gtt ggc atc aac cct      576
   Thr Val Met Tyr Ser Tyr Tyr Thr Leu Thr Val Val Gly Ile Asn Pro
               180                 185                 190
   tgg tgg aag aag tgg atg acc acc atg cag atc atc cag ttc atc acg      624
   Trp Trp Lys Lys Trp Met Thr Thr Met Gln Ile Ile Gln Phe Ile Thr
           195                 200                 205
   ggc tgc gtg tac gtc atg gcg ttc ttc ggc cta tat tat gcc ggg gcg      672
   Gly Cys Val Tyr Val Met Ala Phe Phe Gly Leu Tyr Tyr Ala Gly Ala
       210                 215                 220
   ggc tgc acc tcc aac gtg tac act gcc tgg ttc tcg atg ggg gtc aac      720
   Gly Cys Thr Ser Asn Val Tyr Thr Ala Trp Phe Ser Met Gly Val Asn
   225                 230                 235                 240
   ctc agc ttt ctg tgg ctc ttc gct ctt ttc ttc cgc cgg tca tac agc      768
   Leu Ser Phe Leu Trp Leu Phe Ala Leu Phe Phe Arg Arg Ser Tyr Ser
                   245                 250                 255
   aaa cct agc cgg aag gag tag                                          789
   Lys Pro Ser Arg Lys Glu
               260
<210> 131
   <211> 262
   <212> PRT
   <213> Euglena gracilis
<400> 131
   Met Leu Gly Ala Ile Ala Asp Val Val Leu Arg Gly Pro Ala Ala Phe
   1               5                   10                  15
   His Trp Asp Pro Ala Thr Thr Pro Leu Ala Ser Ile Val Ser Pro Cys
               20                  25                  30
   Val Ala Ser Val Ala Tyr Leu Gly Ala Ile Gly Leu Leu Lys Arg Arg
           35                  40                  45
   Thr Gly Pro Glu Val Arg Ser Lys Pro Phe Glu Leu Leu His Asn Gly
       50                  55                  60
   Leu Leu Val Gly Trp Ser Leu Val Val Leu Leu Gly Thr Leu Tyr Gly
   65                  70                  75                  80
   Ala Phe Gln Arg Val Gln Glu Asp Gly Arg Gly Val Gln Ala Leu Leu
                   85                  90                  95
   Cys Thr Gln Arg Pro Pro Ser Gln Ile Trp Asp Gly Pro Val Gly Tyr
               100                 105                 110
   Phe Thr Tyr Leu Phe Tyr Leu Ala Lys Tyr Trp Glu Leu Ala Asp Thr
           115                 120                 125
   Val Ile Leu Ala Leu Arg Gln Lys Pro Thr Ile Pro Leu His Val Tyr
       130                 135                 140
   His His Ala Val Met Leu Phe Ile Val Trp Ser Trp Phe Ala His Pro
   145                 150                 155                 160
   Trp Leu Glu Gly Ser Trp Trp Cys Ser Leu Val Asn Ser Phe Ile His
                   165                 170                 175
   Thr Val Met Tyr Ser Tyr Tyr Thr Leu Thr Val Val Gly Ile Asn Pro
               180                 185                 190
   Trp Trp Lys Lys Trp Met Thr Thr Met Gln Ile Ile Gln Phe Ile Thr
           195                 200                 205
   Gly Cys Val Tyr Val Met Ala Phe Phe Gly Leu Tyr Tyr Ala Gly Ala
       210                 215                 220
   Gly Cys Thr Ser Asn Val Tyr Thr Ala Trp Phe Ser Met Gly Val Asn
   225                 230                 235                 240
   Leu Ser Phe Leu Trp Leu Phe Ala Leu Phe Phe Arg Arg Ser Tyr Ser
                   245                 250                 255
   Lys Pro Ser Arg Lys Glu
               260
<210> 132
   <211> 789
   <212> DNA
   <213> Euglena gracilis
<220>
   <221> CDS
   <222> (1)..(789)
   <223> delta-5-Elongase
<400> 132
   atg ctg ggg gcc atc gcg gac gtc gtg ctc cgg ggg ccc gcc gca ttc      48
   Met Leu Gly Ala Ile Ala Asp Val Val Leu Arg Gly Pro Ala Ala Phe
   1               5                   10                  15
   cac tgg gac cct gcc acc acc ccg ctc gca tcg atc gtc agc ccc tgt      96
   His Trp Asp Pro Ala Thr Thr Pro Leu Ala Ser Ile Val Ser Pro Cys
               20                  25                  30
   gtg gcc tcc gtg gcg tac ctg ggg gcc atc ggg ctg ctg aag cgc cgc     144
   Val Ala Ser Val Ala Tyr Leu Gly Ala Ile Gly Leu Leu Lys Arg Arg
           35                  40                  45
   act gga ccg gag gtc cgc tcc aag ccc ttc gag ctg cta cac aac ggg     192
   Thr Gly Pro Glu Val Arg Ser Lys Pro Phe Glu Leu Leu His Asn Gly
       50                  55                  60
   ctg ctg gtg ggc tgg tcc ctc gtg gtg ctg ctc ggg acg ctg tac ggc     240
   Leu Leu Val Gly Trp Ser Leu Val Val Leu Leu Gly Thr Leu Tyr Gly
   65                  70                  75                  80
   gcg tac cag cgc gtg cag gag gac ggc cgg ggg gtg cag gcc ctg ctg     288
   Ala Tyr Gln Arg Val Gln Glu Asp Gly Arg Gly Val Gln Ala Leu Leu
                   85                  90                  95
   tgc acc cag cgg cca cca tct cag atc tgg gac ggc ccg gtg ggg tac     336
   Cys Thr Gln Arg Pro Pro Ser Gln Ile Trp Asp Gly Pro Val Gly Tyr
               100                 105                 110
   ttc acg tac ctt ttc tac ctc gcg aag tac tgg gag ctg gtg gac act     384
   Phe Thr Tyr Leu Phe Tyr Leu Ala Lys Tyr Trp Glu Leu Val Asp Thr
           115                 120                 125
   gtc atc ctc gcc ctc cgc cag aag ccc acc atc ccc ctc cac gtc tac     432
   Val Ile Leu Ala Leu Arg Gln Lys Pro Thr Ile Pro Leu His Val Tyr
       130                 135                 140
   cat cac gcc gtc atg ctg ttc att gtg tgg tcg tgg ttc gcg cac ccc     480
   His His Ala Val Met Leu Phe Ile Val Trp Ser Trp Phe Ala His Pro
   145                 150                 155                 160
   tgg ctc gag ggg agc tgg tgg tgc tcc ctg gtc aac tct ttc atc cac     528
   Trp Leu Glu Gly Ser Trp Trp Cys Ser Leu Val Asn Ser Phe Ile His
                   165                 170                 175
   acg gtg atg tac tcg tat tac acc ctg acg gtg gtt ggc atc aac cct     576
   Thr Val Met Tyr Ser Tyr Tyr Thr Leu Thr Val Val Gly Ile Asn Pro
               180                 185                 190
   tgg tgg aag aag tgg atg acc acc atg cag atc atc cag ttc atc acg     624
   Trp Trp Lys Lys Trp Met Thr Thr Met Gln Ile Ile Gln Phe Ile Thr
           195                 200                 205
   ggc tgc gtg tac gtc acg gcg ttc ttc ggc cta tac tat gcc ggg gcg     672
   Gly Cys Val Tyr Val Thr Ala Phe Phe Gly Leu Tyr Tyr Ala Gly Ala
       210                 215                 220
   ggc tgc acc tcc aac gtg tac act gcc tgg ttc tcg atg ggg gtc aac      720
   Gly Cys Thr Ser Asn Val Tyr Thr Ala Trp Phe Ser Met Gly Val Asn
   225                 230                 235                 240
   ctc agc ttt ctg tgg ctc ttc gct ctt ttc ttc cgc cgg tcg tac agc      768
   Leu Ser Phe Leu Trp Leu Phe Ala Leu Phe Phe Arg Arg Ser Tyr Ser
                   245                 250                 255
   aaa cct agc cgg aag gag tag                                          789
   Lys Pro Ser Arg Lys Glu
               260
<210> 133
   <211> 262
   <212> PRT
   <213> Euglena gracilis
<400> 133
   Met Leu Gly Ala Ile Ala Asp Val Val Leu Arg Gly Pro Ala Ala Phe
   1 5 10 15
   His Trp Asp Pro Ala Thr Thr Pro Leu Ala Ser Ile Val Ser Pro Cys
   20 25 30
   Val Ala Ser Val Ala Tyr Leu Gly Ala Ile Gly Leu Leu Lys Arg Arg
   35 40 45
   Thr Gly Pro Glu Val Arg Ser Lys Pro Phe Glu Leu Leu His Asn Gly
   50 55 60
   Leu Leu Val Gly Trp Ser Leu Val Val Leu Leu Gly Thr Leu Tyr Gly
   65 70 75 80
   Ala Tyr Gln Arg Val Gln Glu Asp Gly Arg Gly Val Gln Ala Leu Leu
   85 90 95
   Cys Thr Gln Arg Pro Pro Ser Gln Ile Trp Asp Gly Pro Val Gly Tyr
   100 105 110
   Phe Thr Tyr Leu Phe Tyr Leu Ala Lys Tyr Trp Glu Leu Val Asp Thr
   115 120 125
   Val Ile Leu Ala Leu Arg Gln Lys Pro Thr Ile Pro Leu His Val Tyr
   130 135 140
   His His Ala Val Met Leu Phe Ile Val Trp Ser Trp Phe Ala His Pro
   145 150 155 160
   Trp Leu Glu Gly Ser Trp Trp Cys Ser Leu Val Asn Ser Phe Ile His
   165 170 175
   Thr Val Met Tyr Ser Tyr Tyr Thr Leu Thr Val Val Gly Ile Asn Pro
   180 185 190
   Trp Trp Lys Lys Trp Met Thr Thr Met Gln Ile Ile Gln Phe Ile Thr
           195                 200                 205
   Gly Cys Val Tyr Val Thr Ala Phe Phe Gly Leu Tyr Tyr Ala Gly Ala
       210                 215                 220
   Gly Cys Thr Ser Asn Val Tyr Thr Ala Trp Phe Ser Met Gly Val Asn
   225                 230                 235                 240
   Leu Ser Phe Leu Trp Leu Phe Ala Leu Phe Phe Arg Arg Ser Tyr Ser
                   245                 250                 255
   Lys Pro Ser Arg Lys Glu
               260
<210> 134
   <211> 897
   <212> DNA
   <213> Arabidopsis thaliana
<220>
   <221> CDS
   <222> (1)..(897)
   <223> delta-5-Elongase
<400> 134
   atg gca tct gtt tac tcc acc cta acc tac tgg ctc gtc cac cac ccc      48
   Met Ala Ser Val Tyr Ser Thr Leu Thr Tyr Trp Leu Val His His Pro
   1               5                   10                  15
   tac att gcc aac ttc acg tgg acc gaa ggt gaa aca cta ggc tcc acc      96
   Tyr Ile Ala Asn Phe Thr Trp Thr Glu Gly Glu Thr Leu Gly Ser Thr
               20                  25                  30
   gtt ttc ttt gtc ttt gtc gtc gtc tcc ctt tac ctc tcc gcc aca ttc     144
   Val Phe Phe Val Phe Val Val Val Ser Leu Tyr Leu Ser Ala Thr Phe
           35                  40                  45
   ctc ctc cga tac acc gtc gat tca ctc ccc aca ctc ggt ccc cgc att     192
   Leu Leu Arg Tyr Thr Val Asp Ser Leu Pro Thr Leu Gly Pro Arg Ile
       50                  55                  60
   ctc aaa cca atc aca gcc gtt cac agc ctc att ctc ttc ctc ctc tcc     240
   Leu Lys Pro Ile Thr Ala Val His Ser Leu Ile Leu Phe Leu Leu Ser
   65                  70                  75                  80
   tta acc atg gcc gtt ggt tgc act ctc tcc cta atc tct tcc tcg gac     288
   Leu Thr Met Ala Val Gly Cys Thr Leu Ser Leu Ile Ser Ser Ser Asp
                   85                  90                  95
   ccg aag gcg cgt ctc ttc gac gcc gtt tgt ttc ccc ctc gac gtg aaa     336
   Pro Lys Ala Arg Leu Phe Asp Ala Val Cys Phe Pro Leu Asp Val Lys
               100                 105                 110
   cct aag gga ccg ctt ttc ttt tgg gct caa gtc ttt tac ctc tcg aag     384
   Pro Lys Gly Pro Leu Phe Phe Trp Ala Gln Val Phe Tyr Leu Ser Lys
           115                 120                 125
   atc ctt gag ttc gta gac aca ctt ctc atc ata ctc aac aaa tca atc     432
   Ile Leu Glu Phe Val Asp Thr Leu Leu Ile Ile Leu Asn Lys Ser Ile
       130                 135                 140
   caa cgg ctc tcg ttc ctc cac gtc tac cac cac gca acg gtt gtg att      480
   Gln Arg Leu Ser Phe Leu His Val Tyr His His Ala Thr Val Val Ile
   145                 150                 155                 160
   ttg tgc tac ctc tgg tta cga aca cgt caa tcg atg ttt cct gtt ggg      528
   Leu Cys Tyr Leu Trp Leu Arg Thr Arg Gln Ser Met Phe Pro Val Gly
                   165                 170                 175
   ctc gtg ttg aac tcg acg gtc cat gtg att atg tac ggg tac tat ttc      576
   Leu Val Leu Asn Ser Thr Val His Val Ile Met Tyr Gly Tyr Tyr Phe
               180                 185                 190
   ctc tgc gct atc gga tcg agg ccc aag tgg aag aag ttg gtg acg aat      624
   Leu Cys Ala Ile Gly Ser Arg Pro Lys Trp Lys Lys Leu Val Thr Asn
           195                 200                 205
   ttt caa atg gtt cag ttt gct ttc ggc atg ggg tta gga gcc gct tgg      672
   Phe Gln Met Val Gln Phe Ala Phe Gly Met Gly Leu Gly Ala Ala Trp
       210                 215                 220
   atg ctc cca gag cat tat ttc ggg tcg ggt tgc gcc ggg att tgg aca      720
   Met Leu Pro Glu His Tyr Phe Gly Ser Gly Cys Ala Gly Ile Trp Thr
   225                 230                 235                 240
   gtt tat ttc aat ggt gtg ttt act gct tct cta ttg gct ctc ttc tac      768
   Val Tyr Phe Asn Gly Val Phe Thr Ala Ser Leu Leu Ala Leu Phe Tyr
                   245                 250                 255
   aac ttc cac tcc aag aac tat gag aag act aca acg tcg cct ttg tat      816
   Asn Phe His Ser Lys Asn Tyr Glu Lys Thr Thr Thr Ser Pro Leu Tyr
               260                 265                 270
   aag atc gaa tcc ttt ata ttt att cac gga gag agg tgg gca aat aaa      864
   Lys Ile Glu Ser Phe Ile Phe Ile His Gly Glu Arg Trp Ala Asn Lys
           275                 280                 285
   gcg att aca tta ttt tcc aag aaa aac gat taa                          897
   Ala Ile Thr Leu Phe Ser Lys Lys Asn Asp
       290                 295
<210> 135
   <211> 298
   <212> PRT
   <213> Arabidopsis thaliana
<400> 135
   Met Ala Ser Val Tyr Ser Thr Leu Thr Tyr Trp Leu Val His His Pro
   1               5                   10                  15
   Tyr Ile Ala Asn Phe Thr Trp Thr Glu Gly Glu Thr Leu Gly Ser Thr
               20                  25                  30
   Val Phe Phe Val Phe Val Val Val Ser Leu Tyr Leu Ser Ala Thr Phe
           35                  40                  45
   Leu Leu Arg Tyr Thr Val Asp Ser Leu Pro Thr Leu Gly Pro Arg Ile
       50                  55                  60
   Leu Lys Pro Ile Thr Ala Val His Ser Leu Ile Leu Phe Leu Leu Ser
   65                  70                  75                  80
   Leu Thr Met Ala Val Gly Cys Thr Leu Ser Leu Ile Ser Ser Ser Asp
   85 90 95
   Pro Lys Ala Arg Leu Phe Asp Ala Val Cys Phe Pro Leu Asp Val Lys
   100 105 110
   Pro Lys Gly Pro Leu Phe Phe Trp Ala Gln Val Phe Tyr Leu Ser Lys
   115 120 125
   Ile Leu Glu Phe Val Asp Thr Leu Leu Ile Ile Leu Asn Lys Ser Ile
   130 135 140
   Gln Arg Leu Ser Phe Leu His Val Tyr His His Ala Thr Val Val Ile
   145 150 155 160
   Leu Cys Tyr Leu Trp Leu Arg Thr Arg Gln Ser Met Phe Pro Val Gly
   165 170 175
   Leu Val Leu Asn Ser Thr Val His Val Ile Met Tyr Gly Tyr Tyr Phe
   180 185 190
   Leu Cys Ala Ile Gly Ser Arg Pro Lys Trp Lys Lys Leu Val Thr Asn
   195 200 205
   Phe Gln Met Val Gln Phe Ala Phe Gly Met Gly Leu Gly Ala Ala Trp
   210 215 220
   Met Leu Pro Glu His Tyr Phe Gly Ser Gly Cys Ala Gly Ile Trp Thr
   225 230 235 240
   Val Tyr Phe Asn Gly Val Phe Thr Ala Ser Leu Leu Ala Leu Phe Tyr
   245 250 255
   Asn Phe His Ser Lys Asn Tyr Glu Lys Thr Thr Thr Ser Pro Leu Tyr
   260 265 270
   Lys Ile Glu Ser Phe Ile Phe Ile His Gly Glu Arg Trp Ala Asn Lys
   275 280 285
   Ala Ile Thr Leu Phe Ser Lys Lys Asn Asp
   290 295
<210> 136
   <211> 837
   <212> DNA
   <213> Arabidopsis thaliana
<220>
   <221> CDS
   <222> (1)..(837)
   <223> delta-5-Elongase
<400> 136
   atg gca tca att tac tcc tct tta acc tac tgg ctc gtt aac cac ccc    48
   Met Ala Ser Ile Tyr Ser Ser Leu Thr Tyr Trp Leu Val Asn His Pro
   1               5                   10                  15
   tac atc tcc aat ttt act tgg atc gaa ggt gaa acc cta ggc tcc acc    96
   Tyr Ile Ser Asn Phe Thr Trp Ile Glu Gly Glu Thr Leu Gly Ser Thr
               20                  25                  30
   gtc ttt ttc gta tcc gtc gta gtc tcc gtt tac ctc tcc gcc acg ttc   144
   Val Phe Phe Val Ser Val Val Val Ser Val Tyr Leu Ser Ala Thr Phe
           35                  40                  45
   ctc ctc cga tcc gcc atc gat tca ctc cca tca ctc agt cca cgt atc   192
   Leu Leu Arg Ser Ala Ile Asp Ser Leu Pro Ser Leu Ser Pro Arg Ile
       50                  55                  60
   ctc aaa ccg atc aca gcc gtc cac agc cta atc ctc tgt ctc ctc tcc   240
   Leu Lys Pro Ile Thr Ala Val His Ser Leu Ile Leu Cys Leu Leu Ser
   65                  70                  75                  80
   tta gtc atg gcc gtc ggt tgc act ctc tca ata acc tca tct cac gcg   288
   Leu Val Met Ala Val Gly Cys Thr Leu Ser Ile Thr Ser Ser His Ala
                   85                  90                  95
   tct tca gat ccg atg gcg cgt ttc ctt cac gcg att tgc ttt ccc gtc   336
   Ser Ser Asp Pro Met Ala Arg Phe Leu His Ala Ile Cys Phe Pro Val
               100                 105                 110
   gac gtt aaa cct aac gga ccg ctt ttc ttc tgg gct caa gtc ttc tac   384
   Asp Val Lys Pro Asn Gly Pro Leu Phe Phe Trp Ala Gln Val Phe Tyr
           115                 120                 125
   ctc tcg aag atc ctc gag ttc gga gac acg atc ctc atc ata ctc ggc   432
   Leu Ser Lys Ile Leu Glu Phe Gly Asp Thr Ile Leu Ile Ile Leu Gly
       130                 135                 140
   aaa tca atc caa cgg cta tcc ttc ctc cac gtg tac cac cac gcg acg   480
   Lys Ser Ile Gln Arg Leu Ser Phe Leu His Val Tyr His His Ala Thr
   145                 150                 155                 160
   gtt gtg gtc atg tgt tat ctc tgg ctc cga act cgc caa tcg atg ttt   528
   Val Val Val Met Cys Tyr Leu Trp Leu Arg Thr Arg Gln Ser Met Phe
                   165                 170                 175
   ccg att gcg ctc gtg acg aat tcg acg gta cac gtc atc atg tac ggt   576
   Pro Ile Ala Leu Val Thr Asn Ser Thr Val His Val Ile Met Tyr Gly
               180                 185                 190
   tac tac ttc ctc tgc gcc gtt gga tcg agg ccc aag tgg aag aga ttg   624
   Tyr Tyr Phe Leu Cys Ala Val Gly Ser Arg Pro Lys Trp Lys Arg Leu
           195                 200                 205
   gtg acg gat tgt cag att gtt cag ttt gtt ttc agt ttc ggg tta tcc   672
   Val Thr Asp Cys Gln Ile Val Gln Phe Val Phe Ser Phe Gly Leu Ser
       210                 215                 220
   ggt tgg atg ctc cga gag cac tta ttc ggg tcg ggt tgc acc ggg att   720
   Gly Trp Met Leu Arg Glu His Leu Phe Gly Ser Gly Cys Thr Gly Ile
   225                 230                 235                 240
   tgg gga tgg tgt ttc aac gct gca ttt aat gct tct ctt ttg gct ctc   768
   Trp Gly Trp Cys Phe Asn Ala Ala Phe Asn Ala Ser Leu Leu Ala Leu
                   245                 250                 255
   ttt tcc aac ttc cat tca aag aat tat gtc aag aag cca acg aga gag   816
   Phe Ser Asn Phe His Ser Lys Asn Tyr Val Lys Lys Pro Thr Arg Glu
               260                 265                 270
   gat ggc aaa aaa agc gat tag                                            837
   Asp Gly Lys Lys Ser Asp
           275
<210> 137
   <211> 278
   <212> PRT
   <213> Arabidopsis thaliana
<400> 137
   Met Ala Ser Ile Tyr Ser Ser Leu Thr Tyr Trp Leu Val Asn His Pro
   1               5                   10                  15
   Tyr Ile Ser Asn Phe Thr Trp Ile Glu Gly Glu Thr Leu Gly Ser Thr
               20                  25                  30
   Val Phe Phe Val Ser Val Val Val Ser Val Tyr Leu Ser Ala Thr Phe
           35                  40                  45
   Leu Leu Arg Ser Ala Ile Asp Ser Leu Pro Ser Leu Ser Pro Arg Ile
       50                  55                  60
   Leu Lys Pro Ile Thr Ala Val His Ser Leu Ile Leu Cys Leu Leu Ser
   65                  70                  75                  80
   Leu Val Met Ala Val Gly Cys Thr Leu Ser Ile Thr Ser Ser His Ala
                   85                  90                  95
   Ser Ser Asp Pro Met Ala Arg Phe Leu His Ala Ile Cys Phe Pro Val
               100                 105                 110
   Asp Val Lys Pro Asn Gly Pro Leu Phe Phe Trp Ala Gln Val Phe Tyr
           115                 120                 125
   Leu Ser Lys Ile Leu Glu Phe Gly Asp Thr Ile Leu Ile Ile Leu Gly
       130                 135                 140
   Lys Ser Ile Gln Arg Leu Ser Phe Leu His Val Tyr His His Ala Thr
   145                 150                 155                 160
   Val Val Val Met Cys Tyr Leu Trp Leu Arg Thr Arg Gln Ser Met Phe
                   165                 170                 175
   Pro Ile Ala Leu Val Thr Asn Ser Thr Val His Val Ile Met Tyr Gly
               180                 185                 190
   Tyr Tyr Phe Leu Cys Ala Val Gly Ser Arg Pro Lys Trp Lys Arg Leu
           195                 200                 205
   Val Thr Asp Cys Gln Ile Val Gln Phe Val Phe Ser Phe Gly Leu Ser
       210                 215                 220
   Gly Trp Met Leu Arg Glu His Leu Phe Gly Ser Gly Cys Thr Gly Ile
   225                 230                 235                 240
   Trp Gly Trp Cys Phe Asn Ala Ala Phe Asn Ala Ser Leu Leu Ala Leu
                   245                 250                 255
   Phe Ser Asn Phe His Ser Lys Asn Tyr Val Lys Lys Pro Thr Arg Glu
               260                 265                 270
   Asp Gly Lys Lys Ser Asp
           275
<210> 138
   <211> 1086
   <212> DNA
   <213> Phytophthora infestans
<220>
   <221> CDS
   <222> (1)..(1086)
   <223> omega-3-Desaturase
<400> 138
   atg gcg acg aag gag gcg tat gtg ttc ccc act ctg acg gag atc aag      48
   Met Ala Thr Lys Glu Ala Tyr Val Phe Pro Thr Leu Thr Glu Ile Lys
   1               5                   10                  15
   cgg tcg cta cct aaa gac tgt ttc gag gct tcg gtg cct ctg tcg ctc      96
   Arg Ser Leu Pro Lys Asp Cys Phe Glu Ala Ser Val Pro Leu Ser Leu
               20                  25                  30
   tac tac acc gtg cgt tgt ctg gtg atc gcg gtg gct cta acc ttc ggt     144
   Tyr Tyr Thr Val Arg Cys Leu Val Ile Ala Val Ala Leu Thr Phe Gly
           35                  40                  45
   ctc aac tac gct cgc gct ctg ccc gag gtc gag agc ttc tgg gct ctg     192
   Leu Asn Tyr Ala Arg Ala Leu Pro Glu Val Glu Ser Phe Trp Ala Leu
       50                  55                  60
   gac gcc gca ctc tgc acg ggc tac atc ttg ctg cag ggc atc gtg ttc     240
   Asp Ala Ala Leu Cys Thr Gly Tyr Ile Leu Leu Gln Gly Ile Val Phe
   65                  70                  75                  80
   tgg ggc ttc ttc acg gtg ggc cac gat gcc ggc cac ggc gcc ttc tcg     288
   Trp Gly Phe Phe Thr Val Gly His Asp Ala Gly His Gly Ala Phe Ser
                   85                  90                  95
   cgc tac cac ctg ctt aac ttc gtg gtg ggc act ttc atg cac tcg ctc     336
   Arg Tyr His Leu Leu Asn Phe Val Val Gly Thr Phe Met His Ser Leu
               100                 105                 110
   atc ctc acg ccc ttc gag tcg tgg aag ctc acg cac cgt cac cac cac     384
   Ile Leu Thr Pro Phe Glu Ser Trp Lys Leu Thr His Arg His His His
           115                 120                 125
   aag aac acg ggc aac att gac cgt gac gag gtc ttc tac ccg caa cgc     432
   Lys Asn Thr Gly Asn Ile Asp Arg Asp Glu Val Phe Tyr Pro Gln Arg
       130                 135                 140
   aag gcc gac gac cac ccg ctg tct cgc aac ctg att ctg gcg ctc ggg     480
   Lys Ala Asp Asp His Pro Leu Ser Arg Asn Leu Ile Leu Ala Leu Gly
   145                 150                 155                 160
   gca gcg tgg ctc gcc tat ttg gtc gag ggc ttc cct cct cgt aag gtc      528
   Ala Ala Trp Leu Ala Tyr Leu Val Glu Gly Phe Pro Pro Arg Lys Val
                   165                 170                 175
   aac cac ttc aac ccg ttc gag cct ctg ttc gtg cgt cag gtg tca gct      576
   Asn His Phe Asn Pro Phe Glu Pro Leu Phe Val Arg Gln Val Ser Ala
               180                 185                 190
   gtg gta atc tct ctt ctc gcc cac ttc ttc gtg gcc gga ctc tcc atc      624
   Val Val Ile Ser Leu Leu Ala His Phe Phe Val Ala Gly Leu Ser Ile
           195                 200                 205
   tat ctg agc ctc cag ctg ggc ctt aag acg atg gca atc tac tac tat      672
   Tyr Leu Ser Leu Gln Leu Gly Leu Lys Thr Met Ala Ile Tyr Tyr Tyr
       210                 215                 220
   gga cct gtt ttt gtg ttc ggc agc atg ctg gtc att acc acc ttc cta      720
   Gly Pro Val Phe Val Phe Gly Ser Met Leu Val Ile Thr Thr Phe Leu
   225                 230                 235                 240
   cac cac aat gat gag gag acc cca tgg tac gcc gac tcg gag tgg acg      768
   His His Asn Asp Glu Glu Thr Pro Trp Tyr Ala Asp Ser Glu Trp Thr
                   245                 250                 255
   tac gtc aag ggc aac ctc tcg tcc gtg gac cga tcg tac ggc gcg ctc      816
   Tyr Val Lys Gly Asn Leu Ser Ser Val Asp Arg Ser Tyr Gly Ala Leu
               260                 265                 270
   att gac aac ctg agc cac aac atc ggc acg cac cag atc cac cac ctt      864
   Ile Asp Asn Leu Ser His Asn Ile Gly Thr His Gln Ile His His Leu
           275                 280                 285
   ttc cct atc att ccg cac tac aaa ctc aag aaa gcc act gcg gcc ttc      912
   Phe Pro Ile Ile Pro His Tyr Lys Leu Lys Lys Ala Thr Ala Ala Phe
       290                 295                 300
   cac cag gct ttc cct gag ctc gtg cgc aag agc gac gag cca att atc      960
   His Gln Ala Phe Pro Glu Leu Val Arg Lys Ser Asp Glu Pro Ile Ile
   305                 310                 315                 320
   aag gct ttc ttc cgg gtt gga cgt ctc tac gca aac tac ggc gtt gtg     1008
   Lys Ala Phe Phe Arg Val Gly Arg Leu Tyr Ala Asn Tyr Gly Val Val
                   325                 330                 335
   gac cag gag gcg aag ctc ttc acg cta aag gaa gcc aag gcg gcg acc     1056
   Asp Gln Glu Ala Lys Leu Phe Thr Leu Lys Glu Ala Lys Ala Ala Thr
               340                 345                 350
   gag gcg gcg gcc aag acc aag tcc acg taa                             1086
   Glu Ala Ala Ala Lys Thr Lys Ser Thr
           355                 360
<210> 139
   <211> 361
   <212> PRT
   <213> Phytophthora infestans
<400> 139
   Met Ala Thr Lys Glu Ala Tyr Val Phe Pro Thr Leu Thr Glu Ile Lys
   1               5                   10                  15
   Arg Ser Leu Pro Lys Asp Cys Phe Glu Ala Ser Val Pro Leu Ser Leu
               20                  25                  30
   Tyr Tyr Thr Val Arg Cys Leu Val Ile Ala Val Ala Leu Thr Phe Gly
           35                  40                  45
   Leu Asn Tyr Ala Arg Ala Leu Pro Glu Val Glu Ser Phe Trp Ala Leu
       50                  55                  60
   Asp Ala Ala Leu Cys Thr Gly Tyr Ile Leu Leu Gln Gly Ile Val Phe
   65                  70                  75                  80
   Trp Gly Phe Phe Thr Val Gly His Asp Ala Gly His Gly Ala Phe Ser
                   85                  90                  95
   Arg Tyr His Leu Leu Asn Phe Val Val Gly Thr Phe Met His Ser Leu
               100                 105                 110
   Ile Leu Thr Pro Phe Glu Ser Trp Lys Leu Thr His Arg His His His
           115                 120                 125
   Lys Asn Thr Gly Asn Ile Asp Arg Asp Glu Val Phe Tyr Pro Gln Arg
       130                 135                 140
   Lys Ala Asp Asp His Pro Leu Ser Arg Asn Leu Ile Leu Ala Leu Gly
   145                 150                 155                 160
   Ala Ala Trp Leu Ala Tyr Leu Val Glu Gly Phe Pro Pro Arg Lys Val
                   165                 170                 175
   Asn His Phe Asn Pro Phe Glu Pro Leu Phe Val Arg Gln Val Ser Ala
               180                 185                 190
   Val Val Ile Ser Leu Leu Ala His Phe Phe Val Ala Gly Leu Ser Ile
           195                 200                 205
   Tyr Leu Ser Leu Gln Leu Gly Leu Lys Thr Met Ala Ile Tyr Tyr Tyr
       210                 215                 220
   Gly Pro Val Phe Val Phe Gly Ser Met Leu Val Ile Thr Thr Phe Leu
   225                 230                 235                 240
   His His Asn Asp Glu Glu Thr Pro Trp Tyr Ala Asp Ser Glu Trp Thr
                   245                 250                 255
   Tyr Val Lys Gly Asn Leu Ser Ser Val Asp Arg Ser Tyr Gly Ala Leu
               260                 265                 270
   Ile Asp Asn Leu Ser His Asn Ile Gly Thr His Gln Ile His His Leu
           275                 280                 285
   Phe Pro Ile Ile Pro His Tyr Lys Leu Lys Lys Ala Thr Ala Ala Phe
       290                 295                 300
   His Gln Ala Phe Pro Glu Leu Val Arg Lys Ser Asp Glu Pro Ile Ile
   305 310 315 320
   Lys Ala Phe Phe Arg Val Gly Arg Leu Tyr Ala Asn Tyr Gly Val Val
   325 330 335
   Asp Gln Glu Ala Lys Leu Phe Thr Leu Lys Glu Ala Lys Ala Ala Thr
   340 345 350
   Glu Ala Ala Ala Lys Thr Lys Ser Thr
   355 360
<210> 140
   <211> 1257
   <212> DNA
   <213> Thalassiosira pseudonana
<220>
   <221> CDS
   <222> (1)..(1257)
   <223> omega-3-Desaturase
<400> 140
   atg tac aga tta aca tcc acc ttc ctc atc gca ttg gca ttc tcc tcc 48
   Met Tyr Arg Leu Thr Ser Thr Phe Leu Ile Ala Leu Ala Phe Ser Ser
   1 5 10 15
   tcc atc aat gcc ttc tct cca caa cgg cca cca cgt act atc acc aaa 96
   Ser Ile Asn Ala Phe Ser Pro Gln Arg Pro Pro Arg Thr Ile Thr Lys
   20 25 30
   agt aaa gtc caa agc acc gtg cta ccc ata ccg acc aag gat gat ctg 144
   Ser Lys Val Gln Ser Thr Val Leu Pro Ile Pro Thr Lys Asp Asp Leu
   35 40 45
   aac ttt ctc caa cca caa ctc gat gag aat gat ctc tac ctc gac gat 192
   Asn Phe Leu Gln Pro Gln Leu Asp Glu Asn Asp Leu Tyr Leu Asp Asp
   50 55 60
   gtc aac act cca cca aga gca ggt acc atc atg aag atg ttg ccg aag 240
   Val Asn Thr Pro Pro Arg Ala Gly Thr Ile Met Lys Met Leu Pro Lys
   65 70 75 80
   gaa acg ttc aac att gat aca gca act tca ttg ggt tac ttt ggt atg 288
   Glu Thr Phe Asn Ile Asp Thr Ala Thr Ser Leu Gly Tyr Phe Gly Met
   85 90 95
   gat atg gca gcg gtt gta tcg tcc atg acg ttg cta aat gct att gta 336
   Asp Met Ala Ala Val Val Ser Ser Met Thr Leu Leu Asn Ala Ile Val
   100 105 110
   act tcg gat cag tac cat gct ctt cca ctt cct ctc caa gca gca aca 384
   Thr Ser Asp Gln Tyr His Ala Leu Pro Leu Pro Leu Gln Ala Ala Thr
   115 120 125
   gtg att ccc ttt cag cta ttg gct ggg ttc gcc atg tgg tgt atg tgg 432
   Val Ile Pro Phe Gln Leu Leu Ala Gly Phe Ala Met Trp Cys Met Trp
   130 135 140
   tgc att gga cac gat gct gga cat tct act gtt tcg aag aca aag tgg 480
   Cys Ile Gly His Asp Ala Gly His Ser Thr Val Ser Lys Thr Lys Trp
   145 150 155 160
   atc aac cga gtc gtt ggt gaa gtg gct cat tct gtt gtt tgt ctc acg      528
   Ile Asn Arg Val Val Gly Glu Val Ala His Ser Val Val Cys Leu Thr
                   165                 170                 175
   ccg ttc gtg cct tgg cag atg tcg cat agg aaa cac cat ttg aat cac      576
   Pro Phe Val Pro Trp Gln Met Ser His Arg Lys His His Leu Asn His
               180                 185                 190
   aat cat att gaa aag gac tac tct cat aag tgg tac agt cgc gac gag      624
   Asn His Ile Glu Lys Asp Tyr Ser His Lys Trp Tyr Ser Arg Asp Glu
           195                 200                 205
   ttt gat gat atc cca caa ctc tat aag aca ttt ggc tac aac cca aga      672
   Phe Asp Asp Ile Pro Gln Leu Tyr Lys Thr Phe Gly Tyr Asn Pro Arg
       210                 215                 220
   atg atg caa ctt cca ttc ctc tac ttc atg tat ctt gca ttg gga att      720
   Met Met Gln Leu Pro Phe Leu Tyr Phe Met Tyr Leu Ala Leu Gly Ile
   225                 230                 235                 240
   cca gat ggt ggg cat gtt gtg ttc tac gga aga atg tgg gaa gga gtg      768
   Pro Asp Gly Gly His Val Val Phe Tyr Gly Arg Met Trp Glu Gly Val
                   245                 250                 255
   tca ttg cag aag aag ttt gat gct gct att tct gtg gcc gta tca tgt      816
   Ser Leu Gln Lys Lys Phe Asp Ala Ala Ile Ser Val Ala Val Ser Cys
               260                 265                 270
   gca act gct gga tcg ctt tgg atg aat atg ggt aca gca gac ttc acg      864
   Ala Thr Ala Gly Ser Leu Trp Met Asn Met Gly Thr Ala Asp Phe Thr
           275                 280                 285
   gtg gta tgc atg gtt cct tgg cta gtt cta tcg tgg tgg ctc ttc atg      912
   Val Val Cys Met Val Pro Trp Leu Val Leu Ser Trp Trp Leu Phe Met
       290                 295                 300
   gta aca tac ctt cag cat cat tca gaa gac gga aag cta tac act gat      960
   Val Thr Tyr Leu Gln His His Ser Glu Asp Gly Lys Leu Tyr Thr Asp
   305                 310                 315                 320
   gaa acg ttt aca ttt gaa aag gga gcc ttc gag acc gtg gat cgt tcg     1008
   Glu Thr Phe Thr Phe Glu Lys Gly Ala Phe Glu Thr Val Asp Arg Ser
                   325                 330                 335
   tac ggc aag ttg atc aac cga atg tcg cat cac atg atg gac ggt cac     1056
   Tyr Gly Lys Leu Ile Asn Arg Met Ser His His Met Met Asp Gly His
               340                 345                 350
   gtg gtg cac cac ttg ttc ttt gaa cgt gta cct cac tac aga tta gag     1104
   Val Val His His Leu Phe Phe Glu Arg Val Pro His Tyr Arg Leu Glu
           355                 360                 365
   gca gct acc gaa gct ctt gtg aaa gga atg gat gaa acg gga cag aaa     1152
   Ala Ala Thr Glu Ala Leu Val Lys Gly Met Asp Glu Thr Gly Gln Lys
       370                 375                 380
   cat ttg tac aaa tac att gat act cct gat ttc aat gcc gag att gtc     1200
   His Leu Tyr Lys Tyr Ile Asp Thr Pro Asp Phe Asn Ala Glu Ile Val
   385                 390                 395                 400
   aac gga ttt cgc gac aat tgg ttc ctt gtt gaa gag gag aac atc aaa     1248
   Asn Gly Phe Arg Asp Asn Trp Phe Leu Val Glu Glu Glu Asn Ile Lys
                   405                 410                 415
   agg gag tag                                                         1257
   Arg Glu
<210> 141
   <211> 418
   <212> PRT
   <213> Thalassiosira pseudonana
<400> 141
   Met Tyr Arg Leu Thr Ser Thr Phe Leu Ile Ala Leu Ala Phe Ser Ser
   1               5                   10                  15
   Ser Ile Asn Ala Phe Ser Pro Gln Arg Pro Pro Arg Thr Ile Thr Lys
               20                  25                  30
   Ser Lys Val Gln Ser Thr Val Leu Pro Ile Pro Thr Lys Asp Asp Leu
           35                  40                  45
   Asn Phe Leu Gln Pro Gln Leu Asp Glu Asn Asp Leu Tyr Leu Asp Asp
       50                  55                  60
   Val Asn Thr Pro Pro Arg Ala Gly Thr Ile Met Lys Met Leu Pro Lys
   65                  70                  75                  80
   Glu Thr Phe Asn Ile Asp Thr Ala Thr Ser Leu Gly Tyr Phe Gly Met
                   85                  90                  95
   Asp Met Ala Ala Val Val Ser Ser Met Thr Leu Leu Asn Ala Ile Val
               100                 105                 110
   Thr Ser Asp Gln Tyr His Ala Leu Pro Leu Pro Leu Gln Ala Ala Thr
           115                 120                 125
   Val Ile Pro Phe Gln Leu Leu Ala Gly Phe Ala Met Trp Cys Met Trp
       130                 135                 140
   Cys Ile Gly His Asp Ala Gly His Ser Thr Val Ser Lys Thr Lys Trp
   145                 150                 155                 160
   Ile Asn Arg Val Val Gly Glu Val Ala His Ser Val Val Cys Leu Thr
                   165                 170                 175
   Pro Phe Val Pro Trp Gln Met Ser His Arg Lys His His Leu Asn His
               180                 185                 190
   Asn His Ile Glu Lys Asp Tyr Ser His Lys Trp Tyr Ser Arg Asp Glu
           195                 200                 205
   Phe Asp Asp Ile Pro Gln Leu Tyr Lys Thr Phe Gly Tyr Asn Pro Arg
       210                 215                 220
   Met Met Gln Leu Pro Phe Leu Tyr Phe Met Tyr Leu Ala Leu Gly Ile
   225                 230                 235                 240
   Pro Asp Gly Gly His Val Val Phe Tyr Gly Arg Met Trp Glu Gly Val
   245 250 255
   Ser Leu Gln Lys Lys Phe Asp Ala Ala Ile Ser Val Ala Val Ser Cys
   260 265 270
   Ala Thr Ala Gly Ser Leu Trp Met Asn Met Gly Thr Ala Asp Phe Thr
   275 280 285
   Val Val Cys Met Val Pro Trp Leu Val Leu Ser Trp Trp Leu Phe Met
   290 295 300
   Val Thr Tyr Leu Gln His His Ser Glu Asp Gly Lys Leu Tyr Thr Asp
   305 310 315 320
   Glu Thr Phe Thr Phe Glu Lys Gly Ala Phe Glu Thr Val Asp Arg Ser
   325 330 335
   Tyr Gly Lys Leu Ile Asn Arg Met Ser His His Met Met Asp Gly His
   340 345 350
   Val Val His His Leu Phe Phe Glu Arg Val Pro His Tyr Arg Leu Glu
   355 360 365
   Ala Ala Thr Glu Ala Leu Val Lys Gly Met Asp Glu Thr Gly Gln Lys
   370 375 380
   His Leu Tyr Lys Tyr Ile Asp Thr Pro Asp Phe Asn Ala Glu Ile Val
   385 390 395 400
   Asn Gly Phe Arg Asp Asn Trp Phe Leu Val Glu Glu Glu Asn Ile Lys
   405 410 415
   Arg Glu
<210> 142
   <211> 1086
   <212> DNA
   <213> Ostreococcus tauri
<220>
   <221> CDS
   <222> (1)..(1086)
   <223> delta-12-Desaturase
<400> 142
   atg cag gag ggg gtg cga aac att ccg aac gag tgc ttt gag acg gga      48
   Met Gln Glu Gly Val Arg Asn Ile Pro Asn Glu Cys Phe Glu Thr Gly
   1               5                   10                  15
   cat ctt gaa aga ccc tgg cgt tcc ggc cgg tgt ggg cgc gat ccc ggt      96
   His Leu Glu Arg Pro Trp Arg Ser Gly Arg Cys Gly Arg Asp Pro Gly
               20                  25                  30
   tcg aat tgg ggc gct ggc ttc cgc ttt ttt tcg ctc aag ggg ttt tgg 144
   Ser Asn Trp Gly Ala Gly Phe Arg Phe Phe Ser Leu Lys Gly Phe Trp
   35 40 45
   tgg ccg gcg tgg tgg gcg tac gcg ttc gtg acg ggg acg gcg gcc act 192
   Trp Pro Ala Trp Trp Ala Tyr Ala Phe Val Thr Gly Thr Ala Ala Thr
   50 55 60
   ggg tgt tgg gtc gcc gcg cac gag tgc ggg cac ggc gcg ttc agc gat 240
   Gly Cys Trp Val Ala Ala His Glu Cys Gly His Gly Ala Phe Ser Asp
   65 70 75 80
   aac aag acg ttg caa gat gcg gtt gga tac gtg ttg cac tcg ttg ctc 288
   Asn Lys Thr Leu Gln Asp Ala Val Gly Tyr Val Leu His Ser Leu Leu
   85 90 95
   ttg gtg ccg tac ttt tct tgg cag cga tca cac gcg gtg cat cac tcg 336
   Leu Val Pro Tyr Phe Ser Trp Gln Arg Ser His Ala Val His His Ser
   100 105 110
   agg acg aat cac gtt ctt gag ggc gag acg cac gtg ccg gcg cgc ttg 384
   Arg Thr Asn His Val Leu Glu Gly Glu Thr His Val Pro Ala Arg Leu
   115 120 125
   ggg acg gaa gac gcc aac gtc gtg ttc aag ctt cgc gaa ttg atc ggt 432
   Gly Thr Glu Asp Ala Asn Val Val Phe Lys Leu Arg Glu Leu Ile Gly
   130 135 140
   gaa ggg ccg ttc acg ttt ttc aac ctc gtc ggc gtc ttc gcg ctc gga 480
   Glu Gly Pro Phe Thr Phe Phe Asn Leu Val Gly Val Phe Ala Leu Gly
   145 150 155 160
   tgg ccg att tac ttg ctc acc ggc gcg agc ggc gga ccg gtg cgc ggt 528
   Trp Pro Ile Tyr Leu Leu Thr Gly Ala Ser Gly Gly Pro Val Arg Gly
   165 170 175
   aac acg aac cac ttc tta ccc ttc atg ggc gag aaa ggt aag cac gcg 576
   Asn Thr Asn His Phe Leu Pro Phe Met Gly Glu Lys Gly Lys His Ala
   180 185 190
   ctg ttc ccg ggt aag tgg gcg aag aag gtg tgg cag tct gac atc ggc 624
   Leu Phe Pro Gly Lys Trp Ala Lys Lys Val Trp Gln Ser Asp Ile Gly
   195 200 205
   gtt gtt gcc gtc ctg ggc gcg ctc gcg gct tgg gcg gcg cac agc ggg 672
   Val Val Ala Val Leu Gly Ala Leu Ala Ala Trp Ala Ala His Ser Gly
   210 215 220
   att gcc aca gtg atg gca ctc tac gtc ggc ccg tac atg gtg acc aac 720
   Ile Ala Thr Val Met Ala Leu Tyr Val Gly Pro Tyr Met Val Thr Asn
   225 230 235 240
   ttt tgg ctc gtc ttg tac acg tgg tta cag cac acc gac gtt gac gtg 768
   Phe Trp Leu Val Leu Tyr Thr Trp Leu Gln His Thr Asp Val Asp Val
   245 250 255
   ccg cac ttc gag ggc gac gat tgg aac ttg gtc aag ggg gca ttc atg 816
   Pro His Phe Glu Gly Asp Asp Trp Asn Leu Val Lys Gly Ala Phe Met
   260 265 270
   acg atc gat cgc ccg tac ggc cca gtt ttt gat ttc ttg cac cac cgc 864
   Thr Ile Asp Arg Pro Tyr Gly Pro Val Phe Asp Phe Leu His His Arg
   275 280 285
   atc ggc agc acg cac gtc gcg cac cac atc aac aca cca ttc ccg cat 912
   Ile Gly Ser Thr His Val Ala His His Ile Asn Thr Pro Phe Pro His
   290 295 300
   tac aag gct caa atg gcg acg gat gcg cta aag gag gcg tat ccc gac       960
   Tyr Lys Ala Gln Met Ala Thr Asp Ala Leu Lys Glu Ala Tyr Pro Asp
   305                 310                 315                 320
   ctc tac ctt tac gat cca act ccg atc gcg acc gct acg tgg cgc gtg      1008
   Leu Tyr Leu Tyr Asp Pro Thr Pro Ile Ala Thr Ala Thr Trp Arg Val
                   325                 330                 335
   ggg agc aag tgc atc gcc gtc gtg aag aag gga gac gaa tgg gtg ttc      1056
   Gly Ser Lys Cys Ile Ala Val Val Lys Lys Gly Asp Glu Trp Val Phe
               340                 345                 350
   acg gat aag caa ctc ccg gtc gcg gcg tga                              1086
   Thr Asp Lys Gln Leu Pro Val Ala Ala
           355                 360
<210> 143
   <211> 361
   <212> PRT
   <213> Ostreococcus tauri
<400> 143
   Met Gln Glu Gly Val Arg Asn Ile Pro Asn Glu Cys Phe Glu Thr Gly
   1 5 10 15
   His Leu Glu Arg Pro Trp Arg Ser Gly Arg Cys Gly Arg Asp Pro Gly
   20 25 30
   Ser Asn Trp Gly Ala Gly Phe Arg Phe Phe Ser Leu Lys Gly Phe Trp
   35 40 45
   Trp Pro Ala Trp Trp Ala Tyr Ala Phe Val Thr Gly Thr Ala Ala Thr
   50 55 60
   Gly Cys Trp Val Ala Ala His Glu Cys Gly His Gly Ala Phe Ser Asp
   65 70 75 80
   Asn Lys Thr Leu Gln Asp Ala Val Gly Tyr Val Leu His Ser Leu Leu
   85 90 95
   Leu Val Pro Tyr Phe Ser Trp Gln Arg Ser His Ala Val His His Ser
   100 105 110
   Arg Thr Asn His Val Leu Glu Gly Glu Thr His Val Pro Ala Arg Leu
   115 120 125
   Gly Thr Glu Asp Ala Asn Val Val Phe Lys Leu Arg Glu Leu Ile Gly
   130 135 140
   Glu Gly Pro Phe Thr Phe Phe Asn Leu Val Gly Val Phe Ala Leu Gly
   145 150 155 160
   Trp Pro Ile Tyr Leu Leu Thr Gly Ala Ser Gly Gly Pro Val Arg Gly
   165 170 175
   Asn Thr Asn His Phe Leu Pro Phe Met Gly Glu Lys Gly Lys His Ala
   180 185 190
   Leu Phe Pro Gly Lys Trp Ala Lys Lys Val Trp Gln Ser Asp Ile Gly
   195 200 205
   Val Val Ala Val Leu Gly Ala Leu Ala Ala Trp Ala Ala His Ser Gly
   210 215 220
   Ile Ala Thr Val Met Ala Leu Tyr Val Gly Pro Tyr Met Val Thr Asn
   225 230 235 240
   Phe Trp Leu Val Leu Tyr Thr Trp Leu Gln His Thr Asp Val Asp Val
   245 250 255
   Pro His Phe Glu Gly Asp Asp Trp Asn Leu Val Lys Gly Ala Phe Met
   260 265 270
   Thr Ile Asp Arg Pro Tyr Gly Pro Val Phe Asp Phe Leu His His Arg
   275 280 285
   Ile Gly Ser Thr His Val Ala His His Ile Asn Thr Pro Phe Pro His
   290 295 300
   Tyr Lys Ala Gln Met Ala Thr Asp Ala Leu Lys Glu Ala Tyr Pro Asp
   305 310 315 320
   Leu Tyr Leu Tyr Asp Pro Thr Pro Ile Ala Thr Ala Thr Trp Arg Val
   325 330 335
   Gly Ser Lys Cys Ile Ala Val Val Lys Lys Gly Asp Glu Trp Val Phe
   340 345 350
   Thr Asp Lys Gln Leu Pro Val Ala Ala
   355 360
<210> 144
   <211> 1305
   <212> DNA
   <213> Thalassiosira pseudonana
<220>
   <221> CDS
   <222> (1)..(1305)
   <223> delta-12-Desaturase
<400> 144
   atg gga aag gga gga aga tca gta acc cgc gct caa aca gca gaa aag      48
   Met Gly Lys Gly Gly Arg Ser Val Thr Arg Ala Gln Thr Ala Glu Lys
   1               5                   10                  15
   tca gca cac acc atc caa acc ttc acc gac ggc cga tgg gtc tcc ccc      96
   Ser Ala His Thr Ile Gln Thr Phe Thr Asp Gly Arg Trp Val Ser Pro
               20                  25                  30
   tac aac ccc ctc gca aaa gat gca cct gaa ctc ccc tcc aag ggt gaa      144
   Tyr Asn Pro Leu Ala Lys Asp Ala Pro Glu Leu Pro Ser Lys Gly Glu
           35                  40                  45
   atc aag gcg gtc atc ccc aaa gag tgc ttc gaa cga agc tac ctc cac      192
   Ile Lys Ala Val Ile Pro Lys Glu Cys Phe Glu Arg Ser Tyr Leu His
       50                  55                  60
   tcc atg tac ttc gtc ctc cgt gac acc gtc atg gcc gtg gcc tgc gcc      240
   Ser Met Tyr Phe Val Leu Arg Asp Thr Val Met Ala Val Ala Cys Ala
   65                  70                  75                  80
   tac atc gcc cac tca acg ctc tcc acc gat att ccc tcc gag tta ctg      288
   Tyr Ile Ala His Ser Thr Leu Ser Thr Asp Ile Pro Ser Glu Leu Leu
                   85                  90                  95
   agc gtg gac gca ctc aaa tgg ttc ctc gga tgg aac acc tac gcc ttt      336
   Ser Val Asp Ala Leu Lys Trp Phe Leu Gly Trp Asn Thr Tyr Ala Phe
               100                 105                 110
   tgg atg ggg tgc att ctc acc gga cac tgg gtc cta gcc cat gaa tgt      384
   Trp Met Gly Cys Ile Leu Thr Gly His Trp Val Leu Ala His Glu Cys
           115                 120                 125
   gga cat ggt gca ttc tct ccc tct cag acg ttt aat gac ttt tgg ggg      432
   Gly His Gly Ala Phe Ser Pro Ser Gln Thr Phe Asn Asp Phe Trp Gly
       130                 135                 140
   ttc att atg cat cag gcg gtg ttg gtt ccg tat ttc gcc tgg cag tac      480
   Phe Ile Met His Gln Ala Val Leu Val Pro Tyr Phe Ala Trp Gln Tyr
   145                 150                 155                 160
   tct cat gcg aag cat cat cga cgt acc aac aac att atg gat ggg gag      528
   Ser His Ala Lys His His Arg Arg Thr Asn Asn Ile Met Asp Gly Glu
                   165                 170                 175
   agc cat gtg ccc aat atc gcc aag gaa atg gga ttg aac gag aag aat      576
   Ser His Val Pro Asn Ile Ala Lys Glu Met Gly Leu Asn Glu Lys Asn
               180                 185                 190
   gag cgc agt gga gga tat gcc gcc att cat gag gct att gga gat gga      624
   Glu Arg Ser Gly Gly Tyr Ala Ala Ile His Glu Ala Ile Gly Asp Gly
           195                 200                 205
   ccc ttt gcg atg ttt caa atc ttt gct cac ttg gtg atc ggg tgg cct      672
   Pro Phe Ala Met Phe Gln Ile Phe Ala His Leu Val Ile Gly Trp Pro
       210                 215                 220
   att tac ttg atg gga ttt gct tcc act gga cgt ctc ggt cag gat ggg      720
   Ile Tyr Leu Met Gly Phe Ala Ser Thr Gly Arg Leu Gly Gln Asp Gly
   225                 230                 235                 240
   aag gaa ctt cag gct gga gag atc atc gac cat tac cgt cct tgg agt      768
   Lys Glu Leu Gln Ala Gly Glu Ile Ile Asp His Tyr Arg Pro Trp Ser
                   245                 250                 255
   aag atg ttc ccc acc aag ttg cga ttc aaa att gct ctt tcg aca ctt      816
   Lys Met Phe Pro Thr Lys Leu Arg Phe Lys Ile Ala Leu Ser Thr Leu
               260                 265                 270
   gga gtg att gcc gcc tgg gtt ggg ttg tac ttt gct gca caa gag tat      864
   Gly Val Ile Ala Ala Trp Val Gly Leu Tyr Phe Ala Ala Gln Glu Tyr
           275                 280                 285
   gga gtc ttg ccc gtg gtt ctt tgg tac att ggc cca ctc atg tgg aat      912
   Gly Val Leu Pro Val Val Leu Trp Tyr Ile Gly Pro Leu Met Trp Asn
       290                 295                 300
   cag gcg tgg ctt gtg ctc tac act tgg ctt cag cac aat gat ccc tcc      960
   Gln Ala Trp Leu Val Leu Tyr Thr Trp Leu Gln His Asn Asp Pro Ser
   305                 310                 315                 320
   gtg cct caa tat gga agt gac gaa tgg aca tgg gtc aag gga gct ttg     1008
   Val Pro Gln Tyr Gly Ser Asp Glu Trp Thr Trp Val Lys Gly Ala Leu
                   325                 330                 335
   tcg acg att gat cgc ccg tat ggt atc ttt gac ttc ttc cat cac aag     1056
   Ser Thr Ile Asp Arg Pro Tyr Gly Ile Phe Asp Phe Phe His His Lys
               340                 345                 350
   att gga agc act cac gta gct cat cat ttg ttc cac gag atg cca ttt     1104
   Ile Gly Ser Thr His Val Ala His His Leu Phe His Glu Met Pro Phe
           355                 360                 365
   tac aag gcg gat gtg gct act gcg tcg atc aag ggt ttc ttg gag ccg     1152
   Tyr Lys Ala Asp Val Ala Thr Ala Ser Ile Lys Gly Phe Leu Glu Pro
       370                 375                 380
   aag gga ctt tac aac tat gat cca acg cct tgg tat gtg gcc atg tgg     1200
   Lys Gly Leu Tyr Asn Tyr Asp Pro Thr Pro Trp Tyr Val Ala Met Trp
   385                 390                 395                 400
   agg gtg gcc aag act tgt cat tat att gag gat gtg gat gga gtt cag     1248
   Arg Val Ala Lys Thr Cys His Tyr Ile Glu Asp Val Asp Gly Val Gln
                   405                 410                 415
   tat tat aag agt ttg gag gat gtg cct ttg aag aag gat gcc aag aag     1296
   Tyr Tyr Lys Ser Leu Glu Asp Val Pro Leu Lys Lys Asp Ala Lys Lys
               420                 425                 430
   tct gat tag                                                         1305
   Ser Asp
<210> 145
   <211> 434
   <212> PRT
   <213> Thalassiosira pseudonana
<400> 145
   Met Gly Lys Gly Gly Arg Ser Val Thr Arg Ala Gln Thr Ala Glu Lys
   1               5                   10                  15
   Ser Ala His Thr Ile Gln Thr Phe Thr Asp Gly Arg Trp Val Ser Pro
               20                  25                  30
   Tyr Asn Pro Leu Ala Lys Asp Ala Pro Glu Leu Pro Ser Lys Gly Glu
           35                  40                  45
   Ile Lys Ala Val Ile Pro Lys Glu Cys Phe Glu Arg Ser Tyr Leu His
       50                  55                  60
   Ser Met Tyr Phe Val Leu Arg Asp Thr Val Met Ala Val Ala Cys Ala
   65                  70                  75                  80
   Tyr Ile Ala His Ser Thr Leu Ser Thr Asp Ile Pro Ser Glu Leu Leu
                   85                  90                  95
   Ser Val Asp Ala Leu Lys Trp Phe Leu Gly Trp Asn Thr Tyr Ala Phe
   100 105 110
   Trp Met Gly Cys Ile Leu Thr Gly His Trp Val Leu Ala His Glu Cys
   115 120 125
   Gly His Gly Ala Phe Ser Pro Ser Gln Thr Phe Asn Asp Phe Trp Gly
   130 135 140
   Phe Ile Met His Gln Ala Val Leu Val Pro Tyr Phe Ala Trp Gln Tyr
   145 150 155 160
   Ser His Ala Lys His His Arg Arg Thr Asn Asn Ile Met Asp Gly Glu
   165 170 175
   Ser His Val Pro Asn Ile Ala Lys Glu Met Gly Leu Asn Glu Lys Asn
   180 185 190
   Glu Arg Ser Gly Gly Tyr Ala Ala Ile His Glu Ala Ile Gly Asp Gly
   195 200 205
   Pro Phe Ala Met Phe Gln Ile Phe Ala His Leu Val Ile Gly Trp Pro
   210 215 220
   Ile Tyr Leu Met Gly Phe Ala Ser Thr Gly Arg Leu Gly Gln Asp Gly
   225 230 235 240
   Lys Glu Leu Gln Ala Gly Glu Ile Ile Asp His Tyr Arg Pro Trp Ser
   245 250 255
   Lys Met Phe Pro Thr Lys Leu Arg Phe Lys Ile Ala Leu Ser Thr Leu
   260 265 270
   Gly Val Ile Ala Ala Trp Val Gly Leu Tyr Phe Ala Ala Gln Glu Tyr
   275 280 285
   Gly Val Leu Pro Val Val Leu Trp Tyr Ile Gly Pro Leu Met Trp Asn
   290 295 300
   Gln Ala Trp Leu Val Leu Tyr Thr Trp Leu Gln His Asn Asp Pro Ser
   305 310 315 320
   Val Pro Gln Tyr Gly Ser Asp Glu Trp Thr Trp Val Lys Gly Ala Leu
   325 330 335
   Ser Thr Ile Asp Arg Pro Tyr Gly Ile Phe Asp Phe Phe His His Lys
   340 345 350
   Ile Gly Ser Thr His Val Ala His His Leu Phe His Glu Met Pro Phe
   355 360 365
   Tyr Lys Ala Asp Val Ala Thr Ala Ser Ile Lys Gly Phe Leu Glu Pro
       370                 375                 380
   Lys Gly Leu Tyr Asn Tyr Asp Pro Thr Pro Trp Tyr Val Ala Met Trp
   385                 390                 395                 400
   Arg Val Ala Lys Thr Cys His Tyr Ile Glu Asp Val Asp Gly Val Gln
                   405                 410                 415
   Tyr Tyr Lys Ser Leu Glu Asp Val Pro Leu Lys Lys Asp Ala Lys Lys
               420                 425                 430
   Ser Asp